# EUROPEAN PATENT APPLICATION

(11) **EP 3 940 071 A1**
(43) Date of publication of application: **19.01.2022**
(21) Application number: 20186028.5
(22) Date of filing: 15.07.2020
(51) Int. Cl.: C12N 9/78, C12N 9/88, C12N 9/12, C12N 9/10, C12N 9/80, C12N 9/06, C12N 9/00, C12P 7/44, C12P 17/18, C12N 15/52

(54) **5-METHYLFOLATE PRODUCING MICROORGANISM**

(71) Applicant: Chifeng Pharmaceutical Co., Ltd, Chifeng, Inner Mongolia 024000 (CN)
(72) Inventor: Shi, Mingan, Chifeng, Inner Mongolia 024000 (CN); Xiong, Xin, Chifeng, Inner Mongolia 024000 (CN); Sun, Jia, Chifeng, Inner Mongolia 024000 (CN); Xia, Yunzhong, Chifeng, Inner Mongolia 024000 (CN); Zheng, Shuang, Chifeng, Inner Mongolia 024000 (CN); Cai, Zhigang, Chifeng, Inner Mongolia 024000 (CN); Blazic, Marko, 1230 Domzale (SI); Kogej, Tina, 1000 Ljubljana (SI); Kosec, Gregor, 1000 Ljubljana (SI); Svagelj, Mirjan, 1000 Ljubljana (SI); Horvat, Jaka, 4000 Kranj (SI); Fujs, Stefan, 1000 Ljubljana (SI)
(74) Representative: Zacco GmbH

(57) **Abstract**

The present invention provides a 5-methylfolate producing microorganism which a) has been modified to have a decreased expression and/or activity of a polypeptide having both dihydrofolate synthase activity and folylpolyglutamate synthetase activity compared to an otherwise identical microorganism (reference microorganism); b) has been (further) modified to express a heterologous polypeptide having only dihydrofolate synthase activity; c) has been (further) modified to have an increased expression level of at least one enzyme (such as at least two, at least three, at least four, at least five, at least six, at least seven or at least eight) enzymes involved in the biosynthesis of a 5-methylfolate compared to an otherwise identical microorganism (reference microorganism); and/or d) has been (further) modified to have a decreased expression and/or activity of a polypeptide having 5-methyltetrahydropteroyltriglutamate-homocysteine S-methyltransferase activity compared to an otherwise identical microorganism (reference microorganism).

SEQUENCE LISTING

<110>  Chifeng Pharmaceutical Co., Ltd.

<120>  5-Methylfolate producing microorganism

<130>  P47683EP00

<160>  101

<170>  PatentIn version 3.5

<210>  1
<211>  573
<212>  DNA
<213>  Bacillus subtilis

<400>  1
atgaaagaag ttaataaaga gcaaatcgaa caagctgttc gtcaaatttt agaagcgatc      60

ggagaagacc cgaatagaga agggcttctt gatactccga aaagagtcgc aaagatgtat     120

gccgaagtat tctccggctt gaatgaagat ccaaaagaac atttccagac tatcttcggt     180

gaaaaccatg aggagcttgt tcttgtaaaa gatatagcgt ttcattctat gtgtgagcat     240

caccttgttc ccttttatgg aaaagcacat gttgcatata tcccgcgagg cggaaaggtc     300

acaggactca gcaaactggc acgtgccgtt gaagccgttg caaagcgccc gcagcttcag     360

gaacgcatca cttctacaat tgcagaaagc atcgtagaaa cgcttgatcc gcatggcgta     420

atggtagtgg ttgaagcgga acacatgtgc atgacgatgc gcggtgtaag aaaaccgggt     480

gcgaaaactg tgacttcagc agtcagaggc gtttttaaag atgatgccgc tgcccgtgca     540

gaagtattgg aacatattaa acgccaggac taa                                  573


<210>  2
<211>  363
<212>  DNA
<213>  Bacillus subtilis

<400>  2
atggataaag tttatgtaga aggtatggag ttttacggat atcacggtgt gttcacagaa      60

gaaaacaaac ttggccagcg gtttaaagtc gatttaaccg ctgagctgga tttaagcaaa     120

gctggacaga cagacgacct tgagcaaacg atcaactatg ctgagctcta tcacgtatgt     180

aaagatatcg tggaagggga gcctgtgaaa ttggtggaaa cgctggcgga acgtattgct     240

ggcactgttc tcggaaaatt tcagcctgtt cagcaatgta cggtgaaagt gattaagcca     300

gacccgccaa ttcccggaca ctataaatca gtagcaattg aaattacgag aaaaaagtca     360

tga                                                                   363


<210>  3
<211>  504
<212>  DNA

<213>  Bacillus subtilis

<400>  3
atgaacaaca tagcttatat tgcacttgga tctaatattg gagatagaga aacgtattta      60

aggcaagcag tggctttact gcatcagcat gctgcggtga cagtcactaa agtgtcgtct     120

atttacgaaa ctgacccggt cggatacgaa gatcaagctc aatttttgaa tatggctgtt     180

gaaatcaaga catcattgaa cccttttgaa ctccttgaac tgacgcagca gatagaaaat     240

gaattaggca gaacaaggga agtaagatgg gggccgcgga cggcagacct tgacattttg     300

ttatttaatc gtgaaaatat tgaaacagag caactaattg ttccgcatcc gagaatgtat     360

gagcgtttgt ttgtccttgc gccgcttgcg gaaatttgcc agcaggttga aaaagaggct     420

acaagcgccg aaacagacca agaaggtgta agagtatgga agcagaaatc tggggtagac     480

gaattcgtgc attcagaaag ctga                                            504

<210>  4
<211>  858
<212>  DNA
<213>  Bacillus subtilis

<400>  4
atggcgcagc acacaataga tcaaacacaa gtaatccaca ctaagcccag cgctttatca      60

tataaagaga agacgctggt gatgggaatt ttaaacgtaa cgcctgactc tttctcggac     120

ggcggaaaat atgacagctt ggacaaggcg ctgctgcacg cgaaagagat gatcgatgat     180

ggtgcccata tcattgatat tggaggggaa tcgacaaggc ctggcgctga gtgcgtatct     240

gaggatgagg agatgtccag agtcattccg gtgattgagc ggattacgaa agagcttggt     300

gttcctattt ctgtagacac gtacaaggct tctgtcgcag atgaagcagt gaaagccggt     360

gcatccatta tcaatgatat ttggggagcc aaacatgatc cgaagatggc ttccgttgca     420

gctgaacata atgttccaat tgtactcatg cataaccgcc ctgaaagaaa ctacaatgac     480

ttattgccgg atatgctgtc ggacttaatg gagagtgtaa aaattgctgt tgaggccgga     540

gtagacgaga agaacattat tcttgatcct ggtatcggtt tcgcgaaaac ctatcacgat     600

aacttggcag tgatgaacaa actagagatt ttcagcggat tgggatatcc ggttcttctg     660

gcaacctccc gaaaaagatt catcggacgt gttctggatc ttccgcctga ggagcgggct     720

gagggcacag gcgcgactgt gtgtctcggc attcaaaaag gctgtgacat tgtcagggtc     780

catgatgtaa agcaaattgc cagaatggcg aaaatgatgg acgcgatgct gaataaggga     840

ggggtgcacc atggataa                                                   858

<210>  5
<211>  1293
<212>  DNA
<213>  Bacillus subtilis

<400>  5
ttgtttactg catatcaaga tgcgcgcagc tggattcacg ggaggctgaa attcggggtg      60

aagcccggac ttggtcgaat gaaacagctg atggcgcggt taggacatcc tgaaaaaaaa     120

atccgagcgt tccacgtcgc aggaacaaac ggtaaaggat caactgtcgc ttttatccgt     180

tctatgctgc aggaagccgg atatacggtt ggaacattta catcgcctta tattattacg     240

tttaatgaac ggatcagcgt aaacgggata ccgatttcag atgaggaatg gacagcactc     300

gtcaatcaaa tgaaaccgca cgttgaagcc cttgatcaaa cggaatacgg acagccgaca     360

gaatttgaaa ttatgacagc ttgtgcattt ttatattttg cagagtttca taaggtcgat     420

tttgttattt ttgaaacagg tctgggcggt agattcgatt ctactaatgt ggttgaaccg     480

ctcttaactg tgattacaag catcggacac gatcatatga acattttggg aaacaccatt     540

gaagaaattg caggagaaaa ggccggcatt attaaagaag gtattccaat cgttacagca     600

gttacccagc cggaagcttt acaggtcatc cgccatgaag ccgaacggca tgctgcgccg     660

ttccagtcat tgcatgatgc atgtgttatt ttcaatgaag aggctctgcc tgcaggagaa     720

cagttttcat tcaaaacgga agagaaatgc tatgaagata ttcggacatc tctgattggc     780

acccatcaaa gacaaaatgc tgctttgtcc atttggctgc ggagtggctc caataaggag     840

aatatcgccc atatcagtga cgaagcgtta aggagcgggc ttgtgaaggc tgcttggccg     900

ggacggttag agcttgttca ggaacatcct ccggtatatt tagacggtgc gcataacgaa     960

gaaggtgttg aaaagctggc ggaaacgatg aagcagcgtt tcgccaattc acgcatttct    1020

gtggtgttta gcgcgttaaa ggacaaaccg tatcagaaca tgattaaaag actggaaacc    1080

attgctcatg cgattcattt tgcttctttt gatttcccgc gtgcttccct tgcaaaagat    1140

ctctatgatg caagtgaaat cagcaataaa agctggagtg aagatccaga cgatgtaatc    1200

aaattcatag aaagtaaaaa gggctcaaat gaaattgtgc tgattaccgg atctctttac    1260

tttatttctg acattcgaaa aaggttgaaa taa                                 1293

<210>  6
<211>  507
<212>  DNA
<213>  Bacillus subtilis

<400>  6
atgatttcat tcatttttgc gatggatgcc aacaggctta tcggcaaaga caatgatttg      60

ccgtggcatt tgcccaatga tcttgcatac tttaagaaaa taacatcggg ccattcaatc     120

attatgggcc ggaaaacatt tgaatcgatc ggacgtccgc ttccaaatcg gaaaaatatt     180

gtcgttacct cagcgccgga ttcagaattt cagggatgca cggttgtcag ttcattaaag     240

gatgtactgg acatttgttc aggccctgaa gaatgctttg tgatcggagg ggctcagctc     300

tatacggacc tgttccctta tgcggacaga ctgtatatga cgaaaattca tcacgagttt 360

gagggtgacc gtcactttcc tgaatttgat gaatccaatt ggaagctggt ttcttctgag 420

caggggacca aagacgaaaa aaacccgtat gattacgaat ttctaatgta tgaaaaaaag 480

aattcttcta aagcgggagg attttaa 507

<210> 7
<211> 190
<212> PRT
<213> Bacillus subtilis

<400> 7

Met Lys Glu Val Asn Lys Glu Gln Ile Glu Gln Ala Val Arg Gln Ile
1               5                   10                  15

Leu Glu Ala Ile Gly Glu Asp Pro Asn Arg Glu Gly Leu Leu Asp Thr
            20                  25                  30

Pro Lys Arg Val Ala Lys Met Tyr Ala Glu Val Phe Ser Gly Leu Asn
        35                  40                  45

Glu Asp Pro Lys Glu His Phe Gln Thr Ile Phe Gly Glu Asn His Glu
    50                  55                  60

Glu Leu Val Leu Val Lys Asp Ile Ala Phe His Ser Met Cys Glu His
65                  70                  75                  80

His Leu Val Pro Phe Tyr Gly Lys Ala His Val Ala Tyr Ile Pro Arg
                85                  90                  95

Gly Gly Lys Val Thr Gly Leu Ser Lys Leu Ala Arg Ala Val Glu Ala
            100                 105                 110

Val Ala Lys Arg Pro Gln Leu Gln Glu Arg Ile Thr Ser Thr Ile Ala
        115                 120                 125

Glu Ser Ile Val Glu Thr Leu Asp Pro His Gly Val Met Val Val Val
    130                 135                 140

Glu Ala Glu His Met Cys Met Thr Met Arg Gly Val Arg Lys Pro Gly
145                 150                 155                 160

Ala Lys Thr Val Thr Ser Ala Val Arg Gly Val Phe Lys Asp Asp Ala
                165                 170                 175

Ala Ala Arg Ala Glu Val Leu Glu His Ile Lys Arg Gln Asp
            180                 185                 190

<210>  8
<211>  120
<212>  PRT
<213>  Bacillus subtilis

<400>  8

Met Asp Lys Val Tyr Val Glu Gly Met Glu Phe Tyr Gly Tyr His Gly
1               5                   10                  15

Val Phe Thr Glu Glu Asn Lys Leu Gly Gln Arg Phe Lys Val Asp Leu
            20                  25                  30

Thr Ala Glu Leu Asp Leu Ser Lys Ala Gly Gln Thr Asp Asp Leu Glu
        35                  40                  45

Gln Thr Ile Asn Tyr Ala Glu Leu Tyr His Val Cys Lys Asp Ile Val
    50                  55                  60

Glu Gly Glu Pro Val Lys Leu Val Glu Thr Leu Ala Glu Arg Ile Ala
65                  70                  75                  80

Gly Thr Val Leu Gly Lys Phe Gln Pro Val Gln Gln Cys Thr Val Lys
                85                  90                  95

Val Ile Lys Pro Asp Pro Pro Ile Pro Gly His Tyr Lys Ser Val Ala
            100                 105                 110

Ile Glu Ile Thr Arg Lys Lys Ser
        115                 120


<210>  9
<211>  167
<212>  PRT
<213>  Bacillus subtilis

<400>  9

Met Asn Asn Ile Ala Tyr Ile Ala Leu Gly Ser Asn Ile Gly Asp Arg
1               5                   10                  15

Glu Thr Tyr Leu Arg Gln Ala Val Ala Leu Leu His Gln His Ala Ala
            20                  25                  30

Val Thr Val Thr Lys Val Ser Ser Ile Tyr Glu Thr Asp Pro Val Gly
        35                  40                  45

Tyr Glu Asp Gln Ala Gln Phe Leu Asn Met Ala Val Glu Ile Lys Thr
    50                  55                  60

Ser Leu Asn Pro Phe Glu Leu Leu Glu Leu Thr Gln Gln Ile Glu Asn
65                  70                  75                  80

Glu Leu Gly Arg Thr Arg Glu Val Arg Trp Gly Pro Arg Thr Ala Asp
                85                  90                  95

Leu Asp Ile Leu Leu Phe Asn Arg Glu Asn Ile Glu Thr Glu Gln Leu
            100                 105                 110

Ile Val Pro His Pro Arg Met Tyr Glu Arg Leu Phe Val Leu Ala Pro
        115                 120                 125

Leu Ala Glu Ile Cys Gln Gln Val Glu Lys Glu Ala Thr Ser Ala Glu
    130                 135                 140

Thr Asp Gln Glu Gly Val Arg Val Trp Lys Gln Lys Ser Gly Val Asp
145                 150                 155                 160

Glu Phe Val His Ser Glu Ser
                165

<210>  10
<211>  285
<212>  PRT
<213>  Bacillus subtilis

<400>  10

Met Ala Gln His Thr Ile Asp Gln Thr Gln Val Ile His Thr Lys Pro
1               5                   10                  15

Ser Ala Leu Ser Tyr Lys Glu Lys Thr Leu Val Met Gly Ile Leu Asn
            20                  25                  30

Val Thr Pro Asp Ser Phe Ser Asp Gly Gly Lys Tyr Asp Ser Leu Asp
        35                  40                  45

Lys Ala Leu Leu His Ala Lys Glu Met Ile Asp Asp Gly Ala His Ile
    50                  55                  60

Ile Asp Ile Gly Gly Glu Ser Thr Arg Pro Gly Ala Glu Cys Val Ser
65                  70                  75                  80

Glu Asp Glu Glu Met Ser Arg Val Ile Pro Val Ile Glu Arg Ile Thr
                85                  90                  95

Lys Glu Leu Gly Val Pro Ile Ser Val Asp Thr Tyr Lys Ala Ser Val
            100                 105                 110

Ala Asp Glu Ala Val Lys Ala Gly Ala Ser Ile Ile Asn Asp Ile Trp
        115                 120                 125

Gly Ala Lys His Asp Pro Lys Met Ala Ser Val Ala Ala Glu His Asn
    130                 135                 140

Val Pro Ile Val Leu Met His Asn Arg Pro Glu Arg Asn Tyr Asn Asp
145                 150                 155                 160

Leu Leu Pro Asp Met Leu Ser Asp Leu Met Glu Ser Val Lys Ile Ala
                165                 170                 175

Val Glu Ala Gly Val Asp Glu Lys Asn Ile Ile Leu Asp Pro Gly Ile
            180                 185                 190

Gly Phe Ala Lys Thr Tyr His Asp Asn Leu Ala Val Met Asn Lys Leu
        195                 200                 205

Glu Ile Phe Ser Gly Leu Gly Tyr Pro Val Leu Leu Ala Thr Ser Arg
    210                 215                 220

Lys Arg Phe Ile Gly Arg Val Leu Asp Leu Pro Pro Glu Glu Arg Ala
225                 230                 235                 240

Glu Gly Thr Gly Ala Thr Val Cys Leu Gly Ile Gln Lys Gly Cys Asp
                245                 250                 255

Ile Val Arg Val His Asp Val Lys Gln Ile Ala Arg Met Ala Lys Met
            260                 265                 270

Met Asp Ala Met Leu Asn Lys Gly Gly Val His His Gly
        275                 280                 285

<210>  11
<211>  430
<212>  PRT
<213>  Bacillus subtilis

<400>  11

Met Phe Thr Ala Tyr Gln Asp Ala Arg Ser Trp Ile His Gly Arg Leu
1               5                   10                  15

Lys Phe Gly Val Lys Pro Gly Leu Gly Arg Met Lys Gln Leu Met Ala
            20                  25                  30

Arg Leu Gly His Pro Glu Lys Lys Ile Arg Ala Phe His Val Ala Gly
        35                  40                  45

Thr Asn Gly Lys Gly Ser Thr Val Ala Phe Ile Arg Ser Met Leu Gln
    50                  55                  60

Glu Ala Gly Tyr Thr Val Gly Thr Phe Thr Ser Pro Tyr Ile Ile Thr
65                  70                  75                  80

Phe Asn Glu Arg Ile Ser Val Asn Gly Ile Pro Ile Ser Asp Glu Glu
                85                  90                  95

Trp Thr Ala Leu Val Asn Gln Met Lys Pro His Val Glu Ala Leu Asp
            100                 105                 110

Gln Thr Glu Tyr Gly Gln Pro Thr Glu Phe Glu Ile Met Thr Ala Cys
        115                 120                 125

Ala Phe Leu Tyr Phe Ala Glu Phe His Lys Val Asp Phe Val Ile Phe
    130                 135                 140

Glu Thr Gly Leu Gly Gly Arg Phe Asp Ser Thr Asn Val Val Glu Pro
145                 150                 155                 160

Leu Leu Thr Val Ile Thr Ser Ile Gly His Asp His Met Asn Ile Leu
                165                 170                 175

Gly Asn Thr Ile Glu Glu Ile Ala Gly Glu Lys Ala Gly Ile Ile Lys
            180                 185                 190

Glu Gly Ile Pro Ile Val Thr Ala Val Thr Gln Pro Glu Ala Leu Gln
        195                 200                 205

Val Ile Arg His Glu Ala Glu Arg His Ala Ala Pro Phe Gln Ser Leu
    210                 215                 220

His Asp Ala Cys Val Ile Phe Asn Glu Glu Ala Leu Pro Ala Gly Glu
225                 230                 235                 240

Gln Phe Ser Phe Lys Thr Glu Glu Lys Cys Tyr Glu Asp Ile Arg Thr
                245                 250                 255

Ser Leu Ile Gly Thr His Gln Arg Gln Asn Ala Ala Leu Ser Ile Leu
            260                 265                 270

Ala Ala Glu Trp Leu Asn Lys Glu Asn Ile Ala His Ile Ser Asp Glu
        275                 280                 285

Ala Leu Arg Ser Gly Leu Val Lys Ala Ala Trp Pro Gly Arg Leu Glu
    290                 295                 300

Leu Val Gln Glu His Pro Pro Val Tyr Leu Asp Gly Ala His Asn Glu
305                 310                 315                 320

Glu Gly Val Glu Lys Leu Ala Glu Thr Met Lys Gln Arg Phe Ala Asn
                325                 330                 335

Ser Arg Ile Ser Val Val Phe Ser Ala Leu Lys Asp Lys Pro Tyr Gln
            340                 345                 350

Asn Met Ile Lys Arg Leu Glu Thr Ile Ala His Ala Ile His Phe Ala
        355                 360                 365

Ser Phe Asp Phe Pro Arg Ala Ser Leu Ala Lys Asp Leu Tyr Asp Ala
    370                 375                 380

Ser Glu Ile Ser Asn Lys Ser Trp Ser Glu Asp Pro Asp Asp Val Ile
385                 390                 395                 400

Lys Phe Ile Glu Ser Lys Lys Gly Ser Asn Glu Ile Val Leu Ile Thr
                405                 410                 415

Gly Ser Leu Tyr Phe Ile Ser Asp Ile Arg Lys Arg Leu Lys
            420                 425                 430

<210>  12
<211>  168
<212>  PRT
<213>  Bacillus subtilis

<400>  12

Met Ile Ser Phe Ile Phe Ala Met Asp Ala Asn Arg Leu Ile Gly Lys
1               5                   10                  15

Asp Asn Asp Leu Pro Trp His Leu Pro Asn Asp Leu Ala Tyr Phe Lys
            20                  25                  30

Lys Ile Thr Ser Gly His Ser Ile Ile Met Gly Arg Lys Thr Phe Glu
        35                  40                  45

Ser Ile Gly Arg Pro Leu Pro Asn Arg Lys Asn Ile Val Val Thr Ser
    50                  55                  60

Ala Pro Asp Ser Glu Phe Gln Gly Cys Thr Val Val Ser Ser Leu Lys
65                  70                  75                  80

Asp Val Leu Asp Ile Cys Ser Gly Pro Glu Glu Cys Phe Val Ile Gly
                85                  90                  95

Gly Ala Gln Leu Tyr Thr Asp Leu Phe Pro Tyr Ala Asp Arg Leu Tyr
            100                 105                 110

Met Thr Lys Ile His His Glu Phe Glu Gly Asp Arg His Phe Pro Glu
        115                 120                 125

Phe Asp Glu Ser Asn Trp Lys Leu Val Ser Ser Glu Gln Gly Thr Lys
    130                 135                 140

Asp Glu Lys Asn Pro Tyr Asp Tyr Glu Phe Leu Met Tyr Glu Lys Lys
145                 150                 155                 160

Asn Ser Ser Lys Ala Gly Gly Phe
                165

<210>  13
<211>  683
<212>  DNA
<213>  Artificial Sequence

<220>
<223>  Optimized folE sequence for Bacillus subtilis codon usage

<400>  13
cgcagcatac gcagcgaaat cagcatcacc ggagaatccc aacgaagcca actagtatga      60

aagaagtcaa taaagaacaa attgaacagg cagtgagaca gattcttgaa gcaattggag     120

aagatccgaa cagagagggc ttacttgata caccgaaaag agttgctaaa atgtatgcgg     180

aagtcttttc aggcttaaat gaagatccga aagagcattt tcagacaatt ttcggagaaa     240

accatgaaga gctggtcctt gtgaaagata ttgcgtttca ctcaatgtgc gaacatcacc     300

tggtgccgtt ttacggcaag gcacacgttg cgtatattcc tagaggcgga aaagttacag     360

gcttgtcaaa attagcacgc gcagttgaag ctgttgcaaa aagaccgcaa ttacaggaac     420

gcattacatc tacaattgcg gaatcaattg tcgagacatt agaccctcat ggcgttatgg     480

ttgtcgttga agctgaacac atgtgcatga caatgcgcgg cgtcagaaaa cctggcgcaa     540

aaacagtcac atcagcagtc agaggcgtgt ttaaagatga tgcggcagct cgtgcggaag     600

tcctggaaca tattaaacgc caggactgaa aaagagggga gggaaacatt aatgacgacc     660

tggctaacga gtctcgccga tct                                             683

<210>  14
<211>  454
<212>  PRT
<213>  Artificial Sequence

<220>
<223>  Optimized  folB sequence for Bacillus subtilis codon usage

<400> 14

Thr Thr Cys Thr Thr Thr Thr Thr Gly Cys Gly Cys Cys Ala Gly Gly
1               5                   10                  15

Thr Ala Gly Cys Cys Ala Thr Ala Gly Cys Thr Gly Gly Thr Cys Ala
            20                  25                  30

Thr Ala Thr Gly Ala Thr Gly Gly Ala Thr Ala Ala Ala Gly Thr Thr
        35                  40                  45

Thr Ala Thr Gly Thr Gly Gly Ala Ala Gly Gly Ala Ala Thr Gly Gly
    50                  55                  60

Ala Ala Thr Thr Thr Thr Ala Thr Gly Gly Cys Thr Ala Thr Cys Ala
65                  70                  75                  80

Thr Gly Gly Cys Gly Thr Cys Thr Thr Cys Ala Cys Ala Gly Ala Ala
                85                  90                  95

Gly Ala Gly Ala Ala Cys Ala Ala Ala Thr Thr Gly Gly Gly Ala Cys
            100                 105                 110

Ala Ala Cys Gly Cys Thr Thr Cys Ala Ala Ala Gly Thr Ala Gly Ala
        115                 120                 125

Thr Cys Thr Gly Ala Cys Ala Gly Cys Ala Gly Ala Ala Cys Thr Gly
    130                 135                 140

Gly Ala Thr Thr Thr Ala Thr Cys Ala Ala Ala Ala Gly Cys Ala Gly
145                 150                 155                 160

Gly Ala Cys Ala Ala Ala Cys Ala Gly Ala Cys Gly Ala Cys Cys Thr
                165                 170                 175

Thr Gly Ala Ala Cys Ala Gly Ala Cys Ala Ala Thr Thr Ala Ala Cys
            180                 185                 190

Thr Ala Thr Gly Cys Ala Gly Ala Gly Cys Thr Thr Thr Ala Cys Cys
        195                 200                 205

Ala Thr Gly Thr Cys Thr Gly Thr Ala Ala Ala Gly Ala Cys Ala Thr
    210                 215                 220

Thr Gly Thr Cys Gly Ala Ala Gly Gly Cys Gly Ala Gly Cys Cys Gly
225                 230                 235                 240

Gly Thr Cys Ala Ala Ala Thr Thr Gly Gly Thr Ala Gly Ala Gly Ala

245                             250                             255

Cys Cys Cys Thr Thr Gly Cys Thr Gly Ala Gly Cys Gly Gly Ala Thr
                    260                             265                             270

Ala Gly Cys Thr Gly Gly Cys Ala Cys Ala Gly Thr Thr Thr Thr Ala
            275                             280                             285

Gly Gly Thr Ala Ala Ala Thr Thr Thr Cys Ala Gly Cys Cys Gly Gly
      290                             295                             300

Thr Thr Cys Ala Ala Cys Ala Ala Thr Gly Thr Ala Cys Gly Gly Thr
305                             310                             315                             320

Gly Ala Ala Ala Gly Thr Thr Ala Thr Thr Ala Ala Ala Cys Cys Ala
                        325                             330                             335

Gly Ala Thr Cys Cys Gly Cys Cys Gly Ala Thr Thr Cys Cys Thr Gly
                    340                             345                             350

Gly Cys Cys Ala Cys Thr Ala Thr Ala Ala Ala Thr Cys Ala Gly Thr
            355                             360                             365

Ala Gly Cys Ala Ala Thr Thr Gly Ala Ala Ala Thr Thr Ala Cys Gly
      370                             375                             380

Ala Gly Ala Ala Ala Ala Ala Ala Gly Thr Cys Ala Thr Ala Ala Ala
385                             390                             395                             400

Thr Thr Ala Ala Thr Thr Cys Thr Ala Gly Ala Gly Thr Cys Gly Ala
                        405                             410                             415

Thr Cys Cys Cys Cys Gly Gly Gly Thr Thr Cys Gly Cys Cys Ala Gly
                    420                             425                             430

Cys Ala Ala Thr Gly Ala Cys Thr Ala Cys Cys Gly Gly Cys Ala Gly
            435                             440                             445

Cys Cys Cys Gly Cys Cys
      450

<210>  15
<211>  595
<212>  DNA
<213>  Artificial Sequence

<220>
<223>  Optimized folK sequence for Bacillus subtilis codon usage

<400>  15
ggcggggctt cttttatcga atccagcgtg acatatgatg aacaacattg cgtacattgc      60

gcttggctct aatattggag atagagaaac gtatctgcgc caggccgttg cgttactgca     120

tcaacatgct gcggtcacag ttacaaaagt cagctcaatt tatgaaacag atccggtcgg     180

ctatgaagac caagcccagt ttttaaatat ggcggttgaa attaaaacaa gcctgaatcc     240

gtttgaactt ctggaactga cacagcaaat cgaaaacgaa ctgggccgca cacgcgaagt     300

tagatggggc ccgagaacag cggatttaga cattctgctg tttaacagag aaaacattga     360

aacagagcag ttaattgtcc cgcatcctcg catgtatgaa cgcctgtttg ttcttgcgcc     420

gcttgcggaa atttgccagc aggtcgagaa agaagcgaca agcgcggaaa cggatcaaga     480

aggagttaga gtttggaaac aaaaatcagg cgttgacgaa tttgtacata gcgaaagctg     540

aaaaagaggg gagggaaaca ttaatgaccg accctcatgg aaacccttcc tggcg          595


<210>  16
<211>  948
<212>  DNA
<213>  Artificial Sequence

<220>
<223>  Optimized sul sequence for Bacillus subtilis codon usage

<400>  16
gaccgaccct catggaaacc cttcctggcg catatgatgg cgcagcacac aatagatcaa      60

acacaagtca ttcatacgaa accgagcgcg ctttcatata aagaaaaaac actggtcatg     120

ggcattctta acgttacacc tgattctttt agcgatggtg gaaaatatga cagcttggac     180

aaggcgcttc tgcatgccaa agaaatgatc gacgacggcg cgcacattat tgacatagga     240

ggcgagagca caagaccggg agctgaatgc gtcagcgaag acgaagaaat gtctcgggtc     300

attccggtca ttgaacgcat cacaaaggaa ctcggcgtcc cgatttcagt ggatacatat     360

aaagcatctg tggcagacga agcagtcaaa gcgggcgcat ctattatcaa tgacatttgg     420

ggagcgaaac atgatccgaa gatggcaagc gtcgcagcgg aacataacgt tccaattgtc     480

ctgatgcaca atcggccaga acggaattat aacgaccttc ttccggatat gctgagcgac     540

cttatggaat cagtcaaaat tgcggttgag gcgggcgtgg atgagaaaaa tattatttta     600

gatccgggca tcggcttcgc gaagacatac catgataatc ttgcagtgat gaataagtta     660

gagatcttca gcggacttgg ctatcctgtc ctgctggcta catctcgtaa aagatttatc     720

ggaagagttc ttgatttacc gcctgaagag agagcagagg gcacaggagc gacagtctgc     780

ttgggcattc agaaaggatg cgacatagtg cgtgttcatg atgtcaagca aattgccaga     840

atggcgaaaa tgatggacgc gatgctgaat aagggagggg tgcaccatgg atgaaaaaga     900

ggggagggaa acattaattt ctttttgcgc caggtagcca tagctggt                  948

<210> 17
<211> 598
<212> DNA
<213> Artificial Sequence

<220>
<223> Optimized dfrA sequence for Bacillus subtilis codon usage

<400> 17
gacgacctgg ctaacgagtc tcgccgatct catatgatga tttcatttat tttcgcaatg 60

gacgcgaata gactgatagg caaagacaat gatctgccgt ggcatttacc gaatgacctg 120

gcttatttta aaaaaattac aagcggccat agcatcatta tgggacgtaa aacatttgag 180

tcaattggca gacctcttcc gaacagaaaa aacattgttg tcacatctgc gccggattca 240

gaatttcagg gctgcacagt cgtctcaagc cttaaagacg ttcttgatat ttgcagcgga 300

ccggaagagt gttttgtcat tggcggagcg caattataca cagatctttt tccgtacgcg 360

gatagactgt atatgacaaa aatccaccat gaatttgaag gcgacagaca ctttcctgaa 420

tttgacgaga gcaactggaa actcgtgtct agcgaacagg gcacgaagga tgagaaaaat 480

ccgtatgact atgaatttct tatgtatgaa aagaaaaaca gcagcaaagc gggaggcttt 540

tgaaaaagag gggagggaaa cattaatggc ggggcttctt ttatcgaatc cagcgtga 598


<210> 18
<211> 1460
<212> DNA
<213> Artificial Sequence

<220>
<223> Assembly fragment

<400> 18
gccttttaat cccggcaaca gcttaatcag tacatccatc attccgaagc atccgacatt 60

cgatcattac aaggaattat ttgcgggcaa ggaaagcctt caatatgtgc agtggtatgt 120

caactctatg aagatcagcc tgtttacaat ggcagggtct ttgctctgtg tgacgtttac 180

ggcctatgcg ttttcgcgct ttcggtttaa agggaggaaa tacgctttaa cgctcttttt 240

attgctgcag atgattcctc agttttcagc tttaattgcc ttgtttgtgc tggcgcaaat 300

cttgggaatg atcaatagcc actggctgct aatcttgctt tatatcggcg gcctgatccc 360

gatgaatacg tatttgatga aagggtacat ggattccatt ccgatggatt tagacgaaag 420

cgccaagatt gacggagcca gcagcaccag aatcttcttc cagatcattc tgccattatc 480

aaaaccgatg gcggcagtcg tggccatgaa cggctttacc ggtccgctcg gagattttgt 540

gctgtcctca accatattga gaacgcctga atcatataca ttgcccgtcg gtctattcaa 600

tttagtgaat gatgtcatgg gggccagcta tacgacattt gcggccggag ccctgcttat 660

cagcataccg gttgccgtca tctttattat gctgcaaaag aattttgtgt ccggattaac 720

cgcaggcgga acgaagggct aagagaacaa ggaggagaat gtgatgtcaa agcttgaaaa 780

aacgcacgta acaaaagcaa aatttatgct ccatggggga gactacaacc ccgatcagtg 840

gctggatcgg cccgatattt tagctgacga tatcaaactg atgaagcttt ctcatacgaa 900

tacgttttct gtcggcattt ttgcatggag cgcacttgag ccggaggagg gcgtatatca 960

atttgaatgg ctggatgata tttttgagcg gattcacagt ataggcggcc gggtcatatt 1020

agcaacgccg agcggagccc gtccggcctg gctgtcgcaa acctatccgg aagttttgcg 1080

cgtcaatgcc tcccgcgtca aacagctgca cggcggaagg cacaaccact gcctcacatc 1140

taaagtctac cgagaaaaaa cacggcacat caaccgctac gggtgcgcat gatcgtatgg 1200

ttcactgtcc accaaccaaa actgtgctca gtaccgccaa tatttctccc ttggggggta 1260

caaagaggtg tccctagaag agatccacgc tgtgtaaaaa ttttacaaaa aggtattgac 1320

tttccctaca gggtgtgtaa taatttaatt acaggcgggg gcaaccccgc tcagtaccta 1380

gagcgtaaaa gaggggaggg aaacactagt tggcttcgtt gggattctcc ggtgatgctg 1440

atttcgctgc gtatgctgcg 1460

<210> 19
<211> 1038
<212> DNA
<213> Artificial Sequence

<220>
<223> Synthetized DNA fragment for erythromycin resistance cassette

<400> 19
tctagaaatt aagaaggagg gattcgtcat gttggtattc caaatgcgtt atgtagataa 60

aacatctact gttttgaaac agactaaaaa cagtgattac gcagataaat aaatacgtta 120

gattaattcc taccagtgac taatcttatg actttttaaa cagataacta aaattacaaa 180

caaatcgttt aacttctgta tttgtttata gatgtaatca cttcaggagt gattacatga 240

acaaaaatat aaaatattct caaaactttt taacgagtga aaaagtactc aaccaaataa 300

taaaacaatt gaatttaaaa gaaaccgata ccgtttacga aattggaaca ggtaaagggc 360

atttaacgac gaaactggct aaaataagta aacaggtaac gtctattgaa ttagacagtc 420

atctattcaa cttatcgtca gaaaaattaa aactgaacat tcgtgtcact ttaattcacc 480

aagatattct acagtttcaa ttccctaaca aacagaggta taaaattgtt gggaatattc 540

cttaccattt aagcacacaa attattaaaa aagtggtttt tgaaagccat gcgtctgaca 600

tctatctgat tgttgaagaa ggattctaca agcgtacctt ggatattcac cgaacactag 660

ggttgctctt gcacactcaa gtctcgattc agcaattgct taagctgcca gcggaatgct 720

ttcatcctaa accaaaagta aacagtgtct taataaaact tacccgccat accacagatg 780

ttccagataa atattggaag ctatatacgt actttgtttc aaaatgggtc aatcgagaat 840

atcgtcaact gtttactaaa aatcagtttc atcaagcaat gaaacacgcc aaagtaaaca 900

atttaagtac cgttacttat gagcaagtat tgtctatttt taatagttat ctattattta 960

acgggaggaa ataattctat gagtcgcttt tgtaaatttg gaaagttaca cgttactaaa 1020

gggaatgtag atggatcc 1038

<210> 20
<211> 5390
<212> DNA
<213> Artificial Sequence

<220>
<223> Assembly of artificial folate operon as an expression cassette

<400> 20
tcccggcaac agcttaatca gtacatccat cattccgaag catccgacat tcgatcatta 60

caaggaatta tttgcgggca aggaaagcct tcaatatgtg cagtggtatg tcaactctat 120

gaagatcagc ctgtttacaa tggcagggtc tttgctctgt gtgacgttta cggcctatgc 180

gttttcgcgc tttcggttta aagggaggaa atacgcttta acgctctttt tattgctgca 240

gatgattcct cagttttcag ctttaattgc cttgtttgtg ctggcgcaaa tcttgggaat 300

gatcaatagc cactggctgc taatcttgct ttatatcggc ggcctgatcc cgatgaatac 360

gtatttgatg aaagggtaca tggattccat tccgatggat ttagacgaaa gcgccaagat 420

tgacggagcc agcagcacca gaatcttctt ccagatcatt ctgccattat caaaaccgat 480

ggcggcagtc gtggccatga acggctttac cggtccgctc ggagattttg tgctgtcctc 540

aaccatattg agaacgcctg aatcatatac attgcccgtc ggtctattca atttagtgaa 600

tgatgtcatg ggggccagct atacgacatt tgcggccgga gccctgctta tcagcatacc 660

ggttgccgtc atctttatta tgctgcaaaa gaattttgtg tccggattaa ccgcaggcgg 720

aacgaagggc taagagaaca aggaggagaa tgtgatgtca aagcttgaaa aaacgcacgt 780

aacaaaagca aaatttatgc tccatggggg agactacaac cccgatcagt ggctggatcg 840

gcccgatatt ttagctgacg atatcaaact gatgaagctt tctcatacga atacgttttc 900

tgtcggcatt tttgcatgga gcgcacttga gccggaggag ggcgtatatc aatttgaatg 960

gctggatgat atttttgagc ggattcacag tataggcggc cgggtcatat tagcaacgcc 1020

gagcggagcc cgtccggcct ggctgtcgca aacctatccg gaagttttgc gcgtcaatgc 1080

ctcccgcgtc aaacagctgc acggcggaag gcacaaccac tgcctcacat ctaaagtcta 1140

ccgagaaaaa acacggcaca tcaaccgcta cgggtgcgca tgatcgtatg gttcactgtc 1200

caccaaccaa aactgtgctc agtaccgcca atatttctcc cttggggggt acaaagaggt 1260

gtccctagaa gagatccacg ctgtgtaaaa attttacaaa aaggtattga ctttccctac 1320

agggtgtgta ataatttaat tacaggcggg ggcaaccccg ctcagtacct agagcgtaaa 1380

agaggggagg gaaacactag ttggcttcgt tgggattctc cggtgatgct gatttcgctg 1440

cgtatgctgc gatgaaagaa gtcaataaag aacaaattga acaggcagtg agacagattc 1500

ttgaagcaat tggagaagat ccgaacagag agggcttact tgatacaccg aaaagagttg 1560

ctaaaatgta tgcggaagtc ttttcaggct taaatgaaga tccgaaagag cattttcaga 1620

caattttcgg agaaaaccat gaagagctgg tccttgtgaa agatattgcg tttcactcaa 1680

tgtgcgaaca tcacctggtg ccgttttacg gcaaggcaca cgttgcgtat attcctagag 1740

gcggaaaagt tacaggcttg tcaaaattag cacgcgcagt tgaagctgtt gcaaaaagac 1800

cgcaattaca ggaacgcatt acatctacaa ttgcggaatc aattgtcgag acattagacc 1860

ctcatggcgt tatggttgtc gttgaagctg aacacatgtg catgacaatg cgcggcgtca 1920

gaaaacctgg cgcaaaaaca gtcacatcag cagtcagagg cgtgtttaaa gatgatgcgg 1980

cagctcgtgc ggaagtcctg gaacatatta aacgccagga ctgaaaaaga ggggagggaa 2040

acattatgat gatttcattt attttcgcaa tggacgcgaa tagactgata ggcaaagaca 2100

atgatctgcc gtggcattta ccgaatgacc tggcttattt taaaaaaatt acaagcggcc 2160

atagcatcat tatgggacgt aaaacatttg agtcaattgg cagacctctt ccgaacagaa 2220

aaaacattgt tgtcacatct gcgccggatt cagaatttca gggctgcaca gtcgtctcaa 2280

gccttaaaga cgttcttgat atttgcagcg gaccggaaga gtgttttgtc attggcggag 2340

cgcaattata cacagatctt tttccgtacg cggatagact gtatatgaca aaaatccacc 2400

atgaatttga aggcgacaga cactttcctg aatttgacga gagcaactgg aaactcgtgt 2460

ctagcgaaca gggcacgaag gatgagaaaa atccgtatga ctatgaattt cttatgtatg 2520

aaaagaaaaa cagcagcaaa gcgggaggct tttgaaaaag aggggaggga aacattatga 2580

tgaacaacat tgcgtacatt gcgcttggct ctaatattgg agatagagaa acgtatctgc 2640

gccaggccgt tgcgttactg catcaacatg ctgcggtcac agttacaaaa gtcagctcaa 2700

tttatgaaac agatccggtc ggctatgaag accaagccca gtttttaaat atggcggttg 2760

aaattaaaac aagcctgaat ccgtttgaac ttctggaact gacacagcaa atcgaaaacg 2820

aactgggccg cacacgcgaa gttagatggg gcccgagaac agcggattta gacattctgc 2880

tgtttaacag agaaaacatt gaaacagagc agttaattgt cccgcatcct cgcatgtatg 2940

aacgcctgtt tgttcttgcg ccgcttgcgg aaatttgcca gcaggtcgag aaagaagcga 3000

caagcgcgga aacggatcaa gaaggagtta gagtttggaa acaaaaatca ggcgttgacg 3060

aatttgtaca tagcgaaagc tgaaaaagag gggagggaaa cattatgatg gcgcagcaca 3120

caatagatca aacacaagtc attcatacga aaccgagcgc gctttcatat aaagaaaaaa 3180

cactggtcat gggcattctt aacgttacac ctgattcttt tagcgatggt ggaaaatatg 3240

acagcttgga caaggcgctt ctgcatgcca aagaaatgat cgacgacggc gcgcacatta 3300

ttgacatagg aggcgagagc acaagaccgg gagctgaatg cgtcagcgaa gacgaagaaa 3360

tgtctcgggt cattccggtc attgaacgca tcacaaagga actcggcgtc ccgatttcag 3420

tggatacata taaagcatct gtggcagacg aagcagtcaa agcgggcgca tctattatca 3480

atgacatttg gggagcgaaa catgatccga agatggcaag cgtcgcagcg gaacataacg 3540

ttccaattgt cctgatgcac aatcggccag aacggaatta taacgacctt cttccggata 3600

tgctgagcga ccttatggaa tcagtcaaaa ttgcggttga ggcgggcgtg gatgagaaaa 3660

atattatttt agatccgggc atcggcttcg cgaagacata ccatgataat cttgcagtga 3720

tgaataagtt agagatcttc agcggacttg gctatcctgt cctgctggct acatctcgta 3780

aaagatttat cggaagagtt cttgatttac cgcctgaaga gagagcagag ggcacaggag 3840

cgacagtctg cttgggcatt cagaaaggat gcgacatagt gcgtgttcat gatgtcaagc 3900

aaattgccag aatggcgaaa atgatggacg cgatgctgaa taagggaggg gtgcaccatg 3960

gatgaaaaag aggggaggga aacattatga tggataaagt ttatgtggaa ggaatggaat 4020

tttatggcta tcatggcgtc ttcacagaag agaacaaatt gggacaacgc ttcaaagtag 4080

atctgacagc agaactggat ttatcaaaag caggacaaac agacgacctt gaacagacaa 4140

ttaactatgc agagctttac catgtctgta aagacattgt cgaaggcgag ccggtcaaat 4200

tggtagagac ccttgctgag cggatagctg gcacagtttt aggtaaattt cagccggttc 4260

aacaatgtac ggtgaaagtt attaaaccag atccgccgat tcctggccac tataaatcag 4320

tagcaattga aattacgaga aaaaagtcat aaattaattc tagaaattaa gaaggaggga 4380

ttcgtcatgt tggtattcca aatgcgttat gtagataaaa catctactgt tttgaaacag 4440

actaaaaaca gtgattacgc agataaataa atacgttaga ttaattccta ccagtgacta 4500

atcttatgac tttttaaaca gataactaaa attacaaaca aatcgtttaa cttctgtatt 4560

tgtttataga tgtaatcact tcaggagtga ttacatgaac aaaaatataa aatattctca 4620

aaacttttta acgagtgaaa aagtactcaa ccaaataata aaacaattga atttaaaaga 4680

aaccgatacc gtttacgaaa ttggaacagg taaagggcat ttaacgacga aactggctaa 4740

aataagtaaa caggtaacgt ctattgaatt agacagtcat ctattcaact tatcgtcaga 4800

aaaattaaaa ctgaacattc gtgtcacttt aattcaccaa gatattctac agtttcaatt 4860

ccctaacaaa cagaggtata aaattgttgg gaatattcct taccatttaa gcacacaaat 4920

tattaaaaaa gtggtttttg aaagccatgc gtctgacatc tatctgattg ttgaagaagg 4980

attctacaag cgtaccttgg atattcaccg aacactaggg ttgctcttgc acactcaagt 5040

ctcgattcag caattgctta agctgccagc ggaatgcttt catcctaaac caaaagtaaa 5100

cagtgtctta ataaaactta cccgccatac cacagatgtt ccagataaat attggaagct 5160

atatacgtac tttgtttcaa aatgggtcaa tcgagaatat cgtcaactgt ttactaaaaa 5220

tcagtttcat caagcaatga aacacgccaa agtaaacaat ttaagtaccg ttacttatga 5280

gcaagtattg tctattttta atagttatct attatttaac gggaggaaat aattctatga 5340

gtcgcttttg taaatttgga aagttacacg ttactaaagg gaatgtagat 5390

<210> 21
<211> 2124
<212> DNA
<213> Artificial Sequence

<220>
<223> Tetracycline resistance cassette

<400> 21
aattcttact gcagatagtg tacgtaaaaa gattaaatta ttgcttggtg aaaaaagtct 60

tgcaatggtg caggttgttc tcaatgtcga aaatatgtat ttgtatttaa cgcacgagag 120

caaggacgct attgctaaga agaaacatgt ttatgataag gctgatataa agctaatcaa 180

taattttgat attgaccgtt atgtgacgtt agatgtcgag gaaaagaccg aacttttcaa 240

tgtggttgta tcgcttattc gtgcgtacac tctccaaaat attttgattt tgtatgattt 300

cattgacgaa aatggagaaa cttatgggtt gactataaat ttggttaacg aagttattgc 360

agggaaaact ggttttatga aattgttgtt tgacggagct tatcaacgta gtaagcgtgg 420

aacaaagaac gaagagagat aaaaagttga tctttgtgaa aactacagaa agtaaagaat 480

gaaaagagta atgctaacat agcattacgg attttatgac cgatgatgaa gaaaagaatt 540

tgaaacttag tttatatgtg gtaaaatgtt ttaatcaagt ttaggaggaa ttaattatga 600

agtgtaatta atgtaacagg gttcaattaa aagagggaag cgtatcatta accctataaa 660

ctacgtctgc cctcattatt ggagggtgaa atgtgaatac atcctattca caatcgaatt 720

tacgacacaa ccaaatttta atttggcttt gcattttatc tttttttagc gtattaaatg 780

aaatggtttt gaacgtctca ttacctgata ttgcaaatga ttttaataaa ccacctgcga 840

gtacaaactg ggtgaacaca gcctttatgt taaccttttc cattggaaca gctgtatatg 900

gaaagctatc tgatcaatta ggcatcaaaa ggttactcct atttggaatt ataataaatt 960

gtttcgggtc ggtaattggg tttgttggcc attctttctt ttccttactt attatggctc 1020

gttttattca aggggctggt gcagctgcat ttccagcact cgtaatggtt gtagttgcgc 1080

gctatattcc aaaggaaaat aggggtaaag catttggtct tattggatcg atagtagcca 1140

tgggagaagg agtcggtcca gcgattggtg gaatgatagc ccattatatt cattggtcct 1200

atcttctact cattcctatg ataacaatta tcactgttcc gtttcttatg aaattattaa 1260

agaaagaagt aaggataaaa ggtcattttg atatcaaagg aattatacta atgtctgtag 1320

gcattgtatt ttttatgttg tttacaacat catatagcat ttcttttctt atcgttagcg 1380

tgctgtcatt cctgatattt gtaaaacata tcaggaaagt aacagatcct tttgttgatc 1440

ccggattagg gaaaaatata ccttttatga ttggagttct ttgtggggga attatatttg 1500

gaacagtagc agggtttgtc tctatggttc cttatatgat gaaagatgtt caccagctaa 1560

gtactgccga aatcggaagt gtaattattt tccctggaac aatgagtgtc attattttcg 1620

gctacattgg tgggatactt gttgatagaa gaggtccttt atacgtgtta aacatcggag 1680

ttacatttct ttctgttagc tttttaactg cttcctttct tttagaaaca acatcatggt 1740

tcatgacaat tataatcgta tttgttttag gtgggctttc gttcaccaaa acagttatat 1800

caacaattgt ttcaagtagc ttgaaacagc aggaagctgg tgctggaatg agtttgctta 1860

actttaccag ctttttatca gagggaacag gtattgcaat tgtaggtggt ttattatcca 1920

tacccttact tgatcaaagg ttgttaccta tggaagttga tcagtcaact tatctgtata 1980

gtaatttgtt attacttttt tcaggaatca ttgtcattag ttggctggtt accttgaatg 2040

tatataaaca ttctcaaagg gatttctaaa tcgttaaggg atcaactttg ggagagagtt 2100

caaaattgat cctttctgca gaag 2124

<210> 22
<211> 419
<212> PRT
<213> Lactobacillus reuteri

<400> 22

Met Arg Thr Tyr Glu Gln Ile Asn Ala Gly Phe Asn Arg Gln Met Leu
1               5                   10                  15

Gly Gly Gln Arg Asp Arg Val Lys Phe Leu Arg Arg Ile Leu Thr Arg
            20                  25                  30

Leu Gly Asn Pro Asp Gln Arg Phe Lys Ile Ile His Ile Ala Gly Thr
        35                  40                  45

Asn Gly Lys Gly Ser Thr Gly Thr Met Leu Glu Gln Gly Leu Gln Asn
    50                  55                  60

Ala Gly Tyr Arg Val Gly Tyr Phe Ser Ser Pro Ala Leu Val Asp Asp
65                  70                  75                  80

Arg Glu Gln Ile Lys Val Asn Asp His Leu Ile Ser Lys Lys Asp Phe
                85                  90                  95

Ala Met Thr Tyr Gln Lys Ile Thr Glu His Leu Pro Ala Asp Leu Leu

100                 105                 110

Pro Asp Asp Ile Thr Ile Phe Glu Trp Trp Thr Leu Ile Met Leu Gln
        115                 120                 125

Tyr Phe Ala Asp Gln Lys Val Asp Trp Ala Val Ile Glu Cys Gly Leu
    130                 135                 140

Gly Gly Gln Asp Asp Ala Thr Asn Ile Ile Ser Ala Pro Phe Ile Ser
145                 150                 155                 160

Val Ile Thr His Ile Ala Leu Asp His Thr Arg Ile Leu Gly Pro Thr
                165                 170                 175

Ile Ala Lys Ile Ala Gln Ala Lys Ala Gly Ile Ile Lys Thr Gly Thr
            180                 185                 190

Lys Gln Val Phe Leu Ala Pro His Gln Glu Lys Asp Ala Leu Thr Ile
        195                 200                 205

Ile Arg Glu Lys Ala Gln Gln Gln Lys Val Gly Leu Thr Gln Ala Asp
    210                 215                 220

Ala Gln Ser Ile Val Asp Gly Lys Ala Ile Leu Lys Val Asn His Lys
225                 230                 235                 240

Ile Tyr Lys Val Pro Phe Asn Leu Leu Gly Thr Phe Gln Ser Glu Asn
                245                 250                 255

Leu Gly Thr Val Val Ser Val Phe Asn Phe Leu Tyr Gln Arg Arg Leu
            260                 265                 270

Val Thr Ser Trp Gln Pro Leu Leu Ser Thr Leu Ala Thr Val Lys Ile
        275                 280                 285

Ala Gly Arg Met Gln Lys Ile Ala Asp His Pro Pro Ile Ile Leu Asp
    290                 295                 300

Gly Ala His Asn Pro Asp Ala Ala Lys Gln Leu Thr Lys Thr Ile Ser
305                 310                 315                 320

Lys Leu Pro His Asn Lys Val Ile Met Val Leu Gly Phe Leu Ala Asp
                325                 330                 335

Lys Asn Ile Ser Gln Met Val Lys Ile Tyr Gln Gln Met Ala Asp Glu
            340                 345                 350

Ile Ile Ile Thr Thr Pro Asp His Pro Thr Arg Ala Leu Asp Ala Ser
        355                 360                 365

Ala Leu Lys Ser Val Leu Pro Gln Ala Ile Ile Ala Asn Asn Pro Arg
    370                 375                 380

Gln Gly Leu Val Val Ala Lys Lys Ile Ala Glu Pro Asn Asp Leu Ile
385                 390                 395                 400

Ile Val Thr Gly Ser Phe Tyr Thr Ile Lys Asp Ile Glu Ala Asn Leu
                405                 410                 415

Asp Glu Lys

<210>  23
<211>  406
<212>  PRT
<213>  Ashbya gossypii

<400>  23

Met Glu Leu Gly Leu Gly Arg Ile Thr Gln Val Leu Arg Gln Leu His
1               5                   10                  15

Ser Pro His Glu Arg Met Arg Val Leu His Val Ala Gly Thr Asn Gly
            20                  25                  30

Lys Gly Ser Val Cys Ala Tyr Leu Ala Ala Val Leu Arg Ala Gly Gly
        35                  40                  45

Glu Arg Val Gly Arg Phe Thr Ser Pro His Leu Val His Pro Arg Asp
    50                  55                  60

Ala Ile Thr Val Asp Gly Glu Val Ile Gly Ala Ala Thr Tyr Ala Ala
65                  70                  75                  80

Leu Lys Ala Glu Val Val Ala Ala Gly Thr Cys Thr Glu Phe Glu Ala
                85                  90                  95

Gln Thr Ala Val Ala Leu Thr His Phe Ala Arg Leu Glu Cys Thr Trp
            100                 105                 110

Cys Val Val Glu Val Gly Val Gly Gly Arg Leu Asp Ala Thr Asn Val
        115                 120                 125

Val Pro Gly Gly Arg Lys Leu Cys Ala Ile Thr Lys Val Gly Leu Asp
    130                 135                 140

His Gln Ala Leu Leu Gly Gly Thr Leu Ala Val Val Ala Arg Glu Lys
145                 150                 155                 160

Ala Gly Ile Val Val Pro Gly Val Arg Phe Val Ala Val Asp Gly Thr
                165                 170                 175

Asn Ala Pro Ser Val Leu Ala Glu Val Arg Ala Ala Ala Ala Lys Val
            180                 185                 190

Gly Ala Glu Val His Glu Thr Gly Gly Ala Pro Val Cys Thr Val Ser
        195                 200                 205

Trp Gly Ala Val Ala Ala Ser Ala Leu Pro Leu Ala Gly Ala Tyr Gln
    210                 215                 220

Val Gln Asn Ala Gly Val Ala Leu Ala Leu Leu Asp His Leu Gln Gln
225                 230                 235                 240

Leu Gly Glu Ile Ser Val Ser His Ala Ala Leu Glu Arg Gly Leu Lys
                245                 250                 255

Ala Val Glu Trp Pro Gly Arg Leu Gln Gln Val Glu Tyr Asp Leu Gly
            260                 265                 270

Gly Val His Val Pro Leu Leu Phe Asp Gly Ala His Asn Pro Cys Ala
        275                 280                 285

Ala Glu Glu Leu Ala Arg Phe Leu Asn Glu Arg Tyr Arg Gly Pro Gly
    290                 295                 300

Gly Ser Pro Leu Ile Tyr Val Leu Ala Val Thr Cys Gly Lys Glu Ile
305                 310                 315                 320

Asp Ala Leu Leu Ala Pro Leu Leu Lys Pro His Asp Arg Val Phe Ala
                325                 330                 335

Thr Ser Phe Gly Ala Val Glu Ser Met Pro Trp Val Ala Ala Met Ala
            340                 345                 350

Ser Glu Asp Val Ala Ala Ala Ala Arg Arg Tyr Thr Ala His Val Ser
        355                 360                 365

Ala Val Ala Asp Pro Leu Asp Ala Leu Arg Ala Ala Ala Ala Ala Arg
    370                 375                 380

Gly Asp Ala Asn Leu Val Val Cys Gly Ser Leu Tyr Leu Val Gly Glu
385                 390                 395                 400

Leu Leu Arg Arg Glu His
                405

<210>  24
<211>  1399
<212>  DNA
<213>  Lactobacillus reuteri

<400>  24
ttttactagt atgagaacat acgaacaaat taatgcagga tttaatcgcc agatgctggg      60

cggccagaga gacagagtca agttccttag acgcatcctt acgagacttg gaaaccctga     120

tcagcgcttt aaaattattc atatcgcggg aacgaacggc aaaggatcaa caggcactat     180

gttagaacag ggccttcaga atgcgggata ccgcgtcggc tactttagct ctcctgcgct     240

ggttgatgat cgcgaacaaa ttaaagtcaa tgatcacctt atcagcaaga aagattttgc     300

gatgacctat cagaaaatta cggagcatct gcctgctgac cttctgcctg atgatattac     360

aatctttgag tggtggacgt taatcatgct tcaatacttt gcggatcaaa aggttgactg     420

ggcggtgatt gaatgtggtc ttggcggcca agacgatgcg acaaacatca tctcagcgcc     480

gttcatttca gtcattaccc atatcgctct tgaccacacc cgtatcctgg gccctacaat     540

tgcgaagatt gcgcaagcta aggcaggcat tataaagaca gggactaaac aggttttcct     600

ggcaccacat caagagaagg atgcgttaac aatcattcgc gaaaaagcgc aacagcaaaa     660

ggtcggactg acgcaggcag atgcacagag cattgtggac ggaaaagcta ttttaaaagt     720

gaatcacaag atttacaagg tcccttttaa tctgctgggc acatttcagt cagaaaacct     780

gggaacggtt gttagcgtct ttaactttct gtatcagcgc cgtcttgtca cgtcatggca     840

accgttactt agcacactgg caacagttaa aattgcagga agaatgcaaa aaatcgcgga     900

tcatcctccg atcattcttg atggcgcaca taatccggat gctgcaaagc agcttacaaa     960

gacaattagc aaactcccac ataataaagt cataatggtg ttaggcttcc ttgctgacaa    1020

aaacatttca cagatggtca agatttacca acagatggcg gatgaaatta tcattacaac    1080

gcctgaccat cctacaagag cgctggacgc ctcagccctt aaatcagtct taccgcaagc    1140

aattattgcg aataatcctc gtcagggact ggttgttgct aagaaaattg cagagccgaa    1200

cgatcttatc atcgtcacgg gcagcttcta cacaatcaag gatattgagg caaatttaga    1260

tgagaaataa gcagaggctg tgatcagtct ctgctttttt ttctgcgttc tatttctttt    1320

tcacgttcac ggatgacgtc agtccgatcc cgcaaacggt gtttgtcgat aagaaatatg    1380

aattcgcgtg cgcattgga                                                 1399

<210>  25
<211>  1360
<212>  DNA

<213>  Ashbya gossypii

<400>  25
ttttactagt atggagttag gcttaggccg catcacacaa gtgctgagac aattacatag      60

ccctcatgaa agaatgcgtg tcttacatgt tgcaggaaca aatggcaaag gaagcgtctg     120

tgcgtattta gcggctgttt taagagcggg cggagaaaga gttggcagat ttacaagccc     180

tcacttagtt catccgcgcg atgctatcac agtcgacggc gaagttattg gagcggcgac     240

atatgctgca cttaaagctg aagtcgttgc ggcaggcaca tgcacggagt ttgaagcaca     300

aacggcggtt gcgcttacgc attttgcaag acttgaatgc acatggtgtg tcgtcgaagt     360

gggcgtcggc ggcagattag acgctacaaa tgtcgtccct ggcggacgca aactgtgtgc     420

aattacaaag gttggattag atcatcaggc gttacttggc ggaacactgg ctgttgttgc     480

aagagagaag gccggcattg tggttccggg agtgcgcttt gtcgctgtcg acggcacgaa     540

cgcaccttca gttctggcgg aggttcgggc ggctgcagcg aaagttggcg cagaggtcca     600

tgagacagga ggcgcgccgg tttgcacagt cagctggggt gcggttgctg caagcgcact     660

tccgttagcg ggagcttacc aggtacaaaa cgcgggcgtt gcacttgcac tgcttgatca     720

tcttcaacaa ctgggagaga tctcagtcag ccatgcagca ctggaaagag gactgaaagc     780

agtcgaatgg cctggcagac ttcaacaagt tgagtatgac cttggaggcg tccatgtccc     840

gctgttattt gacggagcac acaatccgtg tgcagcggaa gagcttgcaa gattcttaaa     900

cgagagatac cgcggaccgg gaggatctcc gctgatctat gtgctggctg tcacgtgtgg     960

caaagagatc gacgcacttc ttgcacctct tctgaaaccg cacgatagag tcttcgcaac    1020

cagctttggc gcggttgagt ctatgccgtg ggtcgcagcg atggcaagcg aggatgtcgc    1080

agcggcggcg agacgctaca cagcccacgt ttcagcggtt gcggacccgc tggacgcgtt    1140

acgcgccgca gcggcagcac gcggcgatgc taatctggtc gtctgcggat cattatatct    1200

tgtcggcgaa cttctgcgcc gcgaacatta agcagaggct gtgatcagtc tctgcttttt    1260

tttctgcgtt ctatttcttt ttcacgttca cggatgacgt cagtccgatc ccgcaaacgg    1320

tgtttgtcga taagaaatat gaattcgcgt gcgcattgga                          1360


<210>  26
<211>  20
<212>  DNA
<213>  Artificial Sequence

<220>
<223>  Primer

<400>  26
gcagcgaaat cagcatcacc                                                  20


<210>  27

<211>  20
<212>  DNA
<213>  Artificial Sequence

<220>
<223>  Primer

<400>  27
gactcgttag ccaggtcgtc                                                   20


<210>  28
<211>  20
<212>  DNA
<213>  Artificial Sequence

<220>
<223>  Primer

<400>  28
tcgataaaag aagccccgcc                                                   20


<210>  29
<211>  20
<212>  DNA
<213>  Artificial Sequence

<220>
<223>  Primer

<400>  29
ggtttccatg agggtcggtc                                                   20


<210>  30
<211>  20
<212>  DNA
<213>  Artificial Sequence

<220>
<223>  Primer

<400>  30
gctacctggc gcaaaaagaa                                                   20


<210>  31
<211>  20
<212>  DNA
<213>  Artificial Sequence

<220>
<223>  Primer

<400>  31
cggtagtcat tgctggcgaa                                                   20


<210>  32
<211>  19
<212>  DNA
<213>  Artificial Sequence

<220>
<223>  Primer

<400>  32
acgacctggc taacgagtc                                                    19


<210>  33
<211>  20
<212>  DNA
<213>  Artificial Sequence

<220>
<223>  Primer

<400>  33
ggcggggctt cttttatcga                                                   20


<210>  34
<211>  20
<212>  DNA
<213>  Artificial Sequence

<220>
<223>  Primer

<400>  34
gaccgaccct catggaaacc                                                   20


<210>  35
<211>  20
<212>  DNA
<213>  Artificial Sequence

<220>
<223>  Primer

<400>  35
ttctttttgc gccaggtagc                                                   20


<210>  36
<211>  20
<212>  DNA
<213>  Artificial Sequence

<220>
<223>  Primer

<400>  36
ggagaatccc aacgaagcca                                                   20


<210>  37
<211>  152
<212>  DNA
<213>  Artificial Sequence

<220>
<223>  Fragment containing the Pveg promotor

<400>  37
gcatcactat ctgcagtaaa atcggaattc aattttgtca aaataatttt attgacaacg      60

tcttattaac gttgatataa tttaaatttt atttgacaaa aatgggctcg tgttgtacaa     120

taaatgttac tagagtaaag gaggaaacta gt                                   152


<210>  38
<211>  228
<212>  DNA
<213>  Artificial Sequence

<220>
<223>  p15

<400>  38
gtgcgcatga tcgtatggtt cactgtccac caaccaaaac tgtgctcagt accgccaata      60

tttctccctt ggggggtaca aagaggtgtc cctagaagag atccacgctg tgtaaaaatt     120

ttacaaaaag gtattgactt tccctacagg gtgtgtaata atttaattac aggcgggggc     180

aaccccgctc agtacctaga gcgtaaaaga ggggagggaa acactagt                  228


<210>  39
<211>  25
<212>  DNA
<213>  Artificial Sequence

<220>
<223>  Primer

<400>  39
atctacattc cctttagtaa cgtgt                                            25


<210>  40
<211>  32
<212>  DNA
<213>  Artificial Sequence

<220>
<223>  Primer

<400>  40
aaatctagaa attaagaagg agggattcgt ca                                    32


<210>  41
<211>  34
<212>  DNA
<213>  Artificial Sequence

<220>
<223>  Primer

<400>  41
aaaggatcca tctacattcc ctttagtaac gtgt                                  34

<210>  42
<211>  20
<212>  DNA
<213>  Artificial Sequence

<220>
<223>  Primer

<400>  42
tcccggcaac agcttaatca                                                   20


<210>  43
<211>  20
<212>  DNA
<213>  Artificial Sequence

<220>
<223>  Primer

<400>  43
ggagccgatt ctctgcgtta                                                   20


<210>  44
<211>  20
<212>  DNA
<213>  Artificial Sequence

<220>
<223>  Primer

<400>  44
aaaatgctcc ctgcggctat                                                   20


<210>  45
<211>  21
<212>  DNA
<213>  Artificial Sequence

<220>
<223>  Primer

<400>  45
caatgagagg ggttgctatg a                                                 21


<210>  46
<211>  20
<212>  DNA
<213>  Artificial Sequence

<220>
<223>  Primer

<400>  46
tcgaacggtc aagcacgtta                                                   20


<210>  47
<211>  34
<212>  DNA

<213>  Artificial Sequence

<220>
<223>  Primer

<400>  47
tttgctagca tgataattgg aatatgggca gaag                                34


<210>  48
<211>  34
<212>  DNA
<213>  Artificial Sequence

<220>
<223>  Primer

<400>  48
tttgcggccg ccctcctcgt catttcttca aaag                                34


<210>  49
<211>  6975
<212>  DNA
<213>  Lactococcus lactis

<400>  49
atgataattg gaatatgggc agaagatgaa gcaggtctta tcggtgaagc tgataaaatg      60

ccttggtctt tacctgctga acaacaacat tttaaagaaa caaccatgaa tcaagtgatt     120

ttgatgggac gaaaaacgtt tgaaggcatg aataaacgtg tattgccagg gagaataagt     180

attattttaa ctcgcgatga aacttatcaa tcagataatg aaaaagtgct catcatgcac     240

agccctaagg aagttctaga ttggtaccat aagcaaaata aagacttatt tatcacagga     300

ggagctgaaa ttttagccct ttttgaatct gaacttgaat tgctctatcg aacagttgtt     360

catgaaaaat ttaaaggaga tacttatttt ccaagtacat ttgactttgg aagatttaag     420

ctagtctctg aaaaatttca cgataaagat gagcggaatt cttatacttt tacaattaaa     480

aaatatgaaa aagtgaaaca accatgacaa aatcaatttt tgggcttttc acagctctcc     540

tttgttggat tagcattgtc atcgctattc aatgctttag aaaaaaacgt tggggtctgg     600

gagtattgtt tttactcaat gcttttacga acctcgtaaa tacaattcac gctttttctg     660

gaactttatt ttaaaaaata aaaaaagtgc cttttaagta agccaataac acttactttt     720

tatgttagtg aaatcaggaa taaaataact atgtcaaata cacaaaatcc aaatatacat     780

tgttctttct gtggaaagag tcaagatgat gtaaaaaaat tgattgccgg ttcagacgtt     840

tatatttgta atgaatgtat tgaactttca actcgaatct tagaagaaga attaagagaa     900

gaacaagatt cagaaatgct tgaagttaaa acacctaaag aaatgtttga ccatttaaat     960

gaatatgtga taggtcaaga aaaagcaaaa cgtgcacttg cagttgccgt ttataatcat    1020

tacaaacgaa ttaattttgc agcaagtaaa attgctgaag atattgaact acaaaaatca    1080

aatattctat taatcggacc taccggttct ggtaagactt ttctcgctca aactttagcg 1140

aaatcactca acgttccatt tgcgattgca gatgcgacaa gtttaactga agctggttat 1200

gttggagaag acgttgaaaa tattctctta aaacttttac aagcgagtga tttcaatatt 1260

gaacgtgctg aacgtggaat tatctatatc gatgaaattg ataaaattgc taaaaaatct 1320

gaaaatgtat caattactcg tgacgtttcc ggggaaggtg ttcaacaagc ccttttgaaa 1380

attattgaag gaacggtagc tagtgttcca ccacaaggtg gacgtaaaca tcctaatcaa 1440

gaaatgattc aaattgatac caaaaatatc ttatttattg ttggtggagc ttttgacggg 1500

attgaagaaa ttgtcaaaca acgtttaggt gaaaaaatta ttggttttgg tgccaataat 1560

aaaaaattaa atgacgatga ttcttatatg caagaaatta ttgccgagga cattcaaaaa 1620

ttcggattaa ttcctgaatt tattggtcgt ctgccaattg ttgctgcttt ggaacgtttg 1680

accgaagagg atttgattca aattttgaca gaacctaaaa atgctttgat taaacaatat 1740

aaacaactcc ttttatttga taatgttgaa cttgaatttg aagatgaagc cctcatggca 1800

attgctagaa aagcaattga gcgcaaaaca ggagcgcgtg gacttcgttc aattattgaa 1860

gaagtaatga tggatatcat gtttgaagtt ccaagtcatg aagaaattac aaaagttatt 1920

attaatgaag cagttgttga cggaaaagct gagccacaaa tgattcgaga ggccaagaaa 1980

aaatgaccat aaatacaaat aatctgacaa taacaatttc agcagcatca aaaaaacaat 2040

atccagaaaa tgattggcca gaaattgcct tagctgggcg ttcaaatgtc ggtaaatcaa 2100

gttttattaa tactttactt aatcgtaaaa actttgccag aacttctggt caacctggta 2160

aaacacagtt gctcaatttt tataatattg atgatcaact tcatttcgtt gacgtacctg 2220

gttacggcta cgctcgtgtt tctaaaaagg aacgcgaaaa atggggtaaa atgattgagg 2280

aatatttgac aacaagagaa aatttaaaag cagttgtcag cttagttgat attcgtcatg 2340

aaccctcaga agatgatttg atgatgtatg agtttttgaa atactaccat attccagtga 2400

ttttagttgc gaccaaagcc gataaagttc cacgtggtaa gtggaataaa catgaatcta 2460

ttatcaaaaa agcaatgaaa tttgatagta cagatgattt tattatcttt tcttctactg 2520

ataagacagg atttgaagaa gcttgggaag cgattttaag atatctctga aaatagtgct 2580

atgaagagat tcatagcctt ttctacactt aaaaagagga aatatgtaca aaataaaact 2640

taataatata aaatttaggg cacatattgg tgttctgcca gaagaaaaag ttctcggaca 2700

aaatctcgaa attgatttaa tcgtggaaac aaattttgat ttttcaggaa aagacgaatt 2760

agatgaaact ttgtcttatg ttgatttcta tgaggcaaca aaagcagttg tagaatcttc 2820

aaaagctgat ttaattgaac atgttgcctt tgaaattatt caagcagtaa aggctacttc 2880

agagcgtata tcaacggttg aagtccatct tagaaaatta gccgtaccga ttgaaggaat 2940

ttttgattca gctgaaattg agatgagagg ctaaagctgg tttttaagat aaatatttta 3000

aagagataga agagaaacaa aatcataaaa gattatgtct aaatggagga cttatgcaaa 3060

caacttactt aagcatggga agtaatattg gtgaccgtca gtattattta catgaagcca 3120

ttcgtttatt gggaaaacac cctaaaatta tgattgaaaa agtatcaaat ttttatgaaa 3180

gtactccagt cggcggcgtc aaacaagatg attttactaa tttggcatta aaggtggcaa 3240

cgctacttga acctttggaa ttattatctt ttattcatga agttgagtta tctttgaacc 3300

gtgagcgaaa aattcattgg gggccaagaa caattgatat tgatattatt ttctatgacg 3360

acttagaaat gcaagtagaa aacttggtta ttccacataa agaagctttt aatcgtcttt 3420

ttgtcttgaa acctattttt gaacttattg ataaagactt taaatattat gcgtcaatag 3480

aaaaagcaat agccgaactt tcagtaagtg aacaagagct ccatgtaata aaagaagaaa 3540

aaacaccgag aaatcgtatt gaagatgccg ttaaagagat tctctttgca gtaggtgaaa 3600

atccaaatcg agaaggatta cttgaaactc cagcaagagt agctaaaatg tatgaagaaa 3660

ttctttcgtc acaacgctta agcaagttta atgagtataa actttttgaa attgattctt 3720

ctaaaacgga ttcaatcgtg ttgattaaag atattccttt ttattcaatg tgtgagcatc 3780

atatgttacc attttttggg aaagctcatg ttgcatatat tccagctgat ggaaaaatta 3840

ttggcttgtc aaaaattccc cgtttagttg attatgtttc gcgcaaactc tcggttcaag 3900

aaaatatcac tcatgatatt ggagatattt tgactgatat tttgaatcct aaaggagtgg 3960

cagttcttgt tgaaggacgt catatgtgcg ttgaaatgcg tggagtaaaa aaagtaaatt 4020

ctattactaa aacttcttat tttttaggtg aatttaaaga aaataatgaa aaaagaatgg 4080

aatttttaga aagtctttta tgaaaatctt agaacttaat caagaatctt tttctcttaa 4140

aaatattatc ctaaaatttg atgagttaaa tcacaatgaa atgatttctc ttcaaaaaaa 4200

actttatcga aatggtagtt tgacaagact ggctccagac tccttgttag tagttttaac 4260

aattgatgac ttagcaaaat tgattaatct ttttgaaaat gatgaagata aaaaaatgct 4320

tgaagtgatt tataagcgtc atcaaatcat ttggtcaggt aaaaatttca attttgattt 4380

aactagaaag tcaattgtct attcaatcgt caatgttaca ccagactctt tttatgatgg 4440

aaatccagat aatttaaacc tctctcatat tttaaaaaga gtagaagctg atttagaaaa 4500

tggagcttct gttcttgagc tgggagggaa atcatcgaaa ccaggatatg acgatattag 4560

cccagaagag gaatggaaca gactgaaaga acctattctt gagttgaaaa aaaactttcc 4620

taaagcgatt tttgctgtcg atacggatga agcttatgtc atggaacgag ttttagacgc 4680

tggggttgat attattaacg atattgatgg ttttgataca aatgataaat taaaagtggt 4740

agaaaagtat caaccggctt tagttgctat gaataatggg cgagctggtt ttagttatgc 4800

tgataatgtt tatgaagaac ttccattatt ttttgaaaat aaaaaagaag agttacttca 4860

acttggttta aaagctgagc aaatcgttat tgatcctgga gttggttttt ttaatggaga 4920

ttcaggttca gatagtcttg agcgggttaa agcaactgaa attttaagca gaataggttt 4980

acctcttatg attgcaatct ctcgtaagtc atttatggga aaactcttca atgcccaagg 5040

agatgagcgg cttttttcaa gccttgtcct agaagcgcaa atggttgctg atgggggacg 5100

gattttgcgt gttcatgatg ttaaggagac taaacgttta ctcgatgcaa ttgaaattta 5160

taaggaattt taaaaatgaa tgaagaccta attgctgaaa ttcaagcttt atctgctatt 5220

ggaagtgaag aaaaattttc cgagattatt cgattattga aaaattcgac tttagagctt 5280

cgggggaaaa agaatccaga tttacaattg tcagcaagtg cattagtttt taaaaaacat 5340

aaactatttt ttattgaaca cccttatcaa aaggagcttt tgcttccagc aggtcatgtt 5400

gaactaggag aaaagccatt ggaaactgcg attcgtgagt tccatgaaga aacaggtttt 5460

tcagcgtcag aatcaggcaa gttggtagat gttaacttga ttaatattcc ttacaacaaa 5520

attaagaatg agaaagaaca tcaacacatt gattttcgtt ttctattgga actaaaagaa 5580

aaagaagcag gccttgctga attgcctttt ttccttcttg atagaactga agctcctgat 5640

gaatttaaaa aatattatca atacaaaaga taaagtagaa aaggtcacaa aatgtctata 5700

gaagaagcat tggaatggat acattcacgt ttaaaattta atattcgccc aggcctaagt 5760

cgtgtttcgg cccttttaga attgcttggt catccagaag agtctttgtc aatgattcac 5820

gttgctggaa caaatggaaa aggctccaca gtcgctttca cacgctcaat ctttatgcag 5880

gcaggtctga aggttgcttc tttcacaagt cctttcatca ccacttttgg tgagcggatg 5940

tcgattaatg cactcccgat tgctgatgat aaattaattt attatgtaga aatgatccaa 6000

ccacttgttg ctgaacttga taaagatgct gaactgactg gaattaccga atttgaaatt 6060

atcacggcaa tggcttttaa atatttctct gatgagcagg ttgatttagc ggttattgaa 6120

gttggtttag gtggacttct tgattcaaca aatgtgatta aacctgttgt ttctggaatt 6180

acaacaattg gtttagatca tattgatatt cttggttcga ccattgaaga aatcgcagct 6240

caaaaggctg gaattattaa accaggaatt ccagtagttg ttggaaatat tgaattaaaa 6300

gcacttcggg ttatatggga agtggctaga aaaaatacag cgcgtgttta tcaatttcca 6360

tatgattatc gtacggaagt ggaagaacac gaacatttta atttcttttc tggtcaagaa 6420

gcaatattgg atattgaaaa atctttagtt ggcttacatc aaatagaaaa tgctggtatg 6480

gctattgaac tttctctggt ttatgcaagt aaggttggga ttgaattgac tgaggatgtg 6540

attcgctctg gaattcgtga ggctttttgg ccagctcgta tggaaaaatt gggtgaaaaa 6600

ccactcattt tactggatgg tgctcataat gttcatgcga tgaatcgttt gcttgaaaat 6660

cttagctctg agtttccaga taaaaaaatt acaatcattt tttcagccat taccacaaaa 6720

gatattagtc aaatgataaa aatgcttcaa actgtgaaaa attcgcatct gattttgaca 6780

acttttgatt atccaaaagc tttgaatttg ggagattttc aaagattgga agaagaaggg 6840

gttgaattgg ctccaagttg ggaattagct ttagttcgtg cgcaaaaaaa tttagctgaa 6900

gatgatttgt tattagttac aggctctctc tatttctcat ctcaagttcg tgagtttttg 6960

aaaaaagaga agtaa 6975

<210> 50
<211> 2463
<212> DNA
<213> Artificial Sequence

<220>
<223> Synthetic folate biosynthesis gene fol1-AG

<400> 50
atgcagtccc ttggattcaa gtgtttgctg tctcgcagga gcctgagcag gatatcaatc 60

tgtacaagag gaatgagtag tgctaacggt ggacgaagta atgatactgt gcatatacag 120

agacaggcac tgaaagttgt tgctgggctt gacggatggg gtcaattgca ggcgcaggat 180

gtgaaattga ccatgaatat gaacacagat tttcgtgctt cctcgcagac ggatgatctg 240

aagtactcct tgaattatgc ggtgatttca cgtggggtgc ataggttcgt tgagggctgt 300

ggacggtacc gctctcttgg tcacttggcc agggaggtaa agaagttttc catgaatgag 360

tatccgggta tccaaactat agaggtgggt gcggaggcgg acgcggccca tttgcgatgc 420

ggaagtctgg gcgtcgtggt gaacagcgat gggcatcgtc ctgatgagat tttgctttct 480

ggaatgaagc ttctgacact aataggggtg ttcacttttg aacggcgtcg gaagcagtac 540

gttgacttga agctgtcatt tccgtggccg aaggaggctg gtgaatttcc ggattgccag 600

gaattattgg acgatgttgt gagctatgta gagagagcga attttaaaac ggcagagtct 660

cttgctgaga gtgtagctca cgttgttacc ttgagagagt attttcagct gcatcgtggg 720

ttaccggtaa aagtcaaggt aattaagctt aatgccatta ctgagactga gggagttggt 780

gtgagctgtg taagaagtgc ggatgaattt acggggaaac cgcccttctg ggaagatatt 840

ccaaacgatc gagcagacgt gtttaacctt cctgtattcc agcagccaca tgcatctgtc 900

agtgagtgga atcgtgtgtt tctggcgttt ggatctaata taggggatag gtttgctcac 960

attgagcgaa gcttacgtct acttgcggaa gatcctaaag ttaaactact tcgctcgtcg 1020

tctctgttcg agagtgaacc aatgtacttt aaggagcagt ccccgtttat gaatggcgtt 1080

gtagaagtgc agacacggta tagcccgcac gagttactag agctatgcaa aaggatagaa 1140

tatgaacatt taaaacgtgt caaagagttt gataacggcc ctcgcagcat tgatttagat 1200

attttattgt accaaaatgc aaactttgag catgtggtac tgaactccga ggatttagtt 1260

attcctcatc caaggatgtt ggagagatcg tttgttttag agcctctctg tgaattgttg 1320

gctttccatg aagtgcaccc catttcggct gaatctgtcc aaagtcacct aaaagaattg 1380

taccgtaagg ggaataagga agacattctt gttaaacttg tacctttgcc gggtattccg 1440

tcaaatatac ctacaacgcg atttctgaag tttagacggg agtatgagga ggatcaatcg 1500

acaagcgaat tggttcttag gaccaagtca aatacatatg tcatgggcat cgtgaatgtg 1560

acacctgatt ctttttctga tggatctcct atgtggaatg atgttaatca tttcctctta 1620

aaagtacaaa ggatgatcct tgacgttttg aagttacatg aaaacgttat cattgatatt 1680

ggaggctgtt cgactaggcc tggtagtcag caaccatcag tggaagaaga acttagtcgt 1740

actattcccc taataacagc gatcaggggt tgcagagatt tttcgcaaga gaatgtgatc 1800

atatctatag acacttacag aagtgctgtt gctgaaaagg ccataacagc aggggctgat 1860

attgtgaacg atatttcagg aggtagtttt gatacaaata tgtttaaggt tatcagcgcg 1920

tatccgaatg ttggttatgt gctatcacac ataaggggag atatgactac catgacgagc 1980

ctgaataagt atgatgatac agttggtttg gatggcgttg aagaattcat ttacggtaag 2040

aaacagcact cagaacggac taaggtgatc cggaacattt gtagggaact tgcggagcga 2100

taccagcttg cccttgctag cggaattaag cgctggcaga ttattttgga tccgggtatt 2160

ggttttgcga agaatgctaa acagaactta gatatcatca agcatacccc gtcaattaag 2220

ggttatagtt gtgtgacaca tggacaattt gtaaattttg ccaaccttcc tgtgttgctt 2280

gggccttcca ggaagaactt tattgggact ataattcaag aggcacaggt cgagcgaagg 2340

gactttgcaa cggggactat tgtaggctcc tgtgttggtt atgatgcgga tatcatcagg 2400

gtacatgatg taactaactg tagcaaaagt gctaggttag cggatgagct ttataggaaa 2460

tag 2463

<210> 51
<211> 732
<212> DNA
<213> Artificial Sequence

<220>
<223> Synthetic folate biosynthesis gene fol2-AG

<400> 51
atgtgtcagg ggggcagtaa aggactagtt aggcaggaca cgcccctaaa gacgaggcct 60

gtctcgccat atacgctcca ggccccagtt gaggcggacg gactgtcctg gccgagtgca 120

ggggcacgtg tgcgggtcga ggagggcacg gaggaagagg cggcacgcgc agcccggata 180

gctgatgcag tcaagacgat tttgacggag ctgggcgaag acgtgacgcg ggagggcctg 240

ctggacaccc cgcaacggta cgccaaagcg atgctgtact tcaccaaggg ctaccaagac 300

aatattttga acgatgtgat caataatgct gtgtttgacg aagatcatga cgagatggta 360

attgtgcggg atattgagat ccattcgctg tgcgagcacc acctggtacc cttcttcggg 420

aaggtgcata ttggctacat acctcggagg agagtcctcg ggttgtcgaa gctcgcccgg 480

ctagcggaaa tgtacgcgcg caggctgcag gtgcaggagc ggctgacgaa gcagattgcg 540

atggcattgc aggatatact gcgccctaga ggagtagccg ttgtggtgga ggccacgcat 600

atgtgcatgg tgtcacgggg ggtccagaag tccgggtcct caactgtcac ctcgtgtatg 660

ctgggctgct tcagagacat gcacaagacc cgggaagaat tcttgaacct cttgagaaat 720

agaagtgtat ag 732

<210> 52
<211> 2484
<212> DNA
<213> Artificial Sequence

<220>
<223> DNA fragments FOL1-AG

<400> 52
ttttactagt atgcaatcac tgggctttaa atgtcttctg agcagaagaa gcctgagccg 60

cattagcatc tgcacgagag gaatgagctc agcgaatgga ggaagaagca atgatacagt 120

tcatattcag cgccaggcac ttaaggtcgt tgcgggcctt gatggctggg gacagctgca 180

ggcgcaagac gttaagctga caatgaacat gaacacagac tttcgtgcgt caagccaaac 240

agatgacctt aaatacagcc ttaattacgc tgtgattagc cgtggagtcc accgttttgt 300

cgagggatgc ggaagatacc gtagcctggg acatctggcg agagaggtca aaaagttttc 360

aatgaatgag taccctggca ttcagaccat tgaggtgggt gccgaggcgg acgcggcaca 420

cctgagatgc ggatctttag gcgttgttgt gaatagcgat ggacacagac ctgatgagat 480

cttattgtca ggcatgaaac ttctgacgct gattggagtc tttacattcg agcgtcgcag 540

aaagcaatac gtcgatctga aactgagctt cccgtggcct aaagaagcag gagagttccc 600

ggattgtcag gaacttctgg atgacgttgt gagctacgtc gagagagcga acttcaaaac 660

ggcagagtct ctggcggagt ctgtggcaca cgtggtcaca cttcgcgaat attttcaact 720

tcatcgtggc ttgcctgtca aagtgaaagt cattaagctg aacgcgatca cagaaacgga 780

gggcgtcgga gttagctgtg tcagatctgc cgatgaattt acaggcaagc ctccattttg 840

ggaagacatc ccgaacgata gagcggacgt ctttaattta cctgtgttcc agcaacctca 900

tgcaagcgtt tcagagtgga atagagtgtt tctggcgttt ggctccaaca ttggagatag 960

attcgcgcat atcgagagat ctttacgtct gcttgctgaa gatcctaaag tcaaactgct 1020

tagaagcagc agccttttcg aatctgagcc tatgtatttc aaggagcagt ccccgtttat 1080

gaacggagtc gttgaggtcc aaacgagata ttcaccgcat gaacttttag agttgtgcaa 1140

acgtatcgaa tatgaacacc tgaaacgtgt taaagagttt gataatggcc cgcgttcaat 1200

tgacctggat atcttactgt accagaacgc gaactttgag catgtggtcc ttaattccga 1260

agacctggta attccgcatc ctagaatgct ggaacgcagc ttcgtgctgg agcctttatg 1320

cgagctgctt gcgtttcacg aggttcaccc tatatcagcc gagtcagtgc agagccatct 1380

gaaagaatta tacagaaaag gcaataaaga ggacatttta gtcaagttag tccctctgcc 1440

tggaatccct tctaatattc cgacgacgag atttcttaaa tttagacgcg aatatgaaga 1500

ggaccagtct acatcagaat tagtcctgcg tacgaaaagc aacacatacg ttatgggaat 1560

tgtcaatgtt acgcctgact catttagcga cggctcacct atgtggaacg acgtcaatca 1620

tttccttctg aaggtgcaac gcatgatcct ggatgtcctg aaactgcatg agaatgtcat 1680

tattgatatc ggaggctgct ctacaagacc tggctctcag caaccgagcg ttgaagaaga 1740

gttatcacgc acgattcctc ttattacagc tattcgcggc tgcagagatt tttcacaaga 1800

gaatgttatt atctcaattg acacataccg gtcagcggtc gctgagaaag caattacggc 1860

aggagcggat attgttaatg atatttctgg cggatctttc gatacaaata tgtttaaagt 1920

tatttcagcg tatcctaatg tcggctacgt tctgtcccat atccgtggcg atatgacaac 1980

gatgacgtca ctgaacaaat atgatgacac agtcggctta gatggcgttg aggaatttat 2040

ctatggcaaa aaacaacatt cagaacgtac aaaagtcatc cgtaacatct gtcgcgaact 2100

tgcagaacgc taccagcttg cacttgcttc aggcattaaa cgctggcaaa ttatccttga 2160

tcctggcatt ggcttcgcta aaaatgctaa acaaaacctg gatattatta aacacacgcc 2220

gagcattaaa ggatactcat gtgtgacgca tggacaattt gtgaatttcg cgaatttacc 2280

ggtactgctg ggcccgtctc gcaagaattt catcggcaca attattcagg aggcgcaagt 2340

agaacgcaga gatttcgcaa caggcacgat tgtgggctca tgtgtcggct atgacgctga 2400

tattatccgc gttcacgatg tcacgaattg tagcaagagt gcacgcctgg cggatgaact 2460

gtatcgcaaa taaggatcca tttt 2484

<210> 53
<211> 1090
<212> DNA
<213> Artificial Sequence

<220>
<223> DNA fragments FOL2-AG

<400> 53
tattggatcc tatggttcac tgtccaccaa ccaaaactgt gctcagtacc gccaatattt 60

ctcccttggg gggtacaaag aggtgtccct agaagagatc cacgctgtgt aaaaatttta 120

caaaaaggta ttgactttcc ctacagggtg tgtaataatt taattacagg cgggggcaac 180

cccgctcagt acctagagcg taaaagaggg gagggaaaca ctagtatgtg tcaaggcgga 240

agcaaaggac tggttagaca agacacaccg ctgaaaacaa gacctgtctc accttataca 300

ctgcaagcac ctgtcgaagc agacggatta agctggccga gcgcgggcgc gagagttaga 360

gtggaagagg gaacggagga agaagcagcg cgcgcggcta gaattgcgga tgcagtcaaa 420

acaatattaa cagagctggg cgaagacgtg acaagagaag gtcttctgga cacaccgcag 480

cggtatgcga aagctatgct gtactttacg aagggatacc aagacaacat cctgaacgat 540

gtcattaaca atgcggtttt tgacgaggat catgatgaga tggttatcgt tcgcgacata 600

gagatacaca gcctgtgtga gcatcacctg gtcccatttt tcggcaaggt ccacataggc 660

tacattccga gaagacgtgt cctgggactt tctaaactgg cgcgcttagc tgaaatgtac 720

gcacgcagac tccaggtcca agaacgttta accaaacaga tcgcaatggc actgcaagat 780

atccttcgcc ctagaggcgt ggcagtcgtt gttgaggcta cgcacatgtg catggtctct 840

cgcggagtgc aaaagagcgg atcatcaacg gtaacatcat gtatgctggg atgtttcaga 900

gacatgcaca agacgagaga ggaatttctt aatttactta gaaacagaag cgtttaagca 960

gaggctgtga tcagtctctg cttttttttc tgcgttctat ttctttttca cgttcacgga 1020

tgacgtcagt ccgatcccgc aaacggtgtt tgtcgataag aaatattacg taatatggcc 1080

tcgagtttta 1090

<210> 54
<211> 25
<212> DNA
<213> Artificial Sequence

<220>
<223> Primer

<400> 54
tattggatcc tatggttcac tgtcc 25

<210> 55
<211> 20
<212> DNA
<213> Artificial Sequence

<220>
<223> Primer

<400> 55
gcggtagtgg tgcttacgat 20

<210> 56
<211> 23
<212> DNA
<213> Artificial Sequence

<220>
<223> Primer

<400> 56

tgcagggtct ttattcttca act                                              23

<210>  57
<211>  20
<212>  DNA
<213>  Artificial Sequence

<220>
<223>  Primer

<400>  57
gcggtagtgg tgcttacgat                                                   20

<210>  58
<211>  1221
<212>  DNA
<213>  Ashbya gossypii

<400>  58
atggagctgg gactcgggcg gataacacaa gtgctgcggc agcttcacag cccgcatgaa      60

aggatgcgcg tgctgcacgt ggccggtacg aacggtaagg gctcggtctg tgcgtacctc     120

gcggcagtgc tgcgcgcggg cggagagcga gtgggaaggt tcacgagccc gcacctggtg     180

cacccgcggg atgcgatcac ggtggatggg gaggtgatcg gggcggcgac gtacgcggcc     240

ctgaaggcgg aggtggtcgc ggcggggaca tgcacggagt ttgaggcgca aacggcggtg     300

gcgttgacgc actttgcgcg gttagagtgc acgtggtgtg tggtggaggt gggcgtgggc     360

gggcggctgg acgcaacgaa cgttgtgccg ggaggccgca agctgtgcgc catcaccaag     420

gtaggactag accaccaggc gctgctcggc gggacgctgg cggtggttgc gcgcgagaag     480

gcggggattg tggtgccagg cgtgcggttt gtggcggtgg acggaacgaa cgcgccgagt     540

gtgcttgcgg aggtgcgtgc tgcggcagcg aaggtggggg cggaggtgca cgagacggga     600

ggggcgcctg tgtgtacggt ttcatggggc gcagtggcgg cgagcgcgct gccgctggcg     660

ggtgcgtacc aagtacagaa cgcaggcgtg gcgctagcgc tgctagacca tctgcagcaa     720

ctgggggaga tctcggtgag tcacgcggca ctggagcgcg ggctcaaagc agtggagtgg     780

ccgggacggc tgcagcaggt ggagtacgac ctgggcggcg tccacgtccc actgctcttt     840

gacggggcgc acaacccctg tgctgcagag gagctggctc gctttctcaa tgagaggtac     900

cgcgggcccg gaggctctcc gctcatttac gtactggcgg tgacatgtgg caaagagatc     960

gatgcgcttc tggcgccgct gttaaagccg cacgaccgag tgttcgccac atcgttcgga    1020

gccgtggaga gcatgccgtg ggtagcggct atggcctccg aggacgttgc ggcagcggct    1080

cgtcgctaca cggcgcacgt gtctgcggta gcggacccgc tggacgccct gcgggcggca    1140

gctgccgccc ggggcgacgc aaaccttgtc gtttgcggct cgctttatct tgtgggcgag    1200

ctgctgcgcc gcgagcacta g                                              1221

<210>  59
<211>  20
<212>  DNA
<213>  Artificial Sequence

<220>
<223>  Primer

<400>  59
taggaggcga gagcacaaga                                                   20


<210>  60
<211>  20
<212>  DNA
<213>  Artificial Sequence

<220>
<223>  Primer

<400>  60
gccgagttcc tttgtgatgc                                                   20


<210>  61
<211>  20
<212>  DNA
<213>  Artificial Sequence

<220>
<223>  Primer

<400>  61
gcccgagaac agcggattta                                                   20


<210>  62
<211>  20
<212>  DNA
<213>  Artificial Sequence

<220>
<223>  Primer

<400>  62
cgcaagaaca aacaggcgtt                                                   20


<210>  63
<211>  20
<212>  DNA
<213>  Artificial Sequence

<220>
<223>  Primer

<400>  63
tggcgttatg gttgtcgttg                                                   20


<210>  64
<211>  20

<212>  DNA
<213>  Artificial Sequence

<220>
<223>  Primer

<400>  64
taaacacgcc tctgactgct                                                    20


<210>  65
<211>  20
<212>  DNA
<213>  Artificial Sequence

<220>
<223>  Primer

<400>  65
ggcggagcgc aattatacac                                                    20


<210>  66
<211>  20
<212>  DNA
<213>  Artificial Sequence

<220>
<223>  Primer

<400>  66
caggaaagtg tctgtcgcct                                                    20


<210>  67
<211>  20
<212>  DNA
<213>  Artificial Sequence

<220>
<223>  Primer

<400>  67
gattggccgc ttacacatgg                                                    20


<210>  68
<211>  20
<212>  DNA
<213>  Artificial Sequence

<220>
<223>  Primer

<400>  68
aacgtttggg cttctaccga                                                    20


<210>  69
<211>  20
<212>  DNA
<213>  Artificial Sequence

<220>
<223>  Primer

<400>  69
cagctcgtgt cgtgagatgt                                                    20


<210>  70
<211>  20
<212>  DNA
<213>  Artificial Sequence

<220>
<223>  Primer

<400>  70
agagtgccca actgaatgct                                                    20


<210>  71
<211>  21
<212>  DNA
<213>  Artificial Sequence

<220>
<223>  Primer

<400>  71
gccctgcata aggaatttaa c                                                  21


<210>  72
<211>  23
<212>  DNA
<213>  Artificial Sequence

<220>
<223>  Primer

<400>  72
agcttatgga catacgactg atg                                                23


<210>  73
<211>  20
<212>  DNA
<213>  Artificial Sequence

<220>
<223>  Primer

<400>  73
tgaacggaca gacatggtcg                                                    20


<210>  74
<211>  20
<212>  DNA
<213>  Artificial Sequence

<220>
<223>  Primer

<400>  74
acagcgtgag ccgtatgatt                                                    20


<210>  75
<211>  53
<212>  DNA
<213>  Bacillus subtilis

<400>  75
ctgtatccgc gggtgaaaga gagtgtttta catataaagg aggagaaaca atg               53


<210>  76
<211>  53
<212>  DNA
<213>  Artificial Sequence

<220>
<223>  Regulatory region containing mutation

<400>  76
ctgtatccgc gggtgaaaga gagtgtttta catataaaag aggagaaaca atg               53


<210>  77
<211>  2742
<212>  DNA
<213>  Artificial Sequence

<220>
<223>  BS-FOLOP1-COMB

<400>  77
gcgcgctacg tagggcgcgc tagcggccaa ttttgtcaaa ataattttat tgacaacgtc        60

ttattaacgt tgatataatt taaattttat ttgacaaaaa tgggctcgtg ttgtacaata       120

aatgtatatt aagaggagga gatatatata atgatttatg tcaacggaag atacatggaa       180

gaaaaagacg cagtcctgag cccgtttgat catggctttc tttatggaat tggagttttt       240

gaaacgttcc gcctttatga aggatgtccg tttctgcttg attggcatat cgaacgcctt       300

gaaagagctc tgaaagacct tcaaatcgaa tatacagtta gcaaacatga aattcttgaa       360

atgcttgata aacttcttaa acttaatgat attaaagatg gcaacgcgcg cgtcagactt       420

aatatctcag caggaattag cgataaaggc tttgtcgcgc agacgtatga taaacctacg       480

gttctttgct tcgttaatca acttaaaccg gaaagccttc cgcttcaaaa agaaggcaaa       540

gtccttagca tcagacgcaa tacgcctgaa ggatcattta gactgaaatc tcatcactac       600

cttaataata tgtatgcgaa acgcgaaatt ggcaatgatc cgcgcgtcga aggaatcttt       660

ctgacggagg atggcgcagt tgcagaagga atcatttcta acgttttttg gcgcaaaggc       720

cgctgcattt atacaccgtc acttgataca ggcatcctgg atggagtcac acgcagattt       780

atcattgaaa atgcaaaaga tattggcctt gaacttaaaa caggacgcta tgaacttgaa       840

gcgctgctta cggcggatga agcgtggatg acgaactcag tccttgaaat tattccgttt       900

acaaaaatcg aagaagtcaa ctatggctca cagagcggag aagcaacgtc agcgcttcaa 960

cttctttaca aaaaagaaat aaagaacatg attcatgaaa agggaggaag agcatggcgc 1020

agcacacaat agatcaaaca caagtcattc atacgaaacc gagcgcgctt tcatataaag 1080

aaaaaacact ggtcatgggc attcttaacg ttacacctga ttcttttagc gatggtggaa 1140

aatatgacag cttggacaag gcgcttctgc atgccaaaga aatgatcgac gacggcgcgc 1200

acattattga cataggaggc gagagcacaa gaccgggagc tgaatgcgtc agcgaagacg 1260

aagaaatgtc tcgggtcatt ccggtcattg aacgcatcac aaaggaactc ggcgtcccga 1320

tttcagtgga tacatataaa gcatctgtgg cagacgaagc agtcaaagcg ggcgcatcta 1380

ttatcaatga catttgggga gcgaaacatg atccgaagat ggcaagcgtc gcagcggaac 1440

ataacgttcc aattgtcctg atgcacaatc ggccagaacg gaattataac gaccttcttc 1500

cggatatgct gagcgacctt atggaatcag tcaaaattgc ggttgaggcg ggcgtggatg 1560

agaaaaatat tattttagat ccgggcatcg gcttcgcgaa gacataccat gataatcttg 1620

cagtgatgaa taagttagag atcttcagcg gacttggcta tcctgtcctg ctggctacat 1680

ctcgtaaaag atttatcgga agagttcttg atttaccgcc tgaagagaga gcagagggca 1740

caggagcgac agtctgcttg ggcattcaga aaggatgcga catagtgcgt gttcatgatg 1800

tcaagcaaat tgccagaatg gcgaaaatga tggacgcgat gctgaataag ggaggggtgc 1860

accatggata aagtttatgt ggaaggaatg gaattttatg gctatcatgg cgtcttcaca 1920

gaagagaaca aattgggaca acgcttcaaa gtagatctga cagcagaact ggatttatca 1980

aaagcaggac aaacagacga ccttgaacag acaattaatt atgcagagct ttaccatgtc 2040

tgtaaagaca ttgtcgaagg cgagccggtc aaattggtag agacccttgc tgagcggata 2100

gctggcacag ttttaggtaa atttcagccg gttcaacaat gtacggtgaa agttattaaa 2160

ccagatccgc cgattcctgg ccactataaa tcagtagcaa ttgaaattac gagaaaaaag 2220

tcatgaacaa cattgcgtac attgcgcttg gctctaatat tggagataga gaaacgtatc 2280

tgcgccaggc cgttgcgtta ctgcatcaac atgctgcggt cacagttaca aaagtcagct 2340

caatttatga aacagatccg gtcggctatg aagaccaagc ccagttttta aatatggcgg 2400

ttgaaattaa aacaagcctg aatccgtttg aacttctgga actgacacag caaatcgaaa 2460

acgaactggg ccgcacacgc gaagttagat ggggcccgag aacagcggat ttagacattc 2520

tgctgtttaa cagagaaaac attgaaacag agcagttaat tgtcccgcat cctcgcatgt 2580

atgaacgcct gtttgttctt gcgccgcttg cggaaatttg ccagcaggtc gagaaagaag 2640

cgacaagcgc ggaaacggat caagaaggag ttagagtttg gaaacaaaaa tcaggcgttg 2700

atgaatttgt acatagcgaa agctaagaat tcaattgttg cg 2742

<210>  78
<211>  1239
<212>  DNA
<213>  Artificial Sequence

<220>
<223>  BS-FOLOP2-COMB

<400>  78
gaattcaatt ttgtcaaaat aattttattg acaacgtctt attaacgttg atataattta      60

aatttatatt gacaaaaatg ggctcgtgtt gtacaataaa tgttactaga gtaaaggagg     120

aaactagtat gaaagaagtc aataaagaac aaattgaaca ggcagtgaga cagattcttg     180

aagcaattgg agaagatccg aacagagagg gcttacttga tacaccgaaa agagttgcta     240

aaatgtatgc ggaagtcttt tcaggcttaa atgaagatcc gaaagagcat tttcagacaa     300

ttttcggaga aaaccatgaa gagctggtcc ttgtgaaaga tattgcgttt cactcaatgt     360

gcgaacatca cctggtgccg ttttacggca aggcacacgt tgcgtatatt cctagaggcg     420

gaaaagttac aggcttgtca aaattagcac gcgcagttga agctgttgca aaaagaccgc     480

aattacagga acgcattaca tctacaattg cggaatcaat tgtcgagaca ttagaccctc     540

atggcgttat ggttgtcgtt gaagctgaac acatgtgcat gacaatgcgc ggcgtcagaa     600

aacctggcgc aaaaacagtc acatcagcag tcagaggcgt gtttaaagat gatgcggcag     660

ctcgtgcgga agtcctggaa catattaaac gccaggacta ataaagatag agggtgctat     720

aaatgatttc atttattttc gcaatggacg cgaatagact gataggcaaa gacaatgatc     780

tgccgtggca tttaccgaat gacctggctt attttaaaaa aattacaagc ggccatagca     840

tcattatggg acgtaaaaca tttgagtcaa ttggcagacc tcttccgaac agaaaaaaca     900

ttgttgtcac atctgcgccg gattcagaat ttcagggctg cacagtcgtc tcaagcctta     960

aagacgttct tgatatttgc agcggaccgg aagagtgttt tgtcattggc ggagcgcaat    1020

tatacacaga tctttttccg tacgcggata gactgtatat gacaaaaatc caccatgaat    1080

ttgaaggcga cagacacttt cctgaatttg acgagagcaa ctggaaactc gtgtctagcg    1140

aacagggcac gaaggatgag aaaaatccgt atgactatga atttcttatg tatgaaaaga    1200

aaaacagcag caaagcggga ggcttttaac tcgagtttt                           1239

<210>  79
<211>  415
<212>  PRT
<213>  Bacillus subtilis

<400>  79

Met Lys His Leu Pro Ala Gln Asp Glu Gln Val Phe Asn Ala Ile Lys
1               5                   10                  15

Asn Glu Arg Glu Arg Gln Gln Thr Lys Ile Glu Leu Ile Ala Ser Glu
            20                  25                  30

Asn Phe Val Ser Glu Ala Val Met Glu Ala Gln Gly Ser Val Leu Thr
        35                  40                  45

Asn Lys Tyr Ala Glu Gly Tyr Pro Gly Lys Arg Tyr Tyr Gly Gly Cys
    50                  55                  60

Glu His Val Asp Val Val Glu Asp Ile Ala Arg Asp Arg Ala Lys Glu
65                  70                  75                  80

Ile Phe Gly Ala Glu His Val Asn Val Gln Pro His Ser Gly Ala Gln
                85                  90                  95

Ala Asn Met Ala Val Tyr Phe Thr Ile Leu Glu Gln Gly Asp Thr Val
            100                 105                 110

Leu Gly Met Asn Leu Ser His Gly Gly His Leu Thr His Gly Ser Pro
        115                 120                 125

Val Asn Phe Ser Gly Val Gln Tyr Asn Phe Val Glu Tyr Gly Val Asp
    130                 135                 140

Lys Glu Thr Gln Tyr Ile Asp Tyr Asp Asp Val Arg Glu Lys Ala Leu
145                 150                 155                 160

Ala His Lys Pro Lys Leu Ile Val Ala Gly Ala Ser Ala Tyr Pro Arg
                165                 170                 175

Thr Ile Asp Phe Lys Lys Phe Arg Glu Ile Ala Asp Glu Val Gly Ala
            180                 185                 190

Tyr Phe Met Val Asp Met Ala His Ile Ala Gly Leu Val Ala Ala Gly
        195                 200                 205

Leu His Pro Asn Pro Val Pro Tyr Ala Asp Phe Val Thr Thr Thr Thr
    210                 215                 220

His Lys Thr Leu Arg Gly Pro Arg Gly Gly Met Ile Leu Cys Arg Glu
225                 230                 235                 240

Glu Phe Gly Lys Lys Ile Asp Lys Ser Ile Phe Pro Gly Ile Gln Gly
                245                 250                 255

Gly Pro Leu Met His Val Ile Ala Ala Lys Ala Val Ser Phe Gly Glu
            260                 265                 270

Val Leu Gln Asp Asp Phe Lys Thr Tyr Ala Gln Asn Val Ile Ser Asn
        275                 280                 285

Ala Lys Arg Leu Ala Glu Ala Leu Thr Lys Glu Gly Ile Gln Leu Val
    290                 295                 300

Ser Gly Gly Thr Asp Asn His Leu Ile Leu Val Asp Leu Arg Ser Leu
305                 310                 315                 320

Gly Leu Thr Gly Lys Val Ala Glu His Val Leu Asp Glu Ile Gly Ile
                325                 330                 335

Thr Ser Asn Lys Asn Ala Ile Pro Tyr Asp Pro Glu Lys Pro Phe Val
            340                 345                 350

Thr Ser Gly Ile Arg Leu Gly Thr Ala Ala Val Thr Ser Arg Gly Phe
        355                 360                 365

Asp Gly Asp Ala Leu Glu Glu Val Gly Ala Ile Ile Ala Leu Ala Leu
    370                 375                 380

Lys Asn His Glu Asp Glu Gly Lys Leu Glu Glu Ala Arg Gln Arg Val
385                 390                 395                 400

Ala Ala Leu Thr Asp Lys Phe Pro Leu Tyr Lys Glu Leu Asp Tyr
                405                 410                 415

<210>  80
<211>  1248
<212>  DNA
<213>  Bacillus subtilis

<400>  80
atgaaacatt tacctgcgca agacgaacaa gtgtttaacg ccattaaaaa tgagcgtgaa      60

cgccaacaga ctaagatcga attgattgct tctgagaact ttgtaagtga agcggttatg     120

gaagcacaag gatctgtttt gacaaataag tacgcagaag gatatccggg caaacgctac     180

tacggcggat gcgagcacgt cgatgtcgtt gaagatatcg cccgtgaccg cgcgaaggaa     240

atctttggag cggagcatgt aaacgttcag cctcattcag gcgcacaagc aaacatggca     300

gtgtacttca cgattttgga gcaaggcgat actgtacttg ggatgaacct ctcccacggc     360

ggccatttaa cacacggaag cccagtcaac ttcagcggtg ttcaatataa ctttgttgag     420

tacggcgtag ataaagaaac tcaatatatt gattacgatg acgtgcgtga aaaagccctc     480

gctcataaac cgaagcttat cgtagcagga gcaagtgcgt atcctcgtac aatcgacttt     540

aagaaattcc gtgaaattgc tgatgaagtc ggcgcttact tcatggtgga tatggcacat     600

atcgcaggac ttgttgcggc aggccttcat ccaaacccgg ttccttacgc tgatttcgtt 660

acaacaacaa cacataaaac acttcgcggt cctcgcggcg gtatgatcct ttgccgtgaa 720

gagtttggca agaaaattga taaatcgatc ttccctggaa ttcaaggcgg ccctctgatg 780

cacgttattg ccgcaaaagc tgtttcattc ggtgaagtat tgcaggacga tttcaaaaca 840

tatgcacaaa acgtcatttc aaacgcgaaa cgtctggctg aagccttaac gaaagagggc 900

atccagctcg tttcaggcgg aacagacaac caccttatcc ttgttgacct tcgttcgctc 960

ggactgactg gtaaggttgc ggagcatgta cttgatgaaa ttggtattac gtctaacaaa 1020

aacgcgattc catatgatcc tgaaaaacct ttcgtaacaa gcggcatccg tcttggtaca 1080

gctgctgtaa ccagccgcgg ttttgacgga gacgcattag aagaagtcgg tgctatcatt 1140

gcgcttgcat tgaaaaacca cgaagatgaa ggaaaacttg aagaagcaag acagcgtgta 1200

gctgctctga ctgataaatt tcctttatat aaagaattag attattaa 1248

<210> 81
<211> 300
<212> PRT
<213> Bacillus subtilis

<400> 81

Met Lys Ser Tyr Met Thr Gln Arg Leu Asp Glu Tyr Arg Asp Gly Asn
1               5                   10                  15

Glu Asp Lys Gly Arg Leu Leu Val Ser Cys Pro Asp Gln Pro Gly Ile
            20                  25                  30

Val Ser Ala Val Ser Ala Phe Leu Phe Glu His Gly Ala Asn Ile Ile
        35                  40                  45

Glu Ser Asn Gln Tyr Thr Thr Asp Pro Glu Gly Gly Arg Phe Phe Leu
    50                  55                  60

Arg Ile Glu Phe Asp Cys Ala Gly Ile Arg Glu Lys Lys Ser Ser Leu
65                  70                  75                  80

Gln Ala Ala Phe Ala Ser Val Ala Glu Lys Phe Asp Met Thr Trp Ser
                85                  90                  95

Leu Thr Leu Ala Ser Glu Leu Lys Arg Val Ala Ile Phe Val Ser Lys
            100                 105                 110

Glu Leu His Cys Leu His Glu Leu Ile Trp Glu Trp Gln Thr Gly Asn
        115                 120                 125

Leu Met Ala Glu Ile Ala Val Val Ile Ser Asn His Glu Glu Ala Arg
    130                 135                 140

Glu Leu Val Glu Arg Leu Asn Ile Pro Phe His Tyr Met Lys Ala Asn
145                 150                 155                 160

Lys Asp Ile Arg Ala Glu Val Glu Lys Lys Gln Leu Glu Leu Leu Glu
                165                 170                 175

Gln Tyr Asp Val Asp Val Ile Val Leu Ala Arg Tyr Met Gln Ile Leu
            180                 185                 190

Thr Pro Asp Phe Val Ser Ala His Pro Asn Arg Ile Ile Asn Ile His
        195                 200                 205

His Ser Phe Leu Pro Ala Phe Ile Gly Ala Asn Pro Tyr Lys Arg Ala
    210                 215                 220

Tyr Glu Arg Gly Val Lys Leu Ile Gly Ala Thr Ser His Tyr Val Thr
225                 230                 235                 240

Asn Asp Leu Asp Glu Gly Pro Ile Ile Glu Gln Asp Ile Glu Arg Val
                245                 250                 255

Asp His Arg Asp Asn Ala Glu Ala Leu Lys Asn Ile Gly Arg Thr Ile
            260                 265                 270

Glu Arg Ser Val Leu Ala Arg Ala Val Lys Trp His Leu Glu Asp Arg
        275                 280                 285

Val Ile Val His Glu Asn Lys Thr Ile Val Phe Asn
    290                 295                 300

<210>  82
<211>  903
<212>  DNA
<213>  Bacillus subtilis

<400>  82
atgaaatcat atatgactca gcggttggac gaataccgtg acggaaatga ggataaaggt      60

cggctcttgg tcagctgccc cgatcagccg ggtatcgtct ctgcagtttc cgcgttttta     120

tttgaacacg gtgccaatat tatagaatca aatcaatata cgacagaccc tgaaggcggc     180

cggttcttcc tgagaatcga attcgactgc gcaggcattc gtgaaaaaaa atcatcactt     240

caggcagcgt ttgcctctgt tgcggaaaaa ttcgacatga catggagctt aactttggcg     300

agcgaactga agcgtgtcgc cattttcgtt tcaaaggagc tccactgcct gcatgagctg     360

atttgggaat ggcaaaccgg caacctgatg gcggagatcg ctgttgtcat cagtaaccat     420

gaggaagcga gagagctggt tgagcgcctg aacattccat tccactatat gaaagcgaac 480

aaagacatca gagcggaagt cgaaaagaag cagcttgaac tgctggagca gtacgatgtt 540

gatgtgatcg tgctcgcacg ctatatgcag attctaactc ctgattttgt ttcggctcat 600

ccgaatcgca tcatcaatat ccaccattca ttcctgccag cttttatcgg tgcgaatccg 660

tacaaacggg cctacgagcg cggcgtgaaa ctgatcggtg cgacatctca ttatgtgaca 720

aacgatcttg atgaagggcc gatcattgaa caggatattg agcgtgtgga ccaccgcgat 780

aatgcggaag cgctgaaaaa catcggaaga acaattgagc gcagcgtgct tgcccgtgct 840

gtgaaatggc atttggaaga ccgtgtcatc gttcatgaaa ataaaacaat cgtctttaac 900

tag 903

<210> 83
<211> 612
<212> PRT
<213> Bacillus subtilis

<400> 83

Met Gly Leu Leu Glu Asp Leu Gln Arg Gln Val Leu Ile Gly Asp Gly
1               5                   10                  15

Ala Met Gly Thr Leu Leu Tyr Ser Tyr Gly Ile Asp Arg Cys Phe Glu
            20                  25                  30

Glu Leu Asn Ile Ser Lys Pro Glu Glu Ile Gln Arg Ile His Lys Ala
        35                  40                  45

Tyr Val Glu Ala Gly Ala Asn Ile Ile Gln Thr Asn Thr Tyr Gly Ala
    50                  55                  60

Asn Tyr Ile Lys Leu Ser Arg His Gly Leu Glu Asp Asp Ile Lys Lys
65                  70                  75                  80

Met Asn Gln Glu Ala Val Lys Ile Ala Arg Ala Ser Ala Gly Asp Ala
                85                  90                  95

Tyr Val Leu Gly Thr Met Gly Gly Ile Arg Thr Phe Asn Lys Asn Ala
            100                 105                 110

Tyr Ser Leu Asp Glu Ile Lys Arg Ser Phe Arg Glu Gln Leu Tyr Leu
        115                 120                 125

Leu Leu His Glu Glu Pro Asp Gly Leu Leu Leu Glu Thr Tyr Tyr Asp
    130                 135                 140

Leu Glu Glu Ala Arg Glu Val Leu Lys Ile Ala Arg Lys Glu Thr Asp
145                 150                 155                 160

Leu Pro Ile Met Leu Asn Val Ser Met His Glu Gln Gly Val Leu Gln
                165                 170                 175

Asp Gly Thr Pro Leu Ser Asp Ala Leu Arg Ser Ile Ala Asp Leu Gly
            180                 185                 190

Ala Asp Ile Val Gly Ile Asn Cys Arg Leu Gly Pro Tyr His Met Ile
        195                 200                 205

Glu Ala Leu Ser Glu Val Pro Ile Phe Asp Asp Val Phe Leu Ser Val
    210                 215                 220

Tyr Pro Asn Ser Ser Leu Pro Ser Leu Glu Glu Gly Arg Leu Val Tyr
225                 230                 235                 240

Glu Thr Asp Asp Thr Tyr Phe Gln Asn Ser Ala Ser Glu Phe Arg Lys
                245                 250                 255

Gln Gly Ala Arg Ile Ile Gly Gly Cys Cys Gly Thr Thr Pro Asn His
            260                 265                 270

Ile Arg Ala Met Ala Glu Ala Val Gly Gly Leu Ala Pro Ile Thr Glu
        275                 280                 285

Lys Glu Val Lys Thr Arg Ala Lys Glu Phe Ile Ser Val His His Glu
    290                 295                 300

Arg Thr Glu Pro Gly Leu Asp Glu Ile Ala Ala Lys Lys Arg Ser Ile
305                 310                 315                 320

Ile Val Glu Leu Asp Pro Pro Lys Lys Leu Ser Phe Asp Lys Phe Leu
                325                 330                 335

Ser Ala Ala Ala Glu Leu Lys Glu Ala Gly Ile Asp Ala Leu Thr Leu
            340                 345                 350

Ala Asp Asn Ser Leu Ala Thr Pro Arg Ile Ser Asn Val Ala Cys Gly
        355                 360                 365

Ala Leu Val Lys Gln Gln Leu Asp Met Arg Ser Leu Val His Ile Thr
    370                 375                 380

Cys Arg Asp Arg Asn Ile Ile Gly Leu Gln Ser His Leu Met Gly Leu
385                 390                 395                 400

Asp Thr Leu Gly Leu Asn Asp Val Leu Ala Ile Thr Gly Asp Pro Ser
                405                 410                 415

Lys Ile Gly Asp Phe Pro Gly Ala Thr Ser Val Tyr Asp Leu Thr Ser
            420                 425                 430

Phe Asp Leu Ile Arg Leu Ile Lys Gln Phe Asn Glu Gly Leu Ser Leu
        435                 440                 445

Ser Gly Lys Pro Leu Gly Lys Lys Thr Asn Phe Ser Val Ala Ala Ala
    450                 455                 460

Phe Asn Pro Asn Val Arg His Leu Asp Lys Ala Val Lys Arg Leu Glu
465                 470                 475                 480

Lys Lys Ile Asp Cys Gly Ala Asp Tyr Phe Val Ser Gln Pro Val Tyr
                485                 490                 495

Ser Glu Gln Gln Leu Val Asp Ile His Asn Glu Thr Lys His Leu Lys
            500                 505                 510

Thr Pro Ile Tyr Ile Gly Ile Met Pro Leu Thr Ser Ser Arg Asn Ala
        515                 520                 525

Glu Phe Ile His Asn Glu Ile Pro Gly Ile Lys Leu Ser Asp Thr Ile
    530                 535                 540

Arg Glu Lys Met Ala His Ala Gly Glu Asp Lys Glu Lys Gln Lys Ala
545                 550                 555                 560

Glu Gly Leu Ala Ile Ala Arg Ser Leu Leu Asp Thr Ala Cys Glu Leu
                565                 570                 575

Phe Asn Gly Ile Tyr Leu Ile Thr Pro Phe Leu Arg Ser Asp Leu Thr
            580                 585                 590

Ala Glu Leu Thr Ser Tyr Ile Gln Gln Lys Asp Glu Gln Arg Gln Asn
        595                 600                 605

Ile Phe Leu His
    610

<210>  84
<211>  296
<212>  PRT
<213>  Escherichia coli

<400>  84

Met Ser Phe Phe His Ala Ser Gln Arg Asp Ala Leu Asn Gln Ser Leu
1               5                   10                  15

Ala Glu Val Gln Gly Gln Ile Asn Val Ser Phe Glu Phe Phe Pro Pro
            20                  25                  30

Arg Thr Ser Glu Met Glu Gln Thr Leu Trp Asn Ser Ile Asp Arg Leu
        35                  40                  45

Ser Ser Leu Lys Pro Lys Phe Val Ser Val Thr Tyr Gly Ala Asn Ser
    50                  55                  60

Gly Glu Arg Asp Arg Thr His Ser Ile Ile Lys Gly Ile Lys Asp Arg
65                  70                  75                  80

Thr Gly Leu Glu Ala Ala Pro His Leu Thr Cys Ile Asp Ala Thr Pro
                85                  90                  95

Asp Glu Leu Arg Thr Ile Ala Arg Asp Tyr Trp Asn Asn Gly Ile Arg
            100                 105                 110

His Ile Val Ala Leu Arg Gly Asp Leu Pro Pro Gly Ser Gly Lys Pro
        115                 120                 125

Glu Met Tyr Ala Ser Asp Leu Val Thr Leu Leu Lys Glu Val Ala Asp
    130                 135                 140

Phe Asp Ile Ser Val Ala Ala Tyr Pro Glu Val His Pro Glu Ala Lys
145                 150                 155                 160

Ser Ala Gln Ala Asp Leu Leu Asn Leu Lys Arg Lys Val Asp Ala Gly
                165                 170                 175

Ala Asn Arg Ala Ile Thr Gln Phe Phe Phe Asp Val Glu Ser Tyr Leu
            180                 185                 190

Arg Phe Arg Asp Arg Cys Val Ser Ala Gly Ile Asp Val Glu Ile Ile
        195                 200                 205

Pro Gly Ile Leu Pro Val Ser Asn Phe Lys Gln Ala Lys Lys Phe Ala
    210                 215                 220

Asp Met Thr Asn Val Arg Ile Pro Ala Trp Met Ala Gln Met Phe Asp
225                 230                 235                 240

Gly Leu Asp Asp Asp Ala Glu Thr Arg Lys Leu Val Gly Ala Asn Ile
                245                 250                 255

Ala Met Asp Met Val Lys Ile Leu Ser Arg Glu Gly Val Lys Asp Phe
            260                 265                 270

His Phe Tyr Thr Leu Asn Arg Ala Glu Met Ser Tyr Ala Ile Cys His
        275                 280                 285

Thr Leu Gly Val Arg Pro Gly Leu
    290                 295

<210>  85
<211>  1839
<212>  DNA
<213>  Bacillus subtilis

<400>  85
atgggacttt tagaagattt gcaaagacag gtgttaatcg gtgacggcgc catggggacg      60

ctcctctact cctatggcat tgacaggtgt tttgaggagc tcaatatttc aaagccggag     120

gaaattcagc gcatacataa agcgtacgtt gaagcgggag ccaacattat tcaaacgaat     180

acgtacggag ccaactatat taaattatcc agacacgggc ttgaggatga catcaaaaaa     240

atgaatcagg aggccgtaaa gatcgcgcgg gcttcagcag gcgatgcata cgtgcttgga     300

acgatgggcg gcatacgcac atttaataaa aacgcataca gtcttgacga aattaagcgc     360

agcttccgcg agcagctgta tctgctgctg catgaagagc cggatggctt gctgttagaa     420

acatattatg atttggaaga agcccgtgaa gtgctgaaaa tcgcgcgcaa agagactgat     480

cttccgatta tgctcaatgt ctccatgcac gagcaaggcg tattgcagga cggaacgccg     540

ctttctgacg cattgcgctc gattgcggat ctcggagctg atattgtcgg cattaactgc     600

cgcctcggcc cttatcacat gattgaagcg ctaagcgaag tgccgatttt tgatgacgta     660

ttcttatccg tttatccgaa cagcagtctt ccttcgcttg aagagggacg gctcgtctat     720

gaaacagacg acacgtattt tcaaaacagc gcgtcagaat tccgtaagca aggtgcgcgg     780

attatcggcg gctgctgcgg cacaacgccg aatcacatca gggcgatggc ggaagcggtt     840

ggcgggctgg ctccgattac ggaaaaagaa gtcaaaaccc gggcgaaaga attcatttct     900

gttcatcacg agcgaaccga gcccggcctg gatgagatcg cagcgaaaaa acgctctatt     960

atcgtagagc tggacccgcc gaaaaaacta agctttgaca aatttctgtc cgctgctgct    1020

gaattaaaag aagcgggaat cgatgccctg acactcgccg acaattcttt ggcgaccccg    1080

cggatcagca atgttgcctg cggcgccctt gtcaagcagc agctcgatat gcgttccctt    1140

gtccatatta cgtgccgtga ccgtaacatt atcggcttac agtcacattt aatgggcctt    1200

gatacgttag ggctgaatga cgtgcttgct attacaggtg acccttctaa aatcggggat    1260

tttcctgggg cgacgtctgt atatgattta acatcttttg atttaatcag gctgatcaaa    1320

caattcaatg aaggtctgtc tctgtctgga aaaccgctcg gcaagaaaac gaatttctca 1380

gttgccgccg catttaatcc taacgtccgc catctggata aagcggtaaa acggcttgag 1440

aaaaaaatag attgcggcgc agattatttt gtttctcagc ctgtctattc cgagcagcag 1500

cttgtcgata tccataacga aacaaaacat ctgaagacgc cgatctatat cggcatcatg 1560

ccgctgacaa gcagccggaa tgccgaattc atccataatg aaattcccgg cattaagctg 1620

tctgatacga ttcgcgaaaa aatggcccat gccggtgaag ataaagaaaa gcaaaaggcg 1680

gaaggccttg caattgcacg ctccctgctg gatacggcat gcgagctgtt taacggcatc 1740

tacttgataa cgcctttcct cagatctgac ttaaccgctg agctcacatc atatattcag 1800

caaaaagatg agcagcgcca aaatatcttt ttgcattaa 1839

<210> 86
<211> 891
<212> DNA
<213> Escherichia coli

<400> 86
atgagctttt ttcacgccag ccagcgggat gccctgaatc agagcctggc agaagtccag 60

gggcagatta acgtttcgtt cgagtttttc ccgccgcgta ccagtgaaat ggagcagacc 120

ctgtggaact ccatcgatcg ccttagcagc ctgaaaccga agtttgtatc ggtgacctat 180

ggcgcgaact ccggcgagcg cgaccgtacg cacagcatta ttaaaggcat taaagatcgc 240

actggtctgg aagcggcacc gcatcttact tgcattgatg cgacgcccga cgagctgcgc 300

accattgcac gcgactactg gaataacggt attcgtcata tcgtggcgct gcgtggcgat 360

ctgccgccgg gaagtggtaa gccagaaatg tatgcttctg acctggtgac gctgttaaaa 420

gaagtggcag atttcgatat ctccgtggcg gcgtatccgg aagttcaccc ggaagcaaaa 480

agcgctcagg cggatttgct taatctgaaa cgcaaagtgg atgccggagc caaccgcgcg 540

attactcagt tcttcttcga tgtcgaaagc tacctgcgtt ttcgtgaccg ctgtgtatcg 600

gcgggcattg atgtggaaat tattccggga attttgccgg tatctaactt taaacaggcg 660

aagaaatttg ccgatatgac caacgtgcgt attccggcgt ggatggcgca aatgttcgac 720

ggtctggatg atgatgccga aacccgcaaa ctggttggcg cgaatattgc catggatatg 780

gtgaagattt taagccgtga aggagtgaaa gatttccact tctatacgct taaccgtgct 840

gaaatgagtt acgcgatttg ccatacgctg ggggttcgac ctggtttata a 891

<210> 87
<211> 19
<212> DNA
<213> Artificial Sequence

<220>

<223>  Primer

<400>  87
gggcgcgcta gcggccaat                                                    19


<210>  88
<211>  75
<212>  DNA
<213>  Artificial Sequence

<220>
<223>  Primer

<400>  88
acgttgtcaa taaaattatt ttgacaaaat tgaattctta gctttcgcta tgtacaaatt      60

catcaacgcc tgatt                                                        75


<210>  89
<211>  75
<212>  DNA
<213>  Artificial Sequence

<220>
<223>  Primer

<400>  89
aatcaggcgt tgatgaattt gtacatagcg aaagctaaga attcaatttt gtcaaaataa      60

ttttattgac aacgt                                                        75


<210>  90
<211>  20
<212>  DNA
<213>  Artificial Sequence

<220>
<223>  Primer

<400>  90
gcggtagtgg tgcttacgat                                                   20


<210>  91
<211>  1248
<212>  DNA
<213>  Artificial Sequence

<220>
<223>  Optimized glyA sequence for Bacillus subtilis codon usage

<400>  91
atgaagcacc ttcctgcaca agatgaacaa gtattcaacg ctattaagaa cgaacgcgaa      60

cgtcaacaaa ccaagattga attgatcgct agcgaaaatt tcgtttcaga agcagttatg     120

gaggctcaag gctctgttct tacgaacaaa tatgctgaag gctacccagg caaaagatac     180

tacggaggct gcgaacacgt tgacgtagtc gaagacattg cacgtgaccg cgcaaaagaa     240

atcttcggcg cagaacatgt taacgttcaa cctcacagcg gagcacaagc gaacatggca 300

gtttatttta caatccttga acaaggggac acagtcctgg gcatgaacct ttctcacgga 360

ggccatctta ctcatggtag cccggtgaac ttttctggcg tccaatataa cttcgttgag 420

tacggtgtag ataaagaaac acagtatatt gactatgatg atgtgcgtga aaaagctctt 480

gcgcacaaac ctaaacttat tgttgcggga gcgtcagcgt acccgcgtac cattgatttt 540

aaaaagttcc gggaaattgc tgacgaggtt ggcgcgtact ttatggtgga tatggctcat 600

attgcgggct tggtcgcagc aggactgcac cctaacccgg tgccttacgc cgatttcgtt 660

acaaccacga cacataaaac acttagaggc ccacgtggcg gaatgattct ttgcagagaa 720

gaatttggaa agaaaattga caaatccatt ttccctggca tccagggagg tcctcttatg 780

catgtcattg cagcaaaagc ggtgagcttc ggcgaagtgt tacaagatga tttcaaaacg 840

tatgctcaga acgttatctc taatgcgaaa cgtttagcgg aagcgttgac aaaagaaggt 900

atccaacttg tctcaggagg cacagataat catcttattc tggttgatct tcgctcactt 960

ggattaacag gcaaagttgc ggaacatgtt cttgacgaaa ttggtattac atcaaacaaa 1020

aacgctattc cttacgatcc ggaaaaacct ttcgtaacat ctggtattcg cttaggcaca 1080

gctgcggtta caagccgcgg attcgatggc gatgcgcttg aagaggtagg cgctattatt 1140

gcactggcgt taaaaaatca tgaggatgaa ggaaaacttg aagaggcccg ccagcgtgtc 1200

gcagctctta ctgataaatt cccgctttac aaagaacttg attattga 1248

<210> 92
<211> 903
<212> DNA
<213> Artificial Sequence

<220>
<223> optimized purU sequence for Bacillus subtilis codon usage

<400> 92
atgaagtctt acatgactca acgtttagat gaatatcgtg acggcaacga ggataaagga 60

cgcctcttgg tgtcttgtcc ggaccagcct ggaattgttt ctgcagtttc cgcatttctt 120

tttgagcacg gcgcaaatat catcgaaagc aatcaataca caactgaccc agaaggcggc 180

cgtttcttcc ttcgcattga atttgactgc gcaggaatcc gtgaaaaaaa aagcagctta 240

caggctgctt tcgcatctgt cgctgaaaaa ttcgacatga cttggtcttt aactttggcg 300

tctgagctaa aacgtgttgc tatctttgtg tcaaaagagc tccactgctt acatgaactt 360

atttgggaat ggcaaacagg taacctaatg gcagagattg ccgtagtcat ctcaaaccac 420

gaagaagctc gcgaactggt ggaacgtctt aatatccctt ttcactacat gaaagctaac 480

aaagacatcc gggctgaagt tgaaaaaaaa cagcttgagc tgttagaaca gtatgacgtt 540

gacgtaatcg tgttagcacg ctatatgcag attttaacac cggacttcgt ctctgctcat 600

cctaaccgta ttatcaatat tcaccattct ttcttaccgg cctttatcgg agcaaatcct 660

tataaacgtg cgtatgaacg tggagttaaa ttgatcggcg ccacgtccca ctatgttacg 720

aacgacttag atgaaggtcc tattatcgaa caagatatcg agcgtgttga tcatcgtgat 780

aatgctgaag cactcaaaaa tattggccgt acaatcgagc gctctgttct agctcgtgca 840

gtaaaatggc atcttgagga ccgcgtcatc gttcatgaga ataaaacaat cgtttttaac 900

tga 903

<210> 93
<211> 1839
<212> DNA
<213> Artificial Sequence

<220>
<223> optimized yitJ sequence for Bacillus subtilis codon usage

<400> 93
atgggattac ttgaagatct tcaacgccaa gttctgatcg gagacggagc gatgggaacg 60

cttctgtatt cttacggaat cgacagatgt tttgaagagt taaatatttc taaacctgaa 120

gaaatccaaa gaatccataa agcgtacgtc gaagcaggag caaacatcat tcaaacaaat 180

acatacggag ctaactacat taaacttagc cgccacggcc tggaagacga cattaaaaaa 240

atgaaccaag aagcggtcaa aattgcacgc gcaagcgctg gcgacgcgta tgttcttggc 300

acgatgggag gcatccgcac attcaacaag aatgcttaca gcctggacga gattaaacgc 360

tcattccgtg aacaattata cctgcttctt cacgaagaac cggatggcct tcttttagaa 420

acatattacg atctggaaga ggcacgtgag gttcttaaaa ttgctcgcaa agagacggat 480

cttccgatca tgcttaacgt ctctatgcac gaacaaggcg tgcttcagga tggcacacct 540

cttagcgacg ctttaagaag catcgcggac ctgggcgcag atatcgttgg cattaactgc 600

cgcctgggac cgtaccacat gattgaagca ttgtcagaag tcccgatctt cgatgacgtc 660

ttccttagcg tttacccgaa ctcttcatta ccgagcttag aagaaggacg cttagtgtat 720

gaaacggacg atacatactt ccaaaattct gcaagcgagt tccgtaaaca aggagcgcgt 780

attatcggcg ggtgctgcgg aacaactccg aatcacatta gagcgatggc agaagcagta 840

ggaggtcttg cacctatcac agaaaaagaa gtgaaaacgc gtgctaaaga atttatttct 900

gttcatcacg aacgtacaga accgggctta gacgaaattg ctgctaaaaa gcgcagcatt 960

attgtcgaac ttgatccgcc taaaaaactt tcatttgata aatttctttc agcggcagcg 1020

gagttaaaag aagcaggcat cgatgctctt acattagcgg ataactcatt agcgactcct 1080

cgtatctcta acgtcgcatg cggtgcactt gtgaaacaac aactggatat gcgctcactt 1140

gttcatatca cgtgccgtga ccgcaatatc attggccttc aatctcattt aatgggtctt 1200

gatacattag gtttaaacga tgtacttgcg attacaggtg atccgtcaaa aattggtgac 1260

tttcctggcg caacatcagt gtacgacctg acctcttttg atcttattcg tcttattaaa 1320

caattcaatg aaggattatc tttgtctggt aaacctcttg gcaagaaaac gaacttctca 1380

gtagcggcgg cattcaatcc taatgtgcgt caccttgaca aagcggtcaa aagacttgaa 1440

aaaaaaatcg actgtggagc ggattatttc gtctctcaac cagtatatag tgagcaacaa 1500

ttagtggata tccacaacga aacgaagcac ttaaaaacac caatctacat tggaatcatg 1560

cctcttacgt cctctcgcaa cgctgaattt atccataacg aaattccagg aattaaactt 1620

tctgatacga tccgtgaaaa aatggcccat gctggcgaag acaaggagaa acaaaaggct 1680

gaaggacttg caattgcgcg ttctttactg gacactgctt gcgaattgtt caacggcatc 1740

tatcttatta caccatttct tcgtagcgac ttgacagccg agttgacatc atatattcaa 1800

caaaaggacg aacaacgcca gaatattttt ctgcattaa 1839

<210> 94
<211> 891
<212> DNA
<213> Artificial Sequence

<220>
<223> DNA fragment

<400> 94
atgtcattct tccatgcttc acaacgtgat gcgcttaacc agagcctggc agaagttcag 60

ggccaaatca atgtgtcttt cgaatttttt cctcctcgca caagcgagat ggaacaaaca 120

ctttggaact ccatcgaccg tctttcatca cttaagccga aatttgtctc agtgacgtac 180

ggcgctaaca gcggagaacg cgatagaaca cattcaatca ttaagggaat caaagatcgt 240

acgggattag aagctgctcc tcacttaaca tgtattgatg cgacgccgga tgaacttcgt 300

acaatcgcgc gtgattactg gaacaatggt attcgtcata tcgtcgctct gagaggagat 360

ttgcctcctg gatctggtaa accggagatg tacgcttcag accttgtaac acttcttaaa 420

gaagtggcgg actttgatat ctctgtcgcg gcttaccctg aagttcatcc ggaggcaaaa 480

agcgcgcagg cagacttatt aaaccttaag cgtaaagtag acgcgggtgc taaccgtgcg 540

atcacacagt tcttttttga tgtggaaagt tatttacgtt ttcgcgatcg ttgcgtttcg 600

gcaggcatcg atgttgaaat catccctgga attttaccag ttagcaactt caaacaggcg 660

aaaaaatttg cggatatgac gaacgttcgt attccggcat ggatggcgca aatgtttgac 720

ggacttgacg atgacgcaga aacacgcaaa ttagtcggtg ctaacattgc gatggacatg 780

gttaaaattc tgtcacgcga aggtgtcaag gactttcatt tctacacact taatcgcgct 840

gaaatgagct atgcgatctg ccacacactt ggagtccgtc ctggccttta a 891

<210>  95
<211>  1686
<212>  DNA
<213>  Bacillus subtilis

<400>  95
cttgttgttg ttccgccgtt ttacggcagt cctttatcgg tgacggggac aattgtgctt      60

gtaccggagc cggaacccgt ctacgcgttt acagcgaatc caaatgatca atcagtttct     120

gtgattgata caaacaccga tactgttgtg acaacgattg ctcttccgta caatccggca     180

ggtattgaaa ttacgccaga taaaagcgct gtgtttgtct tacatcccaa caataatgtg     240

atttctgtca tcgattatga cacattaaca gtgacagcaa ctatattgct ggatcagccg     300

cctcgattga ttagatttat ccctaatcat gagtttgctt atgttttcac cggtactgcg     360

gtttatgtga ttggaattga tacgttaact gtggataggt cgattcctgt ggaaggatat     420

gatgttgcaa tcgatcccaa tgggttattc gcctatgttc tgaattttgg aatagtgcaa     480

aaagtggacc taactaccgg tgaagtcaca ggaacaattg aacgagagct tatcgtatca     540

accatagaaa caaattggcc ggagcggtat gcctatgtat tagaacaaga attctttttt     600

aattatttga cggttattga tttaaatacg tttaccatca gcagcaccca agagctggag     660

tatgaagggg aatatcgaat gtttacgagt ggagcagagg tgtatttata tgacggcttc     720

actggcaatt tatattctgt cagtccaaat ggagcaggcg ttataggaaa tgttccgcaa     780

tcagcaacag actatgcgtt taccccgaat ggcgattttc tgtatgcaac tcgttttata     840

gagcagagca tcattgttta caacacagat gattattctg aggaaactgt gatatctctt     900

ggggtttcac cgggtgccat tacgatttaa ccgctgcatg ttgtgcattt ctaaaaggac     960

gtgaaatcat gaaaaagaat gatctttctg ctttacagga aaattgtttc tgtttttgtg    1020

atgaggaaat tagccgggaa gcgccgtttc aagtgccgat agatttccct gagggcttca    1080

aggttgatac ggccgaagcg tcagcggctg ttacatggag tacagacaat ttgtcctgta    1140

tcagtgagcc ttgcttaatc cagactgggc cggaaccaga agatatcggt gttcgatatg    1200

ctgtgagagt acaagggacc attacgcttc ttgtcagtgt gtcgcctgta cgaaatcaat    1260

atggacaagg agacggggct gtttctgtga ttcatactga agagattgat caagttgttt    1320

attatgcagc acagtctggc cgctgtcctg atttcagtca aataacagtg gaagatctgc    1380

tcattgtacc gcctttctac ggcagtccgc ttacacgggt gcgcatgatc gtatggttca    1440

ctgtccacca accaaaactg tgctcagtac cgccaatatt tctcccttgg ggggtacaaa    1500

gaggtgtccc tagaagagat ccacgctgtg taaaaatttt acaaaaaggt attgactttc    1560

cctacagggt gtgtaataat ttaattacag gcgggggcaa ccccgctcag tacctagagc    1620

gtaaaagagg ggagggaaac actagttggc ttcgttggga ttctccggtg atgctgattt    1680

cgctgc                                                                1686

<210>  96
<211>  2126
<212>  DNA
<213>  Artificial Sequence

<220>
<223>  spectinomycin selectable marker

<400>  96
cggtagtcat tgctggcgaa cgcgtctaga gctccggcat aaaattaact aaaataatta      60

ttatgtagat aaaaaattta gaagccaatg aaatctataa ataaactaaa ttaagtttat     120

ttaattaaca actatggata taaaataggt actaatcaaa atagtgaggc gaatatattt     180

gaatacatac gaacaaatca ataaagtgaa aaaaatactt cggaaacatt taaaaaataa     240

ccttattggt acttacatgt ttggatcagg agttgagagt ggactcaaac caaatagtga     300

tcttgacttt ttagtcgtcg tatctgaacc attgacagat caaagtaaag aaatacttat     360

acaaaaaatt agacctattt caaaaaaaat aggagataaa agcaacttac gatatattga     420

attaacaatt attattcagc aagaaatggt accgtggaat catcctccca aacaagaatt     480

tatttatgga gaatggttac aagagcttta tgaacaagga tacattcctc agaaggaatt     540

aaattcagat ttaaccataa tgctttacca agcaaaacga aaaaataaaa gaatatacgg     600

aaattatgac ttagaggaat tactacctga tattccattt tctgatgtga gaagagccat     660

tatggattcg tcagaggaac ttatagataa ttatcaggat gatgaaacca actctatatt     720

aactttatgc cgcatgattt taactatgga cacgggtaaa atcataccaa aagatattgc     780

gggaaatgca gtggctgaat cttctccatt agaacatagg gagagaattt tgttagcagt     840

tcgtagttat cttggagaga atattgaatg gactaatgaa aatgtaaatc ttacgataaa     900

ctatcttaat aaccgcctta agaaacttta acccgggcgc gttgctttcg atgaagtttt     960

accgccatca ccagaccctc aatcgtatgt gtttactgcc aatactggag acagcacggt    1020

ttcggttatt gatgcagatt taaacactgt tgtaaaaaca atcccttttt ctgacgtgcc    1080

aactaatcta ggcgttactt tcgataaagc attcacgtat gtcctccatg gaaataccaa    1140

ccttgtctca gtgattgata ataaaacatt aaccatcata aataccatta ctgttggagg    1200

agggccgaga aaaatcgaat ttgatccaac agatgagttc gcatacgtga tggccgccgg    1260

atctatatat gtgattaaca cggcctctca atcggtcatt gatgtcattc cgatacccgg    1320

cgctttagac tttgctcttg atccgaatgg acagtatgtc tataccgcta atggaagcag    1380

ttggtcggtt gataaatatg atgtaaatac tgggcagttg gtagaaagta tcatagatac    1440

atttgaattt cccagtttaa ttacaactcc ttacgctgga aattttgcgt acgtattgaa    1500

tggtgagctg tggccgaaag gtgtaacgga gattagtttg tcaccattaa gcaggggagg    1560

ggacttcagc cgtttatttg aaacgctgag gacgattgta ttttctctcg atagtactag 1620

agcttatttt caagagccat atagtgaacc gtttctcatc aataacttat acgtagttaa 1680

tacggccaga cagaggatca ttgcaaacgt gtctctcccc ggcgcctttg acctcgccgt 1740

aacgccggac aaacagtata tttatgcggc acagcccaac gacaatgctg tgaccgtgta 1800

ccgcacaagt gattatacag ccgtgacggt gattccggtt ggcgcgggtc cgtctgcgat 1860

tgcgatgtag ctattttata gatcaagaga agcggtgctg tgataagaga atcgtttata 1920

aataaatgat ccgatttgct gaatggcagc caggataatc agaaacagca gttcacttat 1980

agagaaactg agcatccacc aaatcccggc tgctgccagt gctcccgcaa acacttttct 2040

gaacaggctg ccgtgccgga aaagctgccg caaatacgtt cgcatgcaaa acaataaata 2100

caggatcagt agcatcgcaa gtccca 2126

<210> 97
<211> 7709
<212> DNA
<213> Artificial Sequence

<220>
<223> MTHF-OP-A operon

<400> 97
cgcgtttaca gcgaatccaa atgatcaatc agtttctgtg attgatacaa acaccgatac 60

tgttgtgaca acgattgctc ttccgtacaa tccggcaggt attgaaatta cgccagataa 120

aagcgctgtg tttgtcttac atcccaacaa taatgtgatt tctgtcatcg attatgacac 180

attaacagtg acagcaacta tattgctgga tcagccgcct cgattgatta gatttatccc 240

taatcatgag tttgcttatg ttttcaccgg tactgcggtt tatgtgattg gaattgatac 300

gttaactgtg gataggtcga ttcctgtgga aggatatgat gttgcaatcg atcccaatgg 360

gttattcgcc tatgttctga attttggaat agtgcaaaaa gtggacctaa ctaccggtga 420

agtcacagga acaattgaac gagagcttat cgtatcaacc atagaaacaa attggccgga 480

gcggtatgcc tatgtattag aacaagaatt cttttttaat tatttgacgg ttattgattt 540

aaatacgttt accatcagca gcacccaaga gctggagtat gaaggggaat atcgaatgtt 600

tacgagtgga gcagaggtgt atttatatga cggcttcact ggcaatttat attctgtcag 660

tccaaatgga gcaggcgtta taggaaatgt tccgcaatca gcaacagact atgcgtttac 720

cccgaatggc gattttctgt atgcaactcg ttttatagag cagagcatca ttgtttacaa 780

cacagatgat tattctgagg aaactgtgat atctcttggg gtttcaccgg gtgccattac 840

gatttaaccg ctgcatgttg tgcatttcta aaaggacgtg aaatcatgaa aaagaatgat 900

ctttctgctt tacaggaaaa ttgtttctgt ttttgtgatg aggaaattag ccgggaagcg 960

ccgtttcaag tgccgataga tttccctgag ggcttcaagg ttgatacggc cgaagcgtca 1020

gcggctgtta catggagtac agacaatttg tcctgtatca gtgagccttg cttaatccag 1080

actgggccgg aaccagaaga tatcggtgtt cgatatgctg tgagagtaca agggaccatt 1140

acgcttcttg tcagtgtgtc gcctgtacga aatcaatatg gacaaggaga cggggctgtt 1200

tctgtgattc atactgaaga gattgatcaa gttgtttatt atgcagcaca gtctggccgc 1260

tgtcctgatt tcagtcaaat aacagtggaa gatctgctca ttgtaccgcc tttctacggc 1320

agtccgctta cacgggtgcg catgatcgta tggttcactg tccaccaacc aaaactgtgc 1380

tcagtaccgc caatatttct cccttggggg gtacaaagag gtgtccctag aagagatcca 1440

cgctgtgtaa aaattttaca aaaaggtatt gactttccct acagggtgtg taataattta 1500

attacaggcg ggggcaaccc cgctcagtac ctagagcgta aaagagggga gggaaacact 1560

agtatgaagc accttcctgc acaagatgaa caagtattca acgctattaa gaacgaacgc 1620

gaacgtcaac aaaccaagat tgaattgatc gctagcgaaa atttcgtttc agaagcagtt 1680

atggaggctc aaggctctgt tcttacgaac aaatatgctg aaggctaccc aggcaaaaga 1740

tactacggag gctgcgaaca cgttgacgta gtcgaagaca ttgcacgtga ccgcgcaaaa 1800

gaaatcttcg gcgcagaaca tgttaacgtt caacctcaca gcggagcaca agcgaacatg 1860

gcagtttatt ttacaatcct tgaacaaggg gacacagtcc tgggcatgaa cctttctcac 1920

ggaggccatc ttactcatgg tagcccggtg aacttttctg gcgtccaata taacttcgtt 1980

gagtacggtg tagataaaga aacacagtat attgactatg atgatgtgcg tgaaaaagct 2040

cttgcgcaca aacctaaact tattgttgcg ggagcgtcag cgtacccgcg taccattgat 2100

tttaaaaagt tccgggaaat tgctgacgag gttggcgcgt actttatggt ggatatggct 2160

catattgcgg gcttggtcgc agcaggactg caccctaacc cggtgcctta cgccgatttc 2220

gttacaacca cgacacataa aacacttaga ggcccacgtg gcggaatgat tctttgcaga 2280

gaagaatttg gaaagaaaat tgacaaatcc attttccctg gcatccaggg aggtcctctt 2340

atgcatgtca ttgcagcaaa agcggtgagc ttcggcgaag tgttacaaga tgatttcaaa 2400

acgtatgctc agaacgttat ctctaatgcg aaacgtttag cggaagcgtt gacaaaagaa 2460

ggtatccaac ttgtctcagg aggcacagat aatcatctta ttctggttga tcttcgctca 2520

cttggattaa caggcaaagt tgcggaacat gttcttgacg aaattggtat tacatcaaac 2580

aaaaacgcta ttccttacga tccggaaaaa cctttcgtaa catctggtat tcgcttaggc 2640

acagctgcgg ttacaagccg cggattcgat ggcgatgcgc ttgaagaggt aggcgctatt 2700

attgcactgg cgttaaaaaa tcatgaggat gaaggaaaac ttgaagaggc ccgccagcgt 2760

gtcgcagctc ttactgataa attcccgctt tacaaagaac ttgattattg aaaaagaggg 2820

gagggaaaca ttatgatgaa gtcttacatg actcaacgtt tagatgaata tcgtgacggc 2880

aacgaggata aaggacgcct cttggtgtct tgtccggacc agcctggaat tgtttctgca 2940

gtttccgcat ttctttttga gcacggcgca aatatcatcg aaagcaatca atacacaact 3000

gacccagaag gcggccgttt cttccttcgc attgaatttg actgcgcagg aatccgtgaa 3060

aaaaaaagca gcttacaggc tgctttcgca tctgtcgctg aaaaattcga catgacttgg 3120

tctttaactt tggcgtctga gctaaaacgt gttgctatct ttgtgtcaaa agagctccac 3180

tgcttacatg aacttatttg ggaatggcaa acaggtaacc taatggcaga gattgccgta 3240

gtcatctcaa accacgaaga agctcgcgaa ctggtggaac gtcttaatat cccttttcac 3300

tacatgaaag ctaacaaaga catccgggct gaagttgaaa aaaaacagct tgagctgtta 3360

gaacagtatg acgttgacgt aatcgtgtta gcacgctata tgcagatttt aacaccggac 3420

ttcgtctctg ctcatcctaa ccgtattatc aatattcacc attctttctt accggccttt 3480

atcggagcaa atccttataa acgtgcgtat gaacgtggag ttaaattgat cggcgccacg 3540

tcccactatg ttacgaacga cttagatgaa ggtcctatta tcgaacaaga tatcgagcgt 3600

gttgatcatc gtgataatgc tgaagcactc aaaaatattg gccgtacaat cgagcgctct 3660

gttctagctc gtgcagtaaa atggcatctt gaggaccgcg tcatcgttca tgagaataaa 3720

acaatcgttt ttaactgaaa aagaggggag ggaaacatta tgatgggatt acttgaagat 3780

cttcaacgcc aagttctgat cggagacgga gcgatgggaa cgcttctgta ttcttacgga 3840

atcgacagat gttttgaaga gttaaatatt tctaaacctg aagaaatcca aagaatccat 3900

aaagcgtacg tcgaagcagg agcaaacatc attcaaacaa atacatacgg agctaactac 3960

attaaactta gccgccacgg cctggaagac gacattaaaa aaatgaacca agaagcggtc 4020

aaaattgcac gcgcaagcgc tggcgacgcg tatgttcttg gcacgatggg aggcatccgc 4080

acattcaaca agaatgctta cagcctggac gagattaaac gctcattccg tgaacaatta 4140

tacctgcttc ttcacgaaga accggatggc cttcttttag aaacatatta cgatctggaa 4200

gaggcacgtg aggttcttaa aattgctcgc aaagagacgg atcttccgat catgcttaac 4260

gtctctatgc acgaacaagg cgtgcttcag gatggcacac ctcttagcga cgctttaaga 4320

agcatcgcgg acctgggcgc agatatcgtt ggcattaact gccgcctggg accgtaccac 4380

atgattgaag cattgtcaga agtcccgatc ttcgatgacg tcttccttag cgtttacccg 4440

aactcttcat taccgagctt agaagaagga cgcttagtgt atgaaacgga cgatacatac 4500

ttccaaaatt ctgcaagcga gttccgtaaa caaggagcgc gtattatcgg cgggtgctgc 4560

ggaacaactc cgaatcacat tagagcgatg gcagaagcag taggaggtct tgcacctatc 4620

acagaaaaag aagtgaaaac gcgtgctaaa gaatttattt ctgttcatca cgaacgtaca 4680

gaaccgggct tagacgaaat tgctgctaaa aagcgcagca ttattgtcga acttgatccg 4740

cctaaaaaac tttcatttga taaatttctt tcagcggcag cggagttaaa agaagcaggc 4800

atcgatgctc ttacattagc ggataactca ttagcgactc ctcgtatctc taacgtcgca 4860

tgcggtgcac ttgtgaaaca acaactggat atgcgctcac ttgttcatat cacgtgccgt 4920

gaccgcaata tcattggcct tcaatctcat ttaatgggtc ttgatacatt aggtttaaac 4980

gatgtacttg cgattacagg tgatccgtca aaaattggtg actttcctgg cgcaacatca 5040

gtgtacgacc tgacctcttt tgatcttatt cgtcttatta aacaattcaa tgaaggatta 5100

tctttgtctg gtaaacctct tggcaagaaa acgaacttct cagtagcggc ggcattcaat 5160

cctaatgtgc gtcaccttga caaagcggtc aaaagacttg aaaaaaaaat cgactgtgga 5220

gcggattatt tcgtctctca accagtatat agtgagcaac aattagtgga tatccacaac 5280

gaaacgaagc acttaaaaac accaatctac attggaatca tgcctcttac gtcctctcgc 5340

aacgctgaat ttatccataa cgaaattcca ggaattaaac tttctgatac gatccgtgaa 5400

aaaatggccc atgctggcga agacaaggag aaacaaaagg ctgaaggact tgcaattgcg 5460

cgttctttac tggacactgc ttgcgaattg ttcaacggca tctatcttat tacaccattt 5520

cttcgtagcg acttgacagc cgagttgaca tcatatattc aacaaaagga cgaacaacgc 5580

cagaatattt ttctgcatta aattaattct agagctccgg cataaaatta actaaaataa 5640

ttattatgta gataaaaaat ttagaagcca atgaaatcta taaataaact aaattaagtt 5700

tatttaatta acaactatgg atataaaata ggtactaatc aaaatagtga ggcgaatata 5760

tttgaataca tacgaacaaa tcaataaagt gaaaaaaata cttcggaaac atttaaaaaa 5820

taaccttatt ggtacttaca tgtttggatc aggagttgag agtggactca aaccaaatag 5880

tgatcttgac tttttagtcg tcgtatctga accattgaca gatcaaagta aagaaatact 5940

tatacaaaaa attagaccta tttcaaaaaa aataggagat aaaagcaact tacgatatat 6000

tgaattaaca attattattc agcaagaaat ggtaccgtgg aatcatcctc ccaaacaaga 6060

atttatttat ggagaatggt tacaagagct ttatgaacaa ggatacattc ctcagaagga 6120

attaaattca gatttaacca taatgcttta ccaagcaaaa cgaaaaaata aaagaatata 6180

cggaaattat gacttagagg aattactacc tgatattcca ttttctgatg tgagaagagc 6240

cattatggat tcgtcagagg aacttataga taattatcag gatgatgaaa ccaactctat 6300

attaacttta tgccgcatga ttttaactat ggacacgggt aaaatcatac caaaagatat 6360

tgcgggaaat gcagtggctg aatcttctcc attagaacat agggagagaa ttttgttagc 6420

agttcgtagt tatcttggag agaatattga atggactaat gaaaatgtaa atcttacgat 6480

aaactatctt aataaccgcc ttaagaaact ttaacccggg cgcgttgctt tcgatgaagt 6540

tttaccgcca tcaccagacc ctcaatcgta tgtgtttact gccaatactg gagacagcac 6600

ggtttcggtt attgatgcag atttaaacac tgttgtaaaa acaatccctt tttctgacgt 6660

gccaactaat ctaggcgtta ctttcgataa agcattcacg tatgtcctcc atggaaatac 6720

caaccttgtc tcagtgattg ataataaaac attaaccatc ataaatacca ttactgttgg 6780

aggagggccg agaaaaatcg aatttgatcc aacagatgag ttcgcatacg tgatggccgc 6840

cggatctata tatgtgatta acacggcctc tcaatcggtc attgatgtca ttccgatacc 6900

cggcgcttta gactttgctc ttgatccgaa tggacagtat gtctataccg ctaatggaag 6960

cagttggtcg gttgataaat atgatgtaaa tactgggcag ttggtagaaa gtatcataga 7020

tacatttgaa tttcccagtt taattacaac tccttacgct ggaaattttg cgtacgtatt 7080

gaatggtgag ctgtggccga aaggtgtaac ggagattagt ttgtcaccat taagcagggg 7140

aggggacttc agccgtttat ttgaaacgct gaggacgatt gtattttctc tcgatagtac 7200

tagagcttat tttcaagagc catatagtga accgtttctc atcaataact tatacgtagt 7260

taatacggcc agacagagga tcattgcaaa cgtgtctctc cccggcgcct ttgacctcgc 7320

cgtaacgccg gacaaacagt atatttatgc ggcacagccc aacgacaatg ctgtgaccgt 7380

gtaccgcaca agtgattata cagccgtgac ggtgattccg gttggcgcgg gtccgtctgc 7440

gattgcgatg tagctatttt atagatcaag agaagcggtg ctgtgataag agaatcgttt 7500

ataaataaat gatccgattt gctgaatggc agccaggata atcagaaaca gcagttcact 7560

tatagagaaa ctgagcatcc accaaatccc ggctgctgcc agtgctcccg caaacacttt 7620

tctgaacagg ctgccgtgcc ggaaaagctg ccgcaaatac gttcgcatgc aaaacaataa 7680

atacaggatc agtagcatcg caagtccca 7709

<210> 98
<211> 7709
<212> DNA
<213> Artificial Sequence

<220>
<223> MTHF-OP-B operon

<400> 98
cgcgtttaca gcgaatccaa atgatcaatc agtttctgtg attgatacaa acaccgatac 60

tgttgtgaca acgattgctc ttccgtacaa tccggcaggt attgaaatta cgccagataa 120

aagcgctgtg tttgtcttac atcccaacaa taatgtgatt tctgtcatcg attatgacac 180

attaacagtg acagcaacta tattgctgga tcagccgcct cgattgatta gatttatccc 240

taatcatgag tttgcttatg ttttcaccgg tactgcggtt tatgtgattg gaattgatac 300

gttaactgtg gataggtcga ttcctgtgga aggatatgat gttgcaatcg atcccaatgg 360

gttattcgcc tatgttctga attttggaat agtgcaaaaa gtggacctaa ctaccggtga 420

agtcacagga acaattgaac gagagcttat cgtatcaacc atagaaacaa attggccgga 480

gcggtatgcc tatgtattag aacaagaatt cttttttaat tatttgacgg ttattgattt 540

aaatacgttt accatcagca gcacccaaga gctggagtat gaaggggaat atcgaatgtt 600

tacgagtgga gcagaggtgt atttatatga cggcttcact ggcaatttat attctgtcag 660

tccaaatgga gcaggcgtta taggaaatgt tccgcaatca gcaacagact atgcgtttac 720

cccgaatggc gattttctgt atgcaactcg ttttatagag cagagcatca ttgtttacaa 780

cacagatgat tattctgagg aaactgtgat atctcttggg gtttcaccgg gtgccattac 840

gatttaaccg ctgcatgttg tgcatttcta aaaggacgtg aaatcatgaa aaagaatgat 900

ctttctgctt tacaggaaaa ttgtttctgt ttttgtgatg aggaaattag ccgggaagcg 960

ccgtttcaag tgccgataga tttccctgag ggcttcaagg ttgatacggc cgaagcgtca 1020

gcggctgtta catggagtac agacaatttg tcctgtatca gtgagccttg cttaatccag 1080

actgggccgg aaccagaaga tatcggtgtt cgatatgctg tgagagtaca agggaccatt 1140

acgcttcttg tcagtgtgtc gcctgtacga aatcaatatg gacaaggaga cggggctgtt 1200

tctgtgattc atactgaaga gattgatcaa gttgtttatt atgcagcaca gtctggccgc 1260

tgtcctgatt tcagtcaaat aacagtggaa gatctgctca ttgtaccgcc tttctacggc 1320

agtccgctta cacgggtgcg catgatcgta tggttcactg tccaccaacc aaaactgtgc 1380

tcagtaccgc caatatttct cccttggggg gtacaaagag gtgtccctag aagagatcca 1440

cgctgtgtaa aaattttaca aaaaggtatt gactttccct acagggtgtg taataattta 1500

attacaggcg ggggcaaccc cgctcagtac ctagagcgta aaagagggga gggaaacact 1560

agtatgaagc accttcctgc acaagatgaa caagtattca acgctattaa gaacgaacgc 1620

gaacgtcaac aaaccaagat tgaattgatc gctagcgaaa atttcgtttc agaagcagtt 1680

atggaggctc aaggctctgt tcttacgaac aaatatgctg aaggctaccc aggcaaaaga 1740

tactacggag gctgcgaaca cgttgacgta gtcgaagaca ttgcacgtga ccgcgcaaaa 1800

gaaatcttcg gcgcagaaca tgttaacgtt caacctcaca gcggagcaca agcgaacatg 1860

gcagtttatt ttacaatcct tgaacaaggg gacacagtcc tgggcatgaa cctttctcac 1920

ggaggccatc ttactcatgg tagcccggtg aacttttctg gcgtccaata taacttcgtt 1980

gagtacggtg tagataaaga aacacagtat attgactatg atgatgtgcg tgaaaaagct 2040

cttgcgcaca aacctaaact tattgttgcg ggagcgtcag cgtacccgcg taccattgat 2100

tttaaaaagt tccgggaaat tgctgacgag gttggcgcgt actttatggt ggatatggct 2160

catattgcgg gcttggtcgc agcaggactg caccctaacc cggtgcctta cgccgatttc 2220

gttacaacca cgacacataa aacacttaga ggcccacgtg gcggaatgat tctttgcaga 2280

gaagaatttg gaaagaaaat tgacaaatcc attttccctg gcatccaggg aggtcctctt 2340

atgcatgtca ttgcagcaaa agcggtgagc ttcggcgaag tgttacaaga tgatttcaaa 2400

acgtatgctc agaacgttat ctctaatgcg aaacgtttag cggaagcgtt gacaaaagaa 2460

ggtatccaac ttgtctcagg aggcacagat aatcatctta ttctggttga tcttcgctca 2520

cttggattaa caggcaaagt tgcggaacat gttcttgacg aaattggtat tacatcaaac 2580

aaaaacgcta ttccttacga tccggaaaaa cctttcgtaa catctggtat tcgcttaggc 2640

acagctgcgg ttacaagccg cggattcgat ggcgatgcgc ttgaagaggt aggcgctatt 2700

attgcactgg cgttaaaaaa tcatgaggat gaaggaaaac ttgaagaggc ccgccagcgt 2760

gtcgcagctc ttactgataa attcccgctt tacaaagaac ttgattattg aaaaagaggg 2820

gagggaaaca ttatgatgaa gtcttacatg actcaacgtt tagatgaata tcgtgacggc 2880

aacgaggata aaggacgcct cttggtgtct tgtccggacc agcctggaat tgtttctgca 2940

gtttccgcat ttctttttga gcacggcgca aatatcatcg aaagcaatca atacacaact 3000

gacccagaag gcggccgttt cttccttcgc attgaatttg actgcgcagg aatccgtgaa 3060

aaaaaaagca gcttacaggc tgctttcgca tctgtcgctg aaaaattcga catgacttgg 3120

tctttaactt tggcgtctga gctaaaacgt gttgctatct ttgtgtcaaa agagctccac 3180

tgcttacatg aacttatttg ggaatggcaa acaggtaacc taatggcaga gattgccgta 3240

gtcatctcaa accacgaaga agctcgcgaa ctggtggaac gtcttaatat cccttttcac 3300

tacatgaaag ctaacaaaga catccgggct gaagttgaaa aaaaacagct tgagctgtta 3360

gaacagtatg acgttgacgt aatcgtgtta gcacgctata tgcagatttt aacaccggac 3420

ttcgtctctg ctcatcctaa ccgtattatc aatattcacc attctttctt accggccttt 3480

atcggagcaa atccttataa acgtgcgtat gaacgtggag ttaaattgat cggcgccacg 3540

tcccactatg ttacgaacga cttagatgaa ggtcctatta tcgaacaaga tatcgagcgt 3600

gttgatcatc gtgataatgc tgaagcactc aaaaatattg gccgtacaat cgagcgctct 3660

gttctagctc gtgcagtaaa atggcatctt gaggaccgcg tcatcgttca tgagaataaa 3720

acaatcgttt ttaactgaaa aagaggggag ggaaacatta tgatgggatt acttgaagat 3780

cttcaacgcc aagttctgat cggagacgga gcgatgggaa cgcttctgta ttcttacgga 3840

atcgacagat gttttgaaga gttaaatatt tctaaacctg aagaaatcca aagaatccat 3900

aaagcgtacg tcgaagcagg agcaaacatc attcaaacaa atacatacgg agctaactac 3960

attaaactta gccgccacgg cctggaagac gacattaaaa aaatgaacca agaagcggtc 4020

aaaattgcac gcgcaagcgc tggcgacgcg tatgttcttg gcacgatggg aggcatccgc 4080

acattcaaca agaatgctta cagcctggac gagattaaac gctcattccg tgaacaatta 4140

tacctgcttc ttcacgaaga accggatggc cttcttttag aaacatatta cgatctggaa 4200

gaggcacgtg aggttcttaa aattgctcgc aaagagacgg atcttccgat catgcttaac 4260

gtctctatgc acgaacaagg cgtgcttcag gatggcacac ctcttagcga cgctttaaga 4320

agcatcgcgg acctgggcgc agatatcgtt ggcattaact gccgcctggg accgtaccac 4380

atgattgaag cattgtcaga agtcccgatc ttcgatgacg tcttccttag cgtttacccg 4440

aactcttcat taccgagctt agaagaagga cgcttagtgt atgaaacgga cgatacatac 4500

ttccaaaatt ctgcaagcga gttccgtaaa caaggagcgc gtattatcgg cgggtgctgc 4560

ggaacaactc cgaatcacat tagagcgatg gcagaagcag taggaggtct tgcacctatc 4620

acagaaaaag aagtgaaaac gcgtgctaaa gaatttattt ctgttcatca cgaacgtaca 4680

gaaccgggct tagacgaaat tgctgctaaa aagcgcagca ttattgtcga acttgatccg 4740

cctaaaaaac tttcatttga taaatttctt tcagcggcag cggagttaaa agaagcaggc 4800

atcgatgctc ttacattagc ggataactca ttagcgactc ctcgtatctc taacgtcgca 4860

tgcggtgcac ttgtgaaaca acaactggat atgcgctcac ttgttcatat cacgtgccgt 4920

gaccgcaata tcattggcct tcaatctcat ttaatgggtc ttgatacatt aggtttaaac 4980

gatgtacttg cgattacagg tgatccgtca aaaattggtg actttcctgg cgcaacatca 5040

gtgtacgacc tgacctcttt tgatcttatt cgtcttatta aacaattcaa tgaaggatta 5100

tctttgtctg gtaaacctct tggcaagaaa acgaacttct cagtagcggc ggcattcaat 5160

cctaatgtgc gtcaccttga caaagcggtc aaaagacttg aaaaaaaaat cgactgtgga 5220

gcggattatt tcgtctctca accagtatat agtgagcaac aattagtgga tatccacaac 5280

gaaacgaagc acttaaaaac accaatctac attggaatca tgcctcttac gtcctctcgc 5340

aacgctgaat ttatccataa cgaaattcca ggaattaaac tttctgatac gatccgtgaa 5400

aaaatggccc atgctggcga agacaaggag aaacaaaagg ctgaaggact tgcaattgcg 5460

cgttctttac tggacactgc ttgcgaattg ttcaacggca tctatcttat tacaccattt 5520

cttcgtagcg acttgacagc cgagttgaca tcatatattc aacaaaagga cgaacaacgc 5580

cagaatattt ttctgcatta aattaattct agagctccgg cataaaatta actaaaataa 5640

ttattatgta gataaaaaat ttagaagcca atgaaatcta taaataaact aaattaagtt 5700

tatttaatta acaactatgg atataaaata ggtactaatc aaaatagtga ggcgaatata 5760

tttgaataca tacgaacaaa tcaataaagt gaaaaaaata cttcggaaac atttaaaaaa 5820

taaccttatt ggtacttaca tgtttggatc aggagttgag agtggactca aaccaaatag 5880

tgatcttgac tttttagtcg tcgtatctga accattgaca gatcaaagta aagaaatact 5940

tatacaaaaa attagaccta tttcaaaaaa aataggagat aaaagcaact tacgatatat 6000

tgaattaaca attattattc agcaagaaat ggtaccgtgg aatcatcctc ccaaacaaga 6060

atttatttat ggagaatggt tacaagagct ttatgaacaa ggatacattc ctcagaagga 6120

attaaattca gatttaacca taatgcttta ccaagcaaaa cgaaaaaata aaagaatata 6180

cggaaattat gacttagagg aattactacc tgatattcca ttttctgatg tgagaagagc 6240

cattatggat tcgtcagagg aacttataga taattatcag gatgatgaaa ccaactctat 6300

attaacttta tgccgcatga ttttaactat ggacacgggt aaaatcatac caaaagatat 6360

tgcgggaaat gcagtggctg aatcttctcc attagaacat agggagagaa ttttgttagc 6420

agttcgtagt tatcttggag agaatattga atggactaat gaaaatgtaa atcttacgat 6480

aaactatctt aataaccgcc ttaagaaact ttaacccggg cgcgttgctt tcgatgaagt 6540

tttaccgcca tcaccagacc ctcaatcgta tgtgtttact gccaatactg gagacagcac 6600

ggtttcggtt attgatgcag atttaaacac tgttgtaaaa acaatccctt tttctgacgt 6660

gccaactaat ctaggcgtta ctttcgataa agcattcacg tatgtcctcc atggaaatac 6720

caaccttgtc tcagtgattg ataataaaac attaaccatc ataaatacca ttactgttgg 6780

aggagggccg agaaaaatcg aatttgatcc aacagatgag ttcgcatacg tgatggccgc 6840

cggatctata tatgtgatta acacggcctc tcaatcggtc attgatgtca ttccgatacc 6900

cggcgcttta gactttgctc ttgatccgaa tggacagtat gtctataccg ctaatggaag 6960

cagttggtcg gttgataaat atgatgtaaa tactgggcag ttggtagaaa gtatcataga 7020

tacatttgaa tttcccagtt taattacaac tccttacgct ggaaattttg cgtacgtatt 7080

gaatggtgag ctgtggccga aaggtgtaac ggagattagt ttgtcaccat taagcagggg 7140

aggggacttc agccgtttat ttgaaacgct gaggacgatt gtattttctc tcgatagtac 7200

tagagcttat tttcaagagc catatagtga accgtttctc atcaataact tatacgtagt 7260

taatacggcc agacagagga tcattgcaaa cgtgtctctc cccggcgcct ttgacctcgc 7320

cgtaacgccg gacaaacagt atatttatgc ggcacagccc aacgacaatg ctgtgaccgt 7380

gtaccgcaca agtgattata cagccgtgac ggtgattccg gttggcgcgg gtccgtctgc 7440

gattgcgatg tagctatttt atagatcaag agaagcggtg ctgtgataag agaatcgttt 7500

ataaataaat gatccgattt gctgaatggc agccaggata atcagaaaca gcagttcact 7560

tatagagaaa ctgagcatcc accaaatccc ggctgctgcc agtgctcccg caaacacttt 7620

tctgaacagg ctgccgtgcc ggaaaagctg ccgcaaatac gttcgcatgc aaaacaataa 7680

atacaggatc agtagcatcg caagtccca 7709

<210> 99
<211> 1260
<212> DNA
<213> Lactobacillus reuteri

<400> 99
gtgcggacct atgaacaaat taacgcgggc tttaatcgcc aaatgctggg aggacaacgg 60

gatcgggtaa aatttttgcg acgaattctt actcgtttag gaaaccctga ccagcgattt 120

aaaattatcc atattgcagg gacaaacgga aaaggctcga ctgggacaat gttagaacag 180

gggttacaaa atgctggcta ccgggtcggc tattttagca gtcctgcttt ggtagatgat 240

cgggagcaaa ttaaggttaa tgaccatttg attagtaaaa aagattttgc gatgacttac 300

caaaagatca ccgaacattt acccgctgat cttttacctg atgatattac catttttgag 360

tggtggaccc tgataatgct gcaatatttt gcagaccaaa aagtggattg ggcagtcatt 420

gaatgcggtt taggcggtca agatgatgca accaatatta tcagcgcgcc ttttattagt 480

gtgataacgc atattgccct tgaccataca cggattttgg gtccaacgat tgccaaaatt 540

gcccaggcta aagcaggaat tatcaagacc ggtactaaac aagtgttttt agcgccgcac 600

caagaaaaag acgctctcac aattattaga gaaaaggctc aacagcaaaa agtaggatta 660

acgcaagcag atgcccagtc aattgttgat ggaaaggcga tcctgaaagt taatcataaa 720

atttataaag ttccttttaa tttattaggg acatttcaaa gtgaaaattt gggcaccgtt 780

gtcagtgtat ttaattttct ttatcaacga agacttgtta cttcttggca gcctttatta 840

tcgacgcttg cgacagtgaa gattgctggt cgaatgcaga aaatcgctga tcatccccct 900

attattttgg acggggccca taacccagat gcagccaagc aacttaccaa aactatttct 960

aagcttcctc ataataaagt aattatggtt cttggctttc tcgctgataa aaatattagt 1020

caaatggtta aaatttatca gcaaatggca gatgagataa taattactac tcctgaccat 1080

ccaactcgcg cccttgatgc aagtgcgtta aaatcagttc tgcctcaagc gattattgcc 1140

aacaatcccc gacaagggtt agtggtggca aaaaagattg cggagccgaa tgatttaatt 1200

attgtaacgg gttcttttta tacgattaaa gatatcgagg ccaatttaga tgaaaaataa 1260

<210> 100
<211> 762
<212> PRT
<213> Bacillus subtilis

<400> 100

Met Thr Thr Ile Lys Thr Ser Asn Leu Gly Phe Pro Arg Ile Gly Leu
1               5                   10                  15

Asn Arg Glu Trp Lys Lys Ala Leu Glu Ala Tyr Trp Lys Gly Ser Thr
            20                  25                  30

Asp Lys Asp Thr Phe Leu Lys Gln Ile Asp Glu Leu Phe Leu Ser Ala
        35                  40                  45

Val Lys Thr Gln Ile Asp Gln Gln Ile Asp Val Val Pro Val Ser Asp
    50                  55                  60

Phe Thr Gln Tyr Asp His Val Leu Asp Thr Ala Val Ser Phe Asn Trp

65                  70                  75                  80

Ile Pro Lys Arg Phe Arg His Leu Thr Asp Ala Thr Asp Thr Tyr Phe
                85                  90                  95

Ala Ile Ala Arg Gly Ile Lys Asp Ala Val Ser Ser Glu Met Thr Lys
            100                 105                 110

Trp Phe Asn Thr Asn Tyr His Tyr Ile Val Pro Glu Tyr Asp Glu Ser
        115                 120                 125

Ile Glu Phe Arg Leu Thr Arg Asn Lys Gln Leu Glu Asp Tyr Arg Arg
    130                 135                 140

Ile Lys Gln Glu Tyr Gly Val Glu Thr Lys Pro Val Ile Val Gly Pro
145                 150                 155                 160

Tyr Thr Phe Val Thr Leu Ala Lys Gly Tyr Glu Pro Ser Glu Ala Lys
                165                 170                 175

Ala Ile Gln Lys Arg Leu Val Pro Leu Tyr Val Gln Leu Leu Lys Glu
            180                 185                 190

Leu Glu Glu Glu Gly Val Lys Trp Val Gln Ile Asp Glu Pro Ala Leu
        195                 200                 205

Val Thr Ala Ser Ser Glu Asp Val Arg Gly Ala Lys Glu Leu Phe Glu
    210                 215                 220

Ser Ile Thr Ser Glu Leu Ser Ser Leu Asn Val Leu Leu Gln Thr Tyr
225                 230                 235                 240

Phe Asp Ser Val Asp Ala Tyr Glu Glu Leu Ile Ser Tyr Pro Val Gln
                245                 250                 255

Gly Ile Gly Leu Asp Phe Val His Asp Lys Gly Arg Asn Leu Glu Gln
            260                 265                 270

Leu Lys Thr His Gly Phe Pro Thr Asp Lys Val Leu Ala Ala Gly Val
        275                 280                 285

Ile Asp Gly Arg Asn Ile Trp Lys Ala Asp Leu Glu Glu Arg Leu Asp
    290                 295                 300

Ala Val Leu Asp Ile Leu Ser Ile Ala Lys Val Asp Glu Leu Trp Ile
305                 310                 315                 320

Gln Pro Ser Ser Ser Leu Leu His Val Pro Val Ala Lys His Pro Asp
                325                 330                 335

Glu His Leu Glu Lys Asp Leu Leu Asn Gly Leu Ser Tyr Ala Lys Glu
            340                 345                 350

Lys Leu Ala Glu Leu Thr Ala Leu Lys Glu Gly Leu Val Ser Gly Lys
        355                 360                 365

Ala Ala Ile Ser Glu Glu Ile Gln Gln Ala Lys Ala Asp Ile Gln Ala
    370                 375                 380

Leu Lys Gln Phe Ala Thr Gly Ala Asn Ser Glu Gln Lys Lys Glu Leu
385                 390                 395                 400

Glu Gln Leu Thr Asp Lys Asp Phe Lys Arg Pro Ile Pro Phe Glu Glu
                405                 410                 415

Arg Leu Ala Leu Gln Asn Glu Ser Leu Gly Leu Pro Leu Leu Pro Thr
            420                 425                 430

Thr Thr Ile Gly Ser Phe Pro Gln Ser Ala Glu Val Arg Ser Ala Arg
        435                 440                 445

Gln Lys Trp Arg Lys Ala Glu Trp Ser Asp Glu Gln Tyr Gln Asn Phe
    450                 455                 460

Ile Asn Ala Glu Thr Lys Arg Trp Ile Asp Ile Gln Glu Glu Leu Glu
465                 470                 475                 480

Leu Asp Val Leu Val His Gly Glu Phe Glu Arg Thr Asp Met Val Glu
                485                 490                 495

Tyr Phe Gly Glu Lys Leu Ala Gly Phe Ala Phe Thr Lys Tyr Ala Trp
            500                 505                 510

Val Gln Ser Tyr Gly Ser Arg Cys Val Arg Pro Pro Val Ile Tyr Gly
        515                 520                 525

Asp Val Glu Phe Ile Glu Pro Met Thr Val Lys Asp Thr Val Tyr Ala
    530                 535                 540

Gln Ser Leu Thr Ser Lys His Val Lys Gly Met Leu Thr Gly Pro Val
545                 550                 555                 560

Thr Ile Leu Asn Trp Ser Phe Pro Arg Asn Asp Ile Ser Arg Lys Glu
                565                 570                 575

Ile Ala Phe Gln Ile Gly Leu Ala Leu Arg Lys Glu Val Lys Ala Leu
            580                 585                 590

Glu Asp Ala Gly Ile Gln Ile Ile Gln Val Asp Glu Pro Ala Leu Arg
        595                 600                 605

Glu Gly Leu Pro Leu Lys Thr Arg Asp Trp Asp Glu Tyr Leu Thr Trp
    610                 615                 620

Ala Ala Glu Ala Phe Arg Leu Thr Thr Ser Ser Val Lys Asn Glu Thr
625                 630                 635                 640

Gln Ile His Thr His Met Cys Tyr Ser Asn Phe Glu Asp Ile Val Asp
                645                 650                 655

Thr Ile Asn Asp Leu Asp Ala Asp Val Ile Thr Ile Glu His Ser Arg
            660                 665                 670

Ser His Gly Gly Phe Leu Asp Tyr Leu Lys Asn His Pro Tyr Leu Lys
        675                 680                 685

Gly Leu Gly Leu Gly Val Tyr Asp Ile His Ser Pro Arg Val Pro Ser
    690                 695                 700

Thr Glu Glu Met Tyr Asn Ile Ile Val Asp Ala Leu Ala Val Cys Pro
705                 710                 715                 720

Thr Asp Arg Phe Trp Val Asn Pro Asp Cys Gly Leu Lys Thr Arg Gln
                725                 730                 735

Gln Glu Glu Thr Val Ala Ala Leu Lys Asn Met Val Glu Ala Ala Lys
            740                 745                 750

Gln Ala Arg Ala Gln Gln Thr Gln Leu Val
        755                 760

<210>  101
<211>  2289
<212>  DNA
<213>  Bacillus subtilis

<400>  101
atgacaacca tcaaaacatc gaatttagga tttccgagaa tcggactgaa ccgggaatgg      60

aaaaaagcac ttgaagcgta ttggaaaggc agtactgata aagatacgtt tttgaagcaa     120

atcgacgaac tatttttatc cgcagtaaaa acacaaattg accagcagat tgatgttgtg     180

cctgtttctg atttcacaca gtatgaccat gtactcgaca cagcagtcag cttcaactgg     240

atcccgaaac ggttcagaca tttgactgac gctaccgata catacttcgc tatcgcccgc 300

ggaatcaaag acgctgtatc tagtgaaatg acaaaatggt ttaatacaaa ttaccattac 360

atcgttccgg aatatgacga gagcattgag ttccgtctga caagaaacaa acaactcgaa 420

gattatcgcc ggatcaaaca ggaatacggt gtggaaacaa agcctgtgat tgtcggccct 480

tatacgttcg ttacgcttgc taaaggctat gaaccgtctg aagcaaaagc gatccaaaaa 540

cgccttgtgc cattatatgt acagcttttg aaagagcttg aagaagaagg cgtaaaatgg 600

gttcaaatcg atgagccggc gctcgttacc gcctctagtg aagatgtacg cggcgcaaaa 660

gaattatttg aaagcattac aagtgagctt tcatccttga atgtgctttt gcagacgtat 720

tttgattctg ttgatgctta tgaagagctg atctcttacc cggttcaggg aattggcctt 780

gatttcgttc acgacaaagg cagaaacctg gaacagctta aaacacatgg cttcccgaca 840

gataaagtgc tggcagccgg cgttatcgac ggacgcaaca tttggaaagc ggaccttgaa 900

gagcgtctcg atgccgttct tgatattctc agcattgcaa aagttgatga actgtggatt 960

cagccttcca gcagcctgct gcatgttcca gtagcgaaac accctgatga gcatttggaa 1020

aaagacctat tgaacggatt atcctacgca aaagaaaagc tggccgagct gacagctttg 1080

aaagaaggct tagtatcagg aaaagcggcg atcagcgaag agattcagca ggctaaggct 1140

gatatccagg cgcttaaaca gtttgcaaca ggcgccaatt ctgaacaaaa gaaagagctt 1200

gagcaattaa ctgataaaga cttcaagcgc ccgattccgt ttgaagaacg tttagcccta 1260

caaaatgaat ctctcggcct tccgcttttg ccgacgacaa cgatcggcag cttcccgcag 1320

tctgctgaag tgcggagcgc acgccaaaaa tggcggaaag ctgagtggtc cgatgaacag 1380

tatcaaaact ttatcaatgc ggaaacaaaa agatggattg atattcagga agaattggag 1440

cttgatgtcc ttgttcacgg cgaatttgaa cggacagaca tggtcgaata cttcggtgaa 1500

aagctggccg gtttcgcctt cactaaatat gcctgggttc aatcatacgg ctcacgctgt 1560

gtccgcccgc cagtcattta cggagatgtt gaatttattg aaccgatgac agtgaaagac 1620

acagtctacg cacagtcatt gacttccaag catgtgaaag gaatgctgac gggcccggtt 1680

acaatcttaa actggtcttt ccctcgaaac gacatctcga ggaaagaaat cgccttccaa 1740

atcgggcttg cccttcgcaa agaagttaaa gcgcttgaag acgcaggcat tcaaatcatt 1800

caagtcgatg aaccagcgct gcgtgaaggc cttccattga aaacccgcga ttgggatgag 1860

tatttgactt gggcggcaga agctttcaga ttaaccactt cttccgtgaa aaacgagaca 1920

caaattcata cacatatgtg ctacagcaac ttcgaagata tcgttgatac aatcaatgat 1980

cttgatgccg atgtgattac aatcgaacat agcagaagcc acggaggatt tttagattac 2040

ttaaaaaacc acccgtattt gaaaggcctt ggccttggtg tatatgacat tcacagccct 2100

cgtgtgccgt caactgaaga aatgtacaat attatcgttg atgcgcttgc cgtctgtccg 2160

actgaccgct tctgggtaaa tccagactgc ggattgaaaa caagacagca ggaagaaacg 2220

gttgcagcat tgaaaaatat ggttgaagcc gcaaaacagg caagagcaca gcagacacag 2280

ctagtataa 2289

## Description

### Technical field

The invention relates to the field of biotechnology engineering, in particular to a 5-methylfolate, such as 5-methyl-tetrahydrofolate (5-methyl-THF), producing microorganism and to the preparation and use thereof. More specifically, the present invention provides a 5-methylfolate producing microorganism, such as a 5-methylfolate producing bacterium, which a) has been modified to have an increased expression level of at least one enzyme (such as at least two, at least three, at least four, at least five, at least six, at least seven or at least eight enzymes) involved in the biosynthesis of a 5-methylfolate compared to an otherwise identical microorganism that does not carry said modification (reference microorganism); b) has been (further) modified to have a decreased expression and/or activity of an endogenous polypeptide having 5-methyltetrahydropteroyltriglutamate-homocysteine S-methyltransferase activity compared to an otherwise identical microorganism that does not carry said modification (reference microorganism); c) has been (further) modified to have a decreased expression and/or activity of an endogenous polypeptide having both dihydrofolate synthase activity and folylpolyglutamate synthetase activity compared to an otherwise identical microorganism that does not carry said modification (reference microorganism); and/or d) has been (further) modified to express a heterologous polypeptide having only dihydrofolate synthase activity.

### Background

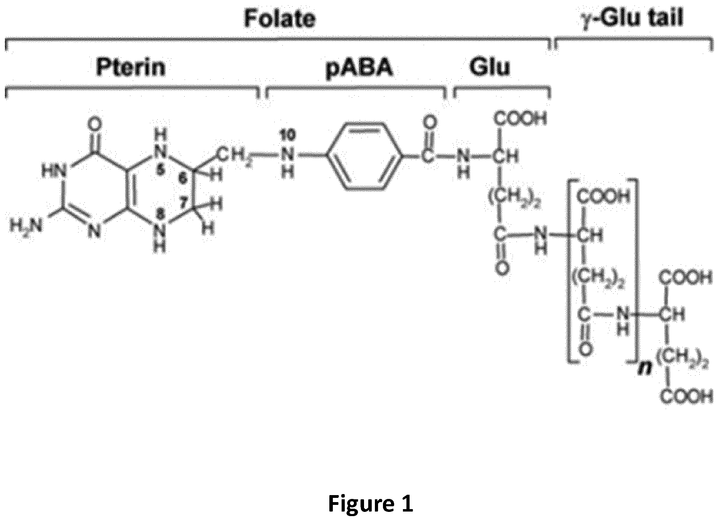

Folate is a general term for folic acid and a number of its derivatives; they differ in the state of oxidation, one-carbon substitution of the pteridine ring and in the number of γ -linked glutamate residues (shown in Fig. 1). The pteridine moiety of folates can exist in three oxidation states: fully oxidized (folic acid), or as the reduced 7,8-dihydrofolate (DHF), or 5,6,7,8-tetrahydrofolate (THF). THF is the co-enzymatically active form of the vitamin that accepts, transfers, and donates C1 groups, which are attached either at the N5 or N10 position or by bridging these positions. The C1 groups also differ in their oxidation state, with folates existing as derivatives of formate (5-formyl-THF (5-FTHF or folinic acid), 10-formyl-THF, 5,10-methenyl-THF, and 5-forminino-THF), methanol (5-methyl-THF; 5-MTHF) or formaldehyde (5,10-methylene-THF). In addition, most naturally occurring folates exist as γ -linked polyglutamate conjugates.

Folic acid (pteroyl-L-glutamic acid) is a synthetic compound, which does not exist in nature. Folic acid is not active as a coenzyme and has to undergo several metabolic steps within the cell to be converted into the metabolically active THF form. However, folic acid is the commercially most important folate compound, produced industrially by chemical synthesis. Mammals cannot synthesize folates and depend on dietary supplementation to maintain normal levels of folates. Low folate status may be caused by low dietary intake, poor absorption of ingested folate and alteration of folate metabolism due to genetic defects or drug interactions. Most countries have established recommended intakes of folate through folic acid supplements or fortified foods. Folates used in diet supplementation include folic acid, folinic acid (5-FTHF, Leucovorin) or 5-MTHF (Scaglione and Panzavolta 2014). Two salt forms of 5-MTHF are currently produced as supplements. Merck Millipore produces Metafolin^{®}, a calcium salt of 5-MTHF, which is a stable crystalline form of the naturally-occurring predominant form of folate. Gnosis S.p.A. developed and patented a glucosamine salt of (6S)-5-MTHF, brand named Quatrefolic^{®}.

Currently, folic acid is industrially primarily produced through chemical synthesis while, unlike other vitamins, microbial production of folic acid on industrial scale is not exploited due to the low yields of folic acid produced by current bacterial strains (Rossi et al., 2016). Although chemically produced folic acid is not a naturally occurring molecule human beings are able to metabolize it into biological active forms of folates by the action of the enzyme dihydrofolate reductase (DHFR). Several reasons support the replacement of chemical synthesis methods by microbial fermentation for commercial production of folates: first, reduced forms of folic acid can be produced by microorganisms, which can be used by humans more efficiently. Most importantly, a single step fermentation process can in principle be much more efficient and environmentally friendly than a multi-stage chemical process.

Previous studies have been done to elucidate folate/folic acid production in microorganisms. Most of microbial application for the production of folates is limited to the fortification of fermented dairy products and to folate-producing probiotics. The optimization of the culture conditions to improve the synthesis of folates have been also carried out, reaching folic acid yields of about 150 µg/g (Hjortmo et al., 2008; Sybesma et al., 2003b). A few studies have described genetically modified strains either of lactic acid bacteria (Sybesma et al., 2003a), yeasts (Walkey et al., 2015) or filamentous fungus (Serrano-Amatriain et al. 2016), which are able to produce folic acid with titers of up to 6,6 mg/L. Another successfully used approach for microbial production of folates is cultivation of yeast or bacterial strains in the presence of *para*-aminobenzoic acid (pABA). Total folate content of up to 22 mg/L were measured in supernatants of these cultures.

(6S)-5-methyltetrahydrofolate (L-5-methyltetrahydrofolate or L-5-methyl-THF) is an active form of folic acid (vitamin B9). Folic acid (pteroyl-L-glutamic acid) is a synthetic compound, produced industrially by chemical synthesis and is not found in fresh natural foods. Folic acid is not active as a coenzyme and has to undergo several metabolic steps within the cell to be converted into the metabolically active folate form. On the other hand, 5-methyl tetrahydrofolate is the predominant form of dietary folate and also predominant active form of folate in the human body, which accounts for approximately 98% of folates in human plasma. Intake of L-5-methyl-THF may have several advantages over intake of folic acid, such as reduced potential for masking the haematological symptoms of vitamin B12 deficiency, reduced interference with drugs that targets dihydrofolate reductase and L-5-methyl-THF does not accumulates in human plasma as unmetabolized vitamin.

The industrial technology for chemical synthesis of folic acid is hampered by a huge environmental burden. So far, microbial fermentation processes to produce folic acid or any of the natural forms of folates, such as 5-methylfolate, have not been competitive due to very low production titers/yields of folates.

Therefore, there is an urgent need to develop new genetically engineering microorganisms for enhancing the production capacity of 5-methylfolate, such as 5-methyl -tetrahydrofolate or a precursor or an intermediate thereof.

### Summary of the Invention

The object of the present invention is to provide genetically engineered microorganisms for enhancing the production capacity of 5-methylfolate (such as 5-methyl-tetrahydrofolate) or a precursor or an intermediate thereof. This object is solved by the present inventors.

The present invention may be summarized by way of the following items.
1. A genetically engineered microorganism, such as bacterium.
2. The genetically engineered microorganism according to item 1, which has been modified to have a decreased expression and/or activity of an endogenous polypeptide having both dihydrofolate synthase activity and folylpolyglutamate synthetase activity compared to an otherwise identical microorganism that does not carry said modification (reference microorganism).
3. The genetically engineered microorganism according to item 2, which has been modified to have a decreased expression level of the endogenous gene encoding said polypeptide having both dihydrofolate synthase activity and folylpolyglutamate synthetase activity compared to an otherwise identical microorganism that does not carry said modification (reference microorganism).
4. The genetically engineered microorganism according to item 3, wherein the expression level of the endogenous gene is decreased by at least 50%, such as by at least 60%, at least 65%, at least 70%, at least 75%, at least 80%, at least 85%, at least 90%, at least 95% or at least 100% compared to the otherwise identical microorganism.
5. The genetically engineered microorganism according to item 3 or 4, wherein the endogenous gene encoding said polypeptide having both dihydrofolate synthase activity and folylpolyglutamate synthetase activity has been inactivated.
6. The genetically engineered microorganism according to item 5, wherein the endogenous gene encoding said polypeptide having both dihydrofolate synthase activity and folylpolyglutamate synthetase activity has been inactivated by deletion of part of or the entire gene sequence.
7. The genetically engineered microorganism according to item 4 or 5, wherein the endogenous gene encoding said polypeptide having both dihydrofolate synthase activity and folylpolyglutamate synthetase activity has been inactivated by introducing or expressing in the microorganism a rare-cutting endonuclease able to selectively inactivating by DNA cleavage the endogenous gene encoding said polypeptide.
8. The genetically engineered microorganism according to item 7, wherein said rare-cutting endonuclease is a transcription activator-like effector (TALE) nuclease, meganuclease, zing-finger nuclease (ZFN), or RNA-guided endonuclease.
9. The genetically engineered microorganism according to item 8, wherein the RNA-guided endonuclease is a catalytically inactive Cas9 protein.
10. The genetically engineered microorganism according to item 13, which comprises (e.g., expresses) a single guide RNA (sgRNA) specifically hybridizing (e.g. binding) under cellular conditions with the genomic DNA encoding said polypeptide.
11. The genetically engineered microorganism according to item 2, wherein the expression of said endogenous polypeptide having both dihydrofolate synthase activity and folylpolyglutamate synthetase activity is decreased (e.g., inhibited) by transcriptional and/or translational repression of the endogenous gene encoding said polypeptide.
12. The genetically engineered microorganism according to item 2, wherein the expression of said endogenous polypeptide having both dihydrofolate synthase activity and folylpolyglutamate synthetase activity is decreased (e.g. inhibited) by introducing or expressing in the microorganism an inhibitory nucleic acid molecule that specifically hybridizes (e.g. binds) under cellular conditions with cellular mRNA and/or genomic DNA encoding said polypeptide.
13. The genetically engineered microorganism according to item 12, wherein the inhibitory nucleic acid molecule is an antisense oligonucleotide, ribozyme or interfering RNA (RNAi) molecule.
14. The genetically engineered microorganism according to item 13, wherein the interfering RNA molecule is a micro RNA (miRNA), small interfering RNA (siRNA) or short hairpin RNA (shRNA).
15. The genetically engineered microorganism according to item 2 or 3, which has been modified to have a decreased activity of an endogenous polypeptide having both dihydrofolate synthase activity and folylpolyglutamate synthetase activity compared to an otherwise identical microorganism that does not carry said modification (reference microorganism).
16. The genetically engineered microorganism according to item 15, wherein the activity of said polypeptide is decreased by at least one active-site mutation resulting in the reduction or loss of activity.
17. The genetically engineered microorganism according to item 16, wherein the at least one active-site mutation is at a position which corresponds to any one of positions 51-54, 75, 114-117, 145, 152-154, 172, 263, 302 and 315 in the amino acid sequence set forth in SEQ ID NO: 11.
18. The genetically engineered microorganism according to item 16 or 17, wherein the at least one active-site mutation is a non-conservative amino acid substitution.
19. The genetically engineered microorganism according to any one of items 2 to 18, wherein the endogenous gene encoding said polypeptide having both dihydrofolate synthase activity and folylpolyglutamate synthetase activity is the gene *folC.*
20. The genetically engineered microorganism according to any one of items 2 to 18, wherein the endogenous gene encoding said polypeptide having both dihydrofolate synthase activity and folylpolyglutamate synthetase activity is the endogenous gene *folC.*
21. The genetically engineered microorganism according to any one of items 2 to 20, wherein the endogenous gene encoding said polypeptide having both dihydrofolate synthase activity and folylpolyglutamate synthetase activity comprises a nucleic acid sequence having at least 70%, such as at least 80%, at least 85%, at least 90%, at least 93%, at least 95%, at least 98% or at least 99%, sequence identity with the nucleic acid sequence set forth in SEQ ID NO: 5
22. The genetically engineered microorganism according to any one of items 2 to 21, wherein the polypeptide having both dihydrofolate synthase activity and folylpolyglutamate synthetase activity encoded by the endogenous gene comprises an amino acid which has at least 70%, such as at least 80%, at least 85%, at least 90%, at least 93%, at least 95%, at least 98% or at least 99%, sequence identity with the amino acid sequence set forth in SEQ ID NO: 11
23. The genetically engineered microorganism according to any one of items 1 to 22, which has been (further) modified to express a heterologous polypeptide having only dihydrofolate synthase activity.
24. The genetically engineered microorganism according to item 23, wherein the heterologous polypeptide having only dihydrofolate synthase activity is derived from a bacterium or fungus, preferably selected from *Lactobacillus reuteri* and *Ashbya gossypii.*
24. The genetically engineered microorganism according to item 23 or 24, wherein the heterologous polypeptide having only dihydrofolate synthase activity comprises an amino acid sequence having at least 70%, such as at least 80%, at least 85%, at least 90%, at least 93%, at least 95%, at least 98% or at least 99%, sequence identity with SEQ ID NO: 22 or 23.
25. The genetically engineered microorganism according to any one of items 1 to 24, which has been (further) modified to have a significantly improved production capacity of a 5-methylfolate (such as 5-methyl-tetrahydrofolate) or a precursor or an intermediate thereof compared to an otherwise identical microorganism that does not carry said modification (reference microorganism).
26. The genetically engineered microorganism according to item 25, wherein the production capacity of a 5-methylfolate (such as 5-methyl-tetrahydrofolate) or a precursor or an intermediate thereof is increased by at least 50%, such as at least 100%, at least 200%, at least 500%, at least 1000%, at least 2000%, at least 5000%, at least 10000%, at least 20000% or at least 50000%, compared to an otherwise identical microorganism that does not carry said modification (reference microorganism).
27. The genetically engineered microorganism according to any one of items 1 to 26, which has been (further) modified to have an increased expression level of at least one gene (such as at least two, at least three, at least four, at least five, at least six, at least seven or at least eight genes) encoding an enzyme involved in the biosynthesis of a 5-methylfolate (such as 5-methyl-tetrahydrofolate) compared to an otherwise identical microorganism that does not carry said modification (reference microorganism).
28. The genetically engineered microorganism according to any one of items 1 to 27, which has been (further) modified to have an increased expression level of at least two genes (such as at least three, at least four, at least five, at least six, at least seven or at least eight genes) encoding enzymes involved in the biosynthesis of a 5-methylfolate (such as 5-methyl-tetrahydrofolate) compared to an otherwise identical microorganism that does not carry said modification (reference microorganism).
29. The genetically engineered microorganism according to any one of items 1 to 28, which has been (further) modified to have an increased expression level of at least three genes (such as at least four, at least five, at least six, at least seven or at least eight genes) encoding enzymes involved in the biosynthesis of a 5-methylfolate compared to an otherwise identical microorganism that does not carry said modification (reference microorganism).
30. The genetically engineered microorganism according to any one of items 1 to 29, which has been (further) modified to have an increased expression level of at least four genes (such as at least five, at least six, at least seven or at least eight genes) encoding enzymes involved in the biosynthesis of a 5-methylfolate (such as 5-methyl-tetrahydrofolate) compared to an otherwise identical microorganism that does not carry said modification (reference microorganism).
31. The genetically engineered microorganism according to any one of items 1 to 30, which has been (further) modified to have an increased expression level of at least five genes (such as at least six genes; at least seven genes or at least eight genes) encoding enzymes involved in the biosynthesis of a 5-methylfolate (such as 5-methyl-tetrahydrofolate) compared to an otherwise identical microorganism that does not carry said modification (reference microorganism).
32. The genetically engineered microorganism according to any one of items 1 to 31, which has been (further) modified to have an increased expression level of at least six genes (such as at least seven genes or at least eight genes) encoding enzymes involved in the biosynthesis of a 5-methylfolate (such as 5-methyl-tetrahydrofolate) compared to an otherwise identical microorganism that does not carry said modification (reference microorganism).
33. The genetically engineered microorganism according to any one of items 1 to 32, which has been (further) modified to have an increased expression level of at least seven genes (such as at least eight genes) encoding enzymes involved in the biosynthesis of a 5-methylfolate (such as 5-methyl-tetrahydrofolate) compared to an otherwise identical microorganism that does not carry said modification (reference microorganism).
34. The genetically engineered microorganism according to any one of items 27 to 33, wherein the at least one gene encoding an enzyme involved in the biosynthesis of a 5-methylfolate (such as 5-methyl-tetrahydrofolate) is selected from the group consisting of *folE*/*mtrA, folB*, *folK*, *folP*/*sul*, *folA*/*dfrA*, *glyA, purU, yitJ and metF.*
35. The genetically engineered microorganism according to any one of items 27 to 34, wherein the enzyme involved in the biosynthesis of a 5-methylfolate, such as 5-methyl-tetrahydrofolate, is selected from the group consisting of: a polypeptide having GTP cyclohydrolase activity, a polypeptide having 7,8-dihydroneopterin aldolase activity, a polypeptide having 2-amino-4-hydroxy-6-hydroxymethyldihydropteridine pyrophosphokinase activity, a polypeptide having dihydropteroate synthase activity, a polypeptide having dihydrofolate reductase activity, a polypeptide having serine hydroxymethyltransferase activity, a polypeptide having formyltetrahydrofolate deformylase activity, and a polypeptide having 5,10-methylenetetrahydrofolate reductase activity.
36. The genetically engineered microorganism according to any one of items 1 to 34, which has been (further) modified to have an increased expression level of a gene encoding a polypeptide having GTP cyclohydrolase activity compared to an otherwise identical microorganism that does not carry said modification (reference microorganism).
37. The genetically engineered microorganism according to any one of items 1 to 36, which has been (further) modified to have an increased expression level of a gene encoding a polypeptide having 7,8-dihydroneopterin aldolase activity compared to an otherwise identical microorganism that does not carry said modification (reference microorganism).
38. The genetically engineered microorganism according to any one of items 1 to 37, which has been (further) modified to have an increased expression level of a gene encoding a polypeptide having 2-amino-4-hydroxy-6-hydroxymethyldihydropteridine pyrophosphokinase activity compared to an otherwise identical microorganism that does not carry said modification (reference microorganism).
39. The genetically engineered microorganism according to any one of items 1 to 38, which has been (further) modified to have an increased expression level of a gene encoding a polypeptide having dihydropteroate synthase activity compared to an otherwise identical microorganism that does not carry said modification (reference microorganism).
40. The genetically engineered microorganism according to any one of items 1 to 39, which has been (further) modified to have an increased expression level of a gene encoding a polypeptide having dihydrofolate reductase activity compared to an otherwise identical microorganism that does not carry said modification (reference microorganism).
41. The genetically engineered microorganism according to any one of items 1 to 40, which has been (further) modified to have an increased expression level of a gene encoding a polypeptide having serine hydroxymethyltransferase activity compared to an otherwise identical microorganism that does not carry said modification (reference microorganism).
42. The genetically engineered microorganism according to any one of items 1 to 41, which has been (further) modified to have an increased expression level of a gene encoding a polypeptide having formyltetrahydrofolate deformylase activity compared to an otherwise identical microorganism that does not carry said modification (reference microorganism).
43. The genetically engineered microorganism according to any one of items 1 to 42, which has been (further) modified to have an increased expression level of a gene encoding a polypeptide having 5,10-methylenetetrahydrofolate reductase activity compared to an otherwise identical microorganism that does not carry said modification (reference microorganism).
44. The genetically engineered microorganism according to any one of items 27 to 43, wherein the at least one gene encoding an enzyme involved in the biosynthesis of a 5-methylfolate (such as 5-methyl-tetrahydrofolate) is heterologous to the genetically engineered microorganism.
45. The genetically engineered microorganism according to any one of items 27 to 44, wherein the at least one gene encoding an enzyme involved in the biosynthesis of a 5-methylfolate is derived from a bacterium or fungus, preferably selected from the genus *Bacillus, Escherichia, Lactococcus* , *Shewanella, Vibrio* and *Ashbya.*
46. The genetically engineered microorganism according to any one of items 27 to 45, wherein the at least one gene encoding an enzyme involved in the biosynthesis of a 5-methylfolate (such as 5-methyl-tetrahydrofolate) is derived from a bacterium or fungus selected from *Bacillus subtiltis*, *Lactobacillus lactis*, *Escherichia coli*, *Shewanella violacea, Vibrio natriegens* or *Ashbya gossypii.*
47. The genetically engineered microorganism according to any one of items 27 to 46, wherein the expression level of at least one gene (such as at least two, at least three, at least four, at least five, at least six, at least seven or at least eight genes) encoding an enzyme involved in the biosynthesis of a 5-methylfolate (such as 5-methyl-tetrahydrofolate) is increased by at least 50%, at least 100, at least 200%, at least 500%, at least 1000%, at least 2000%, at least 5000%, at least 10000%, at least 20000% or at least 50000%, compared to the otherwise identical microorganism (reference microorganism).
48. The genetically engineered microorganism according to any one of items 1 to 47, which has been further modified to have an increased expression level of at least one enzyme (such as at least two, at least three, at least four, at least five, at least six, at least seven or at least eight) enzymes involved in the biosynthesis of a 5-methylfolate (such as 5-methyl-tetrahydrofolate) compared to an otherwise identical microorganism that does not carry said modification (reference microorganism).
49. The genetically engineered microorganism according to any one of items 1 to 47, which has been further modified to have an increased expression level of at least two (such as at least three, at least four, at least five, at least six, at least seven or at least eight) enzymes involved in the biosynthesis of a 5-methylfolate (such as 5-methyl-tetrahydrofolate), compared to an otherwise identical microorganism that does not carry said modification (reference microorganism).
50. The genetically engineered microorganism according to any one of items 1 to 47, which has been further modified to have an increased expression level of at least three (such as at least four, at least five, at least six, at least seven or at least eight) enzymes involved in the biosynthesis of a 5-methylfolate (such as 5-methyl-tetrahydrofolate) compared to an otherwise identical microorganism that does not carry said modification (reference microorganism).
51. The genetically engineered microorganism according to any one of items 1 to 47, which has been further modified to have an increased expression level of at least four (such as at least five, at least six, at least seven or at least eight) enzymes involved in the biosynthesis of a 5-methylfolate (such as 5-methyl-tetrahydrofolate) compared to an otherwise identical microorganism that does not carry said modification (reference microorganism).
52. The genetically engineered microorganism according to any one of items 1 to 47, which has been further modified to have an increased expression level of at least five (such as at least six, at least seven or at least eight) enzymes involved in the biosynthesis of a 5-methylfolate (such as 5-methyl-tetrahydrofolate) compared to an otherwise identical microorganism that does not carry said modification (reference microorganism).
53. The genetically engineered microorganism according to any one of items 1 to 47, which has been further modified to have an increased expression level of at least six (such as at least seven or at least eight) enzymes involved in the biosynthesis of a 5-methylfolate (such as 5-methyl-tetrahydrofolate) compared to an otherwise identical microorganism that does not carry said modification (reference microorganism).
54. The genetically engineered microorganism according to any one of items 1 to 47, which has been (further) modified to have an increased expression level of at least seven (such as at least eight) enzymes involved in the biosynthesis of a 5-methylfolate (such as 5-methyl-tetrahydrofolate) compared to an otherwise identical microorganism that does not carry said modification (reference microorganism).
55. The genetically engineered microorganism according to any one of items 48 to 54, wherein said at least one enzyme involved in the biosynthesis of 5-methylfolate (such as 5-methyl-tetrahydrofolate) is selected from the group consisting of: a polypeptide having GTP cyclohydrolase activity, a polypeptide having 7,8-dihydroneopterin aldolase activity, a polypeptide having 2-amino-4-hydroxy-6-hydroxymethyldihydropteridine pyrophosphokinase activity, a polypeptide having dihydropteroate synthase activity, a polypeptide having dihydrofolate reductase activity, a polypeptide having serine hydroxymethyltransferase activity, a polypeptide having formyltetrahydrofolate deformylase activity, and a polypeptide having 5,10-methylenetetrahydrofolate reductase activity.
56. The genetically engineered microorganism according to any one of items 1 to 55, which has been (further) modified to have an increased expression level of a polypeptide having GTP cyclohydrolase activity compared to an otherwise identical microorganism that does not carry said modification (reference microorganism).
57. The genetically engineered microorganism according to any one of items 1 to 56, which has been (further) modified to have an increased expression level of a polypeptide having 7,8-dihydroneopterin aldolase activity compared to an otherwise identical microorganism that does not carry said modification (reference microorganism).
58. The genetically engineered microorganism according to any one of items 1 to 57, which has been (further) modified to have an increased expression level of a polypeptide having 2-amino-4-hydroxy-6-hydroxymethyldihydropteridine pyrophosphokinase activity compared to an otherwise identical microorganism that does not carry said modification (reference microorganism).
59. The genetically engineered microorganism according to any one of items 1 to 58, which has been (further) modified to have an increased expression level of a polypeptide having dihydropteroate synthase activity compared to an otherwise identical microorganism that does not carry said modification (reference microorganism).
60. The genetically engineered microorganism according to any one of items 1 to 59, which has been (further) modified to have an increased expression level of a polypeptide having dihydrofolate reductase activity compared to an otherwise identical microorganism that does not carry said modification (reference microorganism).
61. The genetically engineered microorganism according to any one of items 1 to 60, which has been (further) modified to have an increased expression level of a polypeptide having serine hydroxymethyltransferase activity compared to an otherwise identical microorganism that does not carry said modification (reference microorganism).
62. The genetically engineered microorganism according to any one of items 1 to 61, which has been (further) modified to have an increased expression level of a polypeptide having formyltetrahydrofolate deformylase activity compared to an otherwise identical microorganism that does not carry said modification (reference microorganism).
63. The genetically engineered microorganism according to any one of items 1 to 62, which has been (further) modified to have an increased expression level of a polypeptide having 5,10-methylenetetrahydrofolate reductase activity compared to an otherwise identical microorganism that does not carry said modification (reference microorganism).
64. The genetically engineered microorganism according to any one of items 48 to 63, wherein the at least one enzyme involved in the biosynthesis of a 5-methylfolate (such as 5-methyl-tetrahydrofolate), respectively the recited polypeptide is heterologous to the genetically engineered microorganism.
65. The genetically engineered microorganism according to any one of items 48 to 64, wherein the at least one enzyme involved in the biosynthesis of a 5-methylfolate, respectively the recited polypeptide is derived from a bacterium or fungus, preferably selected from the genus *Bacillus, Escherichia, Lactococcus, Shewanella, Vibrio* and *Ashbya.*
66. The genetically engineered microorganism according to any one of items 48 to 65, wherein the at least one enzyme involved in the biosynthesis of a 5-methylfolate (such as 5-methyl-tetrahydrofolate), respectively the recited polypeptide is derived from a bacterium or fungus selected from *Bacillus subtiltis*, *Lactobacillus lactis*, *Escherichia coli*, *Shewanella violacea, Vibrio natriegens* or *Ashbya gossypii.*
67. The genetically engineered microorganism according to any one of items 35, 36, 55 and 56, wherein the polypeptide having GTP cyclohydrolase activity comprises an amino acid sequence having at least 70%, such as at least 80%, at least 85%, at least 90%, at least 93%, at least 95%, at least 98% or at least 99%, sequence identity with SEQ ID NO: 7.
68. The genetically engineered microorganism according to any one of items 35, 37, 55 and 57, wherein the polypeptide having 7,8-dihydroneopterin aldolase activity comprises an amino acid sequence having at least 70%, such as at least 80%, at least 85%, at least 90%, at least 93%, at least 95%, at least 98% or at least 99%, sequence identity with SEQ ID NO: 8.
69. The genetically engineered microorganism according to any one of items 35, 38, 55 and 58, wherein the polypeptide having 2-amino-4-hydroxy-6-hydroxymethyl - dihydropteridine pyrophosphokinase activity comprises an amino acid sequence having at least 70%, such as at least 80%, at least 85%, at least 90%, at least 95%, at least 98% or at least 99%, sequence identity with SEQ ID NO: 9.
70. The genetically engineered microorganism according to any one of items 35, 39, 55 or 59, wherein the polypeptide having dihydropteroate synthase activity comprises an amino acid sequence having at least 70%, such as at least 80%, at least 85%, at least 90%, at least 93%, at least 95%, at least 98% or at least 99%, sequence identity with SEQ ID NO: 10.
71. The genetically engineered microorganism according to any one of items 35, 40, 55 and 60, wherein the polypeptide having dihydrofolate reductase activity comprises an amino acid sequence having at least 70%, such as at least 80%, at least 85%, at least 90%, at least 93%, at least 95%, at least 98% or at least 99%, sequence identity with SEQ ID NO: 12.
72. The genetically engineered microorganism according to any one of items 35, 41, 55 and 61, wherein the polypeptide having serine hydroxymethyltransferase activity comprises an amino acid sequence having at least 70%, such as at least 80%, at least 85%, at least 90%, at least 93%, at least 95%, at least 98% or at least 99%, sequence identity with SEQ ID NO: 79.
73. The genetically engineered microorganism according to any one of items 35, 42, 55 and 62, wherein the polypeptide having formyltetrahydrofolate deformylase activity comprises an amino acid sequence having at least 70%, such as at least 80%, at least 85%, at least 90%, at least 95%, at least 98% or at least 99%, sequence identity with SEQ ID NO: 81.
74. The genetically engineered microorganism according to any one of items 35, 43, 55 and 63, wherein the polypeptide having 5,10-methylenetetrahydrofolate reductase activity comprises an amino acid sequence having at least 70%, such as at least 80%, at least 85%, at least 90%, at least 93%, at least 95%, at least 98% or at least 99%, sequence identity with SEQ ID NO: 83.
75. The genetically engineered microorganism according to any one of items 35, 43, 55 and 64, wherein the polypeptide having 5,10-methylenetetrahydrofolate reductase activity comprises an amino acid sequence having at least 70%, such as at least 80%, at least 85%, at least 90%, at least 93%, at least 95%, at least 98% or at least 99%, sequence identity with SEQ ID NO: 84.
76. The genetically engineered microorganism according to any one of items 1 to 75, which has been (further) modified to have a decreased expression and/or activity of an endogenous polypeptide having 5-methyltetrahydropteroyltriglutamate-homocysteine S-methyltransferase activity compared to an otherwise identical microorganism that does not carry said modification (reference microorganism).
77. The genetically engineered microorganism according to item 76, which has been modified to have a decreased expression level of an endogenous gene encoding said polypeptide having 5-methyltetrahydropteroyltriglutamate-homocysteine S-methyltransferase activity compared to an otherwise identical microorganism that does not carry said modification (reference microorganism).
78. The genetically engineered microorganism according to item 77 , wherein the expression level of the endogenous gene is decreased by at least 50%, such as by at least 60%, at least 65%, at least 70%, at least 75%, at least 80%, at least 85%, at least 90%, at least 95% or at least 100% compared to the otherwise identical microorganism.
79. The genetically engineered microorganism according to item 76 or 77, which comprises at least one mutation in the regulatory region of the endogenous gene encoding said polypeptide having 5-methyltetrahydropteroyltriglutamate-homocysteine S-methyltransferase activity, resulting in the decreased expression level.
80. The genetically engineered microorganism according to item 76 or 77, wherein the endogenous gene encoding said polypeptide having 5-methyltetrahydropteroyltriglutamate-homocysteine S-methyltransferase activity has been inactivated.
81. The genetically engineered microorganism according to item 76 or 77, wherein the endogenous gene encoding said polypeptide having 5-methyltetrahydropteroyltriglutamate-homocysteine S-methyltransferase activity has been inactivated by deletion of part of or the entire gene sequence.
82. The genetically engineered microorganism according to item 76, wherein the endogenous gene encoding said polypeptide having 5-methyltetrahydropteroyltriglutamate-homocysteine S-methyltransferase activity has been inactivated by introducing or expressing in the microorganism a rare-cutting endonuclease able to selectively inactivating by DNA cleavage the endogenous gene encoding said polypeptide.
83. The genetically engineered microorganism according to item 82, wherein said rare-cutting endonuclease is a transcription activator-like effector (TALE) nuclease, meganuclease, zing-finger nuclease (ZFN), or RNA-guided endonuclease.
84. The genetically engineered microorganism according to item 83, wherein the RNA-guided endonuclease is a catalytically inactive Cas9 protein.
85. The genetically engineered microorganism according to item 84, which comprises (e.g., expresses) a single guide RNA (sgRNA) specifically hybridizing (e.g. binding) under cellular conditions with the genomic DNA encoding said polypeptide.
86. The genetically engineered microorganism according to item 76, wherein the expression of said endogenous polypeptide having 5-methyltetrahydropteroyltriglutamate-homocysteine S-methyltransferase activity is decreased (e.g., inhibited) by transcriptional and/or translational repression of the endogenous gene encoding said polypeptide.
87. The genetically engineered microorganism according to item 76, wherein the expression of said endogenous polypeptide having 5-methyltetrahydropteroyltriglutamate-homocysteine S-methyltransferase activity is decreased (e.g., inhibited) by introducing or expressing in the microorganism an inhibitory nucleic acid molecule that specifically hybridizes (e.g. binds) under cellular conditions with cellular mRNA and/or genomic DNA encoding said polypeptide.
88. The genetically engineered microorganism according to item 87, wherein the inhibitory nucleic acid molecule is an antisense oligonucleotide, ribozyme or interfering RNA (RNAi) molecule.
89. The genetically engineered microorganism according to item 88, wherein the interfering RNA molecule is a micro RNA (miRNA), small interfering RNA (siRNA) or short hairpin RNA (shRNA).
90. The genetically engineered microorganism according to item 76 or 77, which has been modified to have a decreased activity of an endogenous polypeptide having 5-methyltetrahydropteroyltriglutamate-homocysteine S-methyltransferase activity compared to an otherwise identical microorganism that does not carry said modification (reference microorganism).
91. The genetically engineered microorganism according to item 90, wherein the activity of said polypeptide is decreased by at least one active-site mutation resulting in the reduction or loss of activity.
92. The genetically engineered microorganism according to item 91, wherein the at least one active-site mutation is at a position which corresponds to any one of positions 18, 21, 112, 117, 119, 435-437, 488, 494, 519-521, 565, 601, 603, 605, 645, 647, 669, 730 and 731 in the amino acid sequence set forth in SEQ ID NO: 100.
92. The genetically engineered microorganism according to item 16 or 17, wherein the at least one active-site mutation is a non-conservative amino acid substitution.
93. The genetically engineered microorganism according to any one of items 76 to 92, wherein the endogenous gene encoding said polypeptide having 5-methyltetrahydropteroyltriglutamate-homocysteine S-methyltransferase activity is the gene *metE.*
94. The genetically engineered microorganism according to any one of items 76 to 93, wherein the endogenous gene encoding said polypeptide having 5-methyltetrahydropteroyltriglutamate-homocysteine S-methyltransferase activity comprises a nucleic acid sequence having at least 70%, such as at least 80%, at least 85%, at least 90%, at least 93%, at least 95%, at least 98% or at least 99%, sequence identity with the nucleic acid sequence set forth in SEQ ID NO: 101.
95. The genetically engineered microorganism according to any one of items 76 to 94, wherein the polypeptide having 5-methyltetrahydropteroyltriglutamate-homocysteine S-methyltransferase activity encoded by the endogenous gene comprises an amino acid sequence which has at least 70%, such as at least 80, at least 85%, at least 90%, at least 93%, at least 95%, at least 98% or at least 99%, sequence identity with the amino acid sequence set forth in SEQ ID NO: 100.
96. The genetically engineered microorganism according to any one of items 1 to 95, which is a bacterium.
97. The genetically engineered microorganism according to any one of items 1 to 96, which is a bacterium of the family *Bacillaceae.*
98. The genetically engineered microorganism according to any one of items 1 to 97, which is a bacterium of the genus *Bacillus.*
99. The genetically engineered microorganism according to any one of items 1 to 98, which is a bacterium of the species *Bacillus subtiltis.*
100. A method for preparing a folate, precursor or intermediate thereof, comprising
   i) cultivating a genetically engineered microorganism according to any one of items 1 to 99 in a culture medium under suitable culture conditions to obtain a fermentation product containing said folate, precursor or intermediate thereof; and
   ii) optionally, separating and/ or purifying said folate, precursor or intermediate thereof.
101. The method according to item 100, wherein step i) is carried out at a culture temperature in a range from 32 to 42°C, preferably in a range from 34 to 39°C, more preferably in a range from 36 to 39°C, such as at about 37°C.
102. The method according to item 100 or 101, wherein step i) is carried out for a period in the range from 10 to 70 h, preferably in a range from 24 to 60 h, more preferably in a range from 36 to 50 h.
103. The method according to any one of items 100 to 102, wherein step i) is carried out at a pH in the range of 6 to 8, preferably in a range of 6.5 to 7.5, more preferably in a range from 6.8 to 7.2.
104. The method according to any one of items 100 to 102, wherein the folate is a compound of Formula I: 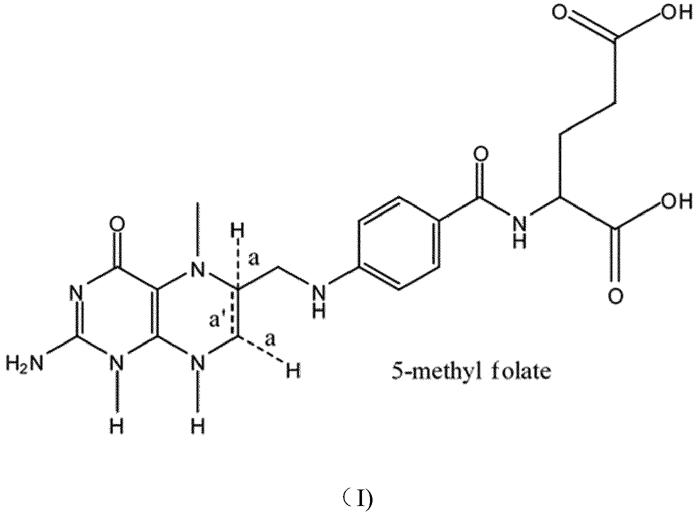 optionally in form of one of the stereoisomers, preferably enantiomers or diastereomers, in form of a racemate or in form of a mixture of at least two of the stereoisomers, preferably enantiomers and/or diastereomers, in any mixing ratio.
105. The method according to any one of items 100 to 104, wherein the folate is a compound of Formula II: 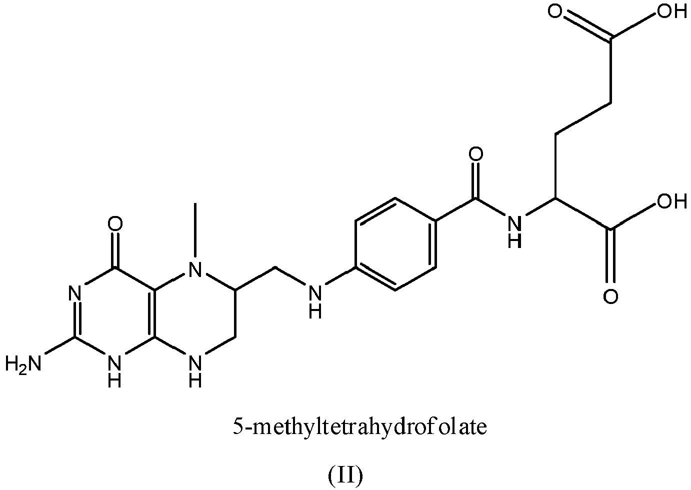 optionally in form of one of the stereoisomers, preferably enantiomers or diastereomers, in form of a racemate or in form of a mixture of at least two of the stereoisomers, preferably enantiomers and/or diastereomers, in any mixing ratio.
106. The method according to any one of items 100 to 105, wherein the folate is a compound of Formula IIa: 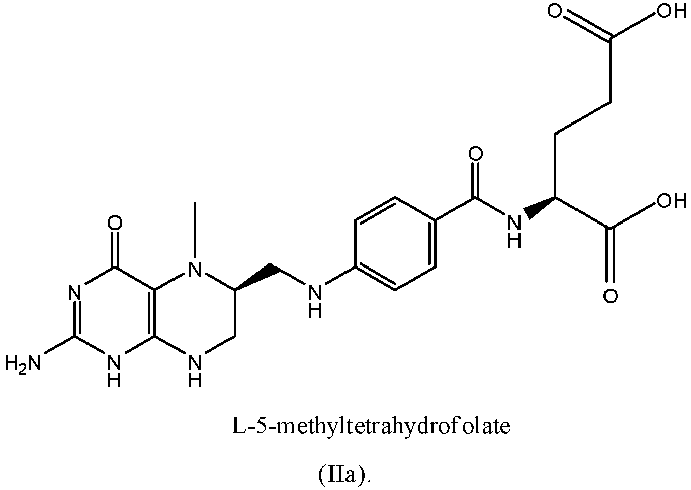
107. The method according to any one of items 100 to 104, wherein the folate is a compound of Formula III: 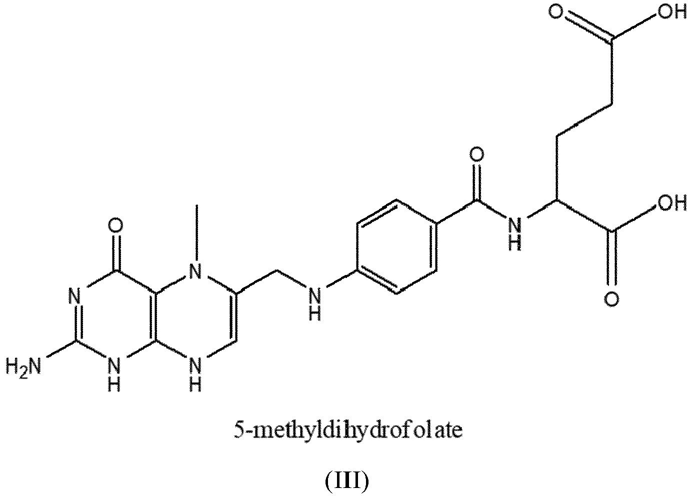 optionally in form of one of the stereoisomers, preferably enantiomers or diastereomers, in form of a racemate or in form of a mixture of at least two of the stereoisomers, preferably enantiomers and/or diastereomers, in any mixing ratio.
108. The method according to any one of items 100 to 107, further comprising the step of adding *para*-aminobenzoic acid (PABA) during the cultivation step (i).
109. The method according to item 108, wherein the *para*-aminobenzoic acid (PABA) is selected from the group consisting of: potassium *para*-aminobenzoate, sodium *para*-aminobenzoate, methyl *para*-aminobenzoate, ethyl *para*-aminobenzoate, butyl *para-*aminobenzoate, or a combination thereof.
110. The method according to any one of items 100 to 109, further comprising subjecting the product obtained in the steps (i) or (ii) to acidic or alkaline conditions to further obtain a derivative compound.
111. A method of preparing a genetically engineered microorganism, comprising any one of the steps (a) to (d) below:
   (a) increased the expression level of at least one (such as at least two, at least three, at least four, at least five, at least six, at least seven or at least eight) enzyme involved in the biosynthesis of a 5-methylfolate (such as 5-methyl-tetrahydrofolate) compared to an otherwise identical microorganism (reference microorganism);
   (b) decreasing the expression and/or activity of an endogenous polypeptide having 5-methyltetrahydropteroyltriglutamate-homocysteine S-methyltransferase activity compared to an otherwise identical microorganism that does not carry said modification (reference microorganism);
   (c) decreasing the expression and/or activity of an endogenous polypeptide having both dihydrofolate synthase activity and folylpolyglutamate synthetase activity compared to an otherwise identical microorganism that does not carry said modification (reference microorganism); and/or
   (d) expressing a heterologous polypeptide having only dihydrofolate synthase activity.
112. The method according to item 111, comprising any one of the steps (a) to (b) below:
   (a) increased the expression level of at least one enzyme involved in the biosynthesis of a 5-methylfolate compared to an otherwise identical microorganism (reference microorganism); and
   (b) decreasing the expression and/or activity of an endogenous polypeptide having 5-methyltetrahydropteroyltriglutamate-homocysteine S-methyltransferase activity compared to an otherwise identical microorganism that does not carry said modification (reference microorganism).
113. The method according to claim 112, further comprising the steps (c) and (d) below:
   (c) decreasing the expression and/or activity of an endogenous polypeptide having both dihydrofolate synthase activity and folylpolyglutamate synthetase activity compared to an otherwise identical microorganism that does not carry said modification (reference microorganism); and
   (d) expressing a heterologous polypeptide having only dihydrofolate synthase activity.
114. The method according to any one of items 111 to 113, comprising the steps of aa) introducing into said microorganism at least one exogenous nucleic acid molecule comprising a nucleic acid sequence encoding an enzyme involved in the biosynthesis of a 5-methylfolate (such as 5-methyl-tetrahydrofolate); bb) inactivating, such as by deleting part of or the entire gene sequence, the endogenous gene encoding said polypeptide having 5-methyltetrahydropteroyltriglutamate-homocysteine S-methyltransferase activity or introducing at least one mutation in the regulatory region of said endogenous gene, which results in the decrease expression level; cc) inactivating, such as by deleting part of or the entire gene sequence, the endogenous gene encoding said polypeptide having both dihydrofolate synthase activity and folylpolyglutamate synthetase activity in said microorganism; and/or dd) introducing into said microorganism an exogenous nucleic acid molecule comprising a nucleic acid sequence encoding a heterologous polypeptide having only dihydrofolate synthase activity.

It should be understood that, within the scope of the present invention, each technical feature of the present invention described above and in the following may be combined with each other to form a preferred technical solution, which is not listed here explicitly due to space limitations.

### Description of Figures

Figure 1 shows the core structure of folates. In natural folates, the pterin ring exists in tetrahydro form (as shown) or in 7,8-dihydro form. The ring is fully oxidized in chemically produced folic acid. Folates usually have a γ-linked polyglutamyl tail of up to about eight residues attached to the first glutamate. One-carbon unit (formyl, methyl, etc.) can be coupled to the N5 and/or N10 positions resulting in synthesis of 5-formyl folates, 10-formyl folates or 5-methyl folates.
Figure 2 shows schematic representation of an example of a folic acid operon consisting of *L. lactis* genes.
Figure 3 shows schematic representation of an example of a folic acid operon consisting of *A. gossypii* genes.
Figure 4 shows schematic representation of an example of a folic acid operon consisting of *B. subtilis* genes.
Figure 5 shows schematic presentation of the FolC disruption cassettes with tetracycline resistance gene (*TetR*), heterologous *folC2*-LR or *folC2*-AG gene under P_{veg} promoter and flanking homology ends for native *folC* target gene disruption. The position of the primers used for PCR amplification of the DNA disruption cassette are denoted as lines.
Figure 6 shows chromatogram of fermentation broth sample. Black: UV signal, red: MS scan signal.
Figure 7 shows schematic representation of oxidation of 10-formyldihydrofolic acid to 10-formylfolic acid in the presence of oxygen, schematic representation of oxidation of 10-formyldihydrofolic acid to 10-formylfolic acid in the presence of hydrogen peroxide and schematic representation of oxidation of 10-formyldihydrofolic acid to 10-formylfolic acid in the presence of sodium periodate.
Figure 8 shows schematic representation of deformylation of 10-formylfolic acid to folic acid in acidic medium.
Figure 9 shows schematic representation of deformylation of 10-formylfolic acid to folic acid in alkaline medium.
Figure 10 shows Folates production bioprocess profile. Folates (mg/L): full stars; Glucose concentration (g/L): empty squares; Acetoin concentration (g/L): full squares; PABA concentration (mg/L): empty circles; PABA feed (mg/L): vertical bars; Optical density: full circles.
Figure 11 shows total folate production titers of *B. subtilis* strain w.t. 168, strain VBB38, strain FL21 and FL23 at the shaker 5 ml scale experiments.

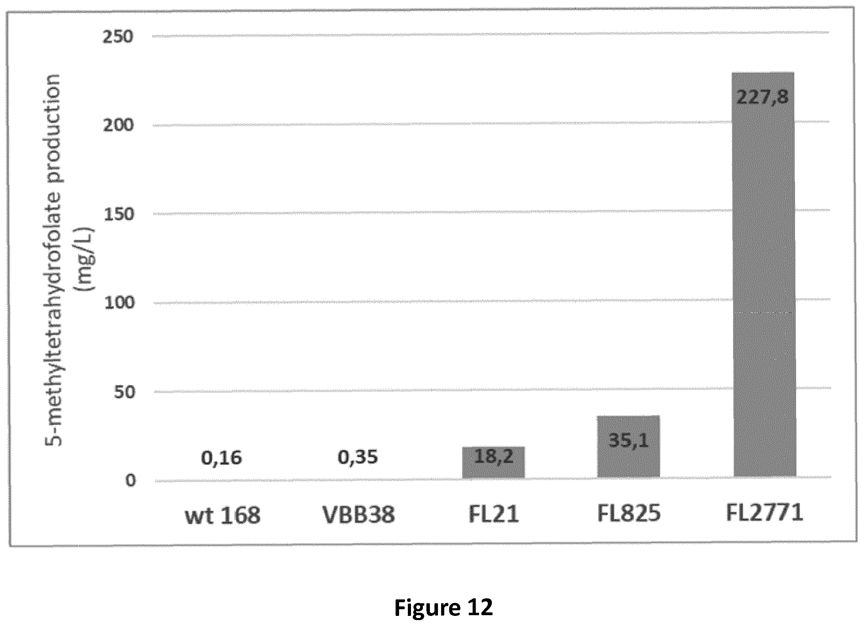

Figure 12 shows 5-methyl folate production titers of *B. subtilis* strain w.t. 168, strain VBB38, strain FL21, FL825 and FL2771 at the shaker 5 ml scale experiments.
Figure 13 shows a schematic presentation of 5-methyl-tetrahydrofolate producing strains development by transformation with artificial methyl-folate operon MTHF-OP-B into the parent strain FL2771.
Figure 14 shoes a schematic presentation of the artificial MTHF-OP folate operons. Constructed operons have spectinomycin selectable marker and the homology for genome integration at ywhL locus. In the MTHF-OP-A operon genes (*glyA, purU and yitJ*) were selected from native organism *B. subtilis* and additional codon-optimized for optimal gene expression. Additionally, homologue gene (*metF*) from *E. coli* was used for construction of alternative to *yitJ* gene in operon MTHF-OP-B.
Figure 15 shows transcription levels of *metE* gene in parent strain FL825 and the descendant strain FL2771.
Figure 16 shows schematic representation of FOL-OP-BS1 a folic acid operon consisting of *B. subtilis* genes.

### Detailed Description

Unless specifically defined herein, all technical and scientific terms used have the same meaning as commonly understood by a skilled artisan in the fields of biochemistry, genetics, and molecular biology.

All methods and materials similar or equivalent to those described herein can be used in the practice or testing of the present invention, with suitable methods and materials being described herein. All publications, patent applications, patents, and other references mentioned herein are incorporated by reference in their entirety. In case of conflict, the present specification, including definitions, will prevail. Further, the materials, methods, and examples are illustrative only and are not intended to be limiting, unless otherwise specified.

The practice of the present invention will employ, unless otherwise indicated, conventional techniques of cell biology, cell culture, molecular biology, transgenic biology, microbiology, recombinant DNA, and immunology, which are within the skill of the art. Such techniques are explained fully in the literature. See, for example, Current Protocols in Molecular Biology (Frederick M. AUSUBEL, 2000, Wiley and son Inc, Library of Congress, USA); Molecular Cloning: A Laboratory Manual, Third Edition, (Sambrook et al, 2001, Cold Spring Harbor, New York: Cold Spring Harbor Laboratory Press); Oligonucleotide Synthesis (M. J. Gait ed., 1984); Mullis et al. U.S. Pat. No. 4,683,195; Nucleic Acid Hybridization (B. D. Harries & S. J. Higgins eds. 1984); Transcription And Translation (B. D. Hames & S. J. Higgins eds. 1984); Culture Of Animal Cells (R. I. Freshney, Alan R. Liss, Inc., 1987); Immobilized Cells And Enzymes (IRL Press, 1986); B. Perbal, A Practical Guide To Molecular Cloning (1984); the series, Methods In ENZYMOLOGY (J. Abelson and M. Simon, eds.-in-chief, Academic Press, Inc., New York), specifically, Vols.154 and 155 (Wu et al. eds.) and Vol. 185, "Gene Expression Technology" (D. Goeddel, ed.); Gene Transfer Vectors For Mammalian Cells (J. H. Miller and M. P. Calos eds., 1987, Cold Spring Harbor Laboratory); and Immunochemical Methods In Cell And Molecular Biology (Mayer and Walker, eds., Academic Press, London, 1987); Handbook Of Experimental Immunology, Volumes I-IV (D. M. Weir and C. C. Blackwell, eds., 1986.

### Genetically engineered microorganism of the invention

In one aspect, the present invention thus provides a genetically engineered microorganism, such as genetically engineered bacterium. Suitably, the genetically engineered microorganism has the ability to produce 5-methylfolate (according to formula (I) shown below), and more specifically 5-methly tetrahydrofolate (5-methyl-THF) (according to formula (II) shown below) including any stereoisomer thereof, such as enantiomer or diastereomer, such (6S)-5-Methyltetrahydrofolate (according to formula (IIa) shown below).

In some bacteria, such as *Bacillus subtilis,* the addition of L-glutamate to dihydropteroate (dihydrofolate synthetase (DHFS) activity, EC 6.3.2.12) and the subsequent additions of L-glutamate to tetrahydrofolate through gamma carboxyl groups (folylpolyglutamate synthetase (FPGS) activity, EC 6.3.2.17) are catalyzed by the same enzyme, FolC. In contrast, in eukaryotes and some other bacteria DHFS and FPGS enzymatic activities are encoded in different genes. *B. subtilis,* as many other bacteria, adds gamma-linked poly-glutamate tails to folates in order to increase solubility and prevent the loss of this essential cofactor into the environment. Thus, the *Bacillus subtilis* FolC possesses folyl-poly-glutamate synthetase (FPGS) activity which catalyzes the polyglutamylation of folates through their gamma-carboxyl groups in addition to its role as dihydrofolate synthase in the *de novo* folate biosynthetic pathway. The folate polyanions cannot be exported out of cells, resulting in enhanced intracellular retention (Sybesma et al., 2003c). In addition, the products of the FPGS enzyme, folyl-polyglutamates, are strong inhibitors of the folate biosynthetic enzymes (McGuire and Bertino, 1981). Therefore, in order to increase the production of folates, the present inventors have abolished the polyglutamylation of folates by knocking-out the native (endogenous) *folC* gene and replaced it with a heterologous *folC* gene encoding only for the essential dihydrofolate synthetase (DHFS) activity, resulting in the addition of only one essential glutamate moiety. Homologs of FolC with only the dihydrofolate synthetase (DHFS) and without folylpolyglutamate (FGPS) synthetase activity can be found in many bacteria species like *Lactobacillus reuteri* and many eukaryotic organisms like *Ashbya gossypii.*

After extensive and intensive research, the inventors have surprisingly found that if the expression level of an endogenous gene encoding a polypeptide having both dihydrofolate synthase activity and folylpolyglutamate synthetase activity, such as the gene *folC*, is reduced in a microorganism, and instead an exogenous gene is introduced encoding a polypeptide having only dihydrofolate synthase activity, only one glutamate is added on the biosynthesized folate, and the production capacity of a folate (such as 5-methyl-tetrahydrofolate), a salt, a precursor, or an intermediate thereof is thereby significantly increased.

Thus, the genetically engineered microorganism of the invention may been modified to have a decreased expression and/or activity of an endogenous polypeptide having both dihydrofolate synthase activity and folylpolyglutamate synthetase activity compared to an otherwise identical microorganism that does not carry said modification (reference microorganism).

According to some embodiments, the genetically engineered microorganism of the invention may been modified to have a decreased expression level of the endogenous gene encoding said polypeptide having both dihydrofolate synthase activity and folylpolyglutamate synthetase activity compared to an otherwise identical microorganism that does not carry said modification (reference microorganism). The expression level of the endogenous gene may, for example, be decreased by at least 50%, such as by at least 60%, at least 65%, at least 70%, at least 75%, at least 80%, at least 85%, at least 90%, at least 95% or at least 100% compared to the otherwise identical microorganism.

According to some embodiments, the endogenous gene encoding said polypeptide having both dihydrofolate synthase activity and folylpolyglutamate synthetase activity has been inactivated, such as by deletion of part of or the entire gene sequence.

According to some embodiments, the endogenous gene encoding said polypeptide having both dihydrofolate synthase activity and folylpolyglutamate synthetase activity has been inactivated by introducing or expressing in the microorganism a rare-cutting endonuclease able to selectively inactivating by DNA cleavage, preferably by double-strand break, the endogenous gene encoding said polypeptide. A rare-cutting endonuclease to be used in accordance of the present invention to inactivate the endogenous gene may, for instance, be a transcription activator-like effector (TALE) nuclease, meganuclease, zing-finger nuclease (ZFN), or RNA-guided endonuclease.

One way to inactivate the endogenous gene encoding said polypeptide having both dihydrofolate synthase activity and folylpolyglutamate synthetase activity is to use the CRISPRi system. The CRISPRi system was developed as a tool for targeted repression of gene expression or for blocking targeted locations on the genome. The CRISPRi system consists of the catalytically inactive, "dead" Cas9 protein (dCas9) and a guide RNA that defines the binding site for the dCas9 to DNA.

Thus, according to some embodiments, the endogenous gene encoding said polypeptide having both dihydrofolate synthase activity and folylpolyglutamate synthetase activity is inactivated by introducing or expressing in the microorganism a RNA-guided endonuclease, such as a catalytically inactive Cas9 protein, and a single guide RNA (sgRNA) specifically hybridizing (e.g. binding) under cellular conditions with the genomic DNA encoding a said polypeptide.

By way of example, if the endogenous gene encoding said polypeptide having both dihydrofolate synthase activity and folylpolyglutamate synthetase activity is to be inhibited in *Bacillus subtiltis*, the single guide RNA (sgRNA) may comprise at least 20 consecutive nucleotides of SEQ ID NO: 5 or its complement.

According to some embodiments, the expression of an endogenous polypeptide having both dihydrofolate synthase activity and folylpolyglutamate synthetase activity is decreased by way of inhibition.

Inhibition of the expression of said endogenous polypeptide having both dihydrofolate synthase activity and folylpolyglutamate synthetase activity may be achieved by any suitable means known in the art. For example, the expression may be inhibited by gene silencing techniques involving the use of inhibitory nucleic acid molecules, such as antisense oligonucleotides, ribozymes or interfering RNA (RNAi) molecules, such as microRNA (miRNA), small interfering RNA (siRNA) or short hairpin RNA (shRNA).

According to some embodiments, the expression of said endogenous polypeptide having both dihydrofolate synthase activity and folylpolyglutamate synthetase activity is decreased (e.g., inhibited) by transcriptional and/or translational repression of the endogenous gene encoding said polypeptide.

According to some embodiments, the expression of said endogenous polypeptide having both dihydrofolate synthase activity and folylpolyglutamate synthetase activity is inhibited by introducing or expressing in the microorganism an inhibitory nucleic acid molecule. For example, the inhibitory nucleic acid molecule may be introduced by way of an exogenous nucleic acid molecule comprising a nucleotide sequence encoding said inhibitory nucleic acid molecule operably linked to a promoter, such as an inducible promoter, that is functional in the microorganism to cause the production of said inhibitory nucleic acid molecule. Suitably, the inhibitory nucleic acid molecule is one that specifically hybridizes (e.g. binds) under cellular conditions with cellular mRNA and/or genomic DNA encoding the endogenous polypeptide. Depending on the target, transcription of the encoding genomic DNA and/or translation of the encoding mRNA is/are inhibited.

According to some embodiments, the inhibitory nucleic acid molecule is an antisense oligonucleotide, ribozyme or interfering RNA (RNAi) molecule. Preferably, such nucleic acid molecule comprises at least 10 consecutive nucleotides of the complement of the cellular mRNA and/or genomic DNA encoding the polypeptide or enzyme of interest (e.g., the cellular mRNA and/or genomic DNA encoding the polypeptide.

By way of example, if the expression of an endogenous polypeptide having both dihydrofolate synthase activity and folylpolyglutamate synthetase activity is to be inhibited in *Bacillus subtilis,* such inhibitory nucleic acid molecule may comprise at least 10 consecutive nucleotides of the complement of SEQ ID NO: 5.

According to some embodiments, the inhibitory nucleic acid is an antisense oligonucleotide. Such antisense oligonucleotide is a nucleic acid molecule (either DNA or RNA) which specifically hybridizes (e.g. binds) under cellular conditions with the cellular mRNA and/or genomic DNA encoding the polypeptide.

According to some embodiments, the inhibitory nucleic acid molecule is a ribozyme, such as a hammerhead ribozyme. A ribozyme molecule is designed to catalytically cleave the mRNA transcript to prevent translation of the polypeptide.

According to some embodiments, the inhibitory nucleic acid molecule is an interfering RNA (RNAi) molecule. RNA interference is a biological process in which RNA molecules inhibit expression, typically causing the destruction of specific mRNA. Exemplary types of RNAi molecules include microRNA (miRNA), small interfering RNA (siRNA) and short hairpin RNA (shRNA). According to some embodiments, the RNAi molecule is a miRNA. According to some embodiments, the RNAi molecule is a siRNA. According to some embodiments, the RNAi molecule is a shRNA.

By way of example, if the expression of an endogenous polypeptide having both dihydrofolate synthase activity and folylpolyglutamate synthetase activity is to be inhibited in *Bacillus subtilis,* the RNAi molecule may be an interfering RNA complementary to SEQ ID NO: 5. The RNAi molecule may be a ribonucleic acid molecule comprising at least 10 consecutive nucleotides of the complement of SEQ ID NO: 5. The RNAi molecule may be a double-stranded ribonucleic acid molecule comprising a first strand identical to 20 to 25, such as 21 to 23, consecutive nucleotides of SEQ ID NO: 5, and a second strand complementary to said first strand.

According to some embodiments, the genetically engineered microorganism of the invention has been modified to have a decreased activity of an endogenous polypeptide having both dihydrofolate synthase activity and folylpolyglutamate synthetase activity compared to an otherwise identical microorganism that does not carry said modification (reference microorganism).

A decrease of the activity of the endogenous polypeptide having both dihydrofolate synthase activity and folylpolyglutamate synthetase activity may be achieved by any suitable means known in the art. For example, the activity may be decrease by introducing one or more mutations in the active site of the polypeptide resulting in the reduction or loss of activity. Thus, according to some embodiments, the activity of the endogenous polypeptide having both dihydrofolate synthase activity and folylpolyglutamate synthetase activity is decreased by at least one active-site mutation resulting in the reduction or loss of activity. The at least one active-site mutation may, for example, be at least one non-conservative amino acid substitution.

By way of example, if the activity of the endogenous polypeptide having both dihydrofolate synthase activity and folylpolyglutamate synthetase activity is to be decreased in *Bacillus subtilis,* the at least one active-site mutation may occur at any one of positions 51-54, 75, 114-117, 145, 152-154, 172, 263, 302 and 315 in the amino acid sequence set forth in SEQ ID NO: 11, which form part of the active site. In case of orthologous polypeptides, the at least one active-site mutation may be at a position which corresponds to any one of positions 51-54, 75, 114-117, 145, 152-154, 172, 263, 302 and 315 in the amino acid sequence set forth in SEQ ID NO: 11. According to some embodiments, the endogenous gene encoding said polypeptide having both dihydrofolate synthase activity and folylpolyglutamate synthetase activity is the gene *folC.*

According to some embodiments, the endogenous gene encoding said polypeptide having both dihydrofolate synthase activity and folylpolyglutamate synthetase activity comprises a nucleic acid sequence having at least 70%, such as at least 80%, at least 85%, at least 90%, at least 93%, at least 95%, at least 98% or at least 99%, sequence identity with the nucleic acid sequence set forth in SEQ ID NO: 5. According to come embodiments, the endogenous gene encoding a polypeptide having both dihydrofolate synthase activity and folylpolyglutamate synthetase activity comprises a nucleic acid sequence having at least 85%, such as at least 90%, sequence identity with the nucleic acid sequence set forth in SEQ ID NO: 5. According to come embodiments, the endogenous gene encoding a polypeptide having both dihydrofolate synthase activity and folylpolyglutamate synthetase activity comprises a nucleic acid sequence having at least 95%, such as at least 98%, sequence identity with the nucleic acid sequence set forth in SEQ ID NO: 5.

According to some embodiments, the polypeptide having both dihydrofolate synthase activity and folylpolyglutamate synthetase activity encoded by the endogenous gene comprises an amino acid which has at least 70%, such as at least 80%, at least 85%, at least 90%, at least 93%, at least 95%, at least 98% or at least 99%, sequence identity with the amino acid sequence set forth in SEQ ID NO: 11. According to some embodiments, the polypeptide having both dihydrofolate synthase activity and folylpolyglutamate synthetase activity encoded by the endogenous gene comprises an amino acid which has at least 85%, such as at least 90%, sequence identity with the amino acid sequence set forth in SEQ ID NO: 11. According to some embodiments, the polypeptide having both dihydrofolate synthase activity and folylpolyglutamate synthetase activity encoded by the endogenous gene comprises an amino acid which has at least 95%, such as at least 98%, sequence identity with the amino acid sequence set forth in SEQ ID NO: 11.

The genetically engineered microorganism of the invention may be (further) modified to express a heterologous polypeptide having only dihydrofolate synthase activity. The heterologous polypeptide having only dihydrofolate synthase activity may, for example, be derived from a bacterium or fungus, preferably selected from *Lactobacillus reuteri* and *Ashbya gossypii.*

According to some embodiments, the heterologous polypeptide having only dihydrofolate synthase activity comprises an amino acid sequence having at least 70%, such as at least 80%, at least 85%, at least 90%, at least 93%, at least 95%, at least 98% or at least 99%, sequence identity with SEQ ID NO: 22 or 23. According to some embodiments, the heterologous polypeptide having only dihydrofolate synthase activity comprises an amino acid sequence having at least 85%, such as at least 90%, sequence identity with SEQ ID NO: 22 or 23. According to some embodiments, the heterologous polypeptide having only dihydrofolate synthase activity comprises an amino acid sequence having at least 95%, such as at least 98%, sequence identity with SEQ ID NO: 22 or 23.

The folate molecule contains one pterin moiety, originating from guanosine triphosphate (GTP), bound to *para*-aminobenzoic acid (pABA) and at least one molecule of glutamic acid. Thus, *de novo* biosynthesis of folate requires three precursors: GTP, pABA and glutamic acid.

Folate biosynthesis proceeds via the conversion of GTP to the 6-hydroxymethyl-7,8-dihydropterin pyrophosphate (DHPPP) in four consecutive steps. The first step is catalyzed by GTP cyclohydrolase I (EC 3.5.4.16) (gene *folE*/*mtrA*) and involves an extensive transformation of GTP, to form a pterin ring structure. Following dephosphorylation, the pterin molecule undergoes aldolase (EC 4.1.2.25) (gene *folB*) and pyrophosphokinase reactions (EC 2.7.6.3) (gene *folK*), which produce the activated pyrophosphorylated DHPPP. Following the first condensation of para-aminobenzoic acid (pABA) with DHPPP catalyzed by dihydropteroate synthase (EC 2.5.1.15) (gene *folP*/*sul*) to produce dihydropteroate. The second condensation is reaction of glutamate with dihydropteroate to form dihydrofolate by dihydrofolate synthase (DHFS) (EC 6.3.2.12) (gene *folC*). Then, DHF is reduced by DHF reductase - DHFR (EC 1.5.1.3) (gene *folA*/*dfrA*) to the biologically active cofactor tetrahydrofolate (THF).

Proteins GlyA, PurU, YitJ and MetF are further involved in the tetrahydrofolate interconversion pathways. Tetrahydrofolate (THF) can be activated by serine hydroxymethyltransferase (gene *glyA*) (EC:2.1.2.1) by converting serine to glycine and subsequently transferring a methyl group to tetrahydrofolate, thus forming 5,10-methylene-tetrahydrofolate (5,10-mTHF). 5,10-mTHF is the major source of C1 units in the cell. Further in the tetrahydrofolate interconversion the *B. subtilis yit J* gene and *Escherichia coli metF* gene are coding for 5,10-methylene-tetrahydrofolate reductase (EC 1.5.1.20), the enzyme that leads to the final formation of 5-methyltetrahydrofolate.

Additionally, enzyme PurU is also important for the tetrahydrofolate interconversion pathways, as it is involved as a formyltetrahydrofolate deformylase (EC 3.5.1.10) in the conversion of 10-formyltetrahydrofolate back to THF, to be further available for 5-methyltetrahydrofolate biosynthesis.

**Table 1. Folate biosynthetic genes**

| **Gene name / synonym:** | **Enzymatic activity and EC number** | **Microorganism (gene source)** | **Protein sequence ID** | **Nucleotide sequence ID** |
|---|---|---|---|---|
| *folC2-AG* / *FOL3* | dihydrofolate synthetase EC: 6.3.2.12 | *Ashbya gossypii* | SEQ ID NO: 23 | SEQ ID NO: 58 |
| *folC2-LR* | dihydrofolate synthetase EC: 6.3.2.12 | *Lactobacillus reuteri* | SEQ ID NO: 22 | SEQ ID NO: 99 |
| *folC* | Bifunctional folylpolyglutamate synthase / dihydrofolate synthase EC: 6.3.2.12 / EC: 6.3.2.17 | *Bacillus subtilis* | SEQ ID NO: 11 | SEQ ID NO: 5 |
| *folE* / *mtrA* | GTP cyclohydrolase EC: 3.5.4.16 | *Bacillus subtilis* | SEQ ID NO: 7 | SEQ ID NO: 1 |
| *folB* | 7,8-dihydroneopterin aldolase EC: 4.1.2.25 | *Bacillus subtilis* | SEQ ID NO: 8 | SEQ ID NO: 2 |
| *folK* | 2-amino-4-hydroxy-6-hydroxymethyldihydropteridin e pyrophosphokinase EC: 2.7.6.3 | *Bacillus subtilis* | SEQ ID NO: 9 | SEQ ID NO: 3 |
| *folP* / *sul* | dihydropteroate synthase EC: 2.5.1.15 | *Bacillus subtilis* | SEQ ID NO: 10 | SEQ ID NO: 4 |
| *folA* / *dfrA* | dihydrofolate reductase EC: 1.5.1.3 | *Bacillus subtilis* | SEQ ID NO: 12 | SEQ ID NO: 6 |
| *glyA* | serine hydroxymethyltransferase EC: 2.1.2.1 | *Bacillus subtilis* | SEQ ID NO: 79 | SEQ ID NO: 80 |
| *purU* / *ykkE* | formyltetrahydrofolate deformylase EC: 3.5.1.10 | *Bacillus subtilis* | SEQ ID NO: 81 | SEQ ID NO: 82 |
| *yitJ* | 5,10-methylenetetrahydrofolate reductase EC: 1.5.1.20 | *Bacillus subtilis* | SEQ ID NO: 83 | SEQ ID NO: 85 |
| *metF* | 5,10-methylenetetrahydrofolate reductase EC: 1.5.1.20 | *Escherichia coli* | SEQ ID NO: 84 | SEQ ID NO: 86 |
| *metE* | methionine synthase or 5-methyltetrahydropteroyltriglut amate---homocysteine S-methyltransferase | *Bacillus subtilis* | SEQ ID NO: 100 | SEQ ID NO: 101 |

The present inventors have found that the introduction or up-regulation of one or more genes involved in the biosynthesis of 5-methyl-THF (such as, *folE*/*mtrA, folB, folK, folP*/*sul, folA*/*dfrA, glyA, purU, yitJ* and *metF*) in a microorganism can also significantly increase the production capacity of 5-methyl-THF, a salt, a precursor, or an intermediate thereof.

Thus, the genetically engineered microorganism of the invention may be (further) modified to have a significantly improved production capacity of a 5-methylfolate (such as 5-methyl-tetrahydrofolate) or a precursor or an intermediate thereof compared to an otherwise identical microorganism that does not carry said modification (reference microorganism). The production capacity of a 5-methylfolate (such as 5-methyl-tetrahydrofolate) or a precursor or an intermediate thereof may, for example, be increased by at least 50%, such as at least 100%, at least 200%, at least 500%, at least 1000%, at least 2000%, at least 5000%, at least 10000%, at least 20000% or at least 50000%, compared to the otherwise identical microorganism (reference microorganism).

According to some embodiments, the genetically engineered microorganism of the invention has been (further) modified to have an increased expression level of at least one gene (such as at least two, at least three, at least four, at least five, at least six, at least seven or at least eight genes) encoding an enzyme involved in the biosynthesis of a 5-methylfolate (such as 5-methyl-tetrahydrofolate) compared to an otherwise identical microorganism that does not carry said modification (reference microorganism). Thus, according to some embodiments, the genetically engineered microorganism of the invention has been (further) modified to have an increased expression level of at least one (such as at least two, at least three, at least four, at least five, at least six, at least seven or at least eight genes) enzymes involved in the biosynthesis of a 5-methylfolate (such as 5-methyl-tetrahydrofolate) compared to an otherwise identical microorganism that does not carry said modification (reference microorganism).

The expression level of the at least one gene (such as at least two, at least three, at least four, at least five, at least six, at least seven or at least eight genes) encoding an enzyme involved in the biosynthesis of a 5-methylfolate (such as 5-methyl-tetrahydrofolate) may, for example, be increased by at least 50%, at least 100 at least 200%, at least 500%, at least 1000%, at least 2000%, at least 5000%, at least 10000%, at least 20000% or at least 50000%, compared to the otherwise identical microorganism (reference microorganism).

According to some embodiments, the enzyme involved in the biosynthesis of a 5-methylfolate (such as 5-methyl-tetrahydrofolate) is selected from selected from the group consisting of: a polypeptide having GTP cyclohydrolase activity, a polypeptide having 7,8-dihydroneopterin aldolase activity, a polypeptide having 2-amino-4-hydroxy-6-hydroxymethyldihydropteridine pyrophosphokinase activity, a polypeptide having dihydropteroate synthase activity, a polypeptide having dihydrofolate reductase activity, a polypeptide having serine hydroxymethyltransferase activity, a polypeptide having formyltetrahydrofolate deformylase activity, and a polypeptide having 5,10-methylenetetrahydrofolate reductase activity.

According to some embodiments, the genetically engineered microorganism of the invention has been (further) modified to have an increased expression level of a gene encoding a polypeptide having GTP cyclohydrolase activity compared to an otherwise identical microorganism that does not carry said modification (reference microorganism). Thus, a genetically engineered microorganism of the invention may have an increased expression level of a polypeptide having GTP cyclohydrolase activity compared to an otherwise identical microorganism that does not carry said modification (reference microorganism).

According to some embodiments, the polypeptide having GTP cyclohydrolase activity comprises an amino acid sequence having at least 70%, such as at least 80%, at least 85%, at least 90%, at least 93%, at least 95%, at least 98% or at least 99%, sequence identity with SEQ ID NO: 7. According to some embodiments, the polypeptide having GTP cyclohydrolase activity comprises an amino acid sequence having at least 85%, such as at least 90%, sequence identity with SEQ ID NO: 7. According to some embodiments, the polypeptide having GTP cyclohydrolase activity comprises an amino acid sequence having at least 95%, such as at least 98%, sequence identity with SEQ ID NO: 7.

According to some embodiments, the genetically engineered microorganism of the invention has been (further) modified to have an increased expression level of a gene encoding a polypeptide having 7,8-dihydroneopterin aldolase activity compared to an otherwise identical microorganism that does not carry said modification (reference microorganism). Thus, a genetically engineered microorganism of the invention may have an increased expression level of a polypeptide having 7,8-dihydroneopterin aldolase activity compared to an otherwise identical microorganism that does not carry said modification (reference microorganism).

According to some embodiments, the polypeptide having 7,8-dihydroneopterin aldolase activity comprises an amino acid sequence having at least 70%, such as at least 80%, at least 85%, at least 90%, at least 93%, at least 95%, at least 98% or at least 99%, sequence identity with SEQ ID NO: 8. According to some embodiments, the polypeptide having 7,8-dihydroneopterin aldolase activity comprises an amino acid sequence having at least 85%, such as at least 90%, sequence identity with SEQ ID NO: 8. According to some embodiments, the polypeptide having 7,8-dihydroneopterin aldolase activity comprises an amino acid sequence having at least 95%, such as at least 98%, sequence identity with SEQ ID NO: 8.

According to some embodiments, the genetically engineered microorganism of the invention has been (further) modified to have an increased expression level of a gene encoding a polypeptide having 2-amino-4-hydroxy-6-hydroxymethyldihydropteridine pyrophosphokinase activity compared to an otherwise identical microorganism that does not carry said modification (reference microorganism). Thus, a genetically engineered microorganism of the invention may have an increased expression level of a polypeptide having 2-amino-4-hydroxy-6-hydroxymethyldihydropteridine pyrophosphokinase activity compared to an otherwise identical microorganism that does not carry said modification (reference microorganism).

According to some embodiments, the polypeptide having 2-amino-4-hydroxy-6-hydroxymethyl- dihydropteridine pyrophosphokinase activity comprises an amino acid sequence having at least 70%, such as at least 80%, at least 85%, at least 90%, at least 95%, at least 98% or at least 99%, sequence identity with SEQ ID NO: 9. According to some embodiments, the polypeptide having 2-amino-4-hydroxy-6-hydroxymethyl-dihydropteridine pyrophosphokinase activity comprises an amino acid sequence having at least 85%, such as at least 90%, sequence identity with SEQ ID NO: 9. According to some embodiments, the polypeptide having 2-amino-4-hydroxy-6-hydroxymethyl-dihydropteridine pyrophosphokinase activity comprises an amino acid sequence having at least 95%, such as at least 98%, sequence identity with SEQ ID NO: 9.

According to some embodiments, the genetically engineered microorganism of the invention has been (further) modified to have an increased expression level of a gene encoding a polypeptide having dihydropteroate synthase activity compared to an otherwise identical microorganism that does not carry said modification (reference microorganism). Thus, a genetically engineered microorganism of the invention may have an increased expression level of a polypeptide having dihydropteroate synthase activity compared to an otherwise identical microorganism that does not carry said modification (reference microorganism).

According to some embodiments, the polypeptide having dihydropteroate synthase activity comprises an amino acid sequence having at least 70%, such as at least 80%, at least 85%, at least 90%, at least 93%, at least 95%, at least 98% or at least 99%, sequence identity with SEQ ID NO: 10. According to some embodiments, the polypeptide having dihydropteroate synthase activity comprises an amino acid sequence having at least 85%, such as at least 90%, sequence identity with SEQ ID NO: 10. According to some embodiments, the polypeptide having dihydropteroate synthase activity comprises an amino acid sequence having at least 95%, such as at least 98%, sequence identity with SEQ ID NO: 10.

According to some embodiments, the genetically engineered microorganism of the invention has been (further) modified to have an increased expression level of a gene encoding a polypeptide having dihydrofolate reductase activity compared to an otherwise identical microorganism that does not carry said modification (reference microorganism). Thus, a genetically engineered microorganism of the invention may have an increased expression level of a polypeptide having dihydrofolate reductase activity compared to an otherwise identical microorganism that does not carry said modification (reference microorganism).

According to some embodiments, the polypeptide having dihydrofolate reductase activity comprises an amino acid sequence having at least 70%, such as at least 80%, at least 85%, at least 90%, at least 93%, at least 95%, at least 98% or at least 99%, sequence identity with SEQ ID NO: 12. According to some embodiments, the polypeptide having dihydrofolate reductase activity comprises an amino acid sequence having at least 85%, such as at least 90%, sequence identity with SEQ ID NO: 12. According to some embodiments, the polypeptide having dihydrofolate reductase activity comprises an amino acid sequence having at least 95%, such as at least 98%, sequence identity with SEQ ID NO: 12.

According to some embodiments, the genetically engineered microorganism of the invention has been (further) modified to have an increased expression level of a gene encoding a polypeptide having serine hydroxymethyltransferase activity compared to an otherwise identical microorganism that does not carry said modification (reference microorganism). Thus, a genetically engineered microorganism of the invention may have an increased expression level of a polypeptide having serine hydroxymethyltransferase activity compared to an otherwise identical microorganism that does not carry said modification (reference microorganism).

According to some embodiments, the polypeptide having serine hydroxymethyltransferase activity comprises an amino acid sequence having at least 70%, such as at least 80%, at least 85%, at least 90%, at least 93%, at least 95%, at least 98% or at least 99%, sequence identity with SEQ ID NO: 79. According to some embodiments, the polypeptide having serine hydroxymethyltransferase activity comprises an amino acid sequence having at least 85%, such as at least 90%, sequence identity with SEQ ID NO: 79. According to some embodiments, the polypeptide having serine hydroxymethyltransferase activity comprises an amino acid sequence having at least 95%, such as at least 98%, sequence identity with SEQ ID NO: 79.

According to some embodiments, the genetically engineered microorganism of the invention has been (further) modified to have an increased expression level of a gene encoding a polypeptide having formyltetrahydrofolate deformylase activity compared to an otherwise identical microorganism that does not carry said modification (reference microorganism). Thus, a genetically engineered microorganism of the invention may have an increased expression level of a polypeptide having formyltetrahydrofolate deformylase activity compared to an otherwise identical microorganism that does not carry said modification (reference microorganism).

According to some embodiments, the polypeptide having formyltetrahydrofolate deformylase activity comprises an amino acid sequence having at least 70%, such as at least 80%, at least 85%, at least 90%, at least 95%, at least 98% or at least 99%, sequence identity with SEQ ID NO: 81. According to some embodiments, the polypeptide having formyltetrahydrofolate deformylase activity comprises an amino acid sequence having at least 85%, such as at least 90%, sequence identity with SEQ ID NO: 81. According to some embodiments, the polypeptide having formyltetrahydrofolate deformylase activity comprises an amino acid sequence having at least 95%, such as at least 98%, sequence identity with SEQ ID NO: 81.

According to some embodiments, the genetically engineered microorganism of the invention has been (further) modified to have an increased expression level of a gene encoding a polypeptide having 5,10-methylenetetrahydrofolate reductase activity compared to an otherwise identical microorganism that does not carry said modification (reference microorganism). Thus, a genetically engineered microorganism of the invention may have an increased expression level of a polypeptide having 5,10-methylenetetrahydrofolate reductase activity compared to an otherwise identical microorganism that does not carry said modification (reference microorganism).

According to some embodiments, the polypeptide having 5,10-methylenetetrahydrofolate reductase activity comprises an amino acid sequence having at least 70%, such as at least 80%, at least 85%, at least 90%, at least 93%, at least 95%, at least 98% or at least 99%, sequence identity with SEQ ID NO: 83. According to some embodiments, the polypeptide having 5,10-methylenetetrahydrofolate reductase activity comprises an amino acid sequence having at least 85%, such as at least 90%, sequence identity with SEQ ID NO: 83. According to some embodiments, the polypeptide having 5,10-methylenetetrahydrofolate reductase activity comprises an amino acid sequence having at least 95%, such as at least 98%, sequence identity with SEQ ID NO: 83.

According to some embodiments, the polypeptide having 5,10-methylenetetrahydrofolate reductase activity comprises an amino acid sequence having at least 70%, such as at least 80%, at least 85%, at least 90%, at least 93%, at least 95%, at least 98% or at least 99%, sequence identity with SEQ ID NO: 84. According to some embodiments, the polypeptide having 5,10-methylenetetrahydrofolate reductase activity comprises an amino acid sequence having at least 85%, such as at least 90%, sequence identity with SEQ ID NO: 84. According to some embodiments, the polypeptide having 5,10-methylenetetrahydrofolate reductase activity comprises an amino acid sequence having at least 95%, such as at least 98%, sequence identity with SEQ ID NO: 84.

According to some embodiments, the at least one gene encoding an enzyme involved in the biosynthesis of a 5-methylfolate (such as 5-methyl-tetrahydrofolate) is selected from the group consisting of *folE*/*mtrA*, *folB*, *folK*, *folP*/*sul*, *folA*/*dfrA*, *glyA*, *purU*, *yitJ* and *metF.*

According to some embodiments, the at least one gene encoding an enzyme involved in the biosynthesis of a 5-methylfolate (such as 5-methyl-tetrahydrofolate) is heterologous to the genetically engineered microorganism.

According to some embodiments, the at least one gene encoding an enzyme involved in the biosynthesis of a 5-methylfolate is derived from a bacterium or fungus, preferably selected from the genus *Bacillus, Escherichia, Lactococcus, Shewanella, Vibrio* and *Ashbya.*

According to some embodiments, the at least one gene encoding an enzyme involved in the biosynthesis of a 5-methylfolate (such as 5-methyl-tetrahydrofolate) is derived from a bacterium or fungus selected from *Bacillus subtiltis*, *Lactobacillus lactis*, *Escherichia coli*, *Shewanella violacea, Vibrio natriegens* or *Ashbya gossypii.*

The present inventors have further found that the down-regulation or deletion of an endogenous gene encoding a polypeptide having 5-methyltetrahydropteroyltriglutamate-homocysteine S-methyltransferase activity (such as the gene *metE*), which is the main enzyme that metabolizes/consumes the 5-methyltetrahydrofolate, in the microorganism can significantly further increase the accumulation and production capacity of a 5-methyltetrahydrofolate, a salt, a precursor, or an intermediate thereof.

Thus, the genetically engineered microorganism of the invention may be (further) modified to have a decreased expression level of an endogenous gene encoding a polypeptide having 5-methyltetrahydropteroyltriglutamate-homocysteine S-methyltransferase activity compared to an otherwise identical microorganism that does not carry said modification (reference microorganism). The expression level of the endogenous gene may, for example, be decreased by at least 50%, such as by at least 60%, at least 65%, at least 70%, at least 75%, at least 80%, at least 85%, at least 90%, at least 95% or at least 100% compared to the otherwise identical microorganism.

According to some embodiments, the genetically engineered microorganism comprises at least one mutation in the regulatory region of the endogenous gene encoding a polypeptide having 5-methyltetrahydropteroyltriglutamate-homocysteine S-methyltransferase activity, resulting in the decreased expression level. For example, the at least one mutation in the regulatory region may be at least one nucleotide substitution at a position in close proximity (e.g., 1 or 2 nucleotides up- or downstream) to the Pribnow box (TATAAT) sequence, resulting in the decreased expression level of the encoded polypeptide. As the particular case may be, for example, if the microorganism is *Bacillus subtilis,* the nucleotide substitution may be at a position located 12 nucleotides upstream from the start codon of the endogenous gene. The at least one nucleotide substitution may, for example, be a substitution of one type of purine by another type of purine (such as guanine to adenine).

According to some embodiments, the genetically engineered microorganism has been modified by replacing the endogenous promoter operatively linked to the endogenous gene encoding the polypeptide having 5-methyltetrahydropteroyltriglutamate-homocysteine S-methyltransferase activity with an exogenous promoter which is weaker in its affinity for RNA polymerase compared to the endogenous promoter. As will be appreciated by those skilled in the art, the weaker affinity for RNA polymerase will result in decreased levels of transcription, and hence decreased levels of the corresponding polypeptide being producing the microorganism.

According to some embodiments, the endogenous gene encoding a polypeptide having 5-methyltetrahydropteroyltriglutamate-homocysteine S-methyltransferase activity has been inactivated, such as by deletion of part of or the entire gene sequence.

According to some embodiments, the endogenous gene encoding said polypeptide having 5-methyltetrahydropteroyltriglutamate-homocysteine S-methyltransferase activity has been inactivated by introducing or expressing in the microorganism a rare-cutting endonuclease able to selectively inactivating by DNA cleavage, preferably by double-strand break, the endogenous gene encoding said polypeptide. A rare-cutting endonuclease to be used in accordance of the present invention to inactivate the endogenous gene may, for instance, be a transcription activator-like effector (TALE) nuclease, meganuclease, zing-finger nuclease (ZFN), or RNA-guided endonuclease.

One way to inactivate the endogenous gene encoding said polypeptide 5-methyltetrahydropteroyltriglutamate-homocysteine S-methyltransferase activity is to use the CRISPRi system as mentioned above. Thus, according to some embodiments, the endogenous gene encoding said polypeptide having 5-methyltetrahydropteroyltriglutamate-homocysteine S-methyltransferase activity is inactivated by introducing or expressing in the microorganism a RNA-guided endonuclease, such as a catalytically inactive Cas9 protein, and a single guide RNA (sgRNA) specifically hybridizing (e.g. binding) under cellular conditions with the genomic DNA encoding said polypeptide.

By way of example, if the endogenous gene encoding said polypeptide having having 5-methyltetrahydropteroyltriglutamate-homocysteine S-methyltransferase activity is to be inhibited in Bacillus subtiltis, the single guide RNA (sgRNA) may comprise at least 20 consecutive nucleotides of SEQ ID NO: 101 or its complement.

According to some embodiments, the expression of an endogenous polypeptide having 5-methyltetrahydropteroyltriglutamate-homocysteine S-methyltransferase activity is decreased by way of inhibition.

Inhibition of the expression of said endogenous polypeptide having 5-methyltetrahydropteroyltriglutamate-homocysteine S-methyltransferase activity may be achieved by any suitable means known in the art. For example, the expression may be inhibited by gene silencing techniques involving the use of inhibitory nucleic acid molecules, such as antisense oligonucleotides, ribozymes or interfering RNA (RNAi) molecules, such as microRNA (miRNA), small interfering RNA (siRNA) or short hairpin RNA (shRNA).

According to some embodiments, the expression of said endogenous polypeptide having 5-methyltetrahydropteroyltriglutamate-homocysteine S-methyltransferase activity is decreased (e.g., inhibited) by transcriptional and/or translational repression of the endogenous gene encoding said polypeptide.

According to some embodiments, the expression of said endogenous polypeptide having 5-methyltetrahydropteroyltriglutamate-homocysteine S-methyltransferase activity is inhibited by introducing or expressing in the microorganism an inhibitory nucleic acid molecule. For example, the inhibitory nucleic acid molecule may be introduced by way of an exogenous nucleic acid molecule comprising a nucleotide sequence encoding said inhibitory nucleic acid molecule operably linked to a promoter, such as an inducible promoter, that is functional in the microorganism to cause the production of said inhibitory nucleic acid molecule. Suitably, the inhibitory nucleic acid molecule is one that specifically hybridizes (e.g. binds) under cellular conditions with cellular mRNA and/or genomic DNA encoding the endogenous polypeptide.

According to some embodiments, the inhibitory nucleic acid molecule is an antisense oligonucleotide, ribozyme or interfering RNA (RNAi) molecule. Preferably, such nucleic acid molecule comprises at least 10 consecutive nucleotides of the complement of the cellular mRNA and/or genomic DNA encoding the polypeptide.

By way of example, if the expression of an endogenous polypeptide having 5-methyltetrahydropteroyltriglutamate-homocysteine S-methyltransferase activity is to be inhibited in *Bacillus subtilis,* such inhibitory nucleic acid molecule may comprise at least 10 consecutive nucleotides of the complement of SEQ ID NO: 101.

According to some embodiments, the inhibitory nucleic acid is an antisense oligonucleotide. Such antisense oligonucleotide is a nucleic acid molecule (either DNA or RNA) which specifically hybridizes (e.g. binds) under cellular conditions with the cellular mRNA and/or genomic DNA encoding the polypeptide.

According to some embodiments, the inhibitory nucleic acid molecule is a ribozyme, such as a hammerhead ribozyme. A ribozyme molecule is designed to catalytically cleave the mRNA transcript to prevent translation of the polypeptide.

According to some embodiments, the inhibitory nucleic acid molecule is an interfering RNA (RNAi) molecule. According to some embodiments, the RNAi molecule is a miRNA. According to some embodiments, the RNAi molecule is a siRNA. According to some embodiments, the RNAi molecule is a shRNA.

By way of example, if the expression of an endogenous polypeptide having 5-methyltetrahydropteroyltriglutamate-homocysteine S-methyltransferase activity is to be inhibited in *Bacillus subtilis,* the RNAi molecule may be an interfering RNA complementary to SEQ ID NO: 101. The RNAi molecule may be a ribonucleic acid molecule comprising at least 10 consecutive nucleotides of the complement of SEQ ID NO: 101. The RNAi molecule may be a double-stranded ribonucleic acid molecule comprising a first strand identical to 20 to 25, such as 21 to 23, consecutive nucleotides of SEQ ID NO: 101, and a second strand complementary to said first strand.

According to some embodiments, the genetically engineered microorganism of the invention has been modified to have a decreased activity of an endogenous polypeptide having 5-methyltetrahydropteroyltriglutamate-homocysteine S-methyltransferase activity compared to an otherwise identical microorganism that does not carry said modification (reference microorganism).

A decrease of the activity of the endogenous polypeptide having having 5-methyltetrahydropteroyltriglutamate-homocysteine S-methyltransferase activity may be achieved by any suitable means known in the art. For example, the activity may be decreased by introducing one or more mutations in the active site of the polypeptide resulting in the reduction or loss of activity. Thus, according to some embodiments, the activity of the endogenous polypeptide having 5-methyltetrahydropteroyltriglutamate-homocysteine S-methyltransferase activity is decreased by at least one active-site mutation resulting in the reduction or loss of activity. The at least one active-site mutation may, for example, be at least one non-conservative amino acid substitution.

By way of example, if the activity of the endogenous polypeptide having having 5-methyltetrahydropteroyltriglutamate-homocysteine S-methyltransferase activity is to be decreased in *Bacillus subtilis,* the at least one active-site mutation may occur at any one of positions 18, 21, 112, 117, 119, 435-437, 488, 494, 519-521, 565, 601, 603, 605, 645, 647, 669, 730 and 731 in the amino acid sequence set forth in SEQ ID NO: 100, which form part of the active site. In case of orthologous polypeptides, the at least one active-site mutation may be at a position which corresponds to any one of positions 18, 21, 112, 117, 119, 435-437, 488, 494, 519-521, 565, 601, 603, 605, 645, 647, 669, 730 and 731 in the amino acid sequence set forth in SEQ ID NO: 100. According to some embodiments, the endogenous gene encoding a polypeptide having 5-methyltetrahydropteroyltriglutamate-homocysteine S-methyltransferase activity is the gene *metE.*

According to some embodiments, the endogenous gene encoding a polypeptide having 5-methyltetrahydropteroyltriglutamate-homocysteine S-methyltransferase activity comprises a nucleic acid sequence having at least 70%, such as at least 80%, at least 85%, at least 90%, at least 93%, at least 95%, at least 98% or at least 99%, sequence identity with the nucleic acid sequence set forth in SEQ ID NO: 101. According to some embodiments, the endogenous gene encoding a polypeptide having 5-methyltetrahydropteroyltriglutamate-homocysteine S-methyltransferase activity comprises a nucleic acid sequence having at least 85%, such as at least 90%, sequence identity with the nucleic acid sequence set forth in SEQ ID NO: 101. According to some embodiments, the endogenous gene encoding a polypeptide having 5-methyltetrahydropteroyltriglutamate-homocysteine S-methyltransferase activity comprises a nucleic acid sequence having at least 95%, such as at least 98%, sequence identity with the nucleic acid sequence set forth in SEQ ID NO: 101.

According to some embodiments, the polypeptide having polypeptide having 5-methyltetrahydropteroyltriglutamate-homocysteine S-methyltransferase activity encoded by the endogenous gene comprises an amino acid which has at least 70%, such as at least 80, at least 85%, at least 90%, at least 93%, at least 95%, at least 98% or at least 99%, sequence identity with the amino acid sequence set forth in SEQ ID NO: 100. According to some embodiments, the polypeptide having polypeptide having 5-methyltetrahydropteroyltriglutamate-homocysteine S-methyltransferase activity encoded by the endogenous gene comprises an amino acid which has at least 85%, such as at least 90%, sequence identity with the amino acid sequence set forth in SEQ ID NO: 100.

According to some embodiments, the polypeptide having polypeptide having 5-methyltetrahydropteroyltriglutamate-homocysteine S-methyltransferase activity encoded by the endogenous gene comprises an amino acid which has at least 95%, such as at least 98%, sequence identity with the amino acid sequence set forth in SEQ ID NO: 100.

Generally, a microorganism as referred to herein may be any suitable microorganism, including single-celled or multicellular microorganisms such as bacteria or yeast.

Bacterial microorganisms may be Gram-positive or Gram-negative bacteria. Non-limiting examples for Gram-negative bacteria include species from the genera *Escherichia, Erwinia, Klebsiella* and *Citrobacter.* Non-limiting examples of Gram-positive bacteria include species from the genera *Bacillus, Lactococcus, Lactobacillus, Geobacillus, Pediococcus, Moorella, Clostridium, Corynebacterium, Streptomyces, Streptococcus,* and *Cellulomonas.*

According to some embodiments, the microorganism is a bacterium, which may be a bacterium of the genus *Bacillus, Lactococcus, Lactobacillus, Clostridium, Corynebacterium, Geobacillus, Streptococcus, Pediococcus, Moorella, Pseudomonas, Streptomyces, Escherichia, Shigella, Acinetobacter, Citrobacter, Salmonella, Klebsiella, Enterobacter, Erwinia, Kluyvera, Serratia, Cedecea, Morganella, Hafnia, Edwardsiella, Providencia, Proteus,* or *Yersinia.*

According to some embodiments, the microorganism is a bacterium of the genus *Escherichia.* A non-limiting example of a bacterium of the genus Escherichia is *Escherichia coli.* According to some embodiments, the microorganism is *Escherichia coli.*

According to some embodiments, the microorganism is a bacterium of the genus *Bacillus.* Non-limiting examples of a bacterium of the genus *Bacillus* are *Bacillus subtitlis, Bacillus amyloliquefaciens, Bacillus licheniformis,* and *Bacillus mojavensis.* According to some embodiments, the microorganism is *Bacillus subtitlis.*

Yeast cells may be derived from e.g., *Saccharomyces, Pichia, Schizosacharomyces, Zygosaccharomyces, Hansenula, Pachyosolen, Kluyveromyces, Debaryomyces, Yarrowia, Candida, Cryptococcus, Komagataella, Lipomyces, Rhodospiridium, Rhodotorula, or Trichosporon.*

According to some embodiments, the microorganism is a yeast of the genus *Saccharomyces.* A non-limiting example of a yeast of the genus Saccharomyces is *Saccharomyces cerevisiae.* According to certain embodiments, the microorganism is *Saccharomyces cerevisiae.*

As noted above, a genetically engineered microorganism of the invention may be modified to express one or more polypeptides as detailed herein, which means that one or more exogenous nucleic acid molecules, such as DNA molecules, which comprise(s) a nucleotide sequence or nucleotide sequences encoding said polypeptide or polypeptides has been introduced in the microorganism. Techniques for introducing exogenous nucleic acid molecule, such as a DNA molecule, into the various host cells are well-known to those of skill in the art, and include transformation (e.g., heat shock or natural transformation), transfection, conjugation, electroporation, microinjection and microparticle bombardment.

Accordingly, a genetically engineered microorganism of the invention may comprise an exogenous nucleic acid molecule comprising a nucleotide sequence encoding a polypeptide as detailed herein. In order to facilitate expression of a polypeptide in the microorganism, the exogenous nucleic acid molecule may comprise suitable regulatory elements such as a promoter that is functional in the host cell to cause the production of an mRNA molecule and that is operably linked to the nucleotide sequence encoding said polypeptide. Promoters useful in accordance with the invention are any known promoters that are functional in a given host cell to cause the production of an mRNA molecule. Many such promoters are known to the skilled person. Such promoters include promoters normally associated with other genes, and/or promoters isolated from any bacteria, yeast, fungi, alga or plant cell. The use of promoters for protein expression is generally known to those of skilled in the art of molecular biology, for example, see Sambrook et al., Molecular cloning: A Laboratory Manual, Cold Spring Harbor Laboratory, Cold Spring Harbor, N.Y. , 1989. Besides a promoter, the exogenous nucleic acid molecule may further comprise at least one regulatory element selected from a 5' untranslated region (5'UTR) and 3' untranslated region (3' UTR). Many such 5' UTRs and 3' UTRs derived from prokaryotes and eukaryotes are well known to the skilled person.

The exogenous nucleic acid molecule may be a vector or part of a vector, such as an expression vector. Normally, such a vector remains extrachromosomal within the microorganism which means that it is found outside of the nucleus or nucleoid region of the microorganism. It is also contemplated by the present invention that the exogenous nucleic acid molecule is stably integrated into the genome of the host cell. Means for stable integration into the genome of a microorganism, e.g., by homologous recombination, are well known to the skilled person.

It is understood that the details given herein with respect to a genetically engineered microorganism apply to other aspects of the invention, in particular to the methods according to the invention, which are described in more detail below.

### Methods of the invention

In a second aspect, the present invention provides a method for preparing a folate, precursor or intermediate thereof. Particularly, a method for preparing a folate, precursor or intermediate thereof comprises:
i) cultivating a genetically engineered microorganism according to the invention in a culture medium under suitable culture conditions to obtain a fermentation product containing said folate, precursor or intermediate thereof; and
ii) optionally, separating and/ or purifying said folate, precursor or intermediate thereof.

The medium employed may be any conventional medium suitable for culturing the host cell in question, and may be composed according to the principles of the prior art. The medium will usually contain all nutrients necessary for the growth and survival of the respective host cell, such as carbon and nitrogen sources and other inorganic salts. Suitable media, e.g. minimal or complex media, are available from commercial suppliers, or may be prepared according to published receipts, e.g. the American Type Culture Collection (ATCC) Catalogue of strains. Non-limiting standard medium well known to the skilled person include Luria Bertani (LB) broth, Sabouraud Dextrose (SD) broth, MS broth, Yeast Peptone Dextrose, BMMY, GMMY, or Yeast Malt Extract (YM) broth, which are all commercially available. A non-limiting example of suitable media for culturing bacterial cells, such as *B. subtilis* or *E. coli* cells, including minimal media and rich media such as Luria Broth (LB), M9 media, M17 media, SA media, MOPS media, Terrific Broth, YT and others. Suitable media for culturing eukaryotic cells, such as yeast cells, are RPMI 1640, MEM, DMEM, all of which may be supplemented with serum and/or growth factors as required by the particular host cell being cultured. The medium for culturing eukaryotic cells may also be any kind of minimal media such as Yeast minimal media.

Suitable conditions for culturing the respective microorganism are well known to the skilled person. Typically, the genetically engineered microorganism is cultured at a temperature ranging from 32 to about 42°C, preferably in a range from 34 to 39°C, more preferably in a range from 36 to 39°C, such as at about 37°C. The pH of the medium may be in a range from 6 to 8, preferably in a range from 6.5 to 7.5, more preferably in a range from 6.8 to 7.2. The cultivation in step i) may be carried out for a period in the range from 10 to 70 h, preferably in a range from 24 to 60 h, more preferably in a range from 36 to 50 h.

The method may further comprise ii) separating and/ or purifying said folate, precursor or intermediate thereof. The folate, precursor or intermediate thereof may be separated and/or purified by any conventional method for isolation and purification chemical compounds from a medium. Well-known purification procedures include centrifugation or filtration, precipitation, and chromatographic methods such as e.g. ion exchange chromatography, gel filtration chromatography, etc.

The folate prepared by the method of the invention is preferably a compound of Formula I: 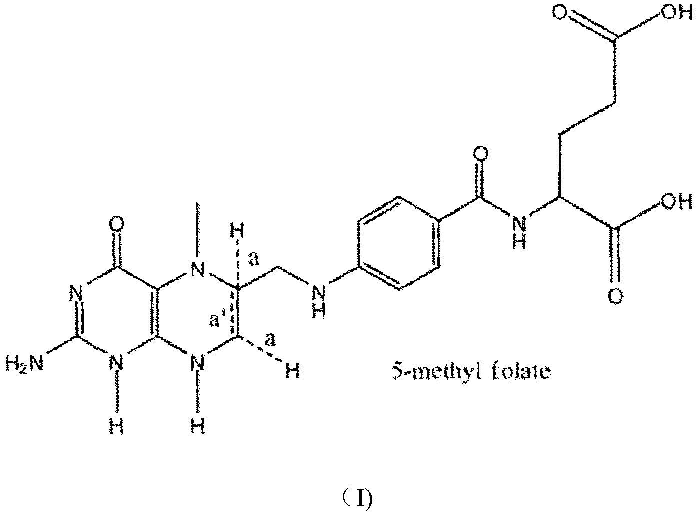 optionally in form of one of the stereoisomers, preferably enantiomers or diastereomers, in form of a racemate or in form of a mixture of at least two of the stereoisomers, preferably enantiomers and/or diastereomers, in any mixing ratio.

In the generic formula (I) above, it is meant that when a is single bond, a' is none, or when a' is a single bond, a is none.

According to certain embodiments, the folate prepared by the method of the invention is compound of Formula II (5-methyltetrahydrofolate): 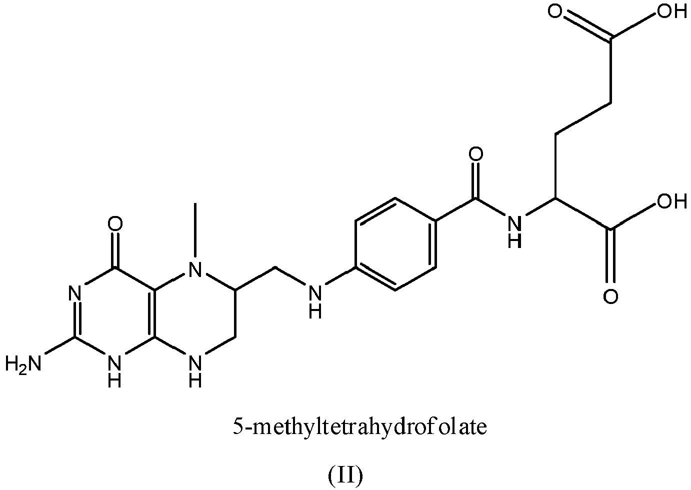 optionally in form of one of the stereoisomers, preferably enantiomers or diastereomers, in form of a racemate or in form of a mixture of at least two of the stereoisomers, preferably enantiomers and/or diastereomers, in any mixing ratio.

According to particular embodiments, the folate prepared by the method of the invention is compound of Formula IIa (L-5-methyltetrahydrofolate; (6S)-5-methyltetrahydrofolate):

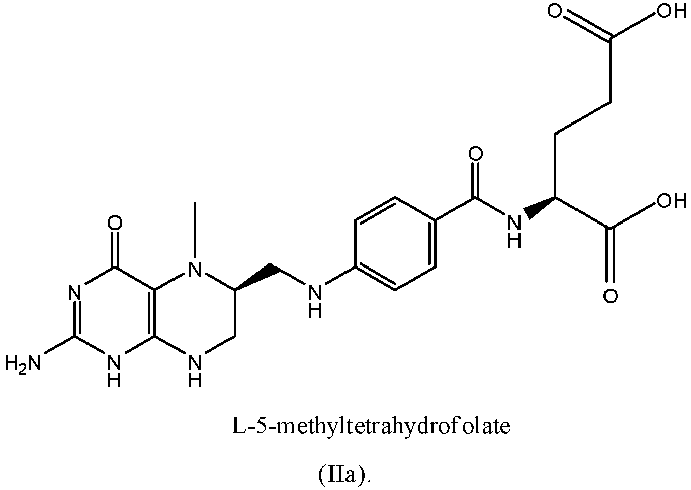

According to certain embodiments, the folate prepared by the method of the invention is compound of Formula III (5-methyldihydrofolate): 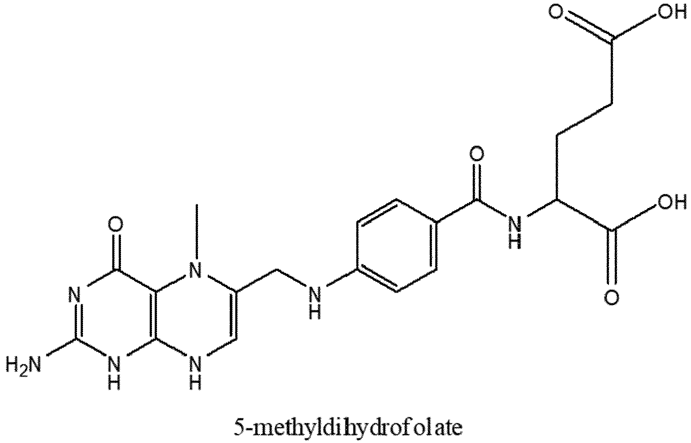 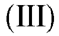 optionally in form of one of the stereoisomers, preferably enantiomers or diastereomers, in form of a racemate or in form of a mixture of at least two of the stereoisomers, preferably enantiomers and/or diastereomers, in any mixing ratio.

The inventors have also surprisingly found that the addition of *para*-aminobenzoic acid (PABA) during the cultivation of the strain, obtained as described above, can significantly further increase the production capacity of a folate, a salt, a precursor, or an intermediate thereof. Thus, according to some embodiments, the method further comprises the step of adding *para*-aminobenzoic acid (PABA) during the cultivation step (i).

The *para*-aminobenzoic acid (PABA) may, for example, be a PABA selected from the group consisting of: potassium *para*-aminobenzoate, sodium *para*-aminobenzoate, methyl *para*-aminobenzoate, ethyl *para*-aminobenzoate, butyl *para*-aminobenzoate, or a combination thereof.

According to some embodiments, the method further comprises subjecting the product obtained in the steps (i) or (ii) to acidic or alkaline conditions to further obtain a derivative compound.

In a further aspect, the present invention provides a method of preparing a genetically engineered microorganism of the present invention. Particularly, the method of preparing a genetically engineered microorganism of the present invention comprises any one (such as all) of the steps (a) to (d) below:
(a) increased the expression level of at least one (such as at least two, at least three, at least four, at least five, at least six, at least seven or at least eight) enzyme involved in the biosynthesis of a 5-methylfolate (such as 5-methyl-tetrahydrofolate) compared to an otherwise identical microorganism (reference microorganism);
(b) decreasing the expression and/or activity of an endogenous polypeptide having 5-methyltetrahydropteroyltriglutamate-homocysteine S-methyltransferase activity compared to an otherwise identical microorganism that does not carry said modification (reference microorganism);
(c) decreasing the expression and/or activity of an endogenous polypeptide having both dihydrofolate synthase activity and folylpolyglutamate synthetase activity compared to an otherwise identical microorganism that does not carry said modification (reference microorganism); and/or
(d) expressing a heterologous polypeptide having only dihydrofolate synthase activity.

According to some embodiments, the method comprises any one of the steps (a) to (b) below:
(a) increased the expression level of at least one enzyme involved in the biosynthesis of a 5-methylfolate compared to an otherwise identical microorganism (reference microorganism); and
(b) decreasing the expression level of an endogenous gene encoding a polypeptide having 5-methyltetrahydropteroyltriglutamate-homocysteine S-methyltransferase activity compared to an otherwise identical microorganism (reference microorganism);
optionally further comprising the steps (c) and (d) below:
(c) decreasing the expression level of the endogenous gene encoding a polypeptide having both dihydrofolate synthase activity and folylpolyglutamate synthetase activity compared to an otherwise identical microorganism (reference microorganism); and
(d) expressing a heterologous polypeptide having only dihydrofolate synthase activity.

According to some embodiments, the method of preparing a genetically engineered microorganism of the present invention comprises the steps of aa) introducing into said microorganism at least one exogenous nucleic acid molecule comprising a nucleic acid sequence encoding an enzyme involved in the biosynthesis of a 5-methylfolate (such as 5-methyl-tetrahydrofolate); bb) inactivating, such as by deleting part of or the entire gene sequence, the endogenous gene encoding a polypeptide having 5-methyltetrahydropteroyltriglutamate-homocysteine S-methyltransferase activity or introducing at least one mutation in the regulatory region of said endogenous gene, which results in the decrease expression level; cc) inactivating, such as by deleting part of or the entire gene sequence, the endogenous gene encoding a polypeptide having both dihydrofolate synthase activity and folylpolyglutamate synthetase activity in said microorganism; and/or dd) introducing into said microorganism an exogenous nucleic acid molecule comprising a nucleic acid sequence encoding a heterologous polypeptide having only dihydrofolate synthase activity.

### Certain definitions

5-methylfolate as used herein means any one of 5-methyltetrahydrofolate and 5-methyldihydrofolate including any stereoisomer form and protonated (acid) or deprotonated (salt) form thereof.

The phrase "ability to produce 5-methlyfolate" means that the microorganism, such as a bacterium, is able to produce, excrete or secrete, and /or cause accumulation of 5-methylfolate in a culture medium or in the microorganism when the microorganism is cultured in the medium. A microorganism may be considered as having the ability to produce 5-methlyfolate, if it expresses all enzymes involved in the biosynthetic pathway resulting in 5-methlyfolate.

The phrase "ability to produce 5-methyl-tetrahydrofolate (5-methyl-THF)" means that the microorganism, such as a bacterium, is able to produce, excrete or secrete, and /or cause accumulation of 5-methyl-THF in a culture medium or in the microorganism when the microorganism is cultured in the medium. A microorganism may be considered as having the ability to produce 5-methly-THF, if it expresses all enzymes involved in the biosynthetic pathway resulting in 5-methly-THF.

As used herein, a "polypeptide having both dihydrofolate synthase activity and folylpolyglutamate synthetase activity" means a polypeptide that catalyzes the reaction: ATP + 7,8-dihydropteroate + L-glutamate <=> ADP + phosphate + 7,8-dihydropteroylglutamate (EC 6.3.2.12) and the reaction: ATP + tetrahydropteroyl-(gamma-Glu)(n) + L-glutamate <=> ADP + phosphate + tetrahydropteroyl-(gamma-Glu)(n+1) (EC 6.3.2.17). A polypeptide having both dihydrofolate synthase activity and folylpolyglutamate synthetase activity is, for example, encoded by the gene *folC* found in, e.g., *Bacillus subtilis.* Further information regarding *folC* of, e.g., *Bacillus subtilis* is available at KEGG (https://www.kegg.jp/kegg/genes.html) under Accession number BSU28080. See also NCBI Reference Sequence: NP_390686.1 for the amino acid sequence *(B. subtilis).*

As used herein, a "polypeptide having only dihydrofolate synthase activity" means a polypeptide that catalyzes only the reaction: ATP + 7,8-dihydropteroate + L-glutamate <=> ADP + phosphate + 7,8-dihydropteroylglutamate (EC 6.3.2.12). A polypeptide having only dihydrofolate synthase activity is, for example, encoded by the gene *folC2* found in, e.g., *Ashbya gossypii* and *Lactobacillus reuteri.* Further information regarding folC2 of, e.g., *Ashbya gossypii* and *Lactobacillus reuteri* is available at KEGG (https://www.kegg.jp/kegg/genes.html) under Accession number AGOS_AEL310C and Lreu_1277, respectively. See also NCBI Reference Sequence: NP_984550.1 (*Ashbya gossypii)* and WP_003668526.1 (*Lactobacillus reuteri*) for the amino acid sequence.

As used herein, a "polypeptide having GTP cyclohydrolase activity" means a polypeptide that catalyzes the reaction: GTP + H(2)O <=> formate + 2-amino-4-hydroxy-6-(erythro-1,2,3-trihydroxypropyl)-dihydropteridine triphosphate (EC 3.5.4.16). A polypeptide having GTP cyclohydrolase activity is, for example, encoded by the gene *folE* found in, e.g., *Bacillus subtilis.* Further information regarding *folE* of, e.g., *Bacillus subtilis* is available at KEGG (https://www.kegg.jp/kegg/genes.html) under Accession number BSU22780. See also NCBI Reference Sequence: NP_390159.1 for the amino acid sequence *(B. subtilis).*

As used herein, a "polypeptide having 7,8-dihydroneopterin aldolase activity" means a polypeptide that catalyzes the reaction: 7,8-dihydroneopterin <=> 6-hydroxymethyl-7,8-dihydropterin + glycolaldehyde (EC 4.1.2.25). A polypeptide having 7,8-dihydroneopterin aldolase activity is, for example, encoded by the gene *folB* found in, e.g., *Bacillus subtilis.* Further information regarding *folB* of, e.g., *Bacillus subtilis* is available at KEGG (https://www.kegg.jp/kegg/genes.html) under Accession number BSU00780. See also NCBI Reference Sequence: NP_387959.1 for the amino acid sequence *(B. subtilis).*

As used herein, a "polypeptide having 2-amino-4-hydroxy-6-hydroxymethyldihydropteridine pyrophosphokinase activity" means a polypeptide that catalyzes the reaction: ATP + 6-hydroxymethyl-7,8-dihydropterin <=> AMP + 6-hydroxymethyl-7,8-dihydropterin diphosphate (EC 2.7.6.3). A polypeptide having 2-amino-4-hydroxy-6-hydroxymethyldihydropteridine pyrophosphokinase activity is, for example, encoded by the *gene folK* found in, e.g., *Bacillus subtilis.* Further information regarding *folK* of, e.g., *Bacillus subtilis* is available at KEGG (https://www.kegg.jp/kegg/genes.html) under Accession number BSU00790. See also NCBI Reference Sequence: NP_387960.1 for the amino acid sequence *(B. subtilis).*

As used herein, a "polypeptide having dihydropteroate synthase activity" means a polypeptide that catalyzes the reaction: 6-hydroxymethyl-7,8-dihydropterin diphosphate + 4-aminobenzoate <=> diphosphate + dihydropteroate (EC 2.5.1.15). A polypeptide having dihydropteroate synthase activity is, for example, encoded by the gene *folP* found in, e.g., *Bacillus subtilis.* Further information regarding *folP* of, e.g., *Bacillus subtilis* is available at KEGG (https://www.kegg.jp/kegg/genes.html) under Accession number BSU00770. See also NCBI Reference Sequence: NP_387958.1 for the amino acid sequence *(B. subtilis).*

As used herein, a "polypeptide having dihydrofolate reductase activity" means a polypeptide that catalyzes the reaction: 5,6,7,8-tetrahydrofolate + NADP(+) <=> 7,8-dihydrofolate + NADPH (EC 1.5.1.3). A polypeptide having dihydrofolate reductase activity is, for example, encoded by the gene *folA* found in, e.g., *Bacillus subtilis.* Further information regarding *folA* of, e.g., *Bacillus subtilis* is available at KEGG (https://www.kegg.jp/kegg/genes.html) under Accession number BSU21810. See also NCBI Reference Sequence: NP_390064.1 for the amino acid sequence *(B. subtilis).*

As used herein, a "polypeptide having serine hydroxymethyltransferase activity" means a polypeptide that catalyzes the reaction: 5,10-methylenetetrahydrofolate + glycine + H(2)O <=> tetrahydrofolate + L-serine (EC 2.1.2.1). A polypeptide having serine hydroxymethyltransferase activity is, for example, encoded by the gene *glyA* found in, e.g., *Bacillus subtilis.* Further information regarding *glyA* of, e.g., *Bacillus subtilis* is available at KEGG (https://www.kegg.jp/kegg/genes.html) under Accession number BSU36900. See also NCBI Reference Sequence: NP_391571.1 for the amino acid sequence *(B. subtilis).*

As used herein, a "polypeptide having formyltetrahydrofolate deformylase activity" means a polypeptide that catalyzes the reaction: 10-formyltetrahydrofolate + H(2)O <=> formate + tetrahydrofolate (EC 5.5.1.10). A polypeptide having formyltetrahydrofolate deformylase activity is, for example, encoded by the gene *purU* found in, e.g., *Bacillus subtilis.* Further information regarding *purU* of, e.g., *Bacillus subtilis* is available at KEGG (https://www.kegg.jp/kegg/genes.html) under Accession number BSU13110. See also NCBI Reference Sequence: NP_389194.2 for the amino acid sequence *(B. subtilis).*

As used herein, a "polypeptide having 5,10-methylenetetrahydrofolate reductase activity" means a polypeptide that catalyzes the reaction: 5-methyltetrahydrofolate + NAD(P)(+) <=> 5,10-methylenetetrahydrofolate + NAD(P)H (EC 1.5.1.20). A polypeptide having 5,10-methylenetetrahydrofolate reductase activity is, for example, encoded by the gene *yitJ* found in, e.g., *Bacillus subtilis.* Further information regarding *yitJ* of, e.g., *Bacillus subtilis* is available at KEGG (https://www.kegg.jp/kegg/genes.html) under Accession number BSU11010. See also NCBI Reference Sequence: NP_388982.1 for the amino acid sequence *(B. subtilis).* A polypeptide having 5,10-methylenetetrahydrofolate reductase activity is, for example, encoded by the gene *metF* found in, e.g., *Escherichia coli.* Further information regarding *metF* of, e.g., *Escherichia coli* is available at KEGG (https://www.kegg.jp/kegg/genes.html) under Accession number b3941. See also NCBI Reference Sequence: NP_418376.1 for the amino acid sequence *(B. subtilis).*

As used herein, a "polypeptide having 5-methyltetrahydropteroyltriglutamate-homocysteine S-methyltransferase activity" means a polypeptide that catalyzes the reaction: 5-methyltetrahydropteroyltri-L-glutamate + L-homocysteine <=> tetrahydropteroyltri-L-glutamate + L-methionine (EC 2.1.1.14). A polypeptide having 5-methyltetrahydropteroyltriglutamate-homocysteine S-methyltransferase activity is, for example, encoded by the gene *metE* found in, e.g., *Bacillus subtilis.* Further information regarding *metE* of, e.g., *Bacillus subtilis* is available at KEGG (https://www.kegg.jp/kegg/genes.html) under Accession number BSU13180. See also NCBI Reference Sequence: NP_389201.2 for the amino acid sequence *(B. subtilis).*

"Heterologous" or "exogenous" as used herein in the context of a gene or nucleic acid molecule refer to a gene or nucleic acid molecule (i.e. DNA or RNA molecule) that does not occur naturally as part of the genome of the microorganism in which it is present or which is found in a location or locations in the genome that differ from that in which it occurs in nature. Thus, a "heterologous" or "exogenous" gene or nucleic acid molecule is not endogenous to the microorganism and has been exogenously introduced into the microorganism. A "heterologous" gene or nucleic acid molecule DNA molecule may be from a different organism, a different species, a different genus or a different kingdom, as the host DNA.

"Heterologous" as used herein in the context of a polypeptide (such as an enzyme) means that a polypeptide is normally not found in or made (i.e. expressed) by the host microorganism, but derived from a different organism, a different species, a different genus or a different kingdom.

As used herein, the term "ortholog" or "orthologs" refers to genes, nucleic acid molecules encoded thereby, i.e., mRNA, or proteins encoded thereby that are derived from a common ancestor gene but are present in different species.

By "decreased expression level" of a gene it is meant that the amount of the transcription product, respectively the amount of the polypeptide encoded by said gene produced by the genetically engineered microoganism is decreased compared to an otherwise identical microorganism that does not carry said modification. More particularly, by "decreased expression level" of a gene it is meant that the amount of the transcription product, respectively the amount of the polypeptide encoded by said gene produced by the genetically engineered microorganism is decreased by at least 10%, such as at least 20%, at least 30%, at least 40%, at least 50% at least 60%, at least 70%, at least 80%, at least 90% or at least 100%, compared to an otherwise identical microorganism that does not carry said modification. The level of expression of a gene can be determined by well-known methods, including PCR, Southern blotting, and the like. In addition, the level of gene expression can be estimated by measuring the amount of mRNA transcribed from the gene using various well-known methods, including Northern blotting, quantitative RT-PCR, and the like. The amount of the polypeptide encoded by the gene can be measured by well-known methods, including ELISA, Immunohistochemistry or Western Blotting and the like.

Expression of a gene can be decreased by introducing a mutation into the gene in the genome of the microorganism so that the intracellular activity of the polypeptide encoded by the gene is decreased as compared to an otherwise identical microorganism that does not carry said mutation. Mutations which result in a decreased expression of the gene include the replacement of one nucleotide or more to cause an amino acid substitution in the polypeptide encoded by the gene (missense mutation), introduction of a stop codon (nonsense mutation), deletion or insertion of nucleotides to cause a frame shift, insertion of a drug-resistance gene, or deletion of a part of the gene or the entire gene (Qiu and Goodman, 1997; Kwon et al., 2000). Expression can also be decreased by modifying an expression regulating sequence such as the promoter, the Shine-Dalgarno (SD) sequence, etc. Expression of the gene can also be decreased by gene replacement (Datsenko and Wanner, 2000), such as the "lambda-red mediated gene replacement". The *lambda*-red mediated gene replacement is a particularly suitable method to inactive one or more genes as described herein.

"Inactivating", "inactivation" and "inactivated", when used in the context of a gene, means that the gene in question no longer expresses a functional protein. It is possible that the modified DNA region is unable to naturally express the gene due to the deletion of a part of or the entire gene sequence, the shifting of the reading frame of the gene, the introduction of missense/nonsense mutation(s), or the modification of an adjacent region of the gene, including sequences controlling gene expression, such as a promoter, enhancer, attenuator, ribosome- binding site, etc. Preferably, a gene of interest is inactivated by deletion of a part of or the entire gene sequence, such as by gene replacement. Inactivation may also be accomplished by introducing or expressing a rare-cutting endonuclease able to selectively inactivating by DNA cleavage, preferably by double-strand break, the gene of interest. A "rare-cutting endonuclease" within the context of the present invention includes transcription activator-like effector (TALE) nucleases, meganucleases, zing-finger nucleases (ZFN), and RNA-guided endonucleases.

The presence or absence of a gene in the genome of a microorganism, such as a bacterium, can be detected by well-known methods, including PCR, Southern blotting, and the like. In addition, the level of gene expression can be estimated by measuring the amount of mRNA transcribed from the gene using various well-known methods, including Northern blotting, quantitative RT-PCR, and the like. The amount of the protein encoded by the gene can be measured by well-known methods, including SDS-PAGE followed by an immunoblotting assay (Western blotting analysis), and the like.

By "increased expression level" of a gene it is meant that the amount of the transcription product, respectively the amount of the polypeptide encoded by said gene produced by the genetically engineered microoganism is increased compared to an otherwise identical microorganism that does not carry said modification. More particularly, by "increased expression level" of a gene it is meant that the amount of the transcription product, respectively the amount of the polypeptide encoded by said gene produced by the genetically engineered microorganism is increased by at least 10%, such as at least 20%, at least 30%, at least 40%, at least 50% at least 60%, at least 70%, at least 80%, at least 90%, at least 100%, at least 150%, at least 200%, at least 300%, at least 400%, at least 500%, at least 600%, at least 700% at least 800%, at least about 900%, at least about 1000%, at least about 2000%, at least about 3000%, at least about 4000%, at least about 5000%, at least about 6000%, at least about 7000%, at least about 8000% at least about 9000% or at least about 10000%, compared to an otherwise identical microorganism that does not carry said modification. The level of expression of a gene can be determined by well-known methods, including PCR, Southern blotting, and the like. In addition, the level of gene expression can be estimated by measuring the amount of mRNA transcribed from the gene using various well-known methods, including Northern blotting, quantitative RT-PCR, and the like. The amount of the polypeptide encoded by the gene can be measured by well-known methods, including ELISA, Immunohistochemistry or Western Blotting and the like.

By "increased expression level" of a polypeptide it is meant that the amount of the polypeptide in question produced by the genetically engineered microorganism is increased compared an otherwise identical microorganism that does not carry said modification. More particularly, by "increased expression level" of a polypeptide it is meant that the amount of the polypeptide in question produced by the genetically engineered microorganism is increased by at least 10%, such as at least 20%, at least 30%, at least 40%, at least 50% at least 60%, at least 70%, at least 80%, at least 90%, at least 100%, at least 150%, at least 200%, at least 300%, at least 400%, at least 500%, at least 600%, at least 700% at least 800%, at least about 900%, at least about 1000%, at least about 2000%, at least about 3000%, at least about 4000%, at least about 5000%, at least about 6000%, at least about 7000%, at least about 8000% at least about 9000% or at least about 10000%, compared an otherwise identical microorganism that does not carry said modification. The amount of a polypeptide produced in a given cell can be determined by any suitable quantification technique known in the art, such as ELISA, Immunohistochemistry or Western Blotting.

An increase in polypeptide expression may be achieved by any suitable means well-known to those skilled in the art. For example, an increase in polypeptide expression may be achieved by increasing the number of copies of the gene or genes encoding the polypeptide in the microorganism, such as by introducing into the microorganism an exogenous nucleic acid, such as a vector, comprising the gene or genes encoding the polypeptide operably linked to a promoter that is functional in the microorganism to cause the production of an mRNA molecule. An increase in polypeptide expression may also be achieved by integration of at least a second copy of the gene or genes encoding the polypeptide into the genome of the microorganism. An increase in polypeptide expression may also be achieved by increasing the strength of the promoter(s) operably linked to the gene or genes encoding the polypeptide. An increase in polypeptide expression may also be achieved by modifying the ribosome binding site on the mRNA molecule encoding the polypeptide. By modifying the sequence of the ribosome binding site the translation initiation rate may be increased, thus increasing the translation efficiency.

As used herein, "decreased", "decreasing" or "decrease of' expression of a polypeptide (such as a polypeptide having both dihydrofolate synthase activity and folylpolyglutamate synthetase activity or a polypeptide having 5-methyltetrahydropteroyltriglutamate-homocysteine S-methyltransferase activity) means that the expression of said polypeptide in a modified microorganism is reduced compared to the expression of said polypeptide in an otherwise identical microorganism that does not carry said modification (control). The expression of a polypeptide in a modified microorganism may be reduced by at least about 10 %, and preferably by at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 99% or 100%, or any percentage, in whole integers between 10% and 100% (e.g., 6%, 7%, 8%, etc.), compared to the expression of said polypeptide in an otherwise identical microorganism that does not carry said modification (control). More particularly, "decreased", "decreasing" or "decrease of' expression of a polypeptide means that the amount of the polypeptide in the microorganism is reduced by at least about 10 %, and preferably by at least about 20%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 99% or 100%, or any percentage, in whole integers between 10% and 100% (e.g., 6%, 7%, 8%, etc.), compared to the amount of said polypeptide in an otherwise identical microorganism that does not carry said modification (control). The expression or amount of a polypeptide in a microorganism can be determined by any suitable means know in the art, including techniques such as ELISA, Immunohistochemistry, Western Blotting or Flow Cytometry.

As used herein, "decreased", "decreasing" or "decrease of' activity of a polypeptide (such as a polypeptide having both dihydrofolate synthase activity and folylpolyglutamate synthetase activity or a polypeptide having 5-methyltetrahydropteroyltriglutamate-homocysteine S-methyltransferase activity) means that the catalytic activity of said polypeptide in a modified microorganism is reduced compared to the catalytic activity of said polypeptide in an otherwise identical microorganism that does not carry said modification (control). The activity of a polypeptide in a modified microorganism may be reduced by at least about 10 %, and preferably by at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 99% or 100%, or any percentage, in whole integers between 10% and 100% (e.g., 6%, 7%, 8%, etc.), compared to the expression of said polypeptide in an otherwise identical microorganism that does not carry said modification (control). The activity of a polypeptide in a microorganism can be determined by any suitable protein and enzyme activity assay.

As used herein, "regulatory region" of a gene refers to a nucleic acid sequence that affect the expression of a coding sequence. Regulatory regions are known in the art and include, but are not limited to, promoters, enhancers, transcription terminators, polyadenylation sites, matrix attachment regions and/or other elements that regulate expression of a coding sequence.

As used herein, "expression" includes any step involved in the production of a polypeptide (e.g., encoded enzyme) including, but not limited to, transcription, post-transcriptional modification, translation, post-translational modification, and secretion.

"Substitution" or "substituted" refers to modification of the polypeptide by replacing one amino acid residue with another, for instance the replacement of an Serine residue with a Glycine or Alanine residue in a polypeptide sequence is an amino acid substitution. When used with reference to a polynucleotide, "substitution" or "substituted" refers to modification of the polynucleotide by replacing one nucleotide with another. For instance the replacement of a cytosine with a thymine in a polynucleotide sequence is a nucleotide substitution.

"Non-conservative substitution", when used with reference to a polypeptide, refers to a substitution of an amino acid in a polypeptide with an amino acid with significantly differing side chain properties. Non-conservative substitutions may use amino acids between, rather than within, the defined groups and affects (a) the structure of the peptide backbone in the area of the substitution (e.g., serine for glycine), (b) the charge or hydrophobicity, or (c) the bulk of the side chain. By way of example and not limitation, an exemplary non-conservative substitution can be an acidic amino acid substituted with a basic or aliphatic amino acid; an aromatic amino acid substituted with a small amino acid; and a hydrophilic amino acid substituted with a hydrophobic amino acid.

"Percentage of sequence identity," "% sequence identity" and "percent identity" are used herein to refer to comparisons between an amino acid sequence and a reference amino acid sequence. The "% sequence identify", as used herein, is calculated from the two amino acid sequences as follows: The sequences are aligned using Version 9 of the Genetic Computing Group's GAP (global alignment program), using the default BLOSUM62 matrix with a gap open penalty of -12 (for the first null of a gap) and a gap extension penalty of - 4 (for each additional null in the gap). After alignment, percentage identity is calculated by expressing the number of matches as a percentage of the number of amino acids in the reference amino acid sequence.

Where a numerical limit or range is stated herein, the endpoints are included. Also, all values and sub ranges within a numerical limit or range are specifically included as if explicitly written out.

Having generally described this invention, a further understanding can be obtained by reference to certain specific examples, which are provided herein for purposes of illustration only, and are not intended to be limiting unless otherwise specified.

### Examples

### Example 1: Identification of folate biosynthetic genes in the genome of Bacillus subtilis

Genes and enzymes involved in the folate biosynthetic pathway are known in the literature and are described in detail in the KEGG database (www.genome.jp/kegg/pathway.html). Nucleotide and protein sequences of key folate biosynthetic genes of *B. subtilis* were obtained by investigating the genome and protein databases of *B. subtilis* using the BLAST algorithm. Sequences of folate biosynthetic genes and enzymes were introduced as "query" and the corresponding *B. subtilis* sequences were identified as "hits." Sequences of folate biosynthetic genes are presented in Table 2 below.

**Table 2. Genes and enzymes involved in folate biosynthesis in Bacillus subtilis.**

| **Gene name:** | **Enzymatic activity** | **NCBI accession number** | **Protein sequence ID** | **Nucleotide sequence *optimized sequence for Bacillus subtilis codon usage** |
|---|---|---|---|---|
| *folE* | GTP cyclohydrolase (*Bacillus subtilis*) | NP_390159 | SEQ ID NO: 7 | SEQ ID NO: 13 |
| *folB* | 7,8-dihydroneopterin aldolase (*Bacillus subtilis*) | NP_387959 | SEQ ID NO: 8 | SEQ ID NO: 14 |
| *folK* | 2-amino-4-hydroxy-6-hydroxymethyldihydro pteridine pyrophosphokinase (*Bacillus subtilis*) | NP_387960 | SEQ ID NO: 9 | SEQ ID NO: 15 |
| *sul* | dihydropteroate synthase (*Bacillus subtilis*) | NP_387958 | SEQ ID NO: 10 | SEQ ID NO: 16 |
| *folC* | bifunctional folylpolyglutamate synthetase / dihydrofolate synthetase (*Bacillus subtilis*) | NP_390686 | SEQ ID NO: 11 | / |
| *dfrA* | dihydrofolate reductase (*Bacillus subtilis*) | NP_390064 | SEQ ID NO: 12 | SEQ ID NO: 17 |
| *glyA* | serine hydroxymethyltransfera se (*Bacillus subtilis*) | NP_391571 | SEQ ID NO: 79 | SEQ ID NO: 91 |
| *purU* | formyltetrahydrofolate deformylase (*Bacillus subtilis*) | NP_389194 | SEQ ID NO: 81 | SEQ ID NO: 92 |
| *yitJ* | 5,10-methyl enetetrahydrofol ate reductase (*Bacillus subtilis*) | NP_388982 | SEQ ID NO: 83 | SEQ ID NO: 93 |

### Example 2: Synthesis of synthetic genes for folic acid biosynthesis, optimized for Bacillus subtilis

The amino acid sequences (SEQ ID NOs: 7, 8, 9, 10, 12, 79, 81 and 83) were used for gene codon optimization (Codon Optimization Tool from IDT Integrated DNA Technologies) in order to improve protein expression in *B. subtilis.* The synthesized DNA fragments (SEQ ID NOs: 13, 14, 15, 16, 17, 91, 92 and 93, respectively) were designed with addition of RBS sequences, regulatory promoter sequence (such as p15 SEQ ID NO:38) for gene overexpression and short adapter sequences at both ends needed for further assembly of folic acid operon expression cassette.

### Example 3: Assembly of folic acid operons

### • Folic acid operon assembled from Bacillus subtilis genes

Key folate biosynthetic genes from *Bacillus subtilis* genes synthesized as DNA fragments (SEQ ID NOs: 13, 14, 15, 16 and 17) were used for assembly of folic acid operon (FOL-OP-BS2). For integration of folic acid operon into *B. subtilis* genome two additional DNA fragments with *lacA* homologies and erythromycin selectable marker (SEQ ID NOs: 18 and 19) were designed and synthesized for stabile genome integration.

In the first step of the folic acid operon assembly PCR amplification of separate DNA fragments was performed with specific set of primers (primer pair SEQ ID NO: 26 and SEQ ID NO: 27 for fragment SEQ ID NO: 13; primer pair SEQ ID NO: 32 and SEQ ID NO: 28 for fragment SEQ ID NO: 17; primer pair SEQ ID NO: 33 and SEQ ID NO: 29 for fragment SEQ ID NO : 15; primer pair SEQ ID NO: 34 and SEQ ID NO: 30 for fragment SEQ ID NO: 16; primer pair SEQ ID NO:35 and SEQ ID NO: 31 for fragment SEQ ID NO: 14).

Fragments were amplified using Eppendorf cycler and Phusion polymerase (Thermo Fisher) with buffer provided by the manufacturer with addition of 200 µM dNTPs, 5% DMSO, 0.5 µM of each primer and approximately 20 ng of template in a final volume of 50 µl for 32 cycles.

Used program: 98 °C 2 min
32 cycles of (98°C 30s, 65°C 15s, 72°C 30s)
72 °C 5 min
10 °C hold

PCR of each fragment was run on 0.8 % agarose gel and cleaned from gel by protocol provided in Wizard PCR cleaning kit (Promega). The fragments were assembled into artificial folate operon by repetitive steps of restriction and ligation. A combination of *NdeI* and *AseI* restriction sites were used in order to assure compatible restriction ends for successful ligation. After each step of ligation, the combined fragments were used as a new template for next PCR amplification. Restriction was done in 50 µl volume with addition of 5 µl FD green buffer, 3 µl of selected enzyme and approximately 1500 ng of PCR fragment. Fragments were cleaned after restriction with Wizard SV Gel and PCR Clean-up system and first two were used in ligation. We used 2,5 U T4 DNA ligase (Thermo Fisher) with buffer provided by manufacturer and addition of 5 % PEG 4000 and both fragments in 1:1 molar ratio to final volume 15 µl. In the next step 1 µl of inactivated ligation was used as a template in new 50 µL PCR with primers SEQ ID NO: 26 and SEQ ID NO :28 and same program (with longer elongation time) and mix as used above. PCR was run on 0.8 % agarose gel, fragment was excised from gel and cleaned. Cleaned new fragment (assembly of SEQ ID NO: 13 and SEQ ID NO: 17) was cut with *Asel* restriction enzyme and after additional cleaning used in ligation with third fragment (SEQ ID NO: 15), already cut with *Ndel* and cleaned after. Following new PCR on ligation as a template, we also added fragment four and five by same protocol to make fragment of up to five folate biosynthetic genes.

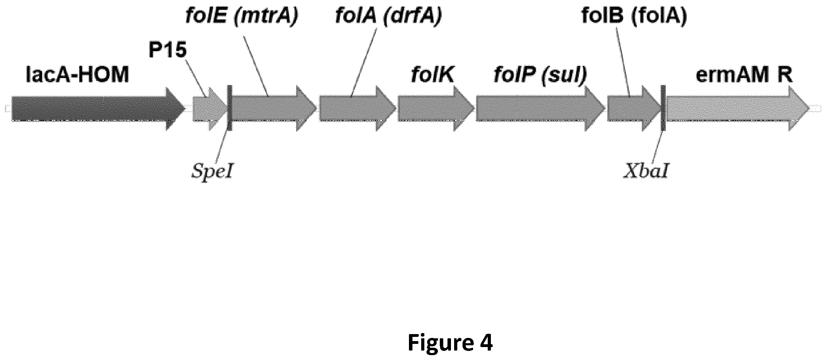

Constructed folic acid operon assembled from *Bacillus subtilis* genes (shown in Fig. 4), was used for transformation (see Example 5) in order to generate strain FL722, after cultivation measurements of total folate was performed (see Example 12).

### • Folic acid operon from Lactococcus lactis subsp. lactis genes

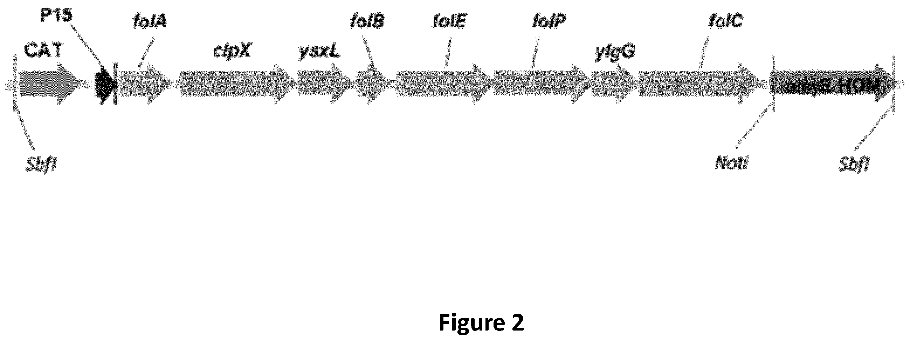

Heterologous genes (*folA, clpX, ysxL, folB, fol*E*, folP, ylgG* and *folC*) from *Lactococcus lactis* subsp. *lactis* operon FOL-OP-LL (SEQ ID NO: 49) were amplified by PCR and isolated genomic DNA was used as a template. Primers for PCR amplification were designed for two separate PCR reactions, where in the 1^{st} PCR reaction primers (SEQ ID NO:45 and SEQ ID NO:46) were used for specific amplification of genes from genomic DNA and in the 2^{nd} PCR reaction primers (SEQ ID NO:47 and SEQ ID NO:48) were used to additionally restriction sites (*NheI* and *NotI*) were introduced at both ends of the operon. The PCR product was subcloned into a low copy vector pFOL1 and the strong constitutive promoter P₁₅ (SEQ ID NO: 38) was added at the start of the FOL-OP-LL operon. For construction of integration cassette for FOL-OP-LL operon, chloramphenicol resistance cassette and downstream homology for amyE locus was introduced. In the final step, the integration cassette was realised from cloning vector by using *SbfI* restriction enzyme and used for self-ligation to achieve multi copy genome integration. Constructed folic acid operon assembled from *Lactococcus lactis subsp. lactis* genes (shown in Fig. 2), was used for transformation in order to generate strain FL84, after cultivation measurements of total folate was performed (see Example 12).

### • Folic acid operon FOL-OP-BS1 assembled from Bacillus subtilis genes

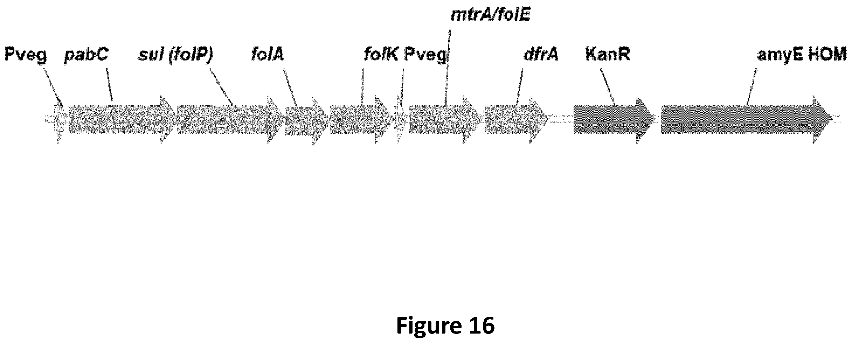

Assembly of FOL-OP-BS1 (artificial folate operon) was carried out from two separate DNA fragments of the synthesis technology (BS-FOLOP1-COMB and BS-FOLOP2-COMB, SEQ ID NOs: 77 and 78). Synthetic DNA fragment BS-FOLOP2-COMB was cloned into low-copy-number plasmids bearing kanamycin resistance cassette and downstream homology for amyE locus. In the final step of constructing the FOL-OP-BS1 integration cassette, the assembly was done in vitro using Gibson assembly protocol with specifically designed primer pair (SEQ ID NOs: 87 and 88) for amplification of BS-FOLOP1-COMB (part A) and primer pair (SEQ ID NO 89 and 90) for amplification of BS-FOLOP2-COMB+KnR+amyE-HOM (part B). Operon FOL-OP-BS1 for folate biosynthesis is under the expression of a strong constitutive promoter Pveg and kanamycin-resistance cassette as a selective marker (Figure 16). Assembled integration cassette FOL-OP-BS1 was PCR amplified and further used for self-ligation to enable multi-copy genome integration into the *B. subtilis* genome at amyE locus.

### • Folic acid operon from Ashbya gossypii (Eremothecium gossypii) genes

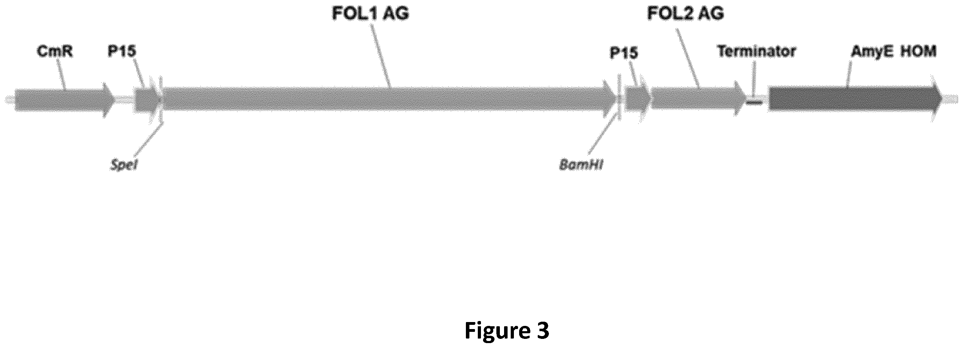

The expression cassette (FOL-OP-AG) from *Ashbya gossypii* (*Eremothecium gossypii*), a known B2 vitamin-producing filamentous fungus, was constructed using two synthetic folate biosynthesis genes, *fol1-AG* (SEQ ID NO:50) and *fol2*-*AG* (SEQ ID NO:51). The genes were codon-optimized for *B. subtilis* optimal expression and synthesized as two separate DNA fragments FOL1-AG (SEQ ID NO:52) and FOL2-AG (SEQ ID NO:53) where additional regulatory promoter sequence (promoter P₁₅) was introduced. The FOL1-AG fragment was first subcloned into a low copy vector pFOL1 using *SpeI*/*BamHI* restriction sites downstream of the chloramphenicol resistance cassette and strong constitutive promoter P₁₅. In the second step the FOL2-AG fragment was subcloned into a low copy vector pFOL2 upstream of the homology for amyE locus using *EcoRV* restriction site. In the next step DNA fragment containing P₁₅-fol2-AG and *amyE* homology was PCR amplified using primers (SEQ ID NO:54 and SEQ ID NO:55) and cloned into plasmid pFOL1 downstream of the chloramphenicol resistance cassette and P₁₅-fol1-AG using *BamHI* restriction site. In the final step, the assembled integration cassette FOL-OP-AG was PCR amplified using primers (SEQ ID NO:56 and SEQ ID NO:57) and PCR product was used for transformation of the cell. Constructed folic acid operon assembled from *Ashbya gossypii* genes (shown in Fig. 3), was used for transformation in order to generate strain FL260, after cultivation measurements of total folate was performed (see Example 12).

### Example 4: Assembly of genetic construct for folC replacement

In order to replace the native folylpolyglutamate synthase (folC), which is capable of attaching multiple glutamate residues to folates, with the variant, capable of attaching only the first glutamate residue in folate biosynthesis we set out to generate the corresponding genetic constructs. The *folC* disruption cassettes were assembled by using *folC* homology ends amplified by PCR from gDNA *B. subtilis* VBB38 by using the corresponding primer pairs SEQ ID NO:43 and SEQ ID NO:44. PCR mix was made with Phusion polymerase (Thermo Fisher) and buffer provided by manufacturer with addition of 5 % DMSO, 200 µM dNTPs and 0,5 µM of each primer to final volume of 50 µL for 32 cycles (annealing temperature 65 °C, elongation time 2 min). The amplified PCR fragment was excised from 0,8 % agarose gel, cleaned with Wizard Gel and PCR Clean-up system kit and phosphorylated with T4 polynucleotide kinase (Thermo Fisher) in buffer A, provided by manufacturer, with addition of 1 mM ATP.

Prepared fragment was ligated in low copy plasmid pET-29c (Novagen), which was previously cut with *FspAl* and *Xhol,* blunt-ended with DNA polymerase 1, Large (Klenow) fragment (Thermo Fisher) and dephosphorylated with FastAP Thermosensitive Alkaline Phosphatase (Thermo Fisher).

Tetracycline resistance cassette (SEQ ID NO:21) was used to disrupt *folC* gene sequence. Tetracycline resistance cassette was inserted into *folC* sequence by cutting plasmid with Bsp119l restriction enzyme, blunt-ended with DNA polymerase 1, Large (Klenow) fragment (Thermo Fisher), dephosphorylated, using FastAP and ligated using T4 DNA ligase (Thermo Fisher).

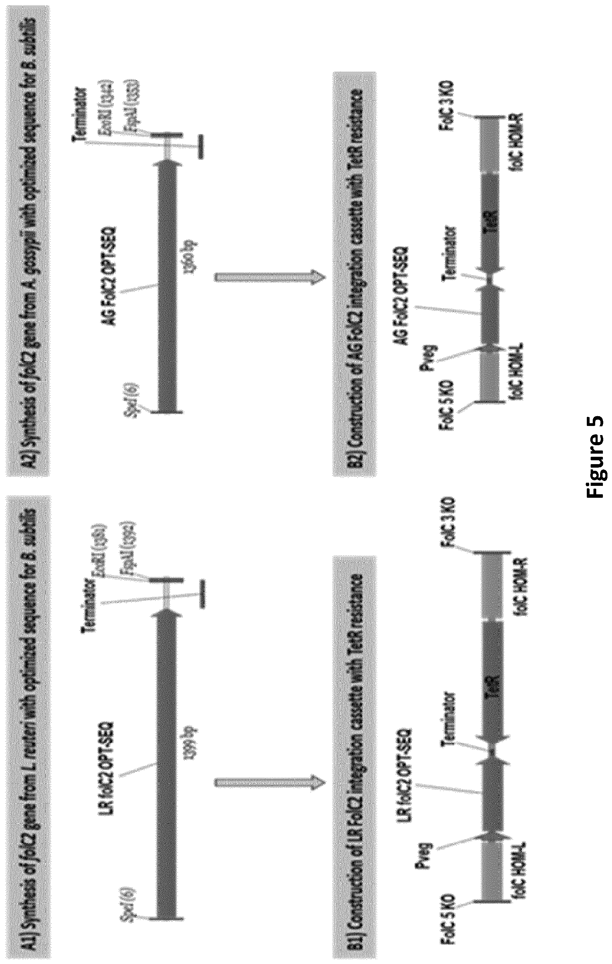

Further, heterologous *folC2* protein sequences from *Lactobacillus reuteri* (*folC2*-LR) (SEQ ID NO:22) and from *Ashbya gossypii* (*folC2*-AG) (SEQ ID NO:23) were used for design codon optimized DNA sequence for *folC2*-LR (*Lactobacillus reuteri*) (SEQ ID NO:24) and for *folC2*-AG (*Ashbya gossypii)* (SEQ ID NO:25) heterologous gene expression. DNA fragments were synthesized (IDT Integrated DNA Technologies) and used for construction of two integration cassettes (shown in Fig. 5). First, we generated a blunt-ended fragment containing the Pveg promotor (SEQ ID NO:37) using DNA polymerase 1, Large (Klenow) fragment (Thermo Fisher) and ligated it in the plasmid with *folC* homology, previously cut with *Xbal* and blunt-ended with DNA polymerase 1, Large (Klenow) fragment (Thermo Fisher).

Next, newly constructed plasmid was cut with *Bcul* and *FspAl* restriction enzymes and dephosphorylated, using FastAP. After that, plasmid was ligated with ordered optimized sequences folC2-AG in folC2-LR, previously cut with *Bcul* and *FspAl* restriction enzymes. In this plasmid tetracycline resistance, previously cut with *EcoRl* restriction enzyme and blunt-ended, was ligated, after restriction of plasmid with *FspAl* and dephosphorylated. Constructed plasmids were used as a template for PCR primers SEQ ID NO:43 and SEQ ID NO:44 in order to generate *folC* disruption/replacement cassette for transformation.

### Example 5: Assembly of folic acid operon constructs for transformation

After assembly of folic acid operon (see Example 3) DNA fragments with folate biosynthetic genes were further cut with *Xbal* restriction enzyme and ligated with synthetized DNA fragment for erythromycin resistance cassette (SEQ ID NO:19) with primers SEQ ID NO:40 and SEQ ID NO:41 (62 °C, 40 s) and cut with *XbaI* to ensure compatible DNA ends for ligation. After ligation whole fragment was PCR amplified with primers (SEQ ID NO: 36 and SEQ ID NO: 39).

In the final step of assembly fragment (SEQ ID NO: 18) with *lacA* homology and regulatory promoter region was added. Fragments were cut with *Spel* restriction enzyme and used in ligation. Ligation mixture was used as PCR template with primers (SEQ ID NO: 42 and SEQ ID NO: 39), with which we finish assembly of artificial folate operon (shown in Fig. 4) as an expression cassette (SEQ ID NO: 20) for genome transformation into B. *subtilis* strains.

### Example 6: Selection of possible Bacillus subtilis host strains for engineering of folate production

Different *Bacillus* strains can be used as starting strains for engineering of folate production (Table 3). *Bacillus* strains can be isolated from nature or obtained from culture collections. Among others, starting strains for folate production can be selected among *Bacillus subtilis* strains that have already been subjected to classical methods of mutagenesis and selection in order to overproduce metabolites related to the purine biosynthetic pathway. For example, strains overproducing riboflavin, inosine and guanosine may be selected. Strains subjected to random mutagenesis and toxic metabolic inhibitors from purine and riboflavin pathway are preferred and are included in Table 3.

**Table 3. Potential non-GMO starting strains of B. subtilis that could be used for development of folate production.**

| **Species** | **Name** | **Alternative name** | **Product** | **Availability** | **Remarks** |
|---|---|---|---|---|---|
| *B. subtilis* | 168 | ATCC6051 | none | yes | Type strain |
| *B. subtilis subsp. spizizenii* | W23 | ATCC 23059/ NRRL B-14472 | none | yes | Type strain |
| *B. subtilis* | RB50 | NRRL B18502 | riboflavin | yes | Developed by Roche/DSM |
| *B. subtilis* | RB58 | ATCC55053 | riboflavin | yes | Containing additional copy of rib operon |
| *B. subtilis* | VNII Genetika 304 | VKPM B2116, VBB38 | riboflavin | yes | Developed by VNII Genetika |
| *B. subtilis* | FERM-P 1657 | | riboflavin | no | Ajinomoto |
| *B. subtilis* | FERM-P 2292 | | riboflavin | no | Ajinomoto |
| *B. subtilis* | AJ12644 | FERM BP-3855 | riboflavin | no | Ajinomoto |
| *B. subtilis* | AJ12643 | FERM BP-3856 | riboflavin | no | Ajinomoto |
| *B. subtilis* | ATCC13952 | | inosine | yes | |
| *B. subtilis* | ATCC19221 | IFO 14123 | guanosine | yes | |
| *B. subtilis* | ATCC13956 | IFO 14124 | inosine | yes | |

VKPM B2116 strain is a hybrid strain of *B. subtilis* 168 strain (most common *B. subtilis* host strain with approx. 4 Mbp genome) with a 6.4 kbp island of DNA from the strain *B. subtilis* W23. Such architecture is common for most *B. subtilis* industrial strains and was obtained by transforming the 168 strain (tryptophan auxotroph *trpC*-) with W23 (prototrophic TrpC+) DNA. It has a 6.4 kbp W23 island in the genome, which is the same as in the commonly used strain *B. subtilis* SMY, which is one of the *B. subtilis* legacy strains with genome publicly available (Ziegler et al., The origins of 168, W23 and other Bacillus subtilis legacy strains, Journal of Bacteriology, 2008, 21, 6983 - 6995). VKPM B2116 strain is a direct descendant of the SMY strain, obtained by classical mutagenesis and selection. Another name for this strain is *B. subtilis* VNII Genetika 304. The description of construction of the strain in described in Soviet Union patent SU908092, filed in 1980. The mutations were obtained by subsequent mutagenesis and selection on metabolic inhibitors. The strain VKPM B2116 is resistant to roseoflavin, a toxic analogue of vitamin B2, due to a mutation in the *ribC* gene, encoding a flavin kinase. This strain is also resistant to 8-azaguanine, toxic analogue of purine bases.

### Example 7: Replacement of folC and generation of the optimum host strain for folic acid production

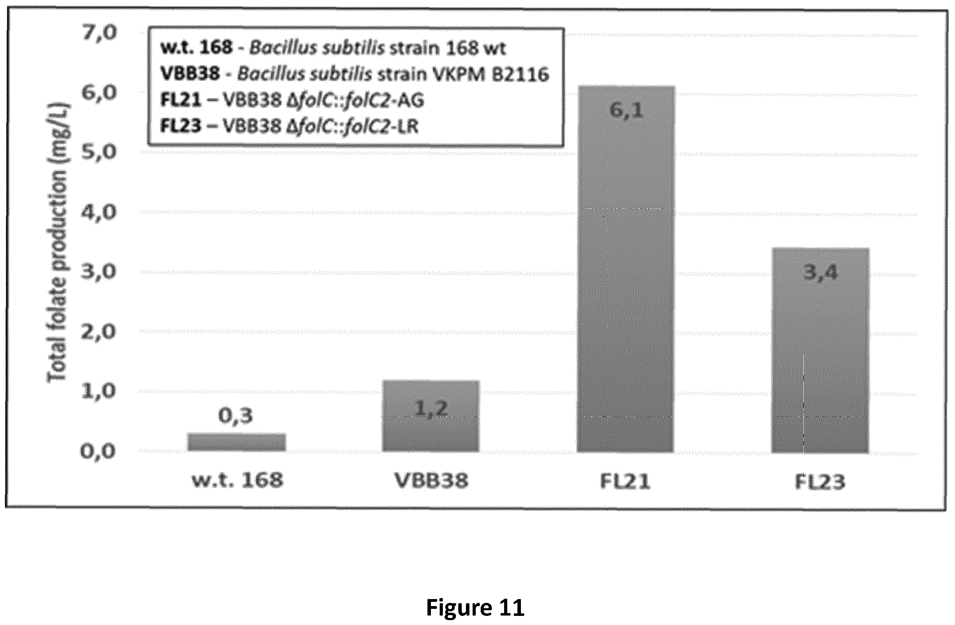

After construction of heterologous *folC2* (folC2-AG or folC2-LR) gene expression cassette (see example 4 and Fig. 5) we have performed transformation of *B. subtilis* VBB38 and *B. subtilis* VBB38Δrib. Expression cassette with homologies for native *folC* gene disruption, was amplified by PCR with primers SEQ ID NO: 43 and SEQ ID NO: 44. After transformation colonies resistant to tetracycline were selected and native *folC* gene replacement, by a heterologous *folC2* gene (*A. gossypii* or *L. reuteri*), was genetically confirmed with cPCR and sequencing of obtained PCR product. New strains were used to test the production yields of the total folates (see Figure 11), and to compare the distribution of the total folates between the supernatant and the cell biomass.

### Example 8: Transformation of Bacillus subtilis

### i) Bacillus subtilis natural competence transformation

10 mL of SpC medium is inoculated from fresh plate of *B. subtilis* and cultured overnight. 1,3 mL of overnight culture is diluted into 10 mL of fresh SpC medium (9x dilution). OD450 is measured and is expected to be around 0.5. Cultures are grown for 3h 10min at 37°C 220 RPM. OD450 is measured again and is expected to be between 1.2-1.6. Cultures are diluted 1:1 with SpII (starvation medium). 3,5 ml of culture is mixed with 3.5 ml of starvation medium and tryptophan in concentration 50 ug/ml is added. Cultures are grown for additional 2h at 37°C, 220 RPM. After incubation cultures are maximally competent for 1h. 500 uL of competent cells is mixed with DNA (5-20 uL, depending on concentration) in 2 mL Eppendorf tube and incubated for 30 min at 37°C with shaking. 300 uL of fresh LB is added for the recovery of competent cells and incubated for additional 30 min at 37°C. Eppendorf tubes are centrifuged at 3000 RPM, 5 min. Pellet is resuspended and plated on LB plates with appropriate antibiotic.

Medium:

### 10x T-base

150 mM ammonium sulfate
800 mM K₂HPO₄
440 mM KH₂PO₄
35 mM sodium citrate

### SpC (minimal culture media)

100 mL 1x T-base
1 mL 50% glucose
1.5 mL1,2% MgSO₄
2 ml 10% yeast extract
2.5 ml 1% casamino acids

### SpII (starvation media)

100 ml 1x T-base
1 ml 50% glucose
7 ml 1,2% MgSO₄
1 ml 10% yeast extract
1 ml 1% casamino acids
0.5 ml 100 mM CaCl₂

### Example 9: Determination of folate operon copy number using qPCR

We used real time quantitative PCR (qPCR) technique for determination of the number of copies of the integrated *B. subtilis* artificial folate operon genes. The copy numbers of the genes *folP, folK, folE, dfrA* and *KnR* (the gene for kanamycin resistance) in the artificial folate operon in the folate-producing *B. subtilis* transformants was estimated by (qPCR) with SYBR Green I detection. The copy number of the gene for kanamycin resistance (*KnR*) and the copy number of the folate biosynthesis genes *folP, folK, folE, dfrA* on artificial *B. subtilis* folate operon were quantified by qPCR. Genomic DNA of the *B. subtilis* strains was isolated with SW Wizard Genomic DNA Purification Kit (Promega). The concentration and purity of gDNA were evaluated spectrophotometrically at OD260 and OD280. The amount of gDNA used in all experiments was equal to the amount of gDNA of the reference strain. A *B. subtilis* with a single copy of artificial folate operon containing the genes *folP*, *folK*, *folE*, *dfrA* and *KnR* was used as a reference strain for relative quantification of the gene copy numbers. A housekeeping gene *DxS,* a single-copy gene in the *B. subtilis* genome, was used as the endogenous control gene. Quantification of gene copy number for the folate biosynthesis genes was performed using specific set of primers (primer pair SEQ ID NO:59 and SEQ ID NO:60 for *folP* gene, primer pair SEQ ID NO:61 and SEQ ID NO:62 for *folK* gene, primer pair SEQ ID NO:63 and SEQ ID NO:64 for *folE* gene, primer pair SEQ ID NO:65 and SEQ ID NO:66 for *dfrA* gene) for quantification of kanamycin resistance marker attached to folate operon (primer pair SEQ ID NO:67 and SEQ ID NO:68) and for reference *DxS* gene primer pair SEQ ID NO:71 and SEQ ID NO:72 were used. The qPCR analysis was run on StepOne^{™} Real-Time PCR System and quantification was performed by using the 2^{-ΔΔCT} method.

The gene copy numbers of the genes in the artificial BS-FOL-OP strains were quantified relatively to the strain with one copy of the genes. The *KnR* gene of the *B. subtilis* strain with one copy number was used as the reference strain for relative quantification of the gene copy numbers of genes in the artificial folate operon in *B. subtilis* transformed strains. The qPCR relative quantification of the genes *folP, folK, folE, dfrA* and *KnR* genes showed 6-fold increase in RQ values compared to *B. subtilis* strain with single copy genes. Folate overproducing strains FL179 and FL722 were confirmed to have multi-copy integration of folic acid synthetic operon.

### Example 10: Cultivation of Bacillus subtilis strains

Serial dilutions from frozen cryovial are made and plated on to MB plates with appropriate antibiotic and incubated for approximately 48 h at 37°C. For further testing at least 10-20 single colonies from MB plates use for each strain. First re-patch 10-20 single colonies on fresh MB plates (with the same concentration of antibiotics) for testing.

For vegetative stage MC medium is used and inoculated with 1 plug per falcon tube (or 5 plugs per baffled Erlenmeyer flask or small portion of patch for microtiter plates). Appropriate antibiotics are added into medium. For microtiter plates 500 ul of medium is used in 96 deep well, for falcon tubes is used 5 ml of medium (in 50 ml falcon tube) and for Erlenmeyer flask 25 ml (in 250 ml flask). Cultures are incubated at 37°C for 18-20 h at 220 RPM.

Inoculation into production medium (MD) is after 18-20 h in vegetative medium. 10 % inoculum is used (50 ul for MW, 0.5 ml for falcon tube and 2.5 ml Erlenmeyer flask). Each strain is tested in two aliquots. For microtiter plates 500 ul of medium is used in 48 deep well, for falcon tubes is used 5 ml of medium and for baffled Erlenmeyer flask 25 ml. Wires are used in falcon tubes for better aeration, as are gauzes used instead of the stoppers on Erlenmeyer flasks. Cultures are incubated at 37°C for 48 h at 220 RPM. After 24 and 48 hours titer of total folates was measured using the microbiological assay, according to the developed procedures

Best candidate strains are retested in the same manner and after several confirmations prepared for testing in bioreactors. 100 ul of frozen culture of selected strain for bioreactor testing is spread on to MB plates with appropriate antibiotic and incubated for approximately 48 h at 37°C. Complete biomass is collected with 2 ml of sterile 20 % glycerol per plate. Collected biomass is distributed into 100 ul aliquots and frozen at -80°C. This is used as working cell bank for bioreactor testing.

### Medium composition:

### 1) MB (plates)

Trypton 10 g/l
Yeast extract 5 g/l
NaCl 5 g/l
Maltose 20 g/l

Agar 20 g/l
pH 7.2-7.4
Autoclaved 30 min, 121°C
After autoclaving and cooling down appropriate antibiotics are added.

### 2) MC (vegetative medium)

Molasses 20 g/l
CSL 20 g/l
Yeast extract 5 g/l
MgSO₄*7H₂O 0.5 g/l
(NH₄)₂SO₄ 5 g/l

Ingredients are mixed together and pH set to 7.2-7.4. KH₂PO₄ - K₂HPO₄ solution is then added in final concentration for KH₂PO₄ 1.5 g/l and K₂HPO₄ 3.5 g/l. Medium is distributed into falcon tubes (5 ml/50 ml-falcon tubes) or Erlenmeyer flasks (25 ml/ 250 ml-baffled Erlenmeyer flask) and autoclaved 30 min, 121°C. Sterile glucose is added after autoclaving in final concentration 7,5 g/l. Antibiotics are added prior to inoculation.

### 3) MD (production medium)

Yeast 20 g/l
Corn steep liquor (CSL) 5 g/l
MgSO₄*7H₂O 0.5 g/l
para-aminobenzoic acid (pABA) 0.5g/L

Ingredients are mixed together and pH set to 7.2-7.4. KH₂PO₄ - K₂HPO₄ solution is then added in final concentration for KH₂PO₄ 1.5 g/l and K₂HPO₄ 3,5 g/l The medium is autoclaved at 121°C for 30 min. Sterile urea solution (20 ml of stock solution, final concentration is 6 g/L), sterile glucose solution (250 ml of stock solution, final concentration is 100 g/L glucose), sterile pABA solution (100 ml of stock solution, final concentration is 0.5 g/L) and 150 ml of sterile water are added after autoclaving to obtain 1 L of MD+pABA500 medium. Appropriate antibiotics were added prior to inoculation. Medium is then distributed into sterile Erlenmeyer flasks (25 ml/ 250 ml-baffled Erlenmeyer flask.

### Example 11: Microbiological assay for quantification of total folates in fermentation broths

A microbiological assay using *Enterococcus hirae* NRRL B-1295 was used for detection of the total folates produced in the strains of *Bacillus subtilis.* The microbiological assay was used for the evaluation of the intracellular (retained in the biomass) and extracellular (released into the culture medium) total folates produced by *B. subtilis.* For the microbiological assay, the indicator organism *Enterococcus hirae* NRRL B-1295 is used, which is auxotrophic for folates or folic acid. *E. hirae* is precultured in the rich growth medium, containing folates (*Lactobacilli* AOAC broth) at 37°C for 18-24 h. It is then washed in the growth medium without folates (folic acid assay medium) to remove the residual folates. The washed *E*. *hirae* culture is inoculated into the assay medium without folic acid. The microbiological assay is set up in 96-well microtiter plates. Appropriately diluted media samples to be assayed and the standard solutions of folic acid are added to the growth medium containing the indicator strain, and the plate is incubated at 37°C for 20 h. The growth response of the indicator organism is proportional to the amount of folic acid/folates present in the media samples/controls. The standard curve is constructed for each assay by adding a set of standard solutions of folic acid to the growth medium and the indicator strain. The growth is measured by measuring the optical density (OD) at 600 nm wavelength. The growth response of *E. hirae* to the test samples is compared quantitatively to that of the known standard solutions. A dilution series containing various concentrations of folic acid is prepared and assayed as described above. The standard curve is obtained by plotting the measured OD₆₀₀ at known concentrations of folic acid. The standard curve is used to calculate the amounts of total folates in the test samples. The indicator organism *E*. *hirae* NRRL B-1295 is used to detect the concentrations of total folates in the range from 0.05 to 0.7 ng/mL in the measured sample. The total extracellular and intracellular folates produced by *B. subtilis* strains can be estimated by adding appropriately diluted test samples to the indicator organism *E. hirae* in folic acid assay medium.

### Example 12: Analysis of total folate yields of different starting strains and initial folC-replaced and folic acid operon amplified strains

The transformants in which *folC* gene was replaced by a heterologous *folC2* gene from either *A. gossypii* (*B. subtilis* strain FL21) or *L. reuteri* (*B. subtilis* strain FL23) and transformants with amplified folic acid operon were tested for total folate amounts at the shaker scale (5 ml production medium MD). After the fermentation, the samples of the fermentation broth (200 µl) was carefully collected to obtain a homogeneous sample and diluted 10 times in the ice-cold extraction buffer (0.1 M phosphate buffer with 1% (w/v) ascorbic acid). The samples were centrifuged at 14,000 rpm and 4°C for 10 min and filter-sterilized (0.22 um pore size). For the microbiological assay samples were serially diluted in the extraction buffer and kept at 4°C until the microbiological assay was set up. In the Table 4 results for selected strains measured by the microbiological assay are presented.

**Table 4. Total folate production of different Bacillus subtills strains in experiments at shaker scale (5 ml)**

| Description of strain | Total folate production (mg/L) |
|---|---|
| *B. subtilis* w.t. 168 | 0.31 |
| VBB38 (*B. subtilis VKPM B2116)* | 1.24 |
| FL23 (*B. subtilis VBB38 folC*::*tet*R P-veg/*folC2*-LR) | 3.4 |
| FL1027 (*B. subtilis VBB38 folC*::*tet*R P-veg/*folC2*-LR; *lacA*::*ermAM* P-15/ FOL-OP-BS2) | 238,0 |
| FL21 (*B. subtilis VBB38 folC*::*tet*R P-veg/*folC2*-AG) | 6.1 |
| FL260 (*B. subtilis VBB38 folC*::*tet*R P-veg/*folC2*-AG; *amyE*::*cat* P-15/ FOL-OP-AG) | 45.7 |
| FL84 (*B. subtilis VBB38 folC*::*tet*R P-veg/*folC2*-AG; *amyE*::*cat* P-15/ FOL-OP-LL) | 55.2 |
| FL179 (*B. subtilis VBB38 folC*::*tet*R P-veg/*folC2*-AG; *amyE::kanR* P-veg/ FOL-OP-BS1) | 573,0 |
| FL722 (*B. subtilis VBB38 folC*::*tet*R P-veg/*folC2*-AG; *lacA*::*ermAM* P-15/FOL-OP-BS2) | 587,4 |

### Example 13: Determination of concentrations folate forms and related compounds using LC-MS and identification of 10-formyl-dihydrofolic acid and 10-formyl folic acid as two main products

In addition to the microbiological assay, our aim was to develop sensitive and versatile analytical method, with reasonably short analytical run time. The method had to be LCMS compatible with volatile mobile phase, and also had to enable UV detection and give good chromatographic separation of as many folate-related analytes as possible.

### Instruments and materials:

The method was developed on Thermo Accela 1250 HPLC instrument with PDA detector, coupled with MS/MS capable mass spectrometer Thermo TSQ Quantum Access MAX, equipped with hESI source. Method has been set-up on Thermo Acclaim RSLC PA2, 150x2.1 mm HPLC column with 2.2 µm particle size. PDA detector is set at 282 nm, with bandwidth 9 nm and 80 Hz scan rate, and also DAD scan from 200-800 nm. Column oven is set at 60 °C and tray cooling at 12 °C. Injection solvent is 10 % methanol in water, with wash and flush volume: 2000 µl. Injection volume is set at 10 µl and can also be set at 1 µl when higher concentrations of analytes are expected. Mobile phase A is 650 mM acetic acid in water, and mobile phase B is methanol. Mobile phase flow is 0.5 ml/min and total run time is 20 min. Method is using gradient program in Table 5 and MS spectrometer parameters described in Table 6.

**Table 5. Gradient program for the chromatographic analysis**

| Time / min | % A | % B |
|---|---|---|
| 0.00 | 100 | 0 |
| 2.00 | 100 | 0 |
| 16.00 | 82 | 18 |
| 16.01 | 100 | 0 |
| 20.00 | 100 | 0 |

**Table 6. MS spectrometer tune parameters and other MS/MS relevant parameters.**

| Tune parameters: | | Other method parameters: | |
|---|---|---|---|
| Ionization | hESI+ | Polarity: | Positive |
| Spray voltage | 4000 V | Scan width (m/z): | 5.000 |
| Vaporizer temperature | 350 °C | Scan time (s): | 0.100 |
| Sheath gas pressure | 55 | Q1 (FWHM): | 0.70 |
| Aux gas pressure | 5 | Micro scans: | 1 |
| Capillary temperature | 300 °C | Data type: | Centroid |
| Tube lens offset | SRM table | Chrom filter P.W. | none |
| Skimmer offset | 0 | MS Acquire time (min): | 15 |
| Collision pressure | 1.0 torr | Divert valve: | none |

LCMS detector is coupled after DAD detector, and analytes are observed in scan from 400-600 m/z mode, in SIM mode at their M.W.+1 and MS/MS mode (Table 6). Standards were prepared with weighting and dissolving in 0.1 M NaOH solution (Table 7 and Table 8) and immediately put to HPLC instrument.

**Table 7. Available standards.**

| Analyte: | Purity: | Source: | Abbreviation: |
|---|---|---|---|
| Folic acid | 91.3 % | Pharmacopoeia | FA |
| Dihydro folic acid | >80.0 % | Sigma | DHF |
| Tetrahydro folate | >65.5 % | Sigma | THF |
| 5-methyl tetrahydro folate | >81.0 % | Carbosynth | 5M-THF, 5-methyl THF |
| 10-formyl folic acid | 91.4% | EDQM | 10F-FA, 10-formyl FA |
| 5-formyl tetrahydro folate | >90.0 % | EDQM | 5F-THF, 5-formyl THF |

**Table 8. Observed standards and their related MS/MS method settings.**

| Analyte: | Parent m/z: | Product(s) m/z: | Collision E: | Tube lens: | Retention time (+/- 0.1 min) |
|---|---|---|---|---|---|
| Folic acid | 442 | 295 | 19 | 50 | 10.2 min |
| Dihydro f.a. | 444 | 178, 297 | 19 | 50 | 10.4 min |
| Tetrahydro f.a. | 446 | 299, 318, 361, 387 | 20 | 50 | 6.1 min |
| 5-methyl THF | 460 | 180, 314 | 20 | 50 | 7.0 min |
| 10-formyl f.a. | 470 | 295, 323 | 20 | 50 | 12.1 min |
| 10-formyl dihydro f.a. | 472 | 297, 325 | 20 | 50 | 10.6 min |
| 5-formyl THF | 474 | 299, 327 | 20 | 50 | 12.6 min |

Method has linear response for MS/MS detection up to 1000 mg/L of analyte, with correlations above 90% for all standards.

### Example 14: Different ratio of folic acid and derivatives production through genetically modified Bacillus subtilis

The transformants in which *folC* gene was replaced by a heterologous *folC2* gene from either *A. gossypii* (*B. subtilis* strain FL21) or *L. reuteri* (*B. subtilis* strain FL23) and transformants with amplified folic acid operon were tested for total folate amounts at the shaker scale (5 ml production medium MD).

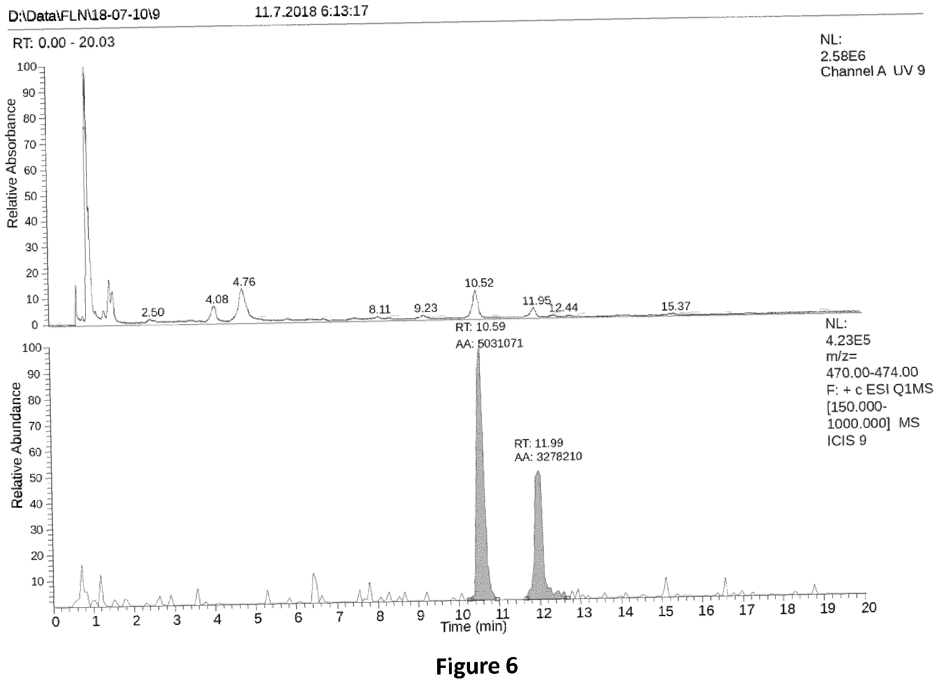

The strains were patched on MB plates with appropriate antibiotics and incubated at 37°C for 2 days. For shake-flasks experiments, the grown strains were transferred to 5 ml of MC (seed) medium in Falcon 50 mL conical centrifuge tubes (1 plug/5 ml) and cultivated on a rotary shaker at 220 RPM and 37 °C for 16 - 18h. A 10-% inoculum of the seed culture was used to inoculate 5 mL of the production medium (MD+pABA500). The strains were cultivated on a rotary shaker at 220 RPM and 37°C for 48h in the dark. After the fermentation, the samples of the fermentation broth (200 µl) was carefully collected to obtain a homogeneous sample and diluted 10 times in the ice-cold extraction buffer (0.1 M phosphate buffer with 1% (w/v) ascorbic acid). The samples were centrifuged at 14,000 rpm and 4°C for 10 min and filter-sterilized (0.22 um pore size). For the quantification of different folate species HPLC method was used as described in Example 13. Results of different *B. subtilis* strain are shown in Table 9 and representative HPLC chromatogram of fermentation broth sample is shown in Figure 6.

**Table 9. Total folate production of different Bacillus subtills strains in experiments at shaker scale (5 ml)**

| **Strain** | **Strain description** | **5M-THF (mg/L)** | **FA (mg/L)** | **10F-DHF (mg/L)** | **10F-FA (mg/L)** |
|---|---|---|---|---|---|
| wt 168 | *B. subtillis* wild type | 0,16 | 0,15 | 0,01 | 0,86 |
| VBB38 | *B. subtilis* VKPM B2116 | 0,35 | 0,01 | 0,02 | 0,09 |
| FL 23 | VBB38 folC::tetR P-veg/folC2-LR | 1,11 | 0,01 | 0,01 | 0,03 |
| FL 21 | VBB38 folC::tetR P-veg/folC2-AG | 18,20 | 0,01 | 0,03 | 2,64 |
| FL 84 | FL21 amyE::cat P-15/ FOL-OP-LL | 25,46 | 0,02 | 0,34 | 18,81 |
| FL 260 | *FL21 amyE::cat P-15*/ *FOL-OP-AG* | 21,67 | 0,04 | 1,52 | 44,50 |
| FL 179 | FL21 amyE::kanR P-veg/ FOL-OP-BS1 | 97,05 | 0,03 | 16,22 | 373,22 |
| FL 722 | FL21 lacA::ermAM P-15/FOL-OP-BS2 | 20,21 | 0,30 | 13,21 | 351,00 |

Strain FL179 with heterologous folC-AG and overexpressed folate biosynthetic genes from *B. subtilis* showed 43297% increased 10-formyl folic acid production compared to the wild type strain *Bacillus subtilis* 168.

### Example 15: Oxidative conversion of 10-formyldihydrofolic acid to 10-formyl folic acid

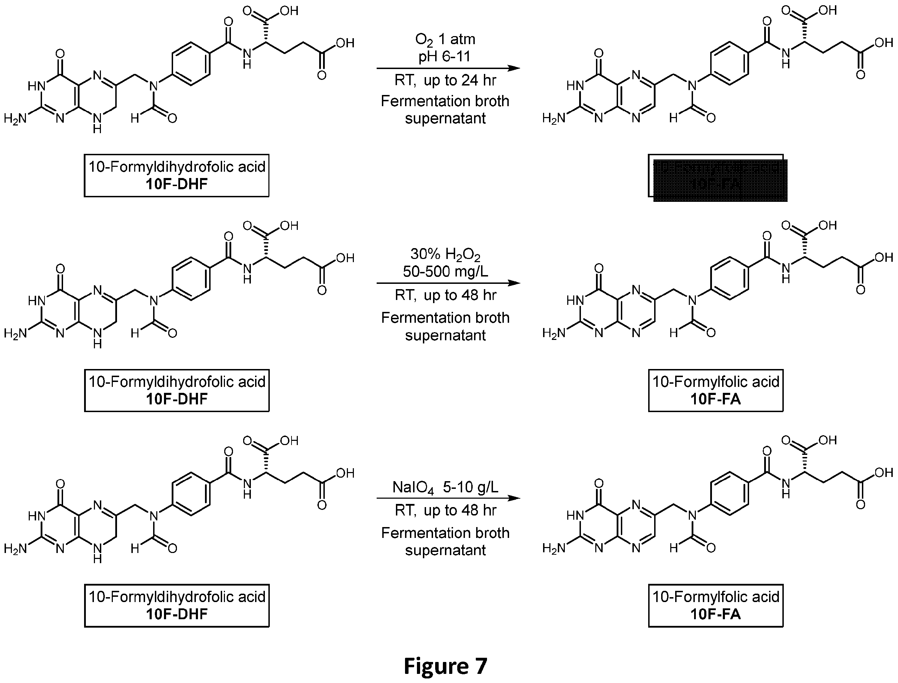

At the end of the fermentation, HPLC analysis of broth detected a relatively high amount (85 Area %) of 10-formyldihydrofolic acid (10F-DHF). Furthermore, we observed that 10-formyldihydrofolic acid can be oxidatively converted to 10-formylfolic acid (see Figure 7). Accordingly, we started to develop a protocol, which will provide a quantitative conversion to 10-formylfolic acid. We anticipate the subsequent deformylation step will provide a folic acid in the highest possible yield. Literature search revealed a report describing the oxidation of thetrahydrofolic acid by air in aqueous solutions at specific pH values (Reed1980). Based on this report, at pH values 4, 7 and 10 the major products of oxidation are *p*-aminobenzoylglutamic acid (PABG) and 6-formylpterin. In addition, 7,8-dihydrofolate intermediate was only detected at pH = 10. We carried out the series of oxidation experiments on the fermentation broth supernatant to facilitate a swift conversion of 10-formyldihydrofolic acid to 10-formylfolic acid. We examined several oxidation reagents such as O₂, H₂O₂ and NaIO₄ (see Figure 7).

**Table 10. Effect of pH on oxidation of 10-formyldihydrofolic acid to 10-formylfolic acid in the fermentation broth supernatant with oxygen.**

| **exp** | **pH** | **oxidant** | **time** | **temp** | **10F-DHF** | **10F-FA** | **FA** | **SUM FOL** |
|---|---|---|---|---|---|---|---|---|
| | | | | | mg/L | mg/L | mg/L | mg/L |
| 1 | 7 | - | 0 hr | 25 °C | 782.9 | 180.2 | 12.4 | 975.5 |
| 2 | 6 | O₂ 1atm | 48 hr | 25 °C | 140.0 | 368.2 | 7.3 | 515.5 |
| 3 | 7 | O₂ 1atm | 48 hr | 25 °C | 253.5 | 366.1 | 10.3 | 611.9 |
| 4 | 8 | O₂ 1atm | 48 hr | 25 °C | 268.1 | 376.5 | 12.0 | 656.5 |
| 5 | 9 | O₂ 1atm | 48 hr | 25 °C | 199.4 | 293.6 | 0 | 493 |
| 6 | 10 | O₂ 1atm | 48 hr | 25 °C | 80.7 | 288.4 | 0 | 369.1 |
| 7 | 11 | O₂ 1atm | 48 hr | 25 °C | 0 | 351.3 | 0 | 351.3 |

Experiments were conducted in 50 mL round bottom flasks using 10 mL of the fermentation broth supernatant. pH values were set by 1.0 M and 0.1 M NaOH solution. Progress of reaction and results were measured by HPLC. The HPLC samples were prepared in the extraction buffer (0.1 M phosphate buffer with 1% (w/v) ascorbic acid). All reactions were stirred protected from the light for 48 hours at ambient temperature (25 °C).

Required pH values were adjusted with 1 M and 0.1 M HCl or NaOH. Reactions at lower pH values are slower and maintain relatively high sum of folates (Table 10, entries 2-4). On the contrary, reactions at higher pH values (Table 10, entries 5-7) improve the consumption of 10-formyldihydrofolic acid albeit significantly reduce the sum of the folates. We anticipate we could use alternative reagents for oxidation such as hydrogen peroxide or sodium periodate.

### Representative experimental procedure:

Fermentation broth was centrifuged at 4,500 rpm and the supernatant decanted. The 10 mL of fermentation broth supernatant was pipetted into the 50 mL round bottom flasks equipped with stirring bars, pH meter and aluminum foil for light protection. Sodium hydroxide or hydrochloric acid (1.0 M and 0.1 M for fine tuning) was added dropwise to set the pH value and reaction was stirred vigorously for 24 hours under the ambient temperature (25 °C). The reaction mixture was purged with an air from the balloon. After 48 hours of stirring, 1 mL of each fermentation broth was diluted in duplicates with 9 mL of extraction buffer (0.1 M phosphate buffer with 1% (w/v) ascorbic acid). The suspensions were stirred on vortex, centrifuged at 4,500 rpm, filtered through 0.22 µm filter and analyzed on HPLC.

**Table 11. Effect of hydrogen peroxide concentration on oxidation of 10-formyldihydrofolic acid to 10-formylfolic acid in the fermentation broth supernatant.**

| **exp** | **oxidant** | **mg/L** | **time** | **temp** | **10F-DHF** | **10F-FA** | **FA** | **SUM FOL** |
|---|---|---|---|---|---|---|---|---|
| | | | | | mg/L | mg/L | mg/L | mg/L |
| 1 | - | 0 | 0 hr | 25 °C | 735.5 | 137.4 | 0 | 872.9 |
| 2 | H₂O₂ | 50 | 24 hr | 25 °C | 390.8 | 299.5 | 12 | 702.4 |
| 3 | H₂O₂ | 100 | 24 hr | 25 °C | 397.3 | 308.7 | 24.2 | 730.2 |
| 4 | H₂O₂ | 250 | 24 hr | 25 °C | 355 | 325.4 | 12.7 | 693.1 |
| 5 | H₂O₂ | 500 | 24 hr | 25 °C | 383.5 | 315.5 | 12.9 | 711.9 |
| 6 | H₂O₂ | 50 | 48 hr | 25 °C | 193.5 | 354.7 | 0 | 548.1 |
| 7 | H₂O₂ | 100 | 48 hr | 25 °C | 183.4 | 529.5 | 0 | 712.8 |
| 8 | H₂O₂ | 250 | 48 hr | 25 °C | 185.9 | 534.3 | 0 | 720.1 |
| 9 | H₂O₂ | 500 | 48 hr | 25 °C | 174.4 | 539.6 | 0 | 714.0 |

Experiments were conducted in 50 mL round bottom flasks using 10 mL of the fermentation broth supernatant. Hydrogen peroxide was added dropwise as 30% solution in water. Progress of reaction and results were measured by HPLC. The HPLC samples were prepared in the extraction buffer (0.1 M phosphate buffer with 1% (w/v) ascorbic acid). All reactions were stirred protected from the light for 48 hours at ambient temperature (25 °C).

Hydrogen peroxide, an alternative oxidant for the oxidative conversion of 10-formyldihydrofolic acid to 10-formylfolic acid was added in concentration range from 50-500 mg/L thus providing more advanced results (Table 11). During the first 24 hours of reaction, the concentration of 10-formyldihydrofolic acid dropped to 50 % of its initial value. Prolongation of reaction to 48 hours provided a good conversion thus maintaining a relatively high sum of total folates.

### Representative experimental procedure:

Fermentation broth was centrifuged at 4,500 rpm and the supernatant decanted. The 10 mL of fermentation broth supernatant was pipetted into the 50 mL round bottom flasks equipped with stirring bars, pH meter and aluminum foil for light protection. Hydrogen peroxide was added dropwise as 30% solution in water and the reaction mixture stirred vigorously for 24-48 hours under the ambient temperature (25 °C). After 48 hours of stirring, 1 mL of each fermentation broth was diluted in duplicates with 9 mL of extraction buffer (0.1 M phosphate buffer with 1% (w/v) ascorbic acid). The suspensions were stirred on vortex, centrifuged at 4,500 rpm, filtered through 0.22 µm filter and analyzed on HPLC.

**Table 12. Effect of sodium periodate concentration on oxidation of 10-formyldihydrofolic acid to 10-formylfolic acid in the fermentation broth supernatant.**

| **exp** | **oxidant** | **mg/L** | **time** | **temp** | **10F-DHF** | **10F-FA** | **FA** | **SUM FOL** |
|---|---|---|---|---|---|---|---|---|
| | | | | | mg/L | mg/L | mg/L | mg/L |
| 1 | - | 0 | 0 hr | 25 °C | 735.5 | 137.4 | 0 | 872.9 |
| 2 | NaIO₄ | 5 | 24 hr | 25 °C | 278.5 | 326.5 | 34.1 | 639.1 |
| 3 | NaIO₄ | 5 | 48 hr | 25 °C | 111.8 | 376.7 | 40.3 | 528.8 |
| 4 | NaIO₄ | 10 | 24 hr | 25 °C | 84.6 | 449.6 | 212.6 | 746.8 |
| 5 | NaIO₄ | 10 | 48 hr | 25 °C | 0 | 575.6 | 251.1 | 826.7 |

Experiments were conducted in 50 mL round bottom flasks using 10 mL of the fermentation broth supernatant. Sodium periodate was added in a single portion. Progress of reaction and results were measured by HPLC. The HPLC samples were prepared in the extraction buffer (0.1 M phosphate buffer with 1% (w/v) ascorbic acid). All reactions were stirred protected from the light for 48 hours at ambient temperature (25 °C).

Sodium periodate is often used as the reagent of choice for capricious substrates. Our initial experimentation with this reagent revealed that the effective concentration for the oxidative conversion is between 1-10 g/L. Sodium periodate was added in two different concentrations, 5 g/L and 10 g/L. During the first 24 hours of reaction, the concertation of 10-formyldihydrofolic acid dropped significantly from its initial value (Table 12). Prolongation of reaction to 48 hours provided an excellent conversion thus maintaining a relatively high sum of total folates.

### Representative experimental procedure:

Fermentation broth was centrifuged at 4,500 rpm and the supernatant decanted. The 10 mL of fermentation broth supernatant was pipetted into the 50 mL round bottom flasks equipped with stirring bars, pH meter and aluminum foil for light protection. Sodium periodate was added in a single portion and the reaction mixture stirred vigorously for 24 hours under the ambient temperature (25 °C). After 48 hours of stirring, 1 mL of each fermentation broth was diluted in duplicates with 9 mL of extraction buffer (0.1 M phosphate buffer with 1% (w/v) ascorbic acid). The suspensions were stirred on vortex, centrifuged at 4,500 rpm, filtered through 0.22 µm filter and analyzed on HPLC.

### Example 16: Production of folates in 5L bioreactor volume

The production of folates can be greatly improved in bioreactors where appropriate conditions are used for the cultivation and production of folates. The process includes the preparation of the pre-culture and the main fed-batch bioprocess.

### i) Preparation of the pre-culture

The pre-culture medium (FOL-MC, Table 13) in flasks is seeded with the working cell bank of strain FL179 and cultivated on a rotary shaker at 37 °C and 220 RPM (2" throw) for 11-14 hours.

### ii) Fed-batch bioprocess

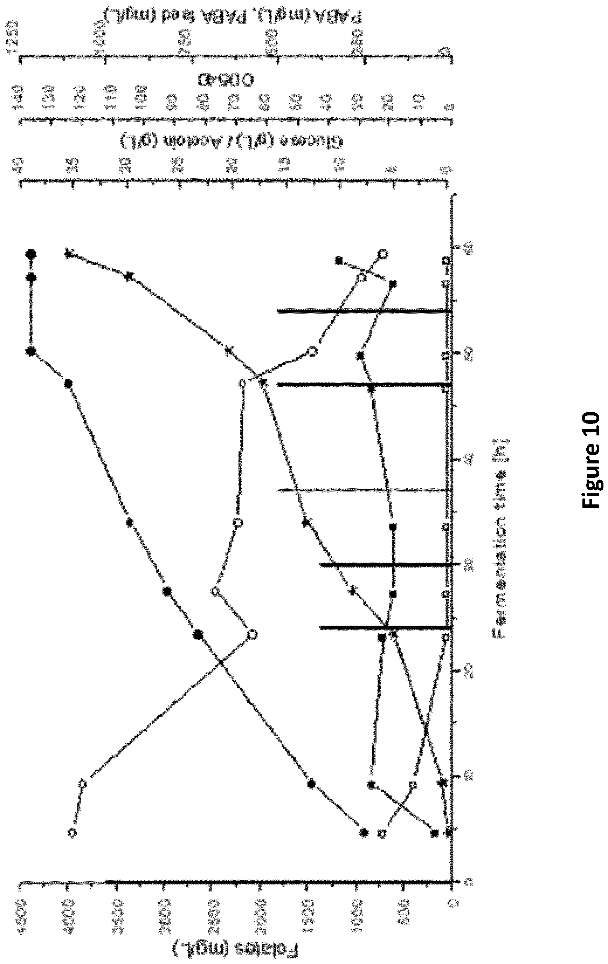

The production of folates is carried out in a 5L bioreactor using the FOL-ME medium (Table 14). The bioreactor starting parameters are Agitation = 600 RPM, Aeration = 1 vvm, pH is controlled at 7 using ammonium hydroxide solution. The bioreactor is inoculated with 10% of the pre-culture. The DO is controlled by agitation and airflow to keep the air saturation above 30%. When glucose in the fermentation broth is depleted, feeding of a glucose and CSL mixture (Table 15) is started. The rate of feed addition needs to be carefully controlled and the feeding rate is controlled at a level, which does not lead to acetoin (not more than 10 g/L) accumulation. If no acetoin is detected in the fermentation broth the feeding rate is too low. *para*-aminobenzoic acid (PABA) concentration in the fermentation broth needs to be measured at regular intervals and kept above 500 mg/L by batch feeding of a concentrated PABA stock solution (50 g/L). The bioprocess is usually finished in 50 hours. Folates production bioprocess profile is shown in Figure 10.

**Table 13. FOL-MC pre-culture medium**

| Component | Amount |
|---|---|
| Molasses | 20g/L |
| Corn steep liquor (CSL) | 20g/L |
| Yeast | 5g/L |
| (NH4)2SO4 | 5g/L |
| MgSO4x7H2O | 0.5g/L |
| KH2PO4 | 1.5g/L |
| K₂HPO₄ | 3.5g/L |
| glucose | 7.5g/L |
| Kanamycin | 10mg/L |
| Tetracycline | 10mg/L |

**Table 14. FOL-ME production medium**

| Component | Amount |
|---|---|
| Soybean powder | 25g/L |
| Corn steep liquor (CSL) | 40g/L |
| Yeast | 5g/L |
| (NH4)2SO4 | 4g/L |
| MgSO4x7H2O | 2.05g/L |
| KH2PO4 | 1.5g/L |
| K₂HPO₄ | 3.5g/L |
| Glucose | 30g/L |
| Kanamycin | 10mg/L |
| Tetracycline | 10mg/L |
| Sodium *para*-aminobenzoate (PABA) | 1 g/L |

**Table 15. Feeding solution (glucose + CSL)**

| Component | Amount |
|---|---|
| Glucose monohydrate | 400g/L |
| Corn steep liquor (CSL) | 310g/L |

### Example 17: Determination of expression levels of folate biosynthetic genes using qPCR

Culture growth conditions: *B. subtilis* culture was grown in LB medium to the exponential phase. The culture was mixed with 2 volumes of the RNA protect Bacteria Reagent (QIAGEN), centrifuged for 10 min at 4500 rpm and frozen at -80°C or processed immediately. Cell pellet was resuspended in 200 µL of TE buffer containing 1 mg/mL lysozyme for 15 min in order to remove the cell wall. RNA was isolated by using QIAGEN Rneasy mini kit according to the manufacturer protocol. The obtained RNA was checked for concentration and quality spectrophotometrically. The isolated RNA was treated with DNase (Ambion kit) and reverse-transcribed to cDNA by using RevertAid H Minus First Strand cDNA Synthesis Kit (Thermo Scientific). The obtained cDNA was diluted and the final yield of cDNA is cca 2.5 ng/µL.

The obtained cDNA was analysed by qPCR analysis (StepOne Real-Time PCR System, Applied Biosystems) with SYBR Green I (Thermo Scientific) detection. The expression of the folate operon genes in the integrated *B. subtilis* artificial folate operon genes *folP, folK, folE*, *dfrA* was quantified by real time quantitative PCR (qPCR) technique.

Internal control gene used as reference for normalization of quantitative qPCR expression data, 16S rRNA gene from *B. subtilis* was used. The expression of the folate biosynthesis genes was determined using specific set of primers (primer pair SEQ ID NO:59 and SEQ ID NO:60 for *folP* gene, primer pair SEQ ID NO:61 and SEQ ID NO:62 for *folK* gene, primer pair SEQ ID NO:63 and SEQ ID NO:64 for *folE* gene, primer pair SEQ ID NO:65 and SEQ ID NO:66 for *dfrA* gene) and for 16S gene selected as internal control primer pair SEQ ID NO:69 and SEQ ID NO:70 were used. The qPCR analysis was run on StepOne^{™} Real-Time PCR System and quantification was performed by using the 2 ^{-ΔΔCT} method.

The best folate producing strain FL722 bearing multicopy of synthetic folate operons at two separate genome locations (*amyE* and *lacA*) was confirmed to have the strongest expression levels of folate biosynthetic genes.

### Example 18: Chemical conversion of 10-formyl folic acid to folic acid

### Acid-Mediated Deformylation

Deformylation of 10-formylfolic acid was conducted on 0.01 mmol scale (5 mg). 10-formylfolic acid was weighed in the 2 mL Eppendorf tube equipped with a stirring bar and suspended in distilled water (1 mL). The suspension was treated with acid (50 equiv., 0.5 mmol) and allowed to stir for 16 hours at ambient temperature. Subsequently, a suspension (200 µL) was diluted with DMSO (800 µL), homogenized on the vortex stirrer and analyzed on HPLC. Results of deformylation are presented in Table 16.

**Table 16. Effect of different acids on of N-deformylation of 10-formylfolic acid**

| **exp** | **solvent** | **acid** | **eq.** | **mmol** | **time** | **temp** | **mmol** | **conv. to FA^{a}** |
|---|---|---|---|---|---|---|---|---|
| 1 | H₂O | HCl | 50 | 0.5 | 16 hr | 25 °C | 0.01 | 98.8% |
| 2 | H₂O | DOWEX | 50 | 0.5 | 16 hr | 25 °C | 0.01 | n.d.^{b} |
| 3 | H₂O | TFA^{c} | 50 | 0.5 | 16 hr | 25 °C | 0.01 | 92.9% |
| 4 | H₂O | TCA^{d} | 50 | 0.5 | 16 hr | 25 °C | 0.01 | 95.3% |
| 5 | H₂O | HCOOH | 50 | 0.5 | 16 hr | 25 °C | 0.01 | 1.1% |
| 6 | H₂O | PTSA^{e} | 50 | 0.5 | 16 hr | 25 °C | 0.01 | 97.8% |
| 7 | H₂O | CH₃COO H | 50 | 0.5 | 16 hr | 25 °C | 0.01 | 0.7% |
| 8 | H₂O | H₂SO₄ | 50 | 0.5 | 16 hr | 25 °C | 0.01 | 100% |

All experiments were conducted in 2 mL Eppendorf tubes using 10-formylfolic acid (5 mg, 0.01 mmol).^{a} Conversion was measured by HPLC.^{b} n.d. - not detected. Neither 10-formylfolic acid nor folic acid were detected in this experiment due to a probable adsorption of the analyte to Dowex 50WX2 resin. ^{c} TFA - Trifluoroacetic acid. ^{d}TCA - Trichloroacetic acid. ^{e} PTSA -*p*-Toluenesulfonic acid.

Deformylation of 10-formylfolic acid with strong inorganic acids proceeded almost quantitatively to folic acid (Table 16, entries 1 and 8). Alternatively, deformylation with stronger organic acids provided folic acid with nearly equal efficiency (Table 16, entries 3,4 and 6). As expected, deformylation with formic and acetic acid provided no conversion (Table 16, entries 5 and 7). HPLC analysis of deformylation using Dowex 50WX2 resin provided no detection for a starting material nor product since analyte probably remained adsorbed to the resin and requires elution.

### Acid-mediated N-deformylation of 10-formylfolic acid in the fermentation broth

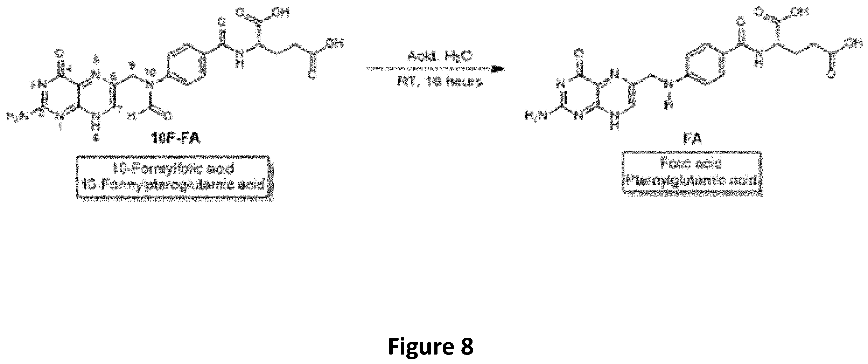

In previous experiments we have illustrated that deformylation of 10-formylfolic acid standard using a strong acid provided a clean conversion to folic acid shown in Fig. 8. Herein we applied the same principle on a more complex system, a fermentation broth. To continue experimenting on biological samples, we have selected a hydrochloric acid (HCl) as a deformylation reagent since it is highly effective and less expensive than other acids we studied. HPLC analysis of fermentation broth from Example 16 showed a substantial amount of 10-formylfolic acid among other folates formed during a biosynthesis (10-formylfolic acid 46 %Area; 5-imidomethyltetrahydrofolic acid 47% Area and 5-methyltetrahydrofolic acid 7 %Area). Samples of fermentation broth were treated with 1 M HCl up to different pH levels (pH = 4, 3, 2, 1 and 0) and stirred for 24 hours at ambient temperature (25 °C) protected from light. According to our HPLC assay, only at lower pH levels (pH = 1 and 0) deformylation provided a modest amount of folic acid. Based on these results, we are confident that acid-mediated deformylation strategy is potentially applicable during downstream processing of folic acid. In order to develop a cost-effective deformylation protocol of formyl folate species in a complex system such as fermentation broth, further optimization of acid amount and reaction temperature is essential.

Well-stirred fermentation broth from Example 16 was pipetted into six 100 mL round bottom flasks equipped with stirring bars and pH electrode. Hydrochloric acid was added dropwise with stirring to reach several pH values (pH = 4, 3, 2, 1, 0) as described in the Table 17.

**Table 17. Acid-mediated deformylation of the fermentation broth 3101**

| **exp** | **V_{FB}** | **V_{HCl}** | **V_{Total}** | **pH** |
|---|---|---|---|---|
| 1 | 50 mL | 0.0 mL | 50 mL | 7.0 |
| 2 | 50 mL | 10.2 mL | 60.2 mL | 4.0 |
| 3 | 50 mL | 15.6 mL | 65.6 mL | 3.0 |
| 4 | 50 mL | 21.4 mL | 71.4 mL | 2.0 |
| 5 | 50 mL | 35.3 mL | 85.3 mL | 1.0 |
| 6 | 50 mL | 59.0 mL | 109.3 mL | 0.0 |

Fermentation mixtures were stirred for 24 hours at ambient temperature (25 °C) shielded from the UV light by a wrapping the flasks in the aluminum foil. A controlled sample was prepared under the exact conditions albeit with the absence of acid (experiment 1). After 24 hours of stirring, 1 mL of each fermentation broth was diluted in duplicates with 9 mL of extraction buffer (0.1 M phosphate buffer with 1% (w/v) ascorbic acid). The suspensions were stirred on vortex, centrifuged at 4500 rpm, filtered through 0.22 µm filter and analyzed on HPLC. The HPLC results were summarized in the Table 18. According to our HPLC assay, only at lower pH levels (pH = 1 and 0) deformylation provided a modest amount of folic acid. In conclusion, we have developed an acid-mediated deformylation of 10-formylfolic acid, a major product of fermentation.

**Table 18. HPLC-based results of acid-mediated deformylation on the fermentation broth from Example 16**

| **exp** | **pH** | **5-FTHF mg/L** | **10-FFA mg/L** | **F SUM mg/L** | **FA mg/L** |
|---|---|---|---|---|---|
| 1 | 7.0 | 432 | 487 | 919 | 0 |
| 2 | 4.0 | 171 | 567 | 738 | 0 |
| 3 | 3.0 | 97 | 632 | 729 | 0 |
| 4 | 2.0 | 76 | 529 | 605 | 0 |
| 5 | 1.0 | 54 | 326 | 549 | 169 |
| 6 | 0.0 | 37 | 116 | 402 | 249 |

### Base-Mediated Deformylation

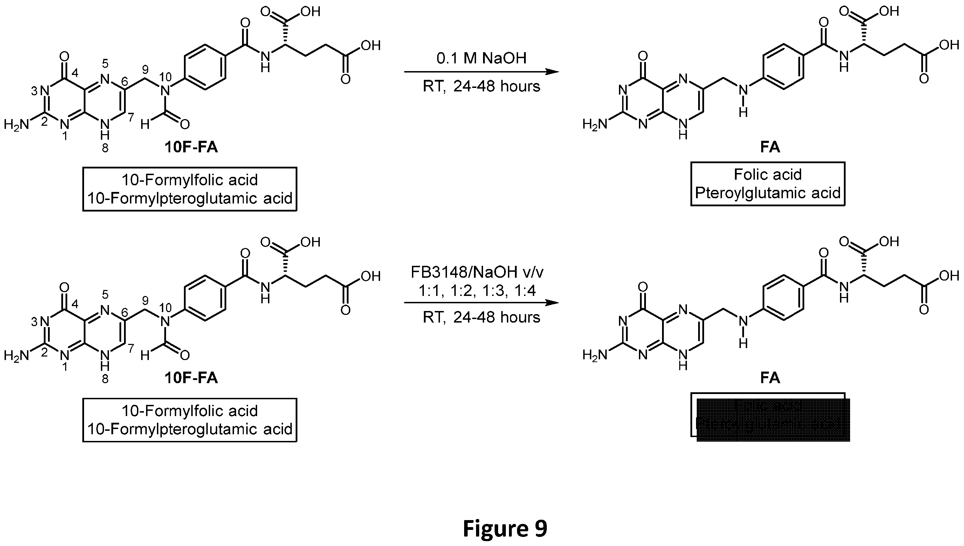

Browsing through the chemical literature, we identified a few reports describing that folic acid displays a greater stability at higher pH values. At such pH values, folic acid exhibit higher solubility which simplifies the synthetic manipulation, purification and downstream processing. Hence, in a series of *N*-deformylation experiments using 0.1 M NaOH, we are aiming toward clean and efficient conversion from 10-formyl folic acid to folic acid (see Figure 9) which will simplify the isolation of target product from the fermentation broth. Initial deformylation experiments were carried out on the analytical standard of 10-formylfolic acid using 0.01 mmol scale (5 mg).

### Representative experimental procedure:

10-formylfolic acid was weighed in the 10 mL round bottom flask equipped with a stirring bar and a rubber septum. The suspension was treated with 0.1 M sodium hydroxide (50 equiv., 0.5 mmol, 5 mL) and allowed to stir for 24-48 hours at ambient temperature protected from light. Subsequently, a solution (100 µL) was diluted with folic acid extraction buffer (900 µL), homogenized on the vortex stirrer and analyzed on HPLC. Three time-dependent aliquots were sampled analyzed on HPLC. Results of deformylation are presented in Table 19. Deformylation of 10-formylfolic acid with 0.1 M NaOH proceeded nearly quantitatively to folic acid during the first sampling after 24 hours (Table 19, entry 1). After stirring for 48 hours, the reaction proceeded to completion according to HPLC analysis. Prolonged stirring under the same conditions disclosed that newly formed folic acid did not undergo to decomposition even after 144 hours (6 days).

**Table 19. Time scale of N-deformylation of 10-formylfolic acid to folic acid in the presence 0.1 M NaOH**

| **exp** | **reagent** | **time** | **temp** | **10-FFA** | | **FA** | |
|---|---|---|---|---|---|---|---|
| | | | | area | mg/L | area | mg/L |
| 1 | NaOH 0.1 M | 24 hr | 25 °C | 16833 | 26 | 1960819 | 1167 |
| | NaOH 0.1 M | 48 hr | 25 °C | 0 | 0 | 2095549 | 1247 |
| | NaOH 0.1 M | 144 hr | 25 °C | 0 | 0 | 2062398 | 1228 |

Experiments were conducted in 10 mL round bottom flasks using 10-formylfolic acid (5 mg, 0.01 mmol). NaOH 0.1 M was added in excess, 50.0 equivalents, 5 mL. Mass concertation of 10-FFA at the beginning of the experiment is approximately 1000 mg/L. Progress of reaction was measured by HPLC. The HPLC samples were prepared in the extraction buffer (0.1 M phosphate buffer with 1% (w/v) ascorbic acid).

### Base-mediated N-deformylation of 10-formylfolic acid in the fermentation broth

In previous experiments we have illustrated that deformylation of 10-formylfolic acid standard using 0.1 M NaOH provided a clean conversion to folic acid shown in Fig. 9. Herein we applied the same principle on a more complex system, a fermentation broth. HPLC analysis of fermentation broth from Example 16 before deformylation showed a substantial amount of 10-formyldihydrofolic acid (10F-DHF; 60 %Area); and 10-formylfolic acid (10F-FA; 40 %Area). Samples of fermentation broth from Example 16 (10 mL) were treated with different v/v ratios of 0.1 M NaOH (1:1, 1:2, 1:3 and 1:4) and stirred for 24 hours at ambient temperature (25 °C) protected from light. According to our HPLC assay, experiments with fermentation broth/NaOH v/v 1:1 and 1:2 did not lead to deformylation but to oxidative conversion of 10-formyldihydrofolic acid to of 10-formylfolic acid as displayed in Table 20 (entries 2 and 3). Subsequently, when the amount of NaOH was increased in respect to fermentation broth (1:3 and 1:4) a significant amount of folic acid was detected by HPLC as displayed in Table 20 (entries 4 and 5). Interestingly, higher amounts of NaOH somewhat hampered the oxidative conversion of 10F-DHF to 10F-FA since a substantial amount of 10F-DHF was detected by HPLC.

### Representative experimental procedure:

Well-stirred fermentation broth from Example 16 (10 mL) was pipetted into the 50-100 mL round bottom flasks equipped with stirring bars and aluminum foil for light protection. Sodium hydroxide (0.1 M) was added dropwise and reaction was stirred vigorously for 24 hours under the ambient temperature (25 °C). After 24 hours of stirring, 1 mL of each fermentation broth was diluted in duplicates with 9 mL of extraction buffer (0.1 M phosphate buffer with 1% (w/v) ascorbic acid). The suspensions were stirred on vortex, centrifuged at 4500 rpm, filtered through 0.22 µm filter and analyzed on HPLC.

**Table 20. Effect of addition of different amounts of NaOH on of N-deformylation of 10-formylfolic acid in fermentation broth.**

| **exp** | **sample** | **time** | **temp** | **10F-DHF** | **10F-FA** | **FA** | **SUM FOL** |
|---|---|---|---|---|---|---|---|
| | | | | mg/L | mg/L | mg/L | mg/L |
| 1 | FB3148 | 0 hr | 25 °C | 455 | 302 | 0 | 757 |
| 2 | FB3148/NaOH 0.1 M (1:1 v/v) | 24 hr | 25 °C | 87 | 428 | 0 | 515 |
| 3 | FB3148/NaOH 0.1 M (1:2 v/v) | 24 hr | 25 °C | 0 | 623 | 0 | 623 |
| 4 | FB3148/NaOH 0.1 M (1:3 v/v) | 24 hr | 25 °C | 47 | 451 | 302 | 800 |
| 5 | FB3148/NaOH 0.1 M (1:4 v/v) | 24 hr | 25 °C | 350 | 284 | 222 | 856 |

Experiments were conducted in 50-100 mL round bottom flasks using the fermentation broth from Example 16 (FB3148, 10 mL). NaOH 0.1 M was added based on the volume/volume ratio in respect to FB3148 (1:1, 1:2, 1:3 and 1:4). Progress of reaction and results were measured by HPLC. The HPLC samples were prepared in the extraction buffer (0.1 M phosphate buffer with 1% (w/v) ascorbic acid). All reactions were stirred protected from the light for 24 hours at ambient temperature (25 °C).

### Example 19: Isolation of 10-formly folic acid

After harvesting, a fermentation broth containing 50 g of folic acid was adjusted to pH=12 using 5M aqueous NaOH. The solution was centrifuged at 10000 rpm for 15 minutes at 4 °C. To a supernatant, 50 g of calcium hydroxide was added and suspension was stirred at room temperature for 2 hours. The resulting suspension was allowed to settle, decanted and the supernatant liquid was filtered with the aid of 100 of diatomaceous earth (Celite). The filter cake was washed with 500 mL of water and filtered. The filtrates were combined and diluted to a final volume of 10 liters. The dilute alkaline solution of clarified folic acid was adjusted to a pH 7.0 with 1N HCl, heated to 70 °C and then cooled to a room temperature. Next, the solution was filtered to remove impurities that precipitate at neutral pH. A clarified filtrate was adjusted to pH =3 using 1N HCl and cooled on ice for 4 hours. The suspension was filtered off and redissolved in 8L of hot alkaline solution with pH= 12 (adjusted with 1M NaOH). To this solution, 50 grams of activated charcoal (1 equivalent/weight of folic acid) was added and the solution was heated to 50 °C and stirred for 30 minutes. The suspension was filtered, the filter cake was washed with 3 L of alkalinized aqueous solution (pH=12 adjusted with NaOH). Filtrates were combined and pH was adjusted to 3.0 utilizing 1N HCl, added during continuous stirring. The resulting slurry was cooled on ice for 24h or overnight. The suspension was filtered off and resuspended in 1L of acidified aqueous solution having a pH=3 (pH was adjusted with 1N HCl). The suspension was again filtered and the resulting filter cake was then frozen and dried to obtain 43 grams of folic acid, which contained 10 % of moisture and assayed 90.1. % folic acid on an anhydrous basis.

### Example 20: Isolation of folic acid

After harvesting, a fermentation broth containing 30 g of folic acid was adjusted to pH=10 using 1M aqueous NaOH. The solution was centrifuged at 10000 rpm for 15 minutes at 4 °C. The resulting supernatant was adjusted to a pH 4.0 with 1N HCl, heated to 70 °C and then cooled to a room temperature. Next, the solution was filtered with the aid of 100 g of Celite. Filter cake was resuspended in 5L of alkaline solution with pH= 10 (adjusted with 1M NaOH). To this solution, 50 grams of activated charcoal (1 equivalent/weight of folic acid) was added and the solution was heated to 50 °C and stirred for 30 minutes. The suspension was filtered, the filter cake was washed with 2 L of alkalinized aqueous solution (pH=12 adjusted with NaOH). Filtrates were combined and pH was adjusted to 3.0 utilizing 1N HCl, added during continuous stirring. The resulting precipitate was cooled on ice for 16-24h or then filtered off and resuspended in 1L of acidified aqueous solution having a pH=3 (pH was adjusted with 1N HCl). The suspension was again filtered and the resulting precipitate cake was dried to obtain 21 grams of 10-formyl folic acid, which was assayed 92 %.

### Example 21. Assembly of 5-Methylfolate operon (MTHF-OP)

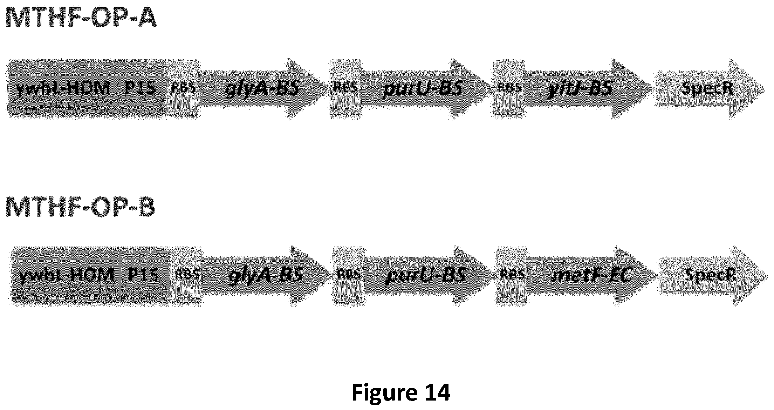

The synthesis of folate biosynthetic genes (glyA, purU yitJ and metF) was carried out as separate synthetic DNA fragments (SEQ ID NO: 91, 92, 93 and 94) with gene nucleotide sequences codon-optimized for *B. subtilis* optimal expression. The fragments were assembled into artificial operon by repetitive steps of restriction and ligation (Example 3). A combination of NdeI and AseI restriction sites is used in order to assure compatible restriction ends for successful ligation. After each step of ligation, the combined fragments were used as a new template for next PCR amplification. Operons were assembled stepwise in order to combine different biosynthetic genes with homologies for ywhL locus (SEQ ID NO: 95) and spectinomycin selectable marker (SEQ ID NO: 96). Integration locus ywhL (uncharacterized protein) was selected as a new chromosome integration site. Folate biosynthetic genes methyl-folate operons (MTHF-OP) were design as a combination of genes involved in the final steps of 5-methyltetrahydrofolate biosynthesis and are under control of a strong constitutive P15 promoter (Figure 14). In the MTHF-OP-A operon (SEQ ID NO: 97) genes (*glyA, purU* and *yitJ*) were selected from native host organism *B. subtilis* and additional codon-optimized for *B. subtilis* optimal gene expression. Additionally, homologue gene (*metF*) from *E. coli* were used for construction of alternative to *yitJ* gene in operon MTHF-OP-B (SEQ ID NO: 98).

### Example 22: Mutagenesis of B. subtilis

Ethyl methanesulfonate (EMS) mutagenesis was performed on *B. subtilis* strain FL825. The culture was grown in liquid LB medium with appropriate antibiotics to the exponential phase. Then, the culture was centrifuged at 3000 RPM for 3 - 5 min, and the supernatant was removed. The cell pellet was washed twice in sterile 0.9-% NaCl, and the supernatant was removed by centrifugation. The pellet was resuspended in 0.9-% NaCl. One hundred µL of cell suspension was diluted in 900 µL of 3% Ethyl methanesulfonate (EMS). The cells were exposed to EMS for corresponding time with constant agitation/mixing. After the incubation, 1 volume of 10-15% Na-thiosulfate was added per 1 volume of cell suspension to stop the mutagenesis reaction. The liquid was removed by centrifugation. The cell pellet was washed in sterile 0.9-% NaCl twice, and the supernatant was removed by centrifugation. Serial dilutions of the mutagenized cells were plated on MB plates with appropriate antibiotic and incubated for approximately 48h at 37°C. Single colonies were further tested for 5-methyltetrahydrofolate production (Example 25).

### Example 23: Determination of the expression level of metE gene using qPCR

*B. subtilis* strain FL2771 was grown in liquid LB medium to the exponential phase. The culture was mixed with 2 volumes of the RNAprotect Bacteria Reagent (QIAGEN), centrifuged for 10 min at 4500 rpm and frozen at -80°C or processed immediately. The cell pellet was resuspended in 200 µL of TE buffer containing 1 mg/mL lysozyme for 15 min to remove the cell wall. RNA was isolated by using RNeasy mini kit (QIAGEN) according to the manufacturer's protocol. The obtained RNA was checked for concentration and quality spectrophotometrically. The isolated RNA was treated with DNase (Ambion kit) and reverse-transcribed to cDNA by using RevertAid H Minus First Strand cDNA Synthesis Kit (Thermo Scientific). The obtained cDNA was diluted and the final yield of cDNA was app 2.5 ng µL.

The obtained cDNA was analysed by real time quantitative PCR (qPCR) technique (StepOne Real-Time PCR System, Applied Biosystems) with SYBR Green I (Thermo Scientific) detection. The expression of the *metE* gene in the 5-Me-THF-producing strain(s) was quantified by qPCR.

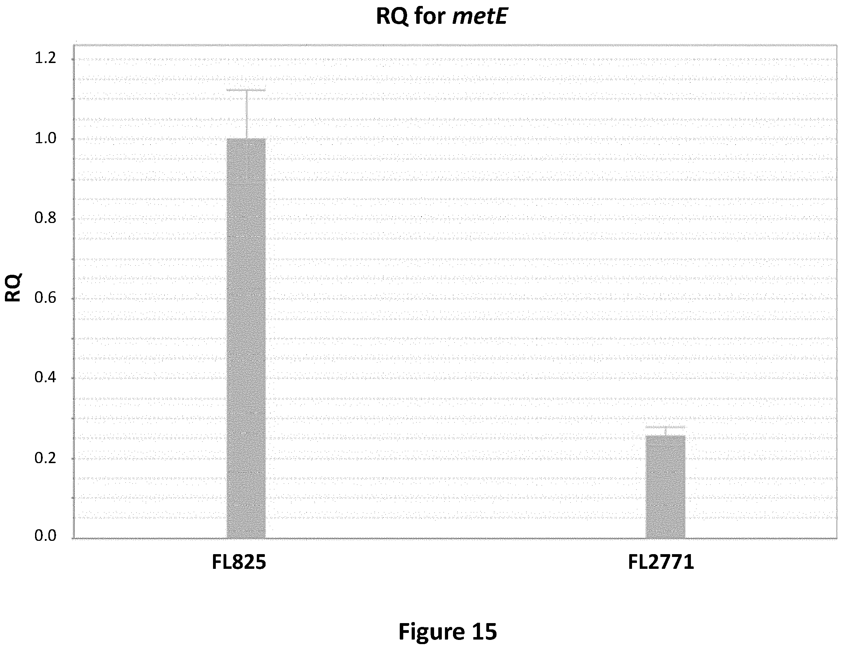

As an internal control gene, 16S rRNA gene from *B. subtilis* was used as a reference for normalization of the quantitative qPCR expression data. *B. subtilis* VBB38 was used as the control strain. The expression of *metE* gene was determined using specific pair of primers *Q_metE_*F (SEQ ID NO:73) and Q*_metE_*R (SEQ ID NO:74) and for 16S gene selected as the internal control gene, primer pair SEQ ID NO:69 and SEQ ID NO:70 were used. The qPCR analysis was run on StepOne^{™} Real-Time PCR System and quantification was performed by using the 2 - ΔΔCT method.
Strain FL2771 had downregulated expression of *metE* gene compared to starting parent strain FL825 for more than 70% (Figure 15).

### Example 24: Generation of 5-Methyl tetrahydrofolate-producing strains

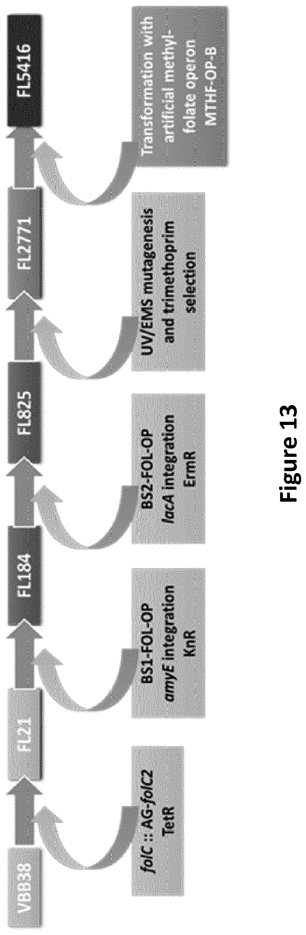

5-Methyl tetrahydrofolate-producing strains were developed from starting strain VBB38 (see Figure 13). Two important genetic modification were performed in order to improve total folate production capacity of the engineered strains. Initially native folC gene in *B. subtilis* was disrupted in order to abolish the synthesis of γ-glutamate tail on folates and replaced with *folC* homolog encoding only for the dihydrofolate synthetase (DHFS) activity, resulting in the addition of only one essential glutamate moiety without the γ-glutamate tail. Therefore, strain FL21 was developed with phenotype capable of enhanced excretion of folates from the cells to the fermentation medium in order to assure high metabolic flow through the folate biosynthetic pathway. In the following step of strain development overexpression of the folate biosynthetic genes (such as, *folE*/*mtrA, foLB, folK, foLP*/*sul, folA*/*dfrA*) were introduced in order to upregulate "*de novo*" biosynthetic pathway (from GTP precursor to THF). Artificial folate operons (BS-FOL-OP1 and BS-FOL-OP2 - see Example 3) were constructed bearing selected folate biosynthetic genes and integrated into *B. subtilis* at multiple genome locations (amyE and lacA locus) in order to provide high level of gene expression. Upregulation of folate biosynthetic genes in newly engineered strain FL184 (BS-FOL-OP1) and strain FL825 (BS-FOL-OP1+ BS-FOL-OP2) has significantly improved production capacity of total folates compared to the starting strain.

Further engineered strains of *B. subtilis* for overproduction of 5-methyl tetrahydrofolate were generated by downregulation/deletion of native *metE* gene and overexpression of genes involved in interconversion of folate forms (from THF towards 5-methyltetrahydrofolate) (such as, *glyA, purU, yitJ* and *metF*). Downregulation of native *metE* gene was achieved by random mutagenesis of parent strain FL825 and mutant strain selection based on their improved 5-methyltetrahydrofolate production. New strain FL2771 was generated, reaching significantly higher titer of 5-methyltetrahydrofolate (more than 220 mg/L) compared with the wild-type strain (0,35 mg/L). The strain FL2771 was further selected for whole genome sequencing. Bioinformatic analyses of FL2771 genome and comparison to whole genome sequence data of ancestor starting strain *B. subtilis* VKPM B2116 was able to rationally connect the several observed SNP variances/mutations, introduced by random mutagenesis during strain development, with folate metabolic cycle. One mutation is located directly upstream of the *metE* gene (SEQ ID NO: 75 and SEQ ID NO: 76), this mutation is located in the regulatory region of the gene coding for methionine synthase involved in consumption of 5-methyltetrahydrofolate.

In the last step of developing 5-methyltetrahydrofolate producing strain, we have performed overexpression of genes (*glyA, purU, yitJ and metF*) crucial for interconversion of folate forms. Overexpression of selected genes was design as a new methyl folate biosynthetic operon (MTHF-OP) constructed from genes involved in final steps of 5-methyltetrahydrofolate biosynthesis (*glyA* and *yitJ*/*metF*) and gene *purU* (formyltetrahydrofolate deformylase) involved in conversion of 10F-THF to the THF in order to improve ratio between 10F-folates and 5-methyltetrahydrofolate. New genetically engineered strain FL5416 has demonstrated to produce the highest 5-methyltetrahydrofolate yield of more than 300 mg/L whereas 5-methyltetrahydrofolate form represented 75% of total folates measured.

### Example 25: Determination of ratio of different forms of folates in genetically engineered strains of B. subtilis

Strains were patched on MB plates with appropriate antibiotics and incubated at 37°C for 2 days. For shake-flasks experiments, the grown strains were transferred to 5 ml of MC (seed) medium in Falcon 50 mL conical centrifuge tubes (1 plug/5 ml) and cultivated on a rotary shaker at 220 RPM and 37 °C for 16 - 18h. A 10-% inoculum of the seed culture was used to inoculate 5 mL of the production medium (MD+pABA500). The strains were cultivated on a rotary shaker at 220 RPM and 37°C for 24h in the dark. After the fermentation, the samples of the fermentation broth (200 µl) was carefully collected to obtain a homogeneous sample and diluted 10 times in the ice-cold extraction buffer (0.1 M phosphate buffer with 1% (w/v) ascorbic acid). The samples were centrifuged at 14,000 rpm and 4°C for 10 min and filter-sterilized (0.22 um pore size). For the quantification of different folate species HPLC method was used as described in Example 13. Results of different *B. subtilis* strain are shown in Table 21 and representative HPLC chromatogram of fermentation broth sample is shown in Figure 6.

**Table 21. Folate production of different Bacillus subtills strains in experiments at shaker scale (5 ml) after 24 hours.**

| Strain | Strain description | 5M-THF (mg/L) | FA (mg/L) | 10F-DHF (mg/L) | 10F-FA (mg/L) |
|---|---|---|---|---|---|
| wt 168 | *B. subtillis* wild type | 0,16 | 0,15 | 0,01 | 0,86 |
| VBB38 | *B. subtilis* VKPM B2116 | 0,35 | 0,01 | 0,02 | 0,09 |
| FL21 | VBB38 folC::tetR P-veg/folC2-AG | 18,20 | 0,01 | 0,03 | 2,64 |
| FL23 | VBB38 folC::tetR P-veg/folC2-LR | 1,11 | 0,01 | 0,01 | 0,03 |
| FL184 | FL21 amyE::kanR P-veg/ FOL-OP-BS1 | 48,5 | 0,03 | 8,1 | 186,6 |
| FL825 | FL184 lacA::EryR P-15/ FOL-OP-BS2 | 35,1 | 0,07 | 14,6 | 328,9 |
| FL2771 | FL825 (EMS mutagenesis) metE* | 227,8 | 0,02 | 6,2 | 99,2 |
| FL5416 | FL2771 ywhL::SpecR P-15/ MTHF-OP-B | 306,5 | 0,02 | 2,7 | 24,0 |

Strain FL5416 with heterologous *folC*-AG and overexpressed folate biosynthetic genes (folate operon FOL-OP-BS1, FOL-OP-BS2 and MTHF-OP-B) showed 191462% increased 5-methylfolate production compared to the wild type strain *Bacillus subtilis* 168.

### Comparative example 1

Total folate production was determined for *B. subtilis* wild type strain "168", our starting non-GMO strain VBB38 (strain VKPM B2116 = *B. subtilis* VNII Genetika 304) and its transformants in which native *folC* gene was replaced in one step by a heterologous *folC2* (FOL3) gene from either *A. gossypii* (*B. subtilis* strain FL21) or *L. reuteri* (*B. subtilis* strain FL23). Strains were tested at the shaker scale (5 ml production medium MD) and total folates were determent by using standard microbiological assay for folate detection.

The result was shown that knockout mutants of deletion of *B. subtilis* native *folC* gene alone without simultaneous heterologous folC2 gene expression were not able to grow in standard cultivation conditions (T=37C, aerobically in nutrient rich LB medium).

### List of references cited in the description

Hjortmo S, Patring J, Andlid T. 2008 Growth rate and medium composition strongly affect folate content in Saccharomyces cerevisiae. Int J Food Microbiol. 123(1-2):93-100.
McGuire JJ and Bertino JR. 1981. Enzymatic synthesis and function of folylpolyglutamates. Mol Cell Biochem 38 Spec No (Pt 1):19-48.
Reed, LS, Archer MC. 1980. Oxidation of tetrahydrofolic acid by air. J Agric Food Chem. 28(4):801-805.
Rossi, M., Raimondi, S., Costantino, L., Amaretti, A., 2016. Folate: Relevance of Chemical and Microbial Production. Industrial Biotechnology of Vitamins, Biopigments, and Antioxidants. Wiley-VCH Verlag GmbH & Co. KGaA, Weinheim, Germany, pp. 103-128.
Scaglione and Panzavolta. 2014. Folate, folic acid and 5-methyltetrahydrofolate are not the same thing. Xenobiotica. 44(5):480-488.
Serrano-Amatriain C, Ledesma-Amaro R, López-Nicolás R, Ros G, Jiménez A, Revuelta JL. 2016. Folic acid production by engineered Ashbya gossypii. Metab Eng. 38:473-482.
Sybesma W, Starrenburg M, Kleerebezem M, Mierau I, de Vos WM, Hugenholtz J. 2003a. Increased production of folate by metabolic engineering of Lactococcus lactis. Appl Environ Microbiol. 69(6):3069-3076.
Sybesma, W., Starrenburg, M., Tijsseling, L., Hoefnagel, M.H.N., Hugenholtz, J., 2003b. Effects of cultivation conditions on folate production by lactic acid bacteria. Applied and Environmental Microbiology. 69(8):4542-4548.
Sybesma W, Van Den Born E, Starrenburg M, Mierau I, Kleerebezem M, De Vos WM, Hugenholtz J. 2003c. Controlled modulation of folate polyglutamyl tail length by metabolic engineering of Lactococcus lactis. Appl Environ Microbiol. 69(12):7101-7107.
Walkey CJ, Kitts DD, Liu Y, van Vuuren HJJ. 2015. Bioengineering yeast to enhance folate levels in wine. Process Biochem 50(2):205-210.
Qui Z and Goodman MF: The Escherichia coli polB locus is identical to dinA, the structural gene for DNA polymerase II. Characterization of Pol II purified from a polB mutant. J Biol Chem. 1997, 272(13): 8611-8617.
Kwon DH, Peña JA, Osato MS, Fox JG, Graham DY, Versalovic J: Frameshift mutations in rdxA and metronidazole resistance in North American Helicobacter pylori isolates. J Antimicrob Chemother 2000, 46(5): 793-796
Datsenko KA, Wanner BL: One-step inactivation of chromosomal genes in Escherichia coli K-12 using PCR products. Proc Natl Acad Sci U S A 2000, 97:6640-6645.

## Claims

1. A genetically engineered microorganism, which has been modified to have an increased expression level of at least one enzyme involved in the biosynthesis of a 5-methylfolate compared to an otherwise identical microorganism that does not carry said modification (reference microorganism) and/or which has been modified to have a decreased expression and/or activity of an endogenous polypeptide having 5-methyltetrahydropteroyltriglutamate-homocysteine S-methyltransferase activity compared to an otherwise identical microorganism that does not carry said modification (reference microorganism).

2. The genetically engineered microorganism according to claim 1, which has been modified to have an increased expression level of at least one enzyme involved in the biosynthesis of a 5-methylfolate compared to an otherwise identical microorganism that does not carry said modification (reference microorganism).

3. The genetically engineered microorganism according to claim 1 or 2, wherein the enzyme involved in the biosynthesis of a 5-methylfolate is selected from the group consisting of: a polypeptide having GTP cyclohydrolase activity, a polypeptide having 7,8-dihydroneopterin aldolase activity, a polypeptide having 2-amino-4-hydroxy-6-hydroxymethyldihydropteridine pyrophosphokinase activity, a polypeptide having dihydropteroate synthase activity, a polypeptide having dihydrofolate reductase activity, a polypeptide having serine hydroxymethyltransferase activity, a polypeptide having formyltetrahydrofolate deformylase activity, and a polypeptide having 5,10-methylenetetrahydrofolate reductase activity.

4. The genetically engineered microorganism according to any one of claims 1 to 3, which has been modified to have an increased expression level of a polypeptide having 5,10-methylenetetrahydrofolate reductase activity compared to an otherwise identical microorganism that does not carry said modification (reference microorganism).

5. The genetically engineered microorganism according to any one of claims 1 to 4, wherein the at least one enzyme involved in the biosynthesis of a 5-methylfolate, respectively the polypeptide is heterologous to the genetically engineered microorganism.

6. The genetically engineered microorganism according to any one of claims 1 to 5, which has been modified to have a decreased expression and/or activity of an endogenous polypeptide having 5-methyltetrahydropteroyltriglutamate-homocysteine S-methyltransferase activity compared to an otherwise identical microorganism that does not carry said modification (reference microorganism).

7. The genetically engineered microorganism according to any one of claims 1 to 6, which has been modified a) to have a decreased expression and/or activity of an endogenous polypeptide having both dihydrofolate synthase activity and folylpolyglutamate synthetase activity compared to an otherwise identical microorganism that does not carry said modification (reference microorganism), and b) to express a heterologous polypeptide having only dihydrofolate synthase activity.

8. The genetically engineered microorganism according to any one of claims 1 to 7, which is a bacterium.

9. The genetically engineered microorganism according to any one of claims 1 to 8, which is a bacterium of the species *Bacillus subtiltis.*

10. A method for preparing a folate, precursor or intermediate thereof, comprising
i) cultivating a genetically engineered microorganism according to any one of claims 1 to 9 in a culture medium under suitable culture conditions to obtain a fermentation product containing said folate, precursor or intermediate thereof; and
ii) optionally, separating and/ or purifying said folate, precursor or intermediate thereof.

11. The method according to claim 10, wherein the folate is a compound of Formula I: 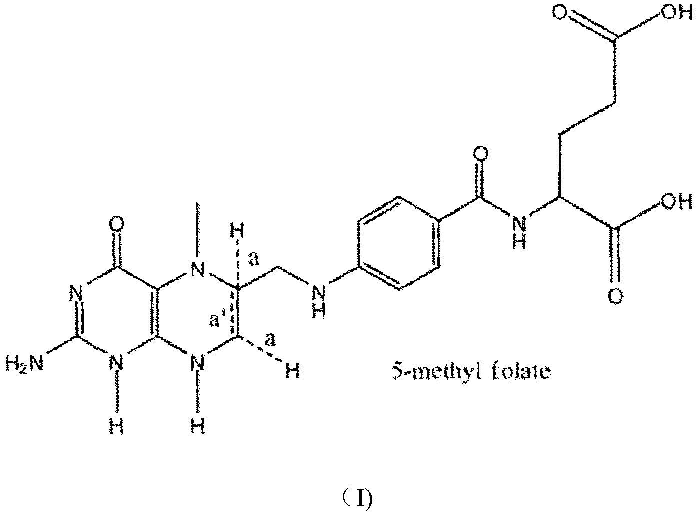 optionally in form of one of the stereoisomers, preferably enantiomers or diastereomers, in form of a racemate or in form of a mixture of at least two of the stereoisomers, preferably enantiomers and/or diastereomers, in any mixing ratio.

12. The method according to claim 10 or 11, wherein the folate is a compound of Formula II: 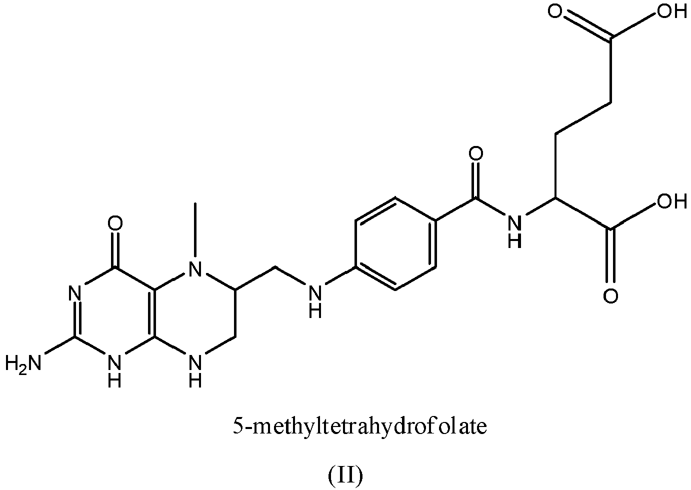 optionally in form of one of the stereoisomers, preferably enantiomers or diastereomers, in form of a racemate or in form of a mixture of at least two of the stereoisomers, preferably enantiomers and/or diastereomers, in any mixing ratio.

13. The method according to any one of claims 10 to 12, wherein the folate is a compound of Formula IIa:

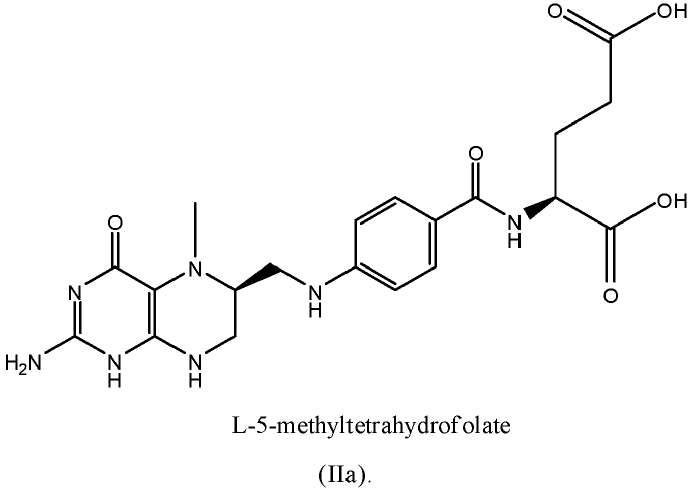

14. A method of preparing a genetically engineered microorganism, comprising any one of the steps (a) to (b) below:
(a) increased the expression level of at least one enzyme involved in the biosynthesis of a 5-methylfolate compared to an otherwise identical microorganism (reference microorganism); and
(b) decreasing the expression and/or activity of an endogenous polypeptide having 5-methyltetrahydropteroyltriglutamate-homocysteine S-methyltransferase activity compared to an otherwise identical microorganism that does not carry said modification (reference microorganism).

15. The method according to claim 14, further comprising the steps (c) and (d) below:
(c) decreasing the expression and/or activity of an endogenous polypeptide having both dihydrofolate synthase activity and folylpolyglutamate synthetase activity compared to an otherwise identical microorganism that does not carry said modification (reference microorganism); and
(d) expressing a heterologous polypeptide having only dihydrofolate synthase activity.
